# EUROPÄISCHE PATENTSCHRIFT

(11) **EP 0 843 658 B1**
(45) Veröffentlichungstag und Bekanntmachung des Hinweises auf die Patenterteilung: **18.12.2002**
(21) Anmeldenummer: 96927634.4
(22) Anmeldetag: 30.07.1996
(51) Int. Cl.: C07C 275/62, C07C 275/50, C07C 271/22, C07C 271/54, C07D 295/215, C07D 213/79, C07C 275/16, C07C 333/04, C07D 295/185, C07D 213/40, C07D 213/74

(54) **ENDOPARASITIZIDE MITTEL AUF BASIS VON DIDEPSIPEPTIDEN, NEUE DIDEPSIPEPTIDE UND VERFAHREN ZU IHRER HERSTELLUNG**
DIDEPSIPEPTIDE-BASED ENDOPARASITICIDES, NEW DIDEPSIPEPTIDES AND PROCESS FOR PREPARING THE SAME
AGENTS ENDOPARASITICIDES A BASE DE DIDEPSIPEPTIDES, NOUVEAUX DIDEPSIPEPTIDES ET LEUR PROCEDE DE PREPARATION

(30) Priorität: 11.08.1995 DE 19529604
(43) Veröffentlichungstag der Anmeldung: 27.05.1998
(73) Patentinhaber: BAYER AG, 51368 Leverkusen (DE)
(72) Erfinder: JESCHKE, Peter, D-51373 Leverkusen (DE); SCHERKENBECK, Jürgen, D-42929 Wermelskirchen (DE); PLANT, Andrew, D-51373 Leverkusen (DE); HARDER, Achim, D-51109 Köln (DE); MENCKE, Norbert, D-51381 Leverkusen (DE)
(86) Internationale Anmeldenummer: EP9603355
(87) Internationale Veröffentlichungsnummer: WO97007093

(56) Entgegenhaltungen:
- WO-A-93/25543
- WO-A-94/19334
- DE-A- 4 341 992
- J. AM. CHEM. SOC. (1993), 115(21), 9774-88, XP002019626 GALLO, ELIZABETH A. ET AL: "Hydrogen-bond-mediated folding in depsipeptide models of.beta.-turns and.alpha.-helical turns"
- CHEMICAL ABSTRACTS, vol. 119, no. 16, 18.Oktober 1993 Columbus, Ohio, US; abstract no. 167592, CROMMEN, JAN ET AL: "Degradable polyphosphazenes for biomedical applications" XP002019631 & J. CONTROLLED RELEASE (1993), 24(1-3), 167-80,
- J. AM. CHEM. SOC. (1991), 113(1), 297-307, XP000604955 MORGAN, BRADLEY, P. ET AL: "Differential binding energy: a detailed evaluation of the influence of hydrogen-bonding and hydrophobic groups on the inhibition of thermolysin by phosphorus-containing inhibitors"
- SYNTHESIS (1988), (12), 947-50, XP002019627 CAVICCHIONI, G. ET AL: "Synthesis of O-(.alpha.-aminoacyl)glycolic and -lactic amides from 2-bromoacetamides or -propanamides with N-protected amino acids"
- CHEMICAL ABSTRACTS, vol. 106, no. 21, 25.Mai 1987 Columbus, Ohio, US; abstract no. 176851, MAMMI, STEFANO ET AL: "Polydepsipeptides. 13. Synthesis and proton NMR analysis of collagen model structures" XP002019632 & INT. J. PEPT. PROTEIN RES. (1986), 28(1), 29-44,
- CHEMICAL ABSTRACTS, vol. 85, no. 24, 13.Dezember 1976 Columbus, Ohio, US; abstract no. 178135, INGWALL, R. T. ET AL: "Polydepsipeptides. 5. Experimental conformational analysis of poly(L-alanyl-L-lactic acid) and related model compounds" XP002019633 & MACROMOLECULES (1976), 9(5), 802-8 CODEN: MAMOBX, 1976,
- J. CHEM. SOC. C (1971), (17), 2890-6, XP002019628 YOUNG, G. T. ET AL: "Amino acids and peptides. XXXIV. Anchimerically assisted coupling reactions. Use of 2-pyridyl thioesters"
- TETRAHEDRON LETTERS, Bd. 24, Nr. 47, 1983, OXFORD GB, Seiten 5219-22, XP002019629 J. MARTINEZ ET AL.: "Carboxymidomethyl esters (CAM esters) as carboxyl protecting groups"
- TETRAHEDRON LETTERS, Nr. 40, 1979, OXFORD GB, Seiten 3811-4, XP002019630 C. GILON ET AL.: "A novel method for the facile synthesis of depsipeptides"
- DATABASE CROSSFIRE Beilsteininformationssysteme GmbH XP002019634 & BIOPOLYMERS, Bd. 16, 1977, Seite 1033, 1040

## Beschreibung

Die vorliegende Erfindung betrifft die Verwendung von Didepsipeptiden zur Bekämpfung von Endoparasiten, neue Didepsipeptide und Verfahren zu ihrer Herstellung.

Bestimmte Didepsipeptide sind als Ausgangsstoffe für endoparasitizid wirksame cyclische Depsipeptide (vgl. Totalsynthese von PF 1022 A: JP-Pat. 05 229 997; Makoto Ohyama et al., Biosci. Biotech. Biochem. 58 (6), 1994, S. 1193-1194; Makio Kobayshi et al., Annu. Rep. Sankyo Res. Lab. 46, 1994, S. 67- 75; Stephen J. Nelson et al., J. Antibiotics 47, (11), 1994, S. 1322-1327; Cyclooktadepsipeptide: WO 93/19053; EP 0 634 408 A1; WO 94/19334; WO 95/07272; EP 626 375; EP 626 376; Cyclohexadepsipeptide: DE-OS 4342 907; WO 93/25543) und offenkettiger Depsipeptide, beispielsweise Oktadepsipeptide (DE-OS 4 341 993), Hexadepsipeptide (DE-OS 4 341 992) oder Tetradepsipeptide (DE-OS 4 341 991) Gegenstand vorveröffentlichter Patentanmeldungen und Publikationen. Einige dieser o. g. Didepsipeptide sind ebenso Gegenstand nicht vorveröffentlichter deutscher Patentanmeldungen (P 44 40 193.0; P 44 01 389.2).

Die vorliegende Erfindung betrifft:
1. Verwendung von Didepsipeptiden der allgemeinen Formel (I) und deren Salze, 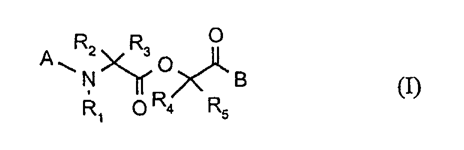 in welcher
   - R¹: für Wasserstoff oder geradkettiges oder verzweigtes Alkyl steht,
   - R¹ und R²: gemeinsam mit den Atomen, an die sie gebunden sind, für einen 5- oder 6-gliedrigen Ring stehen, der gegebenenfalls durch Sauerstoff, Schwefel, Sulfoxyl oder Sulfonyl unterbrochen sein kann und gegebenenfalls substituiert ist,
   - R² und R³: unabhängig voneinander für Wasserstoff, geradkettiges oder verzweigtes Alkyl, Alkenyl, Cycloalkyl, Cycloalkylalkyl, Aryl, Arylalkyl, Heteroaryl, Heteroarylalkyl stehen, die gegebenenfalls substituiert sind, oder
   - R² und R³: gemeinsam für einen spirocyclischen Ring stehen, der gegebenenfalls substituiert ist,
   - R⁴ und R⁵: unabhängig voneinander für Wasserstoff, geradkettiges oder verzweigtes Alkyl, Alkenyl, Cycloalkyl, Cycloalkylalkyl, Aryl, Arylalkyl, Heteroaryl, Heteroarylalkyl stehen, die gegebenenfalls substituiert sind, oder
   - R⁴ und R⁵: gemeinsam für einen spirocyclischen Ring stehen, der gegebenenfalls substituiert ist,
   - A: für Wasserstoff, Alkyl, Aralkyl, Formyl, Alkoxydicarbonyl oder für einen Rest der Gruppe G¹ 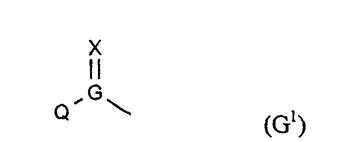 steht,
   worin 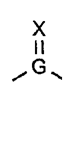 Carboxy, Thiocarboxy, -C=CH-NO₂, -C=CH-CN, -C=N-R⁶, Sulfoxyl, Sulfonyl, -P(O)-OR⁷ oder P(S)-OR⁷ bedeuten kann,
   R⁶ für Wasserstoff, Hydroxy, Alkoxy, Alkylcarbonyl, Halogenalkylcarbonyl, Alkylsulfonyl, Nitro oder Cyan steht, und
   R⁷ für Wasserstoff oder Alkyl steht, und
   - Q: für geradkettiges oder verzweigtes Alkyl, Alkenyl, Alkinyl, Cycloalkyl, Aryl, Arylalkyl, Hetaryl oder Hetarylalkyl, die gegebenenfalls substituiert sind, oder gegebenenfalls für einen Rest aus der Gruppe G² und G³

   R⁸-Y- (G²)

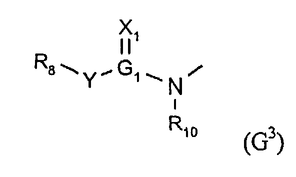

   steht, worin 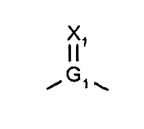 Carboxy, Thiocarboxy oder Sulfonyl bedeuten kann,
   Y für Sauerstoff, Schwefel oder -NR⁹ steht,
   R⁸ für den Fall, daß Y für Stickstoff steht, eine über ein Stickstoffatom verknüpfte cyclische Aminogruppe bedeuten kann,
   R⁸ und R⁹ unabhängig voneinander für Wasserstoff, geradkettiges oder verzweigtes Alkyl, Alkenyl, Alkinyl, Cycloalkyl, Cycloalkylalkyl, Aryl, Arylalkyl, Hetaryl, Hetarylalkyl stehen, die gegebenenfalls substituiert sind, oder
   R⁸ und R⁹ gemeinsam mit dem angrenzenden N-Atom für ein carbocyclisches 5-, 6- oder 7-gliedriges Ringsystem oder für ein 7 bis 10-gliedriges bicyclisches Ringsystem, das gegebenenfalls auch durch Sauerstoff, Schwefel, Sulfoxyl, Sulfonyl, Carbonyl, -N-O-, -N=, -NR¹¹- oder durch quaternisierten Stickstoff unterbrochen sein kann und gegebenenfalls substituiert ist,
   R¹⁰ für Wasserstoff oder Alkyl steht,
   R¹¹ für Wasserstoff, geradkettiges oder verzweigtes Alkyl, Alkenyl, Alkinyl, Cycloalkyl, Cycloalkylalkyl, Alkoxycarbonyl, Alkylcarbonyl, Cycloalkylcarbonyl, Cyan, Aryl, Arylalkyl, Hetaryl, Hetarylalkyl stehen, die gegebenenfalls substituiert sind, steht, und
   - B: für Hydroxy, Alkoxy, Alkenyloxy, Alkinyloxy, Cycloalkyloxy, Cycloalkylalkyloxy, Aryloxy-, Arylalkyloxy, Hetaryloxy, Hetarylalkyloxy stehen, die gegebenenfalls substituiert sind, steht, oder
   für die Reste -NR¹²R¹³, -NR¹⁴-NR¹²R¹³ und -NR¹⁵-OR¹⁶ steht,
   in denen
   R¹² und R¹³ unabhängig voneinander für Wasserstoff, geradkettiges oder verzweigtes Alkyl, Alkylcarbonyl, Alkylsulfonyl, Alkenyl, Alkinyl, Cycloalkyl, Cycloalkylalkyl, Aryl, Arylcarbonyl, Arylsulfonyl, Arylalkyl, Hetaryl, Hetarylcarbonyl, Hetarylsulfonyl oder Hetarylalkyl, die gegebenenfalls substituiert sind, stehen oder
   R¹² und R¹³ gemeinsam mit dem angrenzenden N-Atom für ein carbocyclisches 5-, 6-, 7- oder 8-gliedriges Ringsystem oder für ein 7 bis 10-gliedriges bicyclisches Ringsystem, das gegebenenfalls auch durch Sauerstoff, Schwefel, Sulfoxyl, Sulfonyl, Carbonyl, -N-O-, -N=, -NR¹¹- oder durch quaternisierten Stickstoff unterbrochen sein kann und gegebenenfalls substituiert ist,
   R¹⁴ für Wasserstoff, geradkettiges oder verzweigtes Alkyl, Cycloalkyl, Arylalkyl oder Hetarylalkyl, die gegebenenfalls substituiert sind, steht,
   R¹⁵ und R¹⁶ unabhängig voneinander Wasserstoff, geradkettiges oder verzweigtes Alkyl, Alkylcarbonyl, Alkenyl, Alkinyl, Cycloalkyl, Cycloalkylalkyl, Arylalkyl oder Hetarylalkyl, die gegebenenfalls substituiert sind, bedeuten,
   R¹⁵ und R¹⁶ gemeinsam mit der angrenzenden N-O-Gruppe für einen carbocyclischen 5-, 6- oder 7-gliedrigen Ring stehen,
   sowie deren optische Isomere und Racemate,
   zur Herstellung eines Mittels
   zur Bekämpfung von Endoparasiten in der Medizin und Tiermedizin.

Bevorzugt verwendet werden Didepsipeptide der allgemeinen Formel (I) und deren Salze, 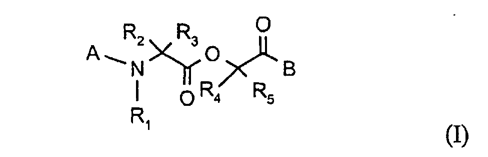 in welcher
- R¹: für Wasserstoff oder geradkettiges oder verzweigtes Alkyl mit bis zu 6 Kohlenstoffatomen steht,
- R¹ und R²: gemeinsam mit den Atomen, an die sie gebunden sind, für einen 5- oder 6- gliedrigen Ring stehen, der gegebenenfalls durch Schwefel, unterbrochen sein kann und gegebenenfalls substituiert ist,
- R² und R³: unabhängig voneinander für Wasserstoff, geradkettiges oder verzweigtes Alkyl mit bis zu 6 Kohlenstoffatomen, Halogenalkyl, Hydroxyalkyl, C₁₋₄-Alkanoyloxyalkyl, C₁₋₂-Alkoxyalkyl, Mercaptoalkyl, C₁₋₂-Alkylthioalkyl, C₁₋₂-Alkylsulfinylalkyl, C₁₋₂-Alkylsulfonylalkyl, Carboxyalkyl, Carbamoylalkyl, Aminoalkyl, C₁₋₆-Alkylaminoalkyl, C₁₋₆-Dialkylaminoalkyl, Guanidinoalkyl, das gegebenenfalls durch einen oder zwei Benzyloxycarbonylreste oder durch einen, zwei, drei oder vier C₁₋₂-Alkylreste substituiert sein kann, C₁₋₄-Alkoxycarbonylaminoalkyl, C₂₋₆-Alkenyl, C₃₋₆-Cycloalkyl, C₃₋₆-Cycloalkyl-C₁₋₂-alkyl sowie für gegebenenfalls substituiertes Aryl, Aryl-C₁₋₂-alkyl, Heteroaryl, Heteroaryl-C₁₋₂-alkyl stehen, oder
- R² und R³: gemeinsam für einen spirocyclischen Ring stehen,
- R⁴ und R⁵: unabhängig voneinander für Wasserstoff, geradkettiges oder verzweigtes Alkyl mit bis zu 6 Kohlenstoffatomen, Halogenalkyl, Hydroxyalkyl, C₁₋₄-Alkanoyloxyalkyl, C₁₋₂-Alkoxyalkyl, Mercaptoalkyl, C₁₋₂-Alkylthioalkyl, C₁₋₂-Alkylsulfinylalkyl, C₁₋₂-Alkylsulfonylalkyl, Carboxyalkyl, Carbamoylalkyl, Aminoalkyl, C₁₋₆-Alkylaminoalkyl, C₁₋₆-Dialkylaminoalkyl, Guanidinoalkyl, das gegebenenfalls durch einen oder zwei Benzyloxycarbonylreste oder durch einen, zwei, drei oder vier C₁₋₂-Alkylreste substituiert sein kann, C₁₋₄-Alkoxycarbonylaminoalkyl, C₂₋₆-Alkenyl, C₃₋₆-Cycloalkyl, C₃₋₆-Cycloalkyl-C₁₋₂-alkyl sowie für gegebenenfalls substituiertes Aryl, Aryl-C₁₋₂-alkyl, Heteroaryl, Heteroaryl-C₁₋₂-alkyl stehen, oder
- R⁴ und R⁵: gemeinsam für einen spirocyclischen Ring stehen,
- A: für Wasserstoff, C₁₋₆-Alkyl, Aryl-C₁₋₂-alkyl, Formyl, C₁₋₄-Alkoxydicarbonyl oder für einen Rest der Gruppe G¹ 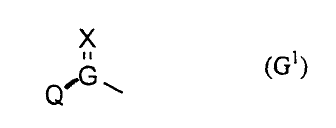 steht,
worin 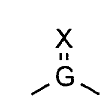 Carboxy, Thiocarboxy, -C=CH-NO₂, -C=CH-CN, -C=N-R⁶, Sulfoxyl, Sulfonyl, -P(O)-OR⁷ oder P(S)-OR⁷ bedeuten kann,
R⁶ für Wasserstoff, Hydroxy, C₁₋₄-Alkoxy, C₁₋₄-Alkylcarbonyl, C₁₋₄-Halogenalkylcarbonyl, C₁₋₄-Alkylsulfonyl, Nitro oder Cyan steht, und
R⁷ für Wasserstoff oder C₁₋₄-Alkyl steht, und
Q für geradkettiges oder verzweigtes C₁₋₆-Alkyl, C₁₋₆-Halogenalkyl, Hydroxy-C₁₋₆-alkyl, C₁₋₄-Alkanoyloxy-C₁₋₆-alkyl, C₁₋₂-Alkoxy-C₁₋₆-alkyl, Mercapto-C₁₋₆-alkyl, C₁₋₂-Alkylthio-C₁₋₆-alkyl, C₁₋₂-Alkylsulfinyl-C₁₋₆-alkyl, C₁₋₂-Alkylsulfonyl-C₁₋₆-alkyl, Carboxy-C₁₋₆-alkyl, Carbamoyl-C₁₋₆-alkyl, Amino-C₁₋₆-alkyl, C₁₋₆-Alkylamino-C₁₋₆-alkyl, C₁₋₆-Dialkylaminoalkyl, C₂₋₆-Alkenyl, C₂₋₆-Alkinyl, C₂₋₆-Halogenalkenyl, C₃₋₆-Cycoalkyl, sowie für gegebenenfalls substituiertes Aryl, Aryl-C₁₋₂-alkyl, Hetaryl oder Hetaryl-C₁₋₂-alkyl, oder gegebenefalls für einen Rest aus der Gruppe G² und G³

R⁸-Y- (G²)

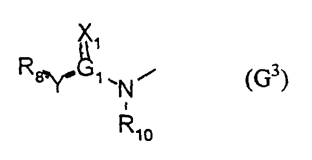

steht,
worin 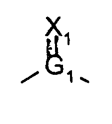 Carboxy, Thiocarboxy oder Sulfonyl bedeuten kann,
Y für Sauerstoff, Schwefel oder -NR⁹ steht,
R⁸ für den Fall, daß Y für Stickstoff steht, eine über ein Stickstoffatom verknüpfte cyclische Aminogruppe bedeuten kann,
R⁸ und R⁹ unabhängig voneinander für Wasserstoff, geradkettiges oder verzweigtes C₁₋₆-Alkyl, C₁₋₆-Alkenyl, C₁₋₆-Alkinyl, C₃₋₆-Cycloalkyl, C₃₋₆-Cycloalkyl-C₁₋₂-alkyl, Aryl, Arylalkyl, Hetaryl, Hetarylalkyl stehen, die gegebenenfalls substituiert sind, oder
R⁸ und R⁹ gemeinsam mit dem angrenzenden N-Atom für ein carbocyclisches 5-, 6- oder 7-gliedriges Ringsystem oder für ein 7 bis 10-gliedriges bicyclisches Ringsystem, das gegebenenfalls auch durch Sauerstoff, Schwefel, Sulfoxyl, Sulfonyl, Carbonyl, -N-O-, -N=, -NR¹¹- oder durch quaternisierten Stickstoff unterbrochen sein kann und gebenenfalls substituiert ist,
R¹⁰ für Wasserstoff oder C₁₋₄-Alkyl steht,
R¹¹ für Wasserstoff, geradkettiges oder verzweigtes C₁₋₆-Alkyl, C₂₋₆-Alkenyl, C₂₋₆-Alkinyl, C₃₋₈-Cycloalkyl, C₃₋₈-Cycloalkyl-C₁₋₂-alkyl, C₁₋₄-Alkoxycarbonyl, C₁₋₄-Alkylcarbonyl, C₃₋₆-Cycloalkylcarbonyl, Cyan, Aryl, Aryl-C₁₋₂-alkyl, Hetaryl, Hetaryl-C₁₋₂-alkyl stehen, die gegebenenfalls substituiert sind, steht, und
B für Hydroxy, C₁₋₆-Alkoxy, C₂₋₆-Alkenyloxy, C₂₋₆-Alkinyloxy, C₃₋₇-Cycloalkyloxy, C₃₋₇-Cycloalkylalkyloxy, Aryloxy-, Aryl-C₁₋₂-alkyloxy, Hetaryloxy, Hetaryl-C₁₋₂-alkyloxy stehen, die gegebenenfalls substituiert sind, steht, oder
für die Reste -NR¹²R¹³, -NR¹⁴-NR¹²R¹³ und -NR¹⁵-OR¹⁶ steht,
in denen
R¹² und R¹³ unabhängig voneinander für Wasserstoff, geradkettiges oder verzweigtes C₁₋₆-Alkyl, C₁₋₆-Alkylcarbonyl, C₁₋₆-Alkylsulfonyl, C₂₋₆-Alkenyl, C₂₋₆-Alkinyl, C₃₋₈-Cycloalkyl, C₃₋₈-Cycloalkyl-C₁₋₂-alkyl, Aryl, Arylcarbonyl, Arylsulfonyl, Aryl-C₁₋₂-alkyl, Hetaryl, Hetarylcarbonyl, Hetarylsulfonyl oder Hetaryl-C₁₋₂-alkyl, die gegebenenfalls substituiert sind, stehen oder
R¹² und R¹³ gemeinsam mit dem angrenzenden N-Atom für ein carbocyclisches 5-, 6-, 7- oder 8-gliedriges Ringsystem oder für ein 7 bis 10-gliedriges bicyclisches Ringsystem, das gegebenenfalls auch durch Sauerstoff, Schwefel, Sulfoxyl, Sulfonyl, Carbonyl, -N-O-, -N=, -NR¹¹- oder durch quaternisierten Stickstoff unterbrochen sein kann und gebenenfalls substituiert ist,
R¹⁴ für Wasserstoff, geradkettiges oder verzweigtes C₁₋₆-Alkyl, C₃₋₆-Cycloalkyl, Aryl-C₁₋₂-alkyl, Hetaryl-C₁₋₂-alkyl, die gegebenenfalls substituiert sind, steht,
R¹⁵ und R¹⁶ unabhängig voneinander Wasserstoff, geradkettiges oder verzweigtes C₁₋₆-Alkyl, C₁₋₆-Alkylcarbonyl, C₂₋₆-Alkenyl, C₂₋₆-Alkinyl, C₃₋₆-Cycloalkyl, C₃₋₆-Cycloalkyl-C₁₋₂-alkyl, Aryl-C₁₋₂-alkyl oder Hetaryl-C₁₋₂-alkyl, die gegebenenfalls substituiert sind, bedeuten,
R¹⁵ und R¹⁶ gemeinsam mit der angrenzenden N-O-Gruppe für einen carbocyclischen 5-, 6- oder 7-gliedrigen Ring stehen,
sowie deren optische Isomere und Racemate,
zur Herstellung eines Miltels
zur Bekämpfung von Endoparasiten in der Medizin und Tiermedizin.

Die Verbindungen der Formel (I) sind teilweise bekannt und lassen sich analog zu bekannten Verfahren herstellen.

Die Erfindung betrifft ferner:
2. Neue Didepsipeptide der allgemeinen Formel (Ia) und deren Salze, 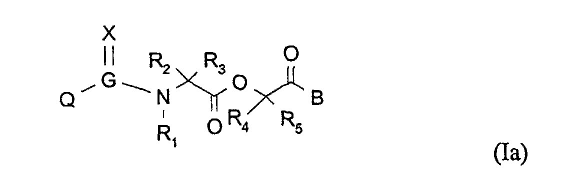 in welcher
   - R¹: für Wasserstoff oder geradkettiges oder verzweigtes C₁₋₄-Alkyl steht, und
   - R¹ und R²: gemeinsam mit den Atomen, an die sie gebunden sind, für einen 5- oder 6-gliedrigen Ring stehen, der gegebenenfalls durch Sauerstoff, Schwefel, Sulfoxyl oder Sulfonyl unterbrochen sein kann und gegebenenfalls substituiert ist,
   - R²: für Wasserstoff, geradkettiges oder verzweigtes Alkyl mit bis zu 6 Kohlenstoffatomen, Alkenyl mit bis zu 4 Kohlenstoffatomen, C₃₋₆-Cycloalkyl, C₃₋₆-Cycloalkyl-C₁₋₂-alkyl, Aryl, Aryl-C₁₋₂-alkyl, Hetaryl, Hetaryl-C₁₋₂-alkyl die gegebenenfalls substituiert sind, steht,
   - R³ und R⁴: für Wasserstoff stehen,
   - R⁵: für Wasserstoff, geradkettiges oder verzweigtes Alkyl mit bis zu 6 Kohlenstoffatomen, Alkenyl mit bis zu 4 Kohlenstoffatomen, C₃₋₆-Cycloalkyl, C₃₋₆-Cycloalkyl-C₁₋₂-alkyl, Aryl, Aryl-C₁₋₂-alkyl, Hetaryl, Hetaryl-C₁₋₂-alkyl die gegebenenfalls substituiert sind, steht, 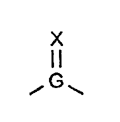 für Carboxy, Thiocarboxy, -C=CH-NO₂, -C=CH-CN, -C=N-R⁶, Sulfoxyl, Sulfonyl, -P(O)-OR⁷ oder P(S)-OR⁷ steht,
   R⁶ für Wasserstoff, Hydroxy, C₁₋₄-Alkoxy, C₁₋₄-Alkylcarbonyl, C₁₋₄-Halogenalkylcarbonyl, C₁₋₄-Alkylsulfonyl, Nitro oder Cyan steht, und
   R⁷ für Wasserstoff oder C₁₋₄-Alkyl steht, und
   - Q: für geradkettiges oder verzweigtes C₁₋₆-Alkyl, C₂₋₆-Alkenyl, C₂₋₆-Alkinyl, C₃₋₆-Cycloalkyl oder Hetaryl-C₁₋₂-alkyl, die gegebenenfalls substituiert sind, oder gegebenenfalls für einen Rest aus der Gruppe G² und G³

   R⁸-Y- (G²)

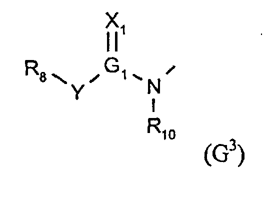

   steht,
   worin 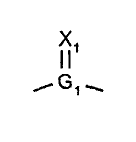 Carboxy, Thiocarboxy oder Sulfonyl bedeuten kann,
   Y für Sauerstoff, Schwefel oder -NR⁹ steht,
   R⁸ für den Fall, daß Y für Stickstoff steht, eine über ein Stickstoffatom verknüpfte cyclische Aminogruppe bedeuten kann,
   R⁸ und R⁹ unabhängig voneinander für Wasserstoff, geradkettiges oder verzweigtes C₁₋₆-Alkyl, C₂₋₆-Alkenyl, C₂₋₆-Alkinyl, C₃₋₆-Cycloalkyl, C₃₋₆-Cycloalkyl-C₁₋₂-alkyl, Hetaryl oder Hetaryl-C₁₋₂-alkyl, die gegebenenfalls substituiert sind, steht, oder
   R⁸ und R⁹ gemeinsam mit dem angrenzenden N-Atom für ein carbocyclisches 5-, 6- oder 7-gliedriges Ringsystem oder für ein 7 bis 10-gliedriges bicyclisches Ringsystem stehen, das gegebenenfalls auch durch Sauerstoff, Schwefel, Sulfoxyl, Sulfonyl, Carbonyl, -N-O-, -N=, -NR¹¹- oder durch quaternisierten Stickstoff unterbrochen sein kann und gegebenenfalls substituiert ist,
   R¹⁰ für Wasserstoff oder C₁₋₄-Alkyl steht, und
   R¹¹ für Wasserstoff, geradkettiges oder verzweigtes C₁₋₆-Alkyl, C₃₋₆-Cycloalkyl, C₂₋₆-Alkenyl, C₂₋₆-Alkinyl, C₃₋₆-Cycloalkyl, C₃₋₆-Cycloalkyl-C₁₋₂-alkyl, C₁₋₄-Alkoxycarbonyl, C₁₋₄-Alkylcarbonyl, C₃₋₆-Cycloalkylcarbonyl, Cyan, Aryl, Aryl-C₁₋₂-alkyl, Hetaryl oder Hetaryl-C₁₋₂-alkyl, die gegebenenfalls substituiert sind, steht, und
   - B: für C₁₋₆-Alkoxy, C₂₋₆-Alkenyloxy, C₂₋₆-Alkinyloxy, C₃₋₇-Cycloalkyloxy, C₃₋₇-Cycloalkyl-C₁₋₂-alkyloxy, Aryloxy-, Aryl-C₁₋₂-alkyloxy, Hetaryloxy, Hetaryl-C₁₋₂-alkyloxy, die gegebenenfalls substituiert sind, steht, oder
   für die Aminoreste -NR¹²R¹³, -NR¹⁴-NR¹²R¹³ und -NR¹⁵-OR¹⁶ steht, in denen
   R¹² und R¹³ unabhängig voneinander für Wasserstoff, geradkettiges oder verzweigtes C₁₋₆-Alkyl, C₁₋₆-Alkylcarbonyl, C₁₋₆-Alkylsulfonyl, C₂₋₆-Alkenyl, C₂₋₆-Alkinyl, C₃₋₈-Cycloalkyl, C₃₋₈-Cycloalkyl-C₁₋₂-alkyl, Aryl, Arylcarbonyl, Arylsulfonyl, Aryl-C₁₋₂-alkyl, Hetaryl, Hetarylcarbonyl, Hetarylsulfonyl oder Hetaryl-C₁₋₂-alkyl, die gegebenenfalls substituiert sind, stehen, oder
   R¹² und R¹³ gemeinsam mit dem angrenzenden N-Atom für ein carbocyclisches 5-, 6-, 7- oder 8-gliedriges Ringsystem oder für ein 7 bis 10-gliedriges bicyclisches Ringsystem stehen, das gegebenenfalls auch durch Sauerstoff, Schwefel, Sulfoxyl, Sulfonyl, Carbonyl, -N-O-, -N=, -NR¹¹- oder durch quaternisierten Stickstoff unterbrochen sein kann und gegebenenfalls substituiert ist,
   R¹⁴ für Wasserstoff, geradkettiges oder verzweigtes C₁₋₆-Alkyl, C₃₋₆-Cycloalkyl, die gegebenenfalls substituiert sind, steht,
   R¹⁵ und R¹⁶ unabhängig voneinander Wasserstoff, geradkettiges oder verzweigtes C₁₋₆-Alkyl, C₁₋₆-Alkylcarbonyl, C₂₋₆-Alkenyl, C₂₋₆-Alkinyl, oder C₃₋₆-Cycloalkyl, die gegebenenfalls substituiert sind, bedeuten, und
   R¹⁵ und R¹⁶ gemeinsam mit der angrenzenden N-O-Gruppe für einen carbocyclischen 5-, 6- oder 7-gliedrigen Ring stehen,
   mit der Maßgabe für den Fall, daß in Formel (Ia) R¹, R⁵ und =G=X gemeinsam für folgende Reste stehen:
   - R¹: steht für Wasserstoff und Methyl,
   - R⁵: steht für Wasserstoff, 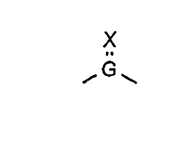 steht für Carboxy,
   die Reste Q und B folgende Bedingung erfüllen müssen:
   - Q: steht für andere Reste als Methyl,
   - B: steht für andere Reste als -NH₂
   sowie mit der Maßgabe für den Fall, daß in Formel (Ia) G² und =G=X gemeinsam für folgende Reste stehen: 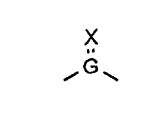 steht für Carboxy,
   - G²: steht für tert-Butyloxy, Benzyloxy und 4-Nitro-benzyloxy,
   der Rest B für andere Reste als tert-Butyloxy, Benzyloxy und 4-Nitrobenzyloxy steht, sowie mit der Maßgabe, daß wenn in Formel (Ia) R⁴ und R⁵ für Wasserstoff stehen, B nicht für NH₂ stehen darf,
   sowie mit der Maßgabe, daß wenn in Formel (Ia) R¹ bis R⁵ für Wasserstoff stehen und 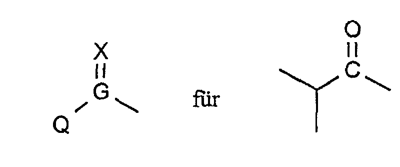 steht,
   - B: nicht für Methylamino steht,
   sowie mit der Maßgabe, daß wenn in Formel (Ia) 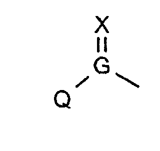 für Benzyloxycarbonyl steht, oder 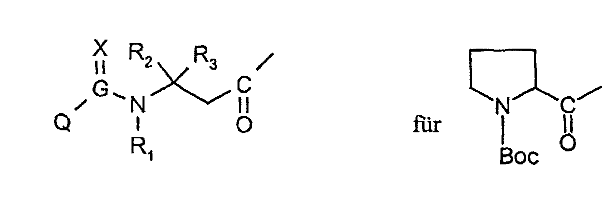 steht,
   - B: nicht für Benzylamino steht,
   sowie mit der Maßgabe, daß für den Fall, daß in Formel (Ia) 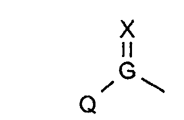 für tert.-Butyloxycarbonyl steht, und R¹ bis R³ für H, entweder R⁴ oder R⁵ für Methyl und das andere für H steht,
   - B: nicht für Ethoxy steht,
   sowie mit der Maßgabe, daß die Verbindung der Formel 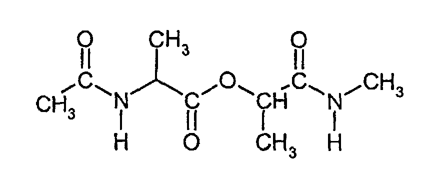 ausgeschlossen ist,
   ferner mit der Maßgabe, daß die Verbindungen
   Methyl-O-(N-carbobenzoxy-L-Leucyl)-hydroxyethanoat,
   Methyl-O-(N-carbobenzoxy-L-Leucyl)-(S)-2-hydroxy-3-phenylpropanoat,
   Methyl-O-(N-carbobenzoxy-L-Leucyl)-(S)-2-hydroxypropanoat,
   Methyl-O-(N-carbobenzoxy-L-Leucyl)-(S)-2-hydroxy-4-methyl-pentanoat,
   Ethyl-N-(benzyloxycarbonyl)-amino-2-(O-glycyl)-glycolat,
   Ethyl-N-(benzyloxycarbonyl)-amino-2-(O-glycyl)-lactat,
   Ethyl-N-(benzyloxycarbonyl)-amino-2-(O-alanyl)-glycolat,
   Ethyl-N-(benzyloxycarbonyl)-amino-2-(O-alanyl)lactat
   ausgeschlossen sind,
   sowie deren optische Isomere und Racemate.
3. Verfahren zur Herstellung der neuen Didepsipeptide der allgemeinen Formel (Ia) und deren Salze, 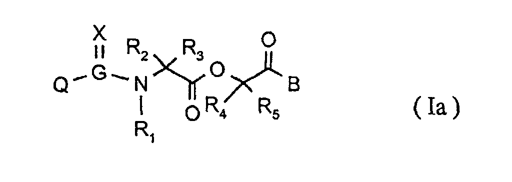 in welcher
   die Reste R¹, R² , R³, R⁴, R⁵, G, Q, X und B die unter Punkt 2 angegebene Bedeutung haben,
   dadurch gekennzeichnet, daß man
   a) N-terminal substituierte Aminosäuren der allgemeinen Formel (II) 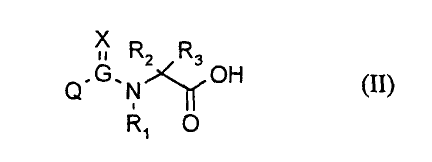 in welcher
      die Reste R¹, R², R³, G, Q, und X die unter Punkt 2 angegebene Bedeutung haben, bzw. deren carboxyaktivierten Derivate oder deren Alkalimetallsalze, gegebenenfalls in Gegenwart eines Katalysators, gegebenenfalls in Gegenwart eines Säurebindemittels und gegebenenfalls in Gegenwart eines Vedünnungsmittels, mit Carbonsäurederivaten der allgemeinen Formel (III) 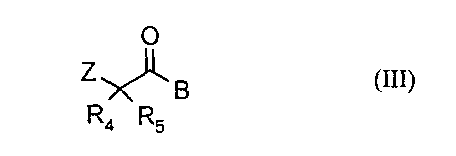 in welcher
      die Reste R⁴, R⁵ und B die unter Punkt 2 angegebene Bedeutung haben und Z für eine geeignete Abgangsgruppe beispielsweise Halogen wie Brom, Chlor Fluor, oder für Hydroxyl steht, umsetzt oder
   b) N-terminal deblockierte Didepsipeptide der allgemeinen Formel (Ib) 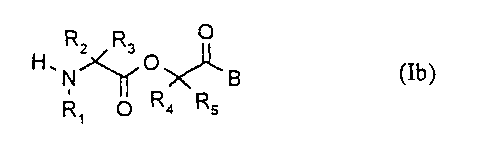 in welcher
      die Reste R¹, R², R³, R⁴, R⁵ und B die unter Punkt 2 angegebene Bedeutung haben, gegebenenfalls in Gegenwart eines Katalysators, gegebenenfalls in Gegenwart eines Säurebindemittels und gegebenenfalls in Gegenwart eines Vedünnungsmittels, mit Verbindungen der allgemeinen Formel (IV) 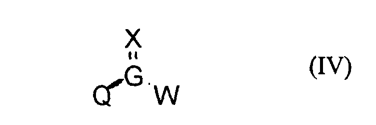 in welcher
      die Reste G, Q, W und X die unter Punkt 2 angegebene Bedeutung haben und W für eine geeignete Abgangsgruppe, wie beispielsweise Halogen, Alkoxy, Alkylthio oder Aryloxy steht, umsetzt oder
   c) N-terminal deblockierte Didepsipeptide der allgemeinen Formel (Ib) 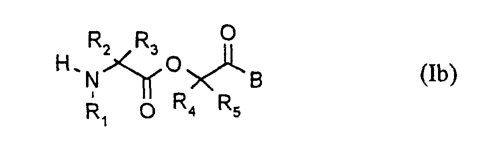 in welcher
      die Reste R¹, R², R³, R⁴, R⁵ und B die unter Punkt 2 angegebene Bedeutung haben, gegebenenfalls in Gegenwart eines Katalysators, gegebenenfalls in Gegenwan eines Säurebindemittels und gegebenenfalls in Gegenwart eines Vedünnungsmittels, mit Verbindungen der allgemeinen Formeln (V) oder (VI)

      R⁸-N=C=X (V)

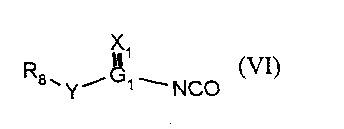

      in welchen
      die Reste R⁸, G¹, X, X¹ und Y die unter Punkt 2 angegebene Bedeutung haben, umsetzt oder
      zur Darstellung der Depsipeptide der allgemeinen Formel (Ia) und deren Salze in der die Gruppe 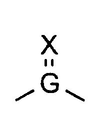 für Carboxy und Y für Sauerstoff steht,
   d) N-terminal deblockierte Didepsipeptide der allgemeinen Formel (Ib) 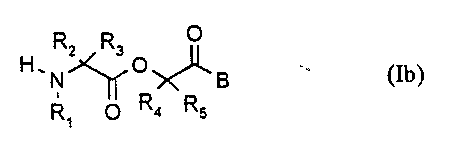 in welcher
      die Reste R¹, R², R³, R⁴, R⁵ und B die unter Punkt 2 angegebene Bedeutung haben,
      in einem ersten Reaktionsschritt mit Kohlendioxid und einem Alkalimetallcarbonat der Formel (VII)

      M₂CO₃ (VII)

      in welcher
      - M: für ein einwertiges Alkalimetallkation, vorzugsweise Lithium, Natrium, Kalium oder Cäsium, insbesondere Kalium oder Cäsium steht,
      dann in einem zweiten Reaktionsschritt das resultierende Alkalimetallsalz der Formel (VIII) 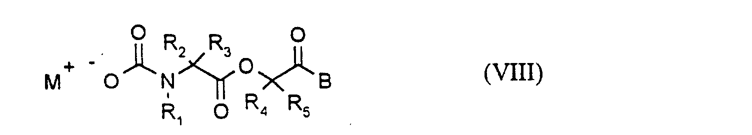 in welcher
      die Reste R¹, R², R³, R⁴, R⁵ und B die unter Punkt 2 angegebene Bedeutung haben,
      - M: für ein salzartig gebundenes Metallkationenäquivalent steht,
      mit Alkylierungsmitteln der Formel (IX)

      R⁸-Hal (IX)

      in welcher
      - R⁸: die unter Punkt 2 angegebene Bedeutung besitzt und
      - Hal: für ein Halogen wie Fluor, Chlor, Brom oder Iod steht,
      gegebenenfalls in Gegenwart eines Verdünnungsmittels und Gegebenenfalls in Gegenwart eines basischen Reaktionshilfsmittels umsetzt, oder
   e) Didepsipeptide der allgemeinen Formel (Ic) 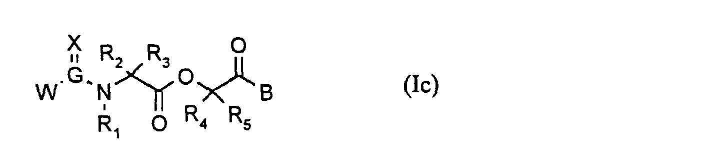 in welcher
      die Reste R¹, R², R³, R⁴, R⁵, G, W, X und B und die unter Punkt 2 und 3b angegebene Bedeutung haben, gegebenenfalls in Gegenwart eines Katalysators, gegebenenfalls in Gegenwart eines Säurebindemittels und gegebenenfalls in Gegenwart eines Verdünnungsmittels, mit Verbindungen der allgemeinen Formel (X)

      R⁸-Y-H (X)

      in welcher
      die Reste R⁸ und Y die weiter oben unter Punkt 2 angegebene Bedeutung haben, umsetzt oder
   f) Didepsipeptide der allgemeinen Formel (Id) 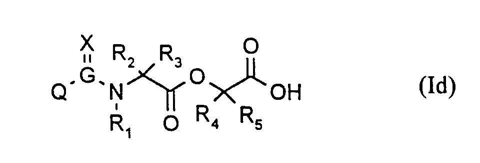 in welcher
      die Reste R¹, R², R³, R⁴, R⁵, G, Q, X und B die unter Punkt 2 angegebene Bedeutung haben bzw. deren carboxyaktivierten Derivate oder deren Alkalimetallsalze, gegebenenfalls in Gegenwart eines Katalysators, gegebenenfalls in Gegenwart eines Säurebindemittels und gegebenenfalls in Gegenwart eines Verdünnungsmittels, mit Verbindungen der allgemeinen Formel (XI)

      H-B (XI)

      in welcher
      der Rest B die weiter oben unter Punkt 2 angegebene Bedeutung hat, umsetzt.

Die substituierten Didepsipeptide und deren Salze sind durch die Formel (I) allgemein definiert.

Die substituierten Didepsipeptide der Formel (I) sowie deren Säureadditionssalze und Metallsalz-Komplexe besitzen sehr gute endoparasitizide, insbesondere anthelmintische Wirkung und können bevorzugt im Bereich der Veterinärmedizin eingesetzt werden.

Gegebenenfalls substituiertes Alkyl allein oder als Bestandteil eines Restes in den allgemeinen Formeln bedeutet geradkettiges oder verzweigtes Alkyl mit vorzugsweise 1 bis 6, insbesondere 1 bis 4 Kohlenstoffatomen. Beispielhaft seien gegebenenfalls substituiertes Methyl, Ethyl, n-Propyl, Isopropyl, n-Butyl, Isobutyl, sec-Butyl, tert-Butyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,2-Dimethylbutyl. 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl und 2-Ethylbutyl genannt. Vorzugsweise seien Methyl, Ethyl, n-Propyl, Isopropyl, n-Butyl, Isobutyl, sec-Butyl und tert-Butyl genannt.

Gegebenenfalls substituiertes Alkenyl allein oder als Bestandteil eines Restes in den allgemeinen Formeln bedeutet geradkettiges oder verzweigtes Alkenyl mit vorzugsweise 1 bis 6, insbesondere 1 bis 4 Kohlenstoffatomen. Beispielhaft seien gegebenenfalls substituiertes Vinyl, 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-bute-nyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-pro-penyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Me-thyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-bute-nyl, 1-Ethyl-3-butenyl, 2-Ethyl-2-butenyl, 2-Ethyl-3-butenyl, 1,1,2-Trimethyl-2-prope-nyl, 1-Ethyl-1-methyl-2-propenyl und 1-Ethyl-2-methyl-2-propenyl genannt. Vorzugs-weise seien gegebenenfalls substituiertes Ethenyl, 2-Propenyl, 2-Butenyl oder 1-Methyl-2-propenyl genannt.

Gegebenenfalls substituiertes Alkinyl allein oder als Bestandteil eines Restes in den allgemeinen Formeln bedeutet geradkettiges oder verzweigtes Alkinyl mit vorzugsweise 1 bis 6, insbesondere 1 bis 4 Kohlenstoffatomen. Beispielhaft seien gegebenenfalls substituiertes Ethinyl, 2-Propinyl, 2-Butinyl, 3-Butinyl, 1-Methyl-2-pro-pinyl, 2-Pentinyl, 3-Pentinyl, 4-Pentinyl, 1-Methyl-3-butinyl, 2-Methyl-3-butinyl, 1-Me-thyl-2-butinyl, 1,1 -Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-bu-tinyl, 1-Ethyl-3-butinyl, 2-Ethyl-3-butinyl und 1-Ethyl-1-methyl-2-propinyl genannt.

Vorzugsweise seien gegebenenfalls substituiertes Ethinyl, 2-Propinyl oder 2-Butinyl genannt.

Gegebenenfalls substituiertes Cycloalkyl allein oder als Bestandteil eines Restes in den allgemeinen Formeln bedeutet mono-, bi- und tricyclisches Cycloalkyl, vorzugsweise mit 3 bis 10, insbesondere mit 3, 5 oder 7 Kohlenstoffatomen.

Beispielhaft seien gegebenenfalls substituiertes Cyclopropyl, Cyclobutyl, Cyclopentyl, Cyclohexyl, Cycloheptyl, Cyclooktyl, Bicyclo[2.2.1]heptyl, Bicyclo[2.2.2]oktyl und Adamantyl genannt.

Halogenalkyl allein oder als Bestandteil eines Restes in den allgemeinen Formeln enthält 1 bis 4, insbesondere 1 oder 2 Kohlenstoffatome und vorzugsweise 1 bis 9, insbesondere 1 bis 5 gleiche oder verschiedene Halogenatome vorzugsweise Fluor, Chlor und Brom, insbesondere Fluor und Chlor. Beispielhaft seien Trifluormethyl, Trichlormethyl, Chlordifluormethyl, Dichlorfluormethyl, Chlormethyl, Brommethyl, 1-Fluorethyl, 2-Fluorethyl, 2, 2-Difluorethyl, 2,2,2-Trifluorethyl, 2,2,2-Trichlorethyl, 2-Chlor-2,2-difluorethyl, Pentafluorethyl und Pentafluor-tert-butyl genannt.

Gegebenenfalls substituiertes Alkoxy allein oder als Bestandteil eines Restes in den allgemeinen Formeln bedeutet geradkettiges oder verzweigtes Alkoxy mit vorzugsweise 1 bis 6, insbesondere 1 bis 4 Kohlenstoffatomen. Beispielhaft seien gegebenenfalls substituiertes Methoxy, Ethoxy, n-Propoxy, Isopropoxy, n-Butoxy, Iso-butoxy, sec-Butoxy und tert-Butoxy genannt.

Gegebenenfalls substituiertes Halogenalkoxy allein oder als Bestandteil eines Restes in den allgemeinen Formeln bedeutet geradkettiges oder verzweigtes Halogenalkoxy mit vorzugsweise 1 bis 6, insbesondere 1 bis 4 Kohlenstoffatomen. Beispielhaft seien gegebenenfalls substituiertes Difluormethoxy, Trifluormethoxy, Trichlormethoxy, Chlordifluormethoxy, 1-Fluorethoxy, 2-Fluorethoxy, 2,2-Difluorethoxy, 1,1,2,2,-Tetrafluorethoxy, 2,2,2-Trifluorethoxy und 2-Chlor-1,1,2-trifluorethoxy genannt.

Gegebenenfalls substituiertes Alkylthio allein oder als Bestandteil eines Restes in den allgemeinen Formeln bedeutet geradkettiges oder verzweigtes Alkylthio mit vorzugsweise 1 bis 6, insbesondere 1 bis 4 Kohlenstoffatomen. Beispielhaft seien gegebenenfalls substituiertes Methylthio, Ethylthio, n-Propylthio, Isopropylthio, n-Butylthio, Isobutylthio, sec-Butylthio und tert-Butylthio genannt.

Gegebenenfalls substituiertes Halogenalkylthio allein oder als Bestandteil eines Restes in den allgemeinen Formeln bedeutet geradkettiges oder verzweigtes Halogenalkylthio mit vorzugsweise 1 bis 6, insbesondere 1 bis 4 Kohlenstoffatomen. Beispielhaft seien gegebenenfalls substituiertes Difluormethylthio, Trifluormethylthio, Trichlormethylthio, Chlordifluormethylthio, 1-Fluorethylthio, 2-Fluorethylthio, 2,2-Di-fluorethylthio, 1,1,2,2,-Tetrafluorethylthio, 2,2,2-Trifluorethylthio und 2-Chlor-1,1,2-trifluorethylthio genannt.

Gegebenenfalls substituiertes Alkylcarbonyl allein oder als Bestandteil eines Restes in den allgemeinen Formeln bedeutet geradkettiges oder verzweigtes Alkylcarbonyl mit vorzugsweise 1 bis 6, insbesondere 1 bis 4 Kohlenstoffatomen. Beispielhaft seien gegebenenfalls substituiertes Methylcarbonyl, Ethylcarbonyl, n-Propylcarbonyl, Isopropylcarbonyl, n-Butylcarbonyl, Isobutylcarbonyl, sec-Butylcarbonyl und tert-Butylcarbonyl genannt.

Gegebenenfalls substituiertes Cycloalkylcarbonyl allein oder als Bestandteil eines Restes in den allgemeinen Formeln bedeutet mono-, bi- und tricyclisches Cycloalkylcarbonyl, vorzugsweise mit 3 bis 10, insbesondere mit 3, 5 oder 7 Kohlenstoffatomen. Beispielhaft seien gegebenenfalls substituiertes Cyclopropylcarbonyl, Cyclobutylcarbonyl, Cyclopentylcarbonyl, Cyclohexylcarbonyl, Cycloheptylcarbonyl, Cyclooktylcarbonyl, Bicyclo[2.2.1]heptylcarbonyl, Bicyclo[2.2.2]oktylcarbonyl und Adamantylcarbonyl genannt.

Gegebenenfalls substituiertes Alkoxycarbonyl allein oder als Bestandteil eines Restes in den allgemeinen Formeln bedeutet geradkettiges oder verzweigtes Alkoxycarbonyl mit vorzugsweise 1 bis 6, insbesondere 1 bis 4 Kohlenstoffatomen. Beispielhaft seien gegebenenfalls substituiertes Methoxycarbonyl, Ethoxycarbonyl, n-Propoxycarbonyl, Isopropoxycarbonyl, n-Butoxycarbonyl, Isobutoxycarbonyl, sec-Butoxycarbonyl und tert-Butoxycarbonyl genannt.

Aryl ist beispielsweise ein ein-, zwei- oder mehrkerniger aromatischer Rest wie Phenyl, Naphthyl, Tetrahydronaphthyl, Indanyl, Fluorenyl und ähnliches, vorzugsweise Phenyl oder Naphthyl.

Gegebenenfalls substituiertes Aryl in den allgemeinen Formeln bedeutet vorzugsweise gegebenenfalls substituiertes Phenyl oder Naphthyl, insbesondere Phenyl.

Gegebenenfalls substituiertes Arylalkyl in den allgemeinen Formeln bedeutet vorzugsweise gegebenenfalls im Arylteil und/oder Alkyl substituiertes Arylalkyl mit vorzugsweise 6 oder 10, insbesondere 8 Kohlenstoffatomen im Arylteil (vorzugsweise Phenyl oder Naphthyl, insbesondere Phenyl) und vorzugsweise 1 bis 4, insbesondere 1 oder 2 Kohlenstoffatomen im Alkylteil, wobei der Alkylteil geradkettig oder verzweigt sein kann. Beispielhaft und vorzugsweise seien gegebenenfalls substituiertes Benzyl und Phenylethyl genannt.

Gegebenenfalls substituiertes Hetaryl allein oder als Bestandteil eines Restes in den allgemeinen Formeln bedeutet 5- bis 7-gliedrige Ringe mit vorzugsweise 1 bis 3, insbesondere 1 oder 2 gleichen oder verschiedenen Heteroatomen. Als Heteroatome in den Heteroaromaten stehen Sauerstoff, Schwefel oder Stickstoff. Beispielhaft und vorzugsweise seien gegebenenfalls substituiertes Furyl, Thienyl, Pyrazolyl, Imid-azolyl, 1,2,3- und 1,2,4-Triazolyl, Isoxazolyl, Thiazolyl, Isothiazolyl, 1,2,3-, 1,3,4-, 1,2,4- und 1,2,5-Oxadiazolyl, Azepinyl, Piperidyl, Pyrrolyl, Pyridyl, Piperazinyl, Pyridazinyl, Pyrimidinyl, Pyrazinyl, 1,3,5-, 1,2,4-und 1,2,3-Triazinyl, 1,2,4-, 1,3,2-, 1,3,6- und 1,2,6-Oxazinyl, Oxepinyl, Thiepinyl und 1,2,4-Diazepinyl genannt.

Die gegebenenfalls substituierten Reste der allgemeinen Formeln können einen oder mehrere, vorzugsweise 1 bis 3, insbesondere 1 bis 2 gleiche oder verschiedene Substituenten tragen. Als Substituenten seien beispielhaft und vorzugsweise aufgeführt:

Alkyl mit vorzugsweise 1 bis 4, insbesondere 1 bis 2 Kohlenstoffatomen, wie Methyl, Ethyl, n-Propyl, Isopropyl, n-Butyl, Isobutyl, sec-Butyl und tert-Butyl; Alkoxy mit vorzugsweise 1 bis 4, insbesondere 1 bis 2 Kohlenstoffatomen, wie Methoxy, Ethoxy, n-Propoxy, Isopropoxy, n-Butoxy, Isobutoxy, sec-Butoxy und tert-Butoxy; Alkylthio wie Methylthio, Ethylthio, n-Propylthio, Isopropylthio, n-Butylthio, Isobutylthio, sec-Butylthio und tert-Butylthio; Halogenalkyl mit vorzugsweise 1 bis 4, insbesondere 1 bis 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor oder Chlor stehen, wie Difluormethyl, Trifluormethyl, Trichlormethyl; Hydroxy; Halogen, vorzugsweise Fluor, Chlor, Brom und Iod, insbesondere Fluor und Chlor; Cyan; Nitro; Amino; Monoalkyl- und Dialkylamino mit vorzugsweise 1 bis 4, insbesondere 1 oder 2 Kohlenstoffatomen je Alkylgruppe, wie Methylamino, Methylethylamino, Dimethyl-amino, n-Propylamino, Isopropylamino, Methyl-n-butylamino; Alkylcarbonylreste wie Methylcarbonyl; Alkoxycarbonyl mit vorzugsweise 2 bis 4, insbesondere 2 bis 3 Kohlenstoffatomen wie Methoxycarbonyl und Ethoxycarbonyl; Alkylsulfinyl mit 1 bis 4, insbesondere 1 bis 2 Kohlenstoffatomen; Halogensulfinyl mit 1 bis 4, insbesondere 1 bis 2 Kohlenstoffatomen und 1 bis 5 Halogenatomen wie Trifluormethylsilfinyl; Sulfonyl (-SO₂-OH); Alkylsulfonyl mit vorzugsweise 1 bis 4, insbesondere 1 oder 2 Kohlenstoffatomen, wie Methylsulfonyl und Ethylsulfonyl; Halogenalkylsulfonyl mit 1 bis 4, insbesondere 1 bis 2 Kohlenstoffatomen und 1 bis 5 Halogenatomen wie Trifluormethylsulfonyl, Perfluor-n-butylsulfonyl, Perfluorisobutylsulfonyl; Arylsulfonyl mit vorzugsweise 6 oder 10 Arylkohlenstoffatomen wie Phenylsulfonyl; Acyl, Aryl, Aryloxy, Hetaryl, Hetaryloxy, die ihrerseits einen der oben genannten Substituenten tragen können sowie den Formiminorest (-HC=N-O-Alkyl).

Als geeignete cyclische Aminogruppen kommen heteroaromatische oder aliphatische Ringsysteme mit einem oder mehreren Stickstoffatomen als Heteroatom in Frage, bei denen die Heterocyclen gesättigt oder ungesättigt, ein Ringsystem oder mehrere kondensierte Ringsysteme sein können, und gegebenenfalls weitere Heteroatome wie Stickstoff, Sauerstoff und Schwefel u.s.w. enthalten. Außerdem können cyclische Aminogruppen auch ein Spiroring oder ein verbrücktes Ringsystem bedeuten. Die Anzahl der Atome, die cyclische Aminogruppen bilden, ist nicht beschränkt, beispielsweise bestehen sie im Falle eines Einringsystems aus 3 bis 8 Atomen und im Falle eines Dreiringsystems aus 7 bis 11 Atomen.

Beispielhaft für cyclischen Aminogruppen mit gesättigten und ungesättigten monocyclischen Gruppen mit einem Stickstoffatom als Heteroatom seien 1-Azetidinyl, Pyrrolidino, 2-Pyrrolin-1-yl, 1-Pyrrolyl, Piperidino, 1,4-Dihydropyridin-1-yl, 1,2,5,6-Tetrahydropyridin-1-yl, Homopiperidino genannt; beispielhaft für cyclischen Aminogruppen mit gesättigten und ungesättigten monocyclischen Gruppen mit zwei oder mehreren Stickstoffatomen als Heteroatome seien 1-Imidazolidinyl, 1-Imidazolyl, 1-Pyrazolyl, 1-Triazolyl, 1-Tetrazolyl, 1-Piperazinyl, 1-Homopiperazinyl, 1,2-Dihydro-pyridazin-1-yl, 1,2-Dihydropyrimidin-1-yl, Perhydropyrimidin-1-yl und 1,4-Diazacycloheptan-1-yl genannt; beispielhaft für cyclischen Aminogruppen mit gesättigten und ungesättigten monocyclischen Gruppen mit einem bis drei Stickstoffatomen und einem bis zwei Schwefelatomen als Heteroatome seien Thiazolidin-3-yl, Isothiazolin-2-yl, Thiomorpholino oder Dioxothiomorpholino genannt; beispielhaft für cyclischen Aminogruppen mit gesättigten und ungesättigten kondensierten cyclischen Gruppen seien Indol-1-yl, 1,2-Dihydrobenzimidazol-1-yl, Perhydropyrrolo[1,2-a]pyrazin-2-yl genannt; beispielhaft für cyclischen Aminogruppen mit spirocyclischen Gruppen sei das 2-Azaspiro[4,5]decan-2-yl genannt; beispielhaft für cyclischen Aminogruppen verbrückten heterocyclischen Gruppen sei das 2-Azabicyclo[2,2,1]heptan-7-yl genannt.

Bevorzugt sind Verbindungen der Formel (Ia) und deren Salze 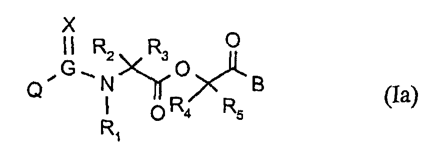 in welcher
- R¹: für Wasserstoff oder geradkettiges oder verzweigtes C₁₋₆-Alkyl, insbesondere Methyl, Ethyl, Propyl, Isopropyl, sec-Butyl, tert-Butyl, steht, und
- R¹ und R²: gemeinsam mit den Atomen, an die sie gebunden sind, für einen 5- oder 6- gliedrigen Ring stehen, der gegebenfalls durch Schwefel, unterbrochen sein kann und gegebenenfalls durch Hydroxy substituiert ist,
- R²: für Wasserstoff, geradkettiges oder verzweigtes Alkyl mit bis zu 6 Kohlenstoffatomen, insbesondere Methyl, Ethyl, Propyl, Isopropyl, sec-Butyl, tert-Butyl, Hydroxy-C₁₋₂-alkyl, insbesondere Hydroxymethyl, 1-Hydroxyethyl, C₁₋₄-Alkanoyloxy-C₁₋₄-alkyl, insbesondere Acetoxymethyl, 1-Acetoxyethyl, C₁₋₄-Alkoxy-C₁₋₄-alkyl, insbesondere Methoxymethyl, 1-Methoxyethyl, Aryl-C₁₋₄-alkoxy-C₁₋₄-alkyl, insbesondere Benzyloxymethyl, 1-Benzyloxymethyl, Mercapto-C₁₋₄-alkyl, insbesondere Mercaptomethyl, C₁₋₄-Alkylthio-C₁₋₄-alkyl, insbesondere Methylsulfinylmethyl, C₁₋₄-Alkylsulfonyl-C₁₋₄-alkyl, insbesondere Methylsulfonylethyl, Carboxy-C₁₋₄-akyl, insbesondere Carboxymethyl, Carboxyethyl, C₁₋₄-Alkoxycarbonyl-C₁₋₄-alkyl, insbesondere Methoxycarbonylmethyl, Ethoxycarbonylmethyl, Aryl-C₁₋₄-alkoxycarbonyl-C₁₋₄-alkyl, insbesondere Benzyloxycarbonylmethyl, Carbamoyl-C₁₋₄-alkyl, insbesondere Carbamoylmethyl, Carbamoylethyl, Amino-C₁₋₄-alkyl, insbesondere Aminopropyl, Aminobutyl, C₁₋₄-Alkylamino-C₁₋₄-alkyl, insbesondere Methylaminopropyl, Methylaminobutyl, C₁₋₄-Dialkylamino-C₁₋₄-alkyl, insbesondere Dimethylaminopropyl, Dimethylaminobutyl, Guanido-C₁₋₄-alkyl, insbesondere Guanidopropyl, C₁₋₄-Alkoxycarbonylamino-C₁₋₄-alkyl, insbesondere tert-Butylcarbonylaminopropyl, tert-Butylcarbonylaminobutyl, Alkenyl mit bis zu 6 Kohlenstoffatomen, insbesondere Vinyl, 2-Propenyl, 2-Butenyl, 1-Methyl-2-propenyl, C₃₋₆-Cycloalkyl-C₁₋₂-alkyl, insbesondere Cyclopropylmethyl, Cyclobutylmethyl, Cyclohexylmethyl, Hetaryl-C₁₋₂-alkyl, insbesondere Benzo[b]thien-1-yl-methyl, Benzo[b]-thien-3-yl, Pyrid-2-yl-methyl, Pyrid-3-yl-methyl, Pyrid-4-yl-methyl, Fur-2-yl-methyl, Fur-3-yl-methyl, Thien-2-yl-methyl, Thien-3-yl-methyl, Indol-3-yl-methyl, N-Methyl-indol-3-yl-methyl, Imidazol-4-yl-methyl, N-Methylimidazol-4-yl-methyl, Aryl-C₁₋₂-alkyl, insbesondere Benzyl steht, die gegebenenfalls durch Reste aus der Reihe Halogen, insbesondere Fluor, Chlor, Brom oder Iod, Hydroxy, C₁₋₄-Alkyl, insbesondere Methyl oder tert-Butyl, C₁₋₄-Halogenalkyl, insbesondere Trifluormethylethyl, Difluormethyl oder Trichlormethyl, C₁₋₄-Alkoxy, insbesondere Methoxy, Ethoxy oder tert-Butyloxy, C₁₋₄-Halogenalkoxy, insbesondere Trifluormethoxy, Difluormethoxy, C₁₋₄-Alkylthio, insbesondere Methylthio, C₁₋₄-Halogenalkylthio, insbesondere Trifluormethylthio, C₁₋₂-Alkylendioxy, insbesondere Methylendioxy oder Ethylendioxy, Nitro, Amino, C₁₋₄-Alkylamino, insbesondere Methylamino, C₁₋₄-Di-alkylamino, insbesondere Dimethylamino, C₃₋₆-Cycloalkylamino, insbesondere Pyrrolidino, Piperidino, C₃₋₆-Cycloalkylthioamino, insbesondere Thiomorpholino und Dioxothiomorpholino, C₃₋₆-Cycloalkyldiamino, insbesondere N-Methylpiperazino, substituiert sein können, und
- R³ und R⁴: für Wasserstoff stehen,
- R⁵: für Wasserstoff, geradkettiges oder verzweigtes Alkyl mit bis zu 6 Kohlenstoffatomen, insbesondere Methyl, Ethyl, Propyl, Isopropyl, sec-Butyl, tert-Butyl, Hydroxy-C₁₋₂-alkyl, insbesondere Hydroxymethyl, 1-Hydroxyethyl, C₁₋₄-Alkanoyloxy-C₁₋₄-alkyl, insbesondere Acetoxymethyl, 1-Acetoxyethyl, C₁₋₄-Alkoxy-C₁₋₄-alkyl, insbesondere Methoxymethyl, 1-Methoxyethyl, Aryl-C₁₋₄-alkoxy-C₁₋₄-alkyl, insbesondere Benzyloxymethyl, 1-Benzyloxymethyl, Mercapto-C₁₋₄-alkyl, insbesondere Mercaptomethyl, C₁₋₄-Alkylthio-C₁₋₄-alkyl, insbesondere Methylsulfinylmethyl, C₁₋₄-Alkylsulfonyl-C₁₋₄-alkyl, insbesondere Methylsulfonylethyl, Carboxy-C₁₋₄-alkyl, insbesondere Carboxymethyl, Carboxyethyl, C₁₋₄-Alkoxycarbonyl-C₁₋₄-alkyl, insbesondere Methoxycarbonylmethyl, Ethoxycarbonylmethyl, ArylC₁₋₄-alkoxycarbonyl-C₁₋₄-alkyl, insbesondere Benzyloxycarbonylmethyl, Carbamoyl-C₁₋₄-alkyl, insbesondere Carbamoylmethyl, Carbamoylethyl, Amino-C₁₋₄-alkyl, insbesondere Aminopropyl, Aminobutyl, C₁₋₄-Alkylamino-C₁₋₄-alkyl, insbesondere Methylaminopropyl, Methylaminobutyl, C₁₋₄-Dialkylamino-C₁₋₄-alkyl, insbesondere Dimethylaminopropyl, Dimethylaminobutyl, Guanido-C₁₋₄-alkyl, insbesondere Guanidopropyl, C₁₋₄-Alkoxycarbonylamino-C₁₋₄-alkyl, insbesondere tert-Butylcarbonylaminopropyl, tert-Butylcarbonylaminobutyl, Alkenyl mit bis zu 6 Kohlenstoffatomen, insbesondere Vinyl, 2-Propenyl, 2-Butenyl, 1-Methyl-2-propenyl, C₃₋₆-Cycloalkyl-C₁₋₂-alkyl, insbesondere Cyclopropylmethyl, Cyclobutylmethyl, Cyclohexylmethyl, Hetaryl-C₁₋₂-alkyl, insbesondere Benzo[b]thien-1-yl-methyl, Benzo[b]-thien-3-yl, Pyrid-2-yl-methyl, Pyrid-3-yl-methyl, Pyrid-4-yl-methyl, Fur-2-yl-methyl, Fur-3-yl-methyl, Thien-2-yl-methyl, Thien-3-yl-methyl, Indol-3-yl-methyl, N-Methyl-indol-3-yl-methyl, Imidazol-4-yl-methyl, N-Methylimidazol-4-yl-methyl, Aryl-C₁₋₂-alkyl, insbesondere Benzyl steht, die gegebenenfalls durch Reste aus der Reihe Halogen, insbesondere Fluor, Chlor, Brom oder Iod, Hydroxy, C₁₋₄-Alkyl, insbesondere Methyl oder tert-Butyl, C₁₋₄-Halogenalkyl, insbesondere Trifluormethylethyl, Difluormethyl oder Trichlormethyl, C₁₋₄-Alkoxy, insbesondere Methoxy, Ethoxy oder tert-Butyloxy, C₁₋₄-Halogenalkoxy, insbesondere Trifluormethoxy, Difluormethoxy, C₁₋₄-Alkylthio, insbesondere Methylthio, C₁₋₄-Halogenalkylthio, insbesondere Trifluormethylthio, C₁₋₂-Mkylendioxy, insbesondere Methylendioxy oder Ethylendioxy, Nitro, Amino, C₁₋₄-Alkylamino, insbesondere Methylamino, C₁₋₄-Di-alkylamino, insbesondere Dimethylamino, C₃₋₆-Cycloalkylamino, insbesondere Pyrrolidino, Piperidino, C₃₋₆-Cycloalkylthioamino, insbesondere Thiomorpholino und Dioxothiomorpholino, C₃₋₆-Cycloalkyldiamino, insbesondere N-Methylpiperazino, substituiert sein können, und 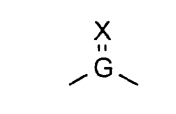 für Carboxy, Thiocarboxy, -C=CH-NO₂, -C=CH-CN, -C=N-R⁶, Sulfoxyl, Sulfonyl, -P(O)-OR⁷ oder P(S)-OR⁷ steht,
R⁶ für Wasserstoff, Hydroxy, C₁₋₄-Alkoxy, insbesondere Methoxy, Ethoxy, sec-Butyloxy, C₁₋₄-Alkylcarbonyl, insbesondere Methylcarbonyl, Ethylcarbonyl, C₁₋₄-Halogenalkylcarbonyl, insbesondere Trifluormethylcarbonyl, Trichlormethylcarbonyl, C₁₋₄-Alkylsulfonyl, insbesondere Methylsulfonyl, Ethylsulfonyl, Propylsulfonyl, Nitro oder Cyan steht, und
R⁷ für Wasserstoff oder C₁₋₄-Alkyl, insbesondere Methyl oder Ethyl steht, und
- Q: für geradkettiges oder verzweigtes C₁₋₄-Alkyl, insbesondere Methyl, Ethyl, Propyl, Isopropyl, sec-Butyl, tert-Butyl, C₁₋₄-Halogenalkyl, insbesondere Trifluormethyl, Trichlormethyl, Chlordifluormethyl, Dichlorfluormethyl, 1-Fluorethyl, Chlormethyl, Brommethyl, 1-Fluorethyl, 2,2,2-Trifluorethyl, 2,2,2-Trichlorethyl, C₂₋₆-Alkenyl, insbesondere Vinyl, 2-Propenyl, 1-Methyl-2-propenyl und 2-Butenyl, C₂₋₆-Halogenalkenyl, insbesondere Difluorvinyl, Dichlorvinyl, 2-Chlor-2-propenyl, 2,3,3-Trifluor-2-propenyl, 2,3,3-Trichlor-2-propenyl, 4,4-Difluor-3-butenyl, C₃₋₆-Cycloalkyl, insbesondere Cyclopropyl, Cyclobutyl, Cyclopentyl und Cyclohexyl, Hetaryl-C₁₋₂-alkyl, insbesondere Pyridylmethyl und Thiazolylmethyl, die gegebenenfalls durch Reste aus der Reihe Halogen, insbesondere Fluor, Chlor, Brom oder Iod, Hydroxy, C₁₋₄-Alkyl, insbesondere Methyl oder tert-Butyl, C₁₋₄-Halogenalkyl, insbesondere Trifluormethyl, Difluormethyl oder Trichlormethyl, C₁₋₄-Alkoxy, insbesondere Methoxy, C₁₋₄-Halogenalkoxy, insbesondere Trifluormethoxy, Difluormethoxy, C₁₋₄-Alkylthio, insbesondere Methylthio, C₁₋₄-Halogenalkylthio, insbesondere Trifluormethylthio, Nitro, Amino, C₁₋₄-Alkylamino, insbesondere Methylamino, C₁₋₄-Di-alkylamino, insbesondere Dimethylamino, substituiert sein können, oder
gegebenenfalls für einen Rest aus der Gruppe G² und G³

R⁸-Y- (G²)

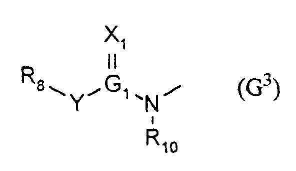

steht,
worin 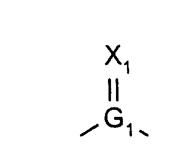 Carboxy, Thiocarboxy oder Sulfonyl bedeuten kann,
Y für Sauerstoff, Schwefel oder -NR⁹ steht,
R⁸ für den Fall, daß Y für Stickstoff steht, eine über ein Stickstoffatom verknüpfte cyclische Aminogruppe, insbesondere 1-Azetidinyl, Pyrrolidino, 2-Pyrrolin-1-yl, 1-Pyrrolyl, Piperidino, 1,4-Dihydropyridin-1-yl, 1-Imidazolidinyl, 1-Imidazolyl, 1-Pyrazolyl, 1-Triazolyl, 1-Tetrazolyl, 1-Piperazinyl, 1-Homopiperazinyl, 1,2-Dihydro-pyridazin-1-yl, 1,2-Dihydropyrimidin-1-yl, Perhydropyrimidin-1-yl, 1,4-Diazacyclo-heptan-1-yl, Thiazolidin-3-yl, Isothiazolin-2-yl, Morpholino, Thiomorpholino, Dioxothiomorpholino, die gegebenenfalls durch Reste aus der Reihe Halogen, insbesondere Fluor, Chlor, Brom oder Iod, C₁₋₄-Alkyl, insbesondere Methyl, Hydoxy-C₁₋₄-Alkyl, insbesondere Hydroxymethyl, Amino-C₁₋₄-Alkyl, insbesondere Aminomethyl, Aminoethyl, C₁₋₄-Monoalkylamino-C₁₋₄-alkyl, insbesondere Methylaminomethyl, Methylaminoethyl, C₁₋₄-Dialkylamino-C₁₋₄-alkyl, insbesondere Dimethylaminomethyl, Dimethylaminoethyl, Amino, Hydroxy, C₁₋₄-Alkoxy, insbesondere Methoxy, C₁₋₄-Alkylcarbonyl, insbesondere Methylcarbonyl, C₁₋₄-Alkoxycarbonyl, insbesondere Methoxycarbonyl, substituiert sein können,
R⁸ und R⁹ unabhängig voneinander für Wasserstoff, geradkettiges oder verzweigtes C₁₋₄-Alkyl, insbesondere Methyl, Ethyl, Propyl, Isopropyl, sec-Butyl, tert-Butyl, C₂₋₄-Alkenyl, insbesondere Vinyl, 2-Propenyl, 1-Methyl-2-propenyl und 2-Butenyl, C₂₋₄-Alkinyl, insbesondere Ethinyl, 2-Propinyl und 2-Butinyl, C₃₋₆-Cycloalkyl, insbesondere Cyclopropyl, Cyclobutyl, Cyclopentyl und Cyclohexyl, C₃₋₆-Cycloalkyl-C₁₋₂-alkyl, insbesondere Cyclopropylmethyl, Cyclobutylmethyl, Cyclopentylmethyl und Cyclohexylmethyl, Hetaryl, insbesondere Pyridyl und Thiazolyl, Hetaryl-C₁₋₂-alkyl, insbesondere Pyridylmethyl und Thiazolylmethyl, die gegebenenfalls durch Reste aus der Reihe Halogen, insbesondere Fluor, Chlor, Brom oder Iod, C₁₋₄-Alkyl, insbesondere Methyl, Hydoxy-C₁₋₄-Alkyl, insbesondere Hydroxymethyl, Amino-C₁₋₄-Alkyl, insbesondere Aminomethyl, Aminoethyl, C₁₋₄-Monoalkylamino-C₁₋₄-alkyl, insbesondere Methylaminomethyl, Methylaminoethyl, C₁₋₄-Dialkylamino-C₁₋₄-alkyl, insbesondere Dimethylaminomethyl, Dimethylaminoethyl, Amino, Hydroxy, C₁₋₄-Alkoxy, insbesondere Methoxy, C₁₋₄-Alkylcarbonyl, insbesondere Methylcarbonyl, C₁₋₄-Alkoxycarbonyl, insbesondere Methoxycarbonyl, substituiert sein können, stehen, oder
R⁸ und R⁹ gemeinsam mit dem angrenzenden N-Atom für ein carbocyclisches 5-, 6- oder 7-gliedriges Ringsystem oder für ein 7 bis 10-gliedriges bicyclisches Ringsystem, das gegebenenfalls auch durch Sauerstoff, Schwefel, Sulfoxyl, Sulfonyl, Carbonyl, -N-O, -N=, -NR¹¹- oder durch quaternisierten Stickstoff unterbrochen sein kann und gebenenfalls durch C₁₋₄-Alkyl, insbesondere Methyl, Hydoxy-C₁₋₄-Alkyl, insbesondere Hydroxymethyl, Amino-C₁₋₄-Alkyl, insbesondere Aminomethyl, Aminoethyl, C₁₋₄-Monoalkylamino-C₁₋₄-alkyl, insbesondere Methylaminomethyl, Methylaminoethyl, C₁₋₄-Dialkylamino-C₁₋₄-alkyl, insbesondere Dimethylaminomethyl, Dimethylaminoethyl, Amino, Hydroxy, C₁₋₄-Alkoxy, insbesondere Methoxy, C₁₋₄-Alkylcarbonyl, insbesondere Methylcarbonyl, C₁₋₄-Alkoxycarbonyl, insbesondere Methoxycarbonyl, Halogen, insbesondere Fluor, Chlor, Brom oder Iod, substituiert ist, stehen,
R¹⁰ für Wasserstoff oder C₁₋₄-Alkyl, insbesondere Methyl, Ethyl, Propyl, Isopropyl, sec-Butyl, tert-Butyl, steht, und
R¹¹ für Wasserstoff, geradkettiges oder verzweigtes C₁₋₆-Alkyl, insbesondere Methyl, Ethyl, Propyl, Isopropyl, sec-Butyl, tert-Butyl, C₂₋₆-Alkenyl, insbesondere Vinyl, 2-Propenyl, 1-Methyl-2-propenyl und 2-Butenyl, C₂₋₆-Alkinyl, insbesondere Ethinyl, 2-Propinyl und 2-Butinyl, C₃₋₆-Cycloalkyl, insbesondere Cyclopropyl, Cyclobutyl, Cyclopentyl und Cyclohexyl, C₃₋₆-Cycloalkyl-C₁₋₂-alkyl, insbesondere Cyclopropylmethyl, Cyclobutylmethyl, Cyclopentylmethyl und Cyclohexylmethyl, C₁₋₄-Alkylcarbonyl, insbesondere Methylcarbonyl, Ethylcarbonyl, n-Propylcarbonyl, Isopropylcarbonyl, n-Butylcarbonyl, Isobutylcarbonyl, sec-Butylcarbonyl, tert-Butylcarbonyl, C₃₋₆-Cycloalkylcarbonyl, insbesondere Cyclopropylcarbonyl, Cyclobutylcarbonyl, Cyclopentylcarbonyl und Cyclohexylcarbonyl, C₁₋₄-Alkoxycarbonyl, insbesondere Methoxycarbonyl, Ethoxycarbonyl, n-Propoxycarbonyl, Isopropoxycarbonyl, n-Butoxycarbonyl, Isobutoxycarbonyl, sec-Butoxycarbonyl, tert-Butoxycarbonyl, Hydoxy-C₁₋₄-alkyl, insbesondere Hydroxymethyl, Hydroxyethyl, Hydroxyethylsulfonylethyl, C₁₋₄-Alkylamino, insbesondere Methylamino, Ethylamino, C₁₋₄-Monoalkylamino-C₁₋₄-alkyl, insbesondere Methylaminomethyl, Methylaminoethyl, C₁₋₄-Dialkylamino-C₁₋₄-alkyl, insbesondere Dimethylaminomethyl, Dimethylaminoethyl, Dimethylaminopropyl, C₃₋₇-Cycloalkylamino-C₁₋₄-alkyl, insbesondere N-Pyrrolidinoethyl, N-Morpholinoethyl, N-Piperidinoethyl, N-Thiomorpholinoethyl, N¹-(N⁴-Methyl-piperazino)-ethyl, C₃₋₆-Cycloalkylaminocarbonyl-C₁₋₂-alkyl, insbesondere N-Morpholinocarbonylmethyl, Cyan, Aryl, insbesondere Phenyl, Aryl-C₁₋₂-alkyl, insbesondere Phenylmethyl, Hetaryl, insbesondere Pyridyl oder Thiazolyl, Hetaryl-C₁₋₂-alkyl, insbesondere Pyridylmethyl und Thiazolylmethyl, die gegebenenfalls durch Reste aus der Reihe Halogen, insbesondere Fluor, Chlor, Brom oder Iod, C₁₋₄-Alkyl, insbesondere Methyl, C₁₋₄-Halogenalkyl, insbesondere Trifluormethyl, Trichlormethyl, Amino, Hydroxy, C₁₋₄-Alkoxy, insbesondere Methoxy, C₁₋₂-Alkylendioxy, insbesondere Methylendioxy oder Ethylendioxy, C₁₋₄-Halogenalkoxy, insbesondere Trifluormethoxy, Difluormethoxy, C₁₋₄-Alkylthio, insbesondere Methylthio, C₁₋₄-Halogenalkylthio, insbesondere Trifluormethylthio, C₁₋₄-Alkylsulfonyl insbesondere Methylsulfonyl, C₁₋₄-Alkylamino, insbesondere Methylamino, Di-C₁₋₄-alkylamino, insbesondere Dimethylamino, C₁₋₄-Alkylcarbonyl, insbesondere Methylcarbonyl, C₁₋₄-Alkoxycarbonyl, insbesondere Methoxycarbonyl, substituiert sein können, steht,
B für geradkettiges oder verzweigtes C₁₋₆-Alkoxy, insbesondere Methoxy, Ethoxy, n-Propoxy, Isopropoxy, n-Butoxy, Isobutoxy, sec-Butoxy und tert-Butoxy, C₂₋₆-Alkenyloxy, Vinyloxy, 2-Propenyloxy, 2-Butenyloxy, 3-Butenyloxy, 1-Methyl-2-propenyloxy, 2-Methyl-2-propenyloxy, 2-Pentenyloxy, 1-Methyl-2-butenyloxy, 2-Methyl-2-butenyloxy, 3-Methyl-2-butenyloxy, 1-Methyl-3-butenyloxy, 1,1-Dimethyl-2-propenyloxy, 1,2-Dimethyl-2-propenyloxy, 1-Ethyl-2-propenyloxy, 2-Hexenyloxy, 1,1-Dimethyl-2-butenyloxy, 1,2-Dimethyl-2-butenyloxy, 1-Ethyl-2-butenyloxy, C₂₋₆-Alkinyloxy, insbesondere Ethinyloxy, 2-Propinyloxy, 2-Butinyloxy, 3-Butinyloxy, 1-Methyl-2-propinyloxy, 2-Pentinyloxy, 1-Methyl-3-butinyl, 2-Methyl-3-butinyloxy, 1,1-Dimethyl-2-propinyloxy, 1-Ethyl-2-propinyloxy und 1-Methyl-2-pentinyloxy, C₃₋₈-Cycloalkyloxy, insbesondere Cyclopropyloxy, Cyclobutyloxy, Cyclopentyloxy, Cyclohexyloxy, Cycloheptyloxy, C₃₋₇-Cycloalkyl-C₁₋₂-alkyloxy, insbesondere Cyclopropylmethoxy, Cyclobutylmethoxy, Cyclopentylmethoxy, Cyclohexylmethoxy, Cycloheptylmethoxy, Aryloxy-, insbesondere Phenoxy, Aryl-C₁₋₂-alkyloxy, insbesondere Benzyloxy, Hetaryloxy, insbesondere Pyridyloxy, Hetaryl-C₁₋₂-alkyloxy, insbesondere Pyridylmethyl und Thiazolylmehyl, die gegebenenfalls durch Reste aus der Reihe Halogen, insbesondere Fluor, Chlor, Brom oder Iod, C₁₋₄-Alkyl, insbesondere Methyl, C₁₋₄-Halogenalkyl, insbesondere Trifluormethyl, Trichlormethyl, Amino, Hydroxy, C₁₋₄-Alkoxy, insbesondere Methoxy, C₁₋₂-Alkylendioxy, insbesondere Methylendioxy oder Ethylendioxy,C₁₋₄-Halogenalkoxy, insbesondere Trifluormethoxy, Difluormethoxy, C₁₋₄-Alkylthio, insbesondere Methylthio, C₁₋₄-Halogenalkylthio, insbesondere Trifluormethylthio, C₁₋₄-Alkylsulfonyl insbesondere Methylsulfonyl, C₁₋₄-Alkylamino, insbesondere Methylamino, Di-C₁₋₄-alkylamino, insbesondere Dimethylamino, C₃₋₆-Cycloalkylamino, insbesondere Pyrrolidino, Piperidino, C₃-C₆-Cycloalkyloxamino, insbesondere Morpholino, C₃₋₆-Cycloalkylthioamino, insbesondere Thiomorpholino und Dioxothiomorpholino, C₃₋₆-Cycloalkyldiamino, insbesondere N-Methylpiperazino, C₁₋₄-Alkylcarbonyl, insbesondere Methylcarbonyl, C₁₋₄-Alkoxycarbonyl, insbesondere Methoxycarbonyl, substituiert sein können, steht, oder
für die Aminoreste -NR¹²R¹³, -NR¹⁴-NR¹²R¹³ und -NR¹⁵-OR¹⁶ steht,
in denen
R¹² und R¹³ unabhängig voneinander für Wasserstoff, geradkettiges oder verzweigtes C₁₋₆-Alkyl, insbesondere Methyl, Ethyl, n-Propyl, Isopropyl, n-Butyl, Isobutyl, sec-Butyl, tert-Butyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, C₁₋₆-Alkylcarbonyl, insbesondere Methylcarbonyl, Ethylcarbonyl, n-Propylcarbonyl, Isopropylcarbonyl, n-Butylcarbonyl, Isobutylcarbonyl, sec-Butylcarbonyl, tert-Butylcarbonyl, Pentylcarbonyl, 1-Methylbutylcarbonyl, 2-Methylbutylcarbonyl, C₁₋₆-Alkylsulfonyl, insbesondere Methylsulfonyl, Ethylsulfonyl, n-Propylsulfonyl, Isopropylsulfonyl, n-Butylsulfonyl, Isobutylsulfonyl, sec-Butylsulfonyl, C₂₋₆-Alkenyl, insbesondere Vinyl, 2-Propenyl, 2-Butenyl, 3-Butenyl, 1-Methyl-2-propenyl, 2-Pentenyl, 3-Pentenyl, 1-Methyl-2-butenyl, 2-Methyl-2-butenyl, 3-Methyl-2-butenyl, 1,1-Dimethyl-2-propenyl, 1,2-Dimethyl-2-propenyl, 1-Ethyl-2-pro-penyl, 2-Hexenyl, 1,2-Dimethyl-3-butenyl, 2,3-Dimethyl-2-butenyl, 1-Ethyl-2-butenyl, 1-Ethyl-3-butenyl, 2-Ethyl-2-butenyl, 2-Ethyl-3-butenyl, C₂₋₆-Alkinyl, insbesondere 2-Propinyl, 2-Butinyl, 3-Butinyl, C₃₋₈-Cycloalkyl, insbesondere Cyclopropyl, Cyclobutyl, Cyclopentyl, Cyclohexyl, Cycloheptyl, Cyclooktyl, C₃₋₈-Cycloalkyl-C₁₋₂-alkyl, insbesondere Cyclopropylmethyl, Cyclobutylmethyl, Cyclopentylmethyl, Cyclohexylmethyl, Cycloheptylmethyl, Hydoxy-C₁₋₄-alkyl, insbesondere Hydroxymethyl, Hydroxyethyl, Hydroxyethylsulfonylethyl, C₁₋₄-Alkylamino, insbesondere Methylamino, Ethylamino, C₁₋₄-Monoalkylamino-C₁₋₄-alkyl, insbesondere Methylaminomethyl, Methylaminoethyl, Methylaminopropyl, C₁₋₄-Dialkylamino-C₁₋₄-alkyl, insbesondere Dimethylaminomethyl, Dimethylaminoethyl, Dimethylaminopropyl, C₃₋₇-Cycloalkylamino-C₁₋₄-alkyl, insbesondere N-Pyrrolidinoethyl, N-Morpholinoethyl, N-Piperidinoethyl, N-Thiomorpholinoethyl, N¹-(N⁴-Methyl-piperazino)-ethyl, C₃₋₆-Cycloalkylaminocarbonyl-C₁₋₂-alkyl, insbesondere N-Morpholinocarbonylmethyl, Aryl, insbesondere Phenyl, Aryl-C₁₋₂-alkyl, insbesondere Benzyl und Phenethyl, Hetaryl, insbesondere Pyridyl, Thiazolyl, Hetaryl-C₁₋₆-alkyl, insbesondere Pyridylmethyl, Thiazolylmethyl, die gegebenenfalls durch Reste aus der Reihe Halogen, insbesondere Fluor, Chlor, Brom oder Iod, Hydroxy, C₁₋₄-Alkyl, insbesondere Methyl oder tert-Butyl, C₁₋₄-Alkoxy, insbesondere Methoxy, Ethoxy oder tert-Butyloxy, Nitro, Amino, C₁₋₄-Alkylamino, insbesondere Methylamino, C₁₋₄-Dialkylamino, insbesondere Dimethylamino, C₃-C₆-Cycloalkylamino, insbesondere Piperidino und Morpholino, substituiert sind, stehen, oder
R¹² und R¹³ gemeinsam mit dem angrenzenden N-Atom für ein carbocyclisches 5-, 6-, 7- oder 8-gliedriges Ringsystem oder für ein 7 bis 10-gliedriges bicyclisches Ringsystem, das gegebenenfalls auch durch Sauerstoff, Schwefel, Sulfoxyl, Sulfonyl, Carbonyl, -N-O-, -N=, -NR¹¹- oder durch quatemisierten Stickstoff unterbrochen sein kann und gegebenenfalls durch Aryl, insbesondere Phenyl, C₁₋₄-Alkyl, insbesondere Methyl, Hydoxy-C₁₋₄-alkyl, insbesondere Hydroxymethyl, Amino-C₁₋₄-alkyl, insbesondere Aminomethyl, Aminoethyl, C₁₋₄-Monoalkylamino-C₁₋₄-alkyl, insbesondere Methylaminomethyl, Methylaminoethyl, C₁₋₄-Dialkylamino-C₁₋₄-alkyl, insbesondere Dimethylaminomethyl, Dimethylaminoethyl. C₃₋₆-Cycloalkylamino, insbesondere Pyrrolidino, Piperidino oder Morpholino, Amino, Hydroxy, Cyan, C₁₋₄-Alkoxy, insbesondere Methoxy, C₁₋₄-Alkylcarbonyl, insbesondere Methylcarbonyl, C₁₋₄-Alkoxycarbonyl, insbesondere Methoxycarbonyl, Halogen, insbesondere Fluor, Chlor, Brom oder Iod, substituiert ist, stehen,oder
einen Rest aus den Gruppen G⁴, G⁵, G⁶,G⁷ und G⁸ 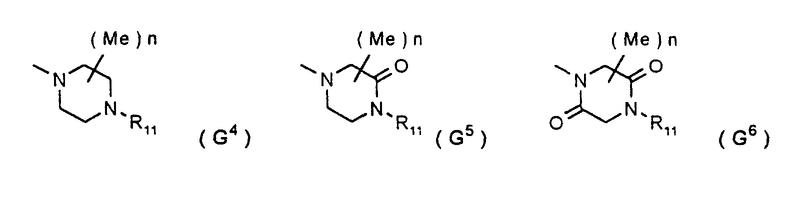 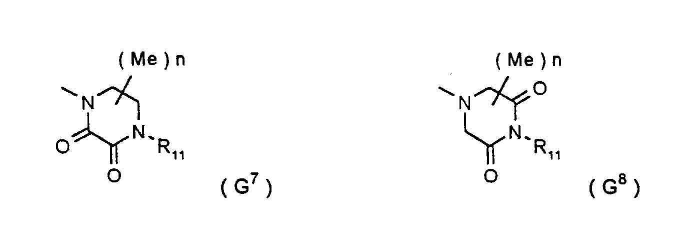 worin
n die Zahlen 0, 1, 2, 3 oder 4 bedeuten kann,
R¹⁴ für Wasserstoff, geradkettiges oder verzweigtes C₁₋₆-Alkyl, insbesondere Methyl, Ethyl, n-Propyl, Isopropyl, n-Butyl, Isobutyl, sec-Butyl, tert-Butyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, C₃₋₆-Cycloalkyl, insbesondere Cyclopropyl, Cyclobutyl, Cyclopentyl, Cyclohexyl, die gegenenenfalls substituiert sind, steht,
R¹⁵ und R¹⁶ unabhängig voneinander Wasserstoff, geradkettiges oder verzweigtes C₁₋₆-Alkyl, insbesondere Methyl, Ethyl, n-Propyl, Isopropyl, n-Butyl, Isobutyl, sec-Butyl, tert-Butyl, Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, Hexyl, 1-Methylpentyl, C₁₋₆-Alkylcarbonyl, insbesondere Methylcarbonyl, Ethylcarbonyl, n-Propylcarbonyl, Isopropylcarbonyl, n-Butylcarbonyl, Isobutylcarbonyl, sec-Butylcarbonyl, tert-Butylcarbonyl, Pentylcarbonyl, 1-Methylbutylcarbonyl, 2-Methylbutylcarbonyl, C₂₋₆-Alkenyl, insbesondere Vinyl, 2-Propenyl, 2-Butenyl, 3-Butenyl, 1-Methyl-2-propenyl, 2-Pentenyl, 3-Pentenyl, 1-Methyl-2-butenyl, 2-Methyl-2-butenyl, 3-Methyl-2-butenyl, 1,1-Dimethyl-2-propenyl, 1,2-Dimethyl-2-propenyl, 1-Ethyl-2-pro-penyl, 2-Hexenyl, 1,2-Dimethyl-3-butenyl, 2,3-Dimethyl-2-butenyl, 1-Ethyl-2-butenyl, 1-Ethyl-3-butenyl, 2-Ethyl-2-butenyl, 2-Ethyl-3-butenyl, C₂₋₆-Alkinyl, insbesondere 2-Propinyl, 2-Butinyl, 3-Butinyl, C₃₋₆-Cycloalkyl, insbesondere Cyclopropyl, Cyclobutyl, Cyclopentyl, Cyclohexyl, die gegebenenfalls substituiert sind, stehen,
R¹⁵ und R¹⁶ gemeinsam mit der angrenzenden N-O-Gruppe für einen carbocyclischen 5-, 6- oder 7-gliedrigen Ring stehen,
mit der Maßgabe für den Fall, daß in Formel (Ia) R¹, R⁵ und =G=X gemeinsam für folgende Reste stehen:
- R¹: steht für Wasserstoff und Methyl,
- R⁵: steht für Wasserstoff, 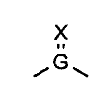
steht für Carboxy,
die Reste Q und B folgende Bedingung erfüllen müssen:
- Q: steht für andere Reste als Methyl,
- B: steht für andere Reste als -NH₂
sowie mit der Maßgabe für den Fall, daß in Formel (Ia) G² und =G=X gemeinsam für folgende Reste stehen: 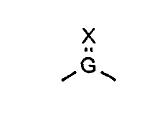 steht für Carboxy,
- G²: steht für tert-Butyloxy, Benzyloxy und 4-Nitro-benzyloxy,
der Rest B für andere Reste als tert-Butyloxy, Benzyloxy und 4-Nitrobenzyloxy steht, sowie mit der Maßgabe, daß wenn in Formel (Ia) R⁴ und R⁵ für Wasserstoff stehen, B nicht für NH₂ stehen darf,
sowie mit der Maßgabe, daß wenn in Formel (Ia) R¹ bis R⁵ für Wasserstoff stehen und 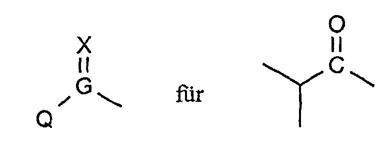 steht,
- B: nicht für Methylamino steht,
sowie mit der Maßgabe, daß wenn in Formel (Ia) 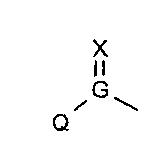 für Benzyloxycarbonyl steht, oder 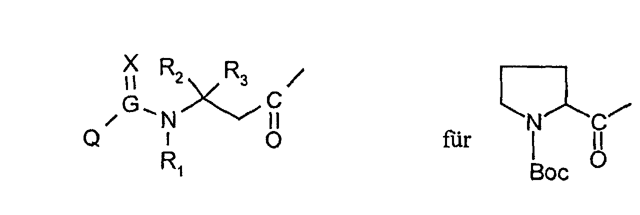 steht,
- B: nicht für Benzylamino steht,
sowie mit der Maßgabe, daß für den Fall, daß in Formel (Ia) 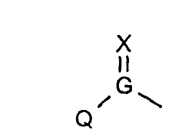 für tert.-Butyloxycarbonyl steht, und R¹ bis R³ für H, und entweder R⁴ oder R⁵ für Methyl und das andere für H steht,
- B: nicht für Ethoxy steht,
sowie mit der Maßgabe, daß die Verbindung der Formel 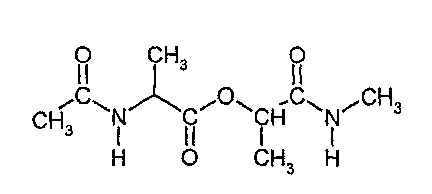 ausgeschlossen ist,
ferner mit der Maßgabe, daß die Verbindungen
Methyl-O-(N-carbobenzoxy-L-Leucyl)-hydroxyethanoat,
Methyl-O-(N-carbobenzoxy-L-Leucyl)-(S)-2-hydroxy-3-phenylpropanoat,
Methyl-O-(N-carbobenzoxy-L-Leucyl)-(S)-2-hydroxypropanoat,
Methyl-O-(N-carbobenzoxy-L-Leucyl)-(S)-2-hydroxy-4-methyl-pentanoat,
Ethyl-N-(benzyloxycarbonyl)-amino-2-(O-glycyl)-glycolat,
Ethyl-N-(benzyloxycarbonyl)-amino-2-(O-glycyl)-lactat,
Ethyl-N-(benzyloxycarbonyl)-amino-2-(O-alanyl)-glycolat,
Ethyl-N-(benzyloxycarbonyl)-amino-2-(O-alanyl)lactat
ausgeschlossen sind,
sowie deren optische Isomere und Racemate.

Besonders bevorzugt sind Verbindungen der Formel (Ia) und deren Salze 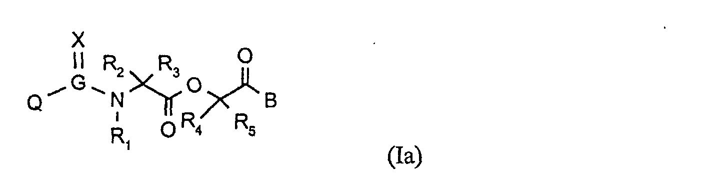 in welcher
- R¹: für Wasserstoff, geradkettiges oder verzweigtes C₁₋₄-Alkyl, insbesondere Methyl, Ethyl steht,
- R²: für Wasserstoff, geradkettiges oder verzweigtes C₁₋₄-Alkyl insbesondere Methyl, Ethyl, Propyl, Isopropyl, sec-Butyl steht,
- R³ und R⁴: für Wasserstoff stehen,
- R⁵: für Wasserstoff, geradkettiges oder verzweigtes C₁₋₄-Alkyl, insbesondere Methyl, Ethyl, Propyl, Isopropyl, sec-Butyl, Hetaryl-C₁₋₂-alkyl, insbesondere Pyrid-2-yl-methyl, Pyrid-3-yl-methyl, Pyrid-4-yl-methyl, Thien-2-yl-methyl, Thien-3-yl-methyl, Aryl-C₁₋₂-alkyl, insbesondere Benzyl steht, die gegebenenfalls durch Reste aus der Reihe Halogen, insbesondere Fluor, Chlor, Brom oder Iod, Hydroxy, C₁₋₄-Alkyl, insbesondere Methyl oder tert-Butyl, C₁₋₄-Alkoxy, insbesondere Methoxy, Ethoxy oder tert-Butyloxy, C₁₋₂-Alkylendioxy, insbesondere Methylendioxy oder Ethylendioxy, Nitro, Amino, C₁₋₄-Alkylamino, insbesondere Methylamino, C₁₋₄-Dialkylamino, insbesondere Dimethylamino, C₃₋₆-Cycloalkylamino, insbesondere Pyrrolidino, Piperidino, C₃₋₆-Cycloalkylthioamino, insbesondere Thiomorpholino und Dioxothiomorpholino, C₃₋₆-Cycloalkyldiamino, insbesondere N-Methyl-piperazino, substituiert sein können, und 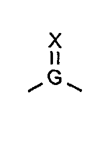 für Carboxy, -C=CH-NO₂, -C=CH-CN, -C=N-R⁶ oder Sulfonyl steht,
R⁶ C₁₋₄-Halogenalkylcarbonyl, insbesondere Trifluormethylcarbonyl, Trichlormethylcarbonyl, Alkyl-C₁₋₄-sulfonyl, insbesondere Methylsulfonyl, Ethylsulfonyl, Nitro oder Cyan steht, und
- Q: für einen Rest aus der Gruppe G² und G³

R⁸-Y- (G²)

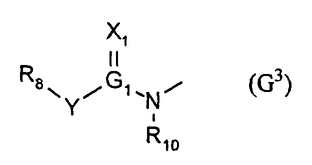

steht,
worin 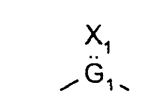 Carboxy oder Sulfonyl bedeuten kann,
- Y: für Sauerstoff oder -NR⁹ steht,
- R⁸: für den Fall, daß Y für Stickstoff steht, eine über ein Stickstoffatom verknüpfte cyclische Aminogruppe, insbesondere Pyrrolidino, 2-Pyrrolin-1-yl, 1-Pyrrolyl, Piperidino, 1,4-Dihydropyridin-1-yl, 1-Piperazinyl, 1-Homopiperazinyl, Morpholino, Thiomorpholino, Dioxothiomorpholino, die gegebenenfalls durch Reste aus der Reihe Halogen, insbesondere Fluor, Chlor, Brom oder Iod, C₁₋₄-Alkyl, insbesondere Methyl, Hydoxy-C₁₋₄-alkyl, insbesondere Hydroxymethyl, Amino-C₁₋₄-alkyl, insbesondere Aminomethyl, Aminoethyl, C₁₋₄-Monoalkylamino-C₁₋₄-alkyl, insbesondere Methylaminomethyl, Methylaminoethyl, C₁₋₄-Dialkylamino-C₁₋₄-alkyl, insbesondere Dimethylaminomethyl, Dimethylaminoethyl, Amino, Hydroxy, C₁₋₄-Alkoxy, insbesondere Methoxy, C₁₋₄-Alkylcarbonyl, insbesondere Methylcarbonyl, C₁₋₄-Alkoxycarbonyl, insbesondere Methoxycarbonyl, substituiert sein können, bedeutet,
- R⁸ und R⁹: unabhängig voneinander für geradkettiges oder verzweigtes C₁₋₄-Alkyl, insbesondere Methyl, Ethyl, Propyl, Isopropyl, sec-Butyl, C₂₋₄-Alkenyl, insbesondere Vinyl, 2-Propenyl, 1-Methyl-2-propenyl, C₂₋₄-Alkinyl, insbesondere Ethinyl, 2-Propinyl, C₃₋₆-Cycloalkyl, insbesondere Cyclopropyl, Hetaryl-C₁₋₂-alkyl, insbesondere Pyridylmethyl und Thiazolylmethyl, die gegebenenfalls durch Reste aus der Reihe Halogen, insbesondere Fluor, Chlor, Brom oder Iod, C₁₋₄-Alkyl, insbesondere Methyl, Hydoxy-C₁₋₄-alkyl, insbesondere Hydroxymethyl, Amino-C₁₋₄-alkyl, insbesondere Aminomethyl, Aminoethyl, C₁₋₄-Monoalkylamino-C₁₋₄-alkyl, insbesondere Methylaminomethyl, Methylaminoethyl, C₁₋₄-Dialkylamino-C₁₋₄-alkyl, insbesondere Dimethylaminomethyl, Dimethylaminoethyl, Amino, Hydroxy, C₁₋₄-Alkoxy, insbesondere Methoxy, C₁₋₄-Alkylcarbonyl, insbesondere Methylcarbonyl, C₁₋₄-Alkoxycarbonyl, insbesondere Methoxycarbonyl, substituiert sein können, stehen, oder
- R⁸ und R⁹: gemeinsam mit dem angrenzenden N-Atom für ein carbocyclisches 5-, 6- oder 7-gliedriges Ringsystem, das gegebenenfalls auch durch Sauerstoff, Schwefel, Sulfoxyl, Sulfonyl, Carbonyl, -N=, -NR¹¹- oder durch quaternisierten Stickstoff unterbrochen sein kann und gebenenfalls durch C₁₋₄-Alkyl, insbesondere Methyl, Hydoxy-C₁₋₄-alkyl, insbesondere Hydroxymethyl, Amino-C₁₋₄-alkyl, insbesondere Aminomethyl, Aminoethyl, C₁₋₄-Monoalkylamino-C₁₋₄-alkyl, insbesondere Methylaminomethyl, Methylaminoethyl, C₁₋₄-Dialkylamino-C₁₋₄-alkyl, insbesondere Dimethylaminomethyl, Dimethylaminoethyl, Amino, Hydroxy, C₁₋₄-Alkoxy, insbesondere Methoxy, C₁₋₄-Alkylcarbonyl, insbesondere Methylcarbonyl, C₁₋₄-Alkoxycarbonyl, insbesondere Methoxycarbonyl, Halogen, insbesondere Fluor, Chlor, Brom oder Iod, substituiert ist, stehen,
- R¹⁰: für Wasserstoff oder C₁₋₄-Alkyl, insbesondere Methyl steht, und
- R¹¹: für Wasserstoff, geradkettiges oder verzweigtes C₁₋₆-Alkyl, insbesondere Methyl, Ethyl, C₂₋₆-Alkenyl, insbesondere Vinyl, C₃₋₆-Cycloalkyl, insbesondere Cyclopropyl, C₁₋₄-Alkylcarbonyl, insbesondere Methylcarbonyl, C₃₋₆-Cycloalkylcarbonyl, insbesondere Cyclopropylcarbonyl, C₁₋₄-Alkoxycarbonyl, insbesondere Methoxycarbonyl, Ethoxycarbonyl, tert-Butoxycarbonyl, Hydroxy-C₁₋₄-alkyl, insbesondere Hydroxyethyl, Hydroxysulfonylethyl, C₁₋₄-Alkylamino, insbesondere Ethylamino, C₁₋₄-Monoalkylamino-C₁₋₄-alkyl, insbesondere Methylaminoethyl, C₁₋₄-Dialkylamino-C₁₋₄-alkyl, insbesondere Dimethylaminoethyl, C₃₋₇-Cycloalkylamino-C₁₋₄-alkyl, insbesondere N-Morpholinoethyl, N-Piperidinoethyl, C₃₋₆-Cycloalkylaminocarbonyl-C₁₋₂-alkyl, insbesondere N-Morpholinocarbonylmethyl, Cyan, Aryl, insbesondere Phenyl, Aryl-C₁₋₂-alkyl, insbesondere Phenylmethyl, Hetaryl, insbesondere Pyridyl oder Thiazolyl, Hetaryl-C₁₋₂-alkyl, insbesondere Pyridylmethyl und Thiazolylmethyl, die gegebenenfalls durch Reste aus der Reihe Halogen, insbesondere Fluor, Chlor, Brom oder Iod, C₁₋₄-Alkyl, insbesondere Methyl, C₁₋₄-Halogenalkyl, insbesondere Trifluormethyl, Trichlormethyl, Amino, Hydroxy, C₁₋₄-Alkoxy, insbesondere Methoxy, C₁₋₂-Alkylendioxy, insbesondere Methylendioxy oder Ethylendioxy,C₁₋₄-Halogenalkoxy, insbesondere Trifluormethoxy, Difluormethoxy, C₁₋₄-Alkylthio, insbesondere Methylthio, C₁₋₄-Halogenalkylthio, insbesondere Trifluormethylthio, C₁₋₄-Alkylsulfonyl insbesondere Methylsulfonyl, C₁₋₄-Alkylamino, insbesondere Methylamino, Di-C₁₋₄-alkylamino, insbesondere Dimethylamino, C₁₋₄-Alkylcarbonyl, insbesondere Methylcarbonyl, C₁₋₄-Alkoxycarbonyl, insbesondere Methoxycarbonyl, substituiert sein können, steht,
- B: für geradkettiges oder verzweigtes C₁₋₆-Alkoxy, insbesondere Methoxy, Ethoxy, n-Propoxy, Isopropoxy, n-Butoxy, Isobutoxy, sec-Butoxy und tert-Butoxy, C₂₋₆-Alkenyloxy, Vinyloxy, 2-Propenyloxy, 1,1-Dimethyl-2-propenyloxy, 1,2-Dimethyl-2-propenyloxy, 1-Ethyl-2-propenyloxy, C₂₋₆-Alkinyloxy, insbesondere 2-Propinyloxy, 1-Methyl-2-propinyloxy, 1,1-Dimethyl-2-propinyloxy, C₃₋₈-Cycloalkyloxy, insbesondere Cyclopropyloxy, Cyclohexyloxy, C₃₋₇-Cycloalkyl-C₁₋₂-alkyloxy, insbesondere Cyclopropylmethoxy, Cyclohexylmethoxy, Aryloxy-, insbesondere Phenoxy, Aryl-C₁₋₂-alkyloxy, insbesondere Benzyloxy, Hetaryloxy, insbesondere Pyridyloxy, Hetaryl-C₁₋₂-alkyloxy, insbesondere Pyridylmethyl und Thiazolylmehyl, die gegebenenfalls durch Reste aus der Reihe Halogen, insbesondere Fluor, Chlor, Brom oder Iod, Hydroxy, C₁₋₄-Alkyl, insbesondere Methyl, C₁₋₄-Halogenalkyl, insbesondere Trifluormethyl, Trichlormethyl, Amino, Hydroxy, C₁₋₄-Alkoxy, insbesondere Methoxy, C₁₋₂-Alkylendioxy, insbesondere Methylendioxy oder Ethylendioxy, C₁₋₄-Alkylamino, insbesondere Methylamino, C₁₋₄-Dialkylamino, insbesondere Dimethylamino, C₃₋₆-Cycloalkylamino, insbesondere Pyrrolidino, Piperidino, C₃₋₆-Cycloalkylthioamino, insbesondere Thiomorpholino und Dioxothiomorpholino, C₃₋₆-Cycloalkyldiamino, insbesondere N-Methylpiperazino, substituiert sein können, steht, oder für die Aminoreste -NR¹²R¹³, -NR¹⁴-NR¹²R¹³ und -NR¹⁵-OR¹⁶ steht,
in denen
R¹² und R¹³ unabhängig voneinander für Wasserstoff, geradkettiges oder verzweigtes C₁₋₆-Alkyl, insbesondere Methyl, Ethyl, n-Propyl, Isopropyl, n-Butyl, Isobutyl, sec-Butyl, tert-Butyl, 1-Methylbutyl, 2,2-Dimethylpropyl, C₁₋₆-Alkylcarbonyl, insbesondere Methylcarbonyl, sec-Butylcarbonyl, C₁₋₆-Alkylsulfonyl, insbesondere Methylsulfonyl, Ethylsulfonyl, C₂₋₆-Alkenyl, insbesondere Vinyl, 2-Propenyl, 1,2-Dimethyl-2-propenyl, 2,3-Dimethyl-2-butenyl, 1-Ethyl-2-butenyl, C₂₋₆-Alkinyl, insbesondere 2-Propinyl, 2-Butinyl, C₃₋₈-Cycloalkyl, insbesondere Cyclopropyl, Cyclohexyl, C₃₋₈-Cycloalkyl-C₁₋₂-alkyl, insbesondere Cyclopropylmethyl, Cyclobutylmethyl, Hydoxy-C₁₋₄-alkyl, insbesondere Hydroxymethyl, Hydroxyethyl, Hydroxyethylsulfonylethyl, C₁₋₄-Alkylamino, insbesondere Methylamino, Ethylamino, C₁₋₄-Monoalkylamino-C₁₋₄-alkyl, insbesondere Methylaminomethyl, Methylaminoethyl, C₁₋₄-Dialkylamino-C₁₋₄-alkyl, insbesondere Dimethylaminomethyl, Dimethylaminoethyl, Dimethylaminopropyl, C₃₋₇-Cycloalkylamino-C₁₋₄-alkyl, insbesondere N-Pyrrolidinoethyl, N-Morpholinoethyl, N-Piperidinoethyl, N-Thiomorpholino-ethyl, N¹-(N⁴-Methylpiperazino)-ethyl, C₃₋₆-Cycloalkylaminocarbonyl-C₁₋₂-alkyl, insbesondere N-Morpholinocarbonylmethyl, Aryl, insbesondere Phenyl, Aryl-C₁₋₂-alkyl, insbesondere Benzyl, Hetaryl-C₁₋₆-alkyl, insbesondere Pyridylmethyl, Thiazolylmethyl, die gegebenenfalls durch Reste aus der Reihe Halogen, insbesondere Fluor, Chlor. Brom oder Iod, Hydroxy, C₁₋₄-Alkyl, insbesondere Methyl oder tert-Butyl, C₁₋₄-Alkoxy, insbesondere Methoxy, Ethoxy oder tert-Butyloxy, Nitro, Amino, C₁₋₄-Alkylamino, insbesondere Methylamino, C₁₋₄-Dialkylamino, insbesondere Dimethylamino. substituiert sind, stehen, oder
R¹² und R¹³ gemeinsam mit dem angrenzenden N-Atom für ein carbocyclisches 5-, 6-, 7- oder 8-gliedriges Ringsystem, das gegebenenfalls auch durch Sauerstoff, Schwefel, Sulfonyl, Carbonyl, -N=, -NR¹¹- oder durch quaternisierten Stickstoff unterbrochen sein kann und gegebenenfalls durch C₁₋₄-Alkyl, insbesondere Methyl, Hydoxy-C₁₋₄-alkyl, insbesondere Hydroxymethyl, Amino-C₁₋₄-alkyl, insbesondere Aminomethyl, Aminoethyl, C₁₋₄-Monoalkylamino-C₁₋₄-alkyl, insbesondere Methylaminomethyl, Methylaminoethyl, C₁₋₄-Dialkylamino-C₁₋₄-alkyl, insbesondere Dimethylaminoethyl, C₃₋₆-Cycloalkylamino, insbesondere Pyrrolidino, Piperidino oder Morpholino, Amino, Hydroxy, C₁₋₄-Alkoxy, insbesondere Methoxy, C₁₋₄-Alkylcarbonyl, insbesondere Methylcarbonyl, C₁₋₄-Alkoxycarbonyl, insbesondere Methoxycarbonyl, substituiert ist, stehen, oder
einen Rest aus den Gruppen G⁴, G⁵, G⁶,G⁷ und G⁸ 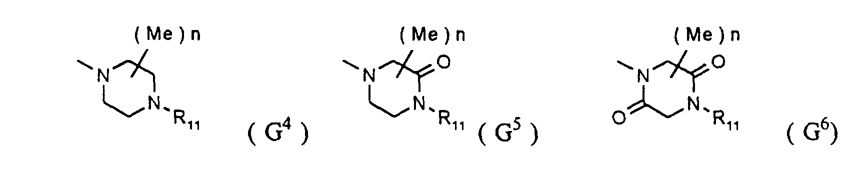 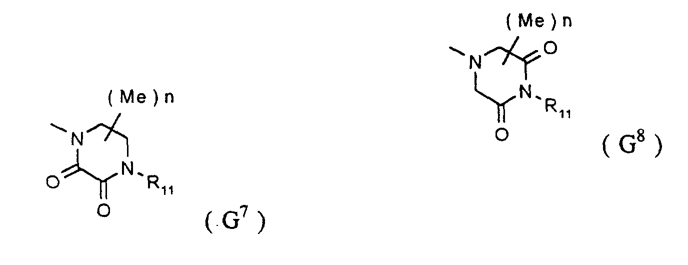 worin
- n: die Zahlen 0, 1, 2, oder 3 bedeuten kann,
- R¹⁴: für Wasserstoff, geradkettiges oder verzweigtes C₁₋₆-Alkyl, insbesondere Methyl, Ethyl, sec-Butyl, C₃₋₆-Cycloalkyl, insbesondere Cyclopropyl steht,
- R¹⁵ und R¹⁶: unabhängig voneinander Wasserstoff, geradkettiges oder verzweigtes C₁₋₆-Alkyl, insbesondere Methyl, Ethyl, sec-Butyl, C₁₋₆-Alkylcarbonyl, insbesondere Methylcarbonyl, sec-Butylcarbonyl, C₂₋₆-Alkenyl, insbesondere 2-Propenyl, 1-Methyl-2-propenyl, 1,1-Dimethyl-2-propenyl, C₂₋₆-Alkinyl, insbesondere 2-Propinyl, C₃₋₆-Cycloalkyl, insbesondere Cyclopropyl stehen,
- R¹⁵ und R¹⁶: gemeinsam mit der angrenzenden N-O-Gruppe für einen carbocyclischen 5-, 6- oder 7-gliedrigen Ring stehen,
mit der Maßgabe für den Fall, daß in Formel (Ia) R¹, R⁵ und =G=X gemeinsam für folgende Reste stehen:
- R¹: steht für Wasserstoff und Methyl,
- R⁵: steht fiir Wasserstoff, 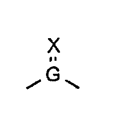 steht für Carboxy,
die Reste Q und B folgende Bedingung erfüllen müssen:
Q steht für andere Reste als Methyl,
B steht für andere Reste als -NH₂ sowie mit der Maßgabe für den Fall, daß in Formel (Ia) G² und =G=X gemeinsam für folgende Reste stehen: 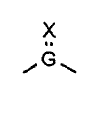 steht für Carboxy,
G² steht für tert-Butyloxy, Benzyloxy und 4-Nitro-benzyloxy, der Rest B für andere Reste als tert-Butyloxy, Benzyloxy und 4-Nitrobenzyloxy steht, sowie mit der Maßgabe, daß wenn in Formel (Ia) R⁴ und R⁵ für Wasserstoff stehen, B nicht für NH₂ stehen darf,
sowie mit der Maßgabe, daß wenn in Formel (Ia) R¹ bis R⁵ für Wasserstoff stehen und 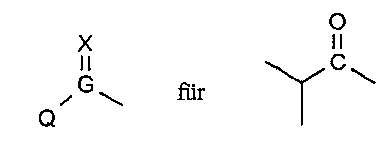 steht,
B nicht für Methylamino steht, sowie mit der Maßgabe, daß wenn in Formel (Ia) 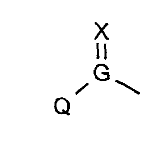 für Benzyloxycarbonyl steht, oder 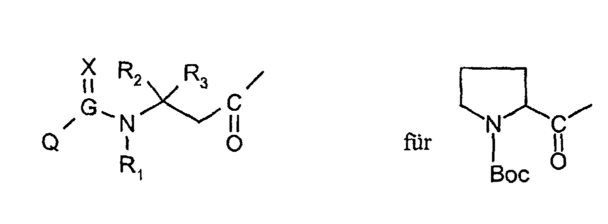 steht,
B nicht für Benzylamino steht, sowie mit der Maßgabe, daß für den Fall, daß in Formel (Ia) 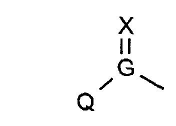 für tert.-Butyloxycarbonyl steht, und R¹ bis R³ für H, und entweder R⁴ oder R⁵ für Methyl und das andere für H steht,
B nicht für Ethoxy steht, sowie mit der Maßgabe, daß die Verbindung der Formel 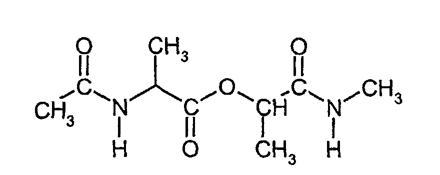 ausgeschlossen ist,
ferner mit der Maßgabe, daß die Verbindungen
Methyl-O-(N-carbobenzoxy-L-Leucyl)-hydroxyethanoat,
Methyl-O-(N-carbobenzoxy-L-Leucyl)-(S)-2-hydroxy-3-phenylpropanoat,
Methyl-O-(N-carbobenzoxy-L-Leucyl)-(S)-2-hydroxypropanoat,
Methyl-O-(N-carbobenzoxy-L-Leucyl)-(S)-2-hydroxy-4-methyl-pentanoat,
Ethyl-N-(benzyloxycarbonyl)-amino-2-(O-glycyl)-glycolat,
Ethyl-N-(benzyloxycarbonyl)-amino-2-(O-glycyl)-lactat,
Ethyl-N-(benzyloxycarbonyl)-amino-2-(O-alanyl)-glycolat,
Ethyl-N-(benzyloxycarbonyl)-amino-2-(O-alanyl)lactat
ausgeschlossen sind,
sowie deren optische Isomere und Racemate.

Ganz besonders bevorzugt sind Verbindungen der Formel (Ia) und deren Salze 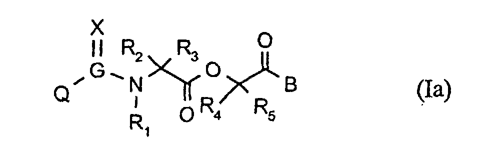 in welcher
- R¹: für geradkettiges oder verzweigtes C₁₋₄-Alkyl, insbesondere Methyl steht,
- R²: für geradkettiges oder verzweigtes C₁₋₄-Alkyl, insbesondere Methyl oder Ethyl, steht,
- R³ und R⁴: für Wasserstoff stehen,
- R⁵: Wasserstoff, für geradkettiges oder verzweigtes C₁₋₄-Alkyl, insbesondere Methyl oder Ethyl steht, 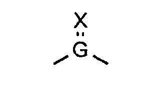 für Carboxy oder Sulfonyl steht,
- Q: für einen Rest aus der Gruppe G² und G³

R⁸-Y- (G²)

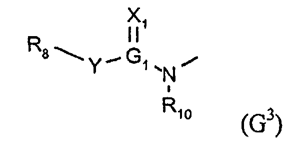

steht,
worin 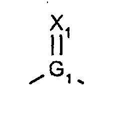 Carboxy oder Sulfonyl bedeuten kann,
- Y: für Sauerstoff oder -NR⁹ steht,
- R⁸: für den Fall, daß Y für Stickstoff steht, eine über ein Stickstoffatom verknüpfte cyclische Aminogruppe, insbesondere Pyrrolidino, 2-Pyrrolin-1-yl, 1-Pyrrolyl, Piperidino, 1-Piperazinyl, Morpholino, Thiomorpholino, Dioxothiomorpholino, bedeutet,
- R⁸ und R⁹: unabhängig voneinander für geradkettiges oder verzweigtes C₁₋₄-Alkyl, insbesondere Methyl, Ethyl, Propyl, Isopropyl, sec-Butyl, C₂₋₄-Alkenyl, insbesondere Vinyl, 2-Propenyl, 1-Methyl-2-propenyl und C₂₋₄-Alkinyl, insbesondere Ethinyl, 2-Propinyl, C₃₋₆-Cycloalkyl, insbesondere Cyclopropyl, Hetaryl-C₁₋₂-alkyl, insbesondere 2-Chlor-pyrid-5-yl-methyl und 2-Chlor-thiazol-5-yl-methyl, stehen, oder
- R⁸ und R⁹: gemeinsam mit dem angrenzenden N-Atom für ein carbocyclisches 5-, 6- oder 7-gliedriges Ringsystem, das gegebenenfalls auch durch Sauerstoff, Schwefel, Sulfonyl, Carbonyl, -N=, -NR¹¹- oder durch quaternisierten Stickstoff unterbrochen sein kann und gebenenfalls durch C₁₋₄-Alkyl, insbesondere Methyl, C₁₋₄-Dialkylamino-C₁₋₄-alkyl, insbesondere Dimethylaminomethyl, Hydroxy, C₁₋₄-Alkoxycarbonyl, besonders bevorzugt jedoch für Methoxycarbonyl, substituiert ist, insbesondere Pyrrolidino, 3-Oxopyrrolidino, Morpholino, 2,6-Dimethylmorpholino, Thiomorpholino, Dioxothiomorpholino stehen,
- R¹⁰: für Wasserstoff oder C₁₋₄-Alkyl, insbesondere Methyl steht,
- R¹¹: geradkettiges oder verzweigtes C₁₋₆-Alkyl, insbesondere Methyl, C₂₋₆-Alkenyl, insbesondere Vinyl, C₃₋₆-Cycloalkyl, insbesondere Cyclopropyl, C₁₋₄-Alkylcarbonyl, insbesondere Methylcarbonyl, C₃₋₆-Cycloalkylcarbonyl, insbesondere Cyclopropylcarbonyl, Cyclohexylcarbonyl, C₁₋₄-Alkoxycarbonyl, insbesondere Methoxycarbonyl, Hydroxy-C₁₋₄-alkyl, insbesondere Hydroxyethyl, Hydroxysulfonylethyl, C₁₋₄-Alkylamino, insbesondere Ethylamino, C₁₋₄-Dialkylamino-C₁₋₄-alkyl, insbesondere Dimethylaminoethyl, C₃₋₇-Cycloalkylamino-C₁₋₄-alkyl, insbesondere N-Morpholinoethyl, N-Piperidinoethyl, C₃₋₆-Cycloalkylaminocarbonyl-C₁₋₂-alkyl, insbesondere N-Morpholinocarbonylmethyl, steht,
- B: für geradkettiges oder verzweigtes C₁₋₆-Alkoxy, insbesondere Methoxy, Ethoxy, n-Propoxy, sec-Butoxy und tert-Butoxy, C₂₋₆-Alkenyloxy, 2-Propenyloxy, 1,1-Dimethyl-2-propenyloxy, C₂₋₆-Alkinyloxy, insbesondere 2-Propinyloxy, 1-Methyl-2-propinyloxy, C₃₋₈-Cycloalkyloxy, insbesondere Cyclopropyloxy, Aryl-C₁₋₂-alkyloxy, insbesondere Benzyloxy, Hetaryloxy, insbesondere Pyridyloxy, Hetaryl-C₁₋₂-alkyloxy, insbesondere Pyridylmethyloxy und Thiazolylmehyloxy, die gegebenenfalls durch Reste aus der Reihe Halogen, insbesondere Fluor, Chlor, Brom oder Iod, Amino, Hydroxy, C₁₋₄-Alkoxy, insbesondere Methoxy, C₁₋₄-Alkylamino, insbesondere Methylamino, C₁₋₄-Dialkylamino, insbesondere Dimethylamino, C₃₋₆-Cycloalkylamino, insbesondere Piperidino, C₃₋₆-Cycloalkylthioamino, insbesondere Dioxothiomorpholino, C₃₋₆-Cycloalkyldiamino, insbesondere N-Methyl-piperazino, substituiert sein können, steht, oder für die Aminoreste -NR¹²R¹³, -NR¹⁴-NR¹²R¹³ und -NR¹⁵-OR¹⁶ steht,
in denen
R¹² und R¹³ unabhängig voneinander für Wasserstoff, geradkettiges oder verzweigtes C₁₋₆-Alkyl, insbesondere Methyl, Ethyl, C₁₋₆-Alkylcarbonyl, insbesondere Methylcarbonyl, C₁₋₆-Alkylsulfonyl, insbesondere Methylsulfonyl, C₂₋₆-Alkenyl, insbesondere 2-Propenyl, 1-Methyl-2-propenyl, C₂₋₆-Alkinyl, insbesondere 2-Propinyl, C₃₋₈-Cycloalkyl, insbesondere Cyclopropyl, Cyclohexyl, C₃₋₈-Cycloalkyl-C₁₋₂-alkyl, insbesondere Cyclopropylmethyl, Hydoxy-C₁₋₄-alkyl, insbesondere Hydroxymethyl, Hydroxyethylsulfonylethyl, C₁₋₄-Dialkylamino-C₁₋₄-alkyl, insbesondere Dimethylaminoethyl, C₃₋₇-Cycloalkylamino-C₁₋₄-alkyl, insbesondere N-Morpholinoethyl, N¹-(N⁴-Methyl-piperazino)-ethyl, C₃₋₆-Cycloalkylaminocarbonyl-C₁₋₂-alkyl, insbesondere N-Morpholinocarbonylmethyl, stehen, oder
R¹² und R¹³ gemeinsam mit dem angrenzenden N-Atom für ein carbocyclisches 5-, 6-, 7- oder 8-gliedriges Ringsystem, das gegebenenfalls auch durch Sauerstoff, Schwefel, Carbonyl, -N=, -NR¹¹- oder durch quaternisierten Stickstoff unterbrochen sein kann und gegebenenfalls durch C₁₋₄-Alkyl, insbesondere Methyl, C₁₋₄-Dialkylamino-C₁₋₄-alkyl, insbesondere Dimethylaminoethyl, C₃₋₆-Cycloalkylamino, insbesondere Piperidino, Hydroxy, C₁₋₄-Alkoxy, insbesondere Methoxy, C₁₋₄-Alkylcarbonyl insbesondere Methylcarbonyl, C₁₋₄-Alkoxycarbonyl, insbesondere Methoxycarbonyl, substituiert ist, stehen, oder
einen Rest aus den Gruppen G⁴, G⁵, G⁶,G⁷ und G⁸ 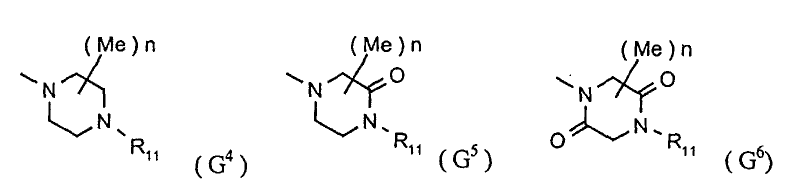 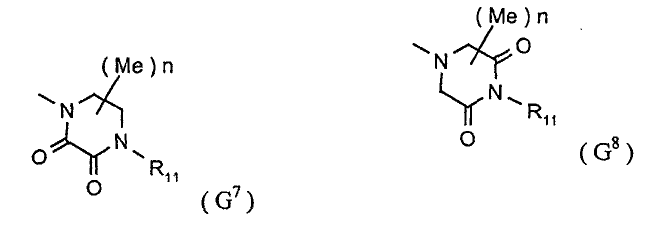 worin
- n: die Zahlen 0, 1 oder 2 bedeuten kann,
- R¹⁴: für Wasserstoff, geradkettiges oder verzweigtes C₁₋₆-Alkyl, insbesondere Methyl steht,
- R¹⁵ und R¹⁶: unabhängig voneinander Wasserstoff, geradkettiges oder verzweigtes C₁₋₆-Alkyl, insbesondere Methyl, C₁₋₆-Alkylcarbonyl, insbesondere Methylcarbonyl, sec-Butylcarbonyl, C₂₋₆-Alkenyl, insbesondere 2-Propenyl, C₂₋₆-Alkinyl, insbesondere 2-Propinyl, C₃₋₆-Cycloalkyl, insbesondere Cyclopropyl steht,
mit der Maßgabe für den Fall, daß in Formel (Ia) R¹, R⁵ und =G=X gemeinsam für folgende Reste stehen:
- R¹: steht für Wasserstoff und Methyl,
- R⁵: steht für Wasserstoff, 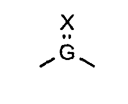 steht für Carboxy,
die Reste Q und B folgende Bedingung erfüllen müssen:
Q steht für andere Reste als Methyl,
B steht für andere Reste als -NH₂ sowie mit der Maßgabe für den Fall, daß in Formel (Ia) G² und =G=X gemeinsam für folgende Reste stehen: 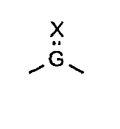 steht für Carboxy,
G² steht für tert-Butyloxy, Benzyloxy und 4-Nitro-benzyloxy, der Rest B für andere Reste als tert-Butyloxy, Benzyloxy und 4-Nitrobenzyloxy steht, sowie mit der Maßgabe, daß wenn in Formel (Ia) R⁴ und R⁵ für Wasserstoff stehen, B nicht für NH₂ stehen darf,
sowie mit der Maßgabe, daß wenn in Formel (Ia) R¹ bis R⁵ für Wasserstoff stehen und 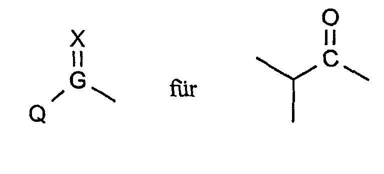 steht,
B nicht für Methylamino steht, sowie mit der Maßgabe, daß wenn in Formel (Ia) 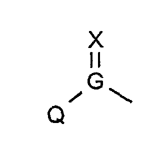 für Benzyloxycarbonyl steht, oder 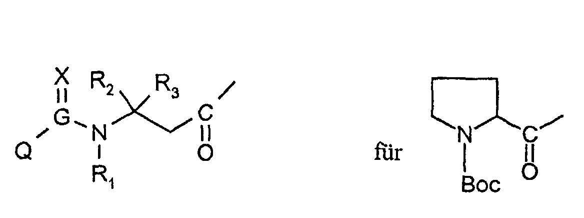 steht,
B nicht für Benzylamino steht, sowie mit der Maßgabe, daß für den Fall, daß in Formel (Ia) 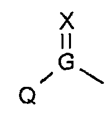 für tert.-Butyloxycarbonyl steht, und R¹ bis R³ für H, und entweder R⁴ oder R⁵ für Methyl und das andere für H steht,
B nicht für Ethoxy steht, sowie mit der Maßgabe, daß die Verbindung der Formel 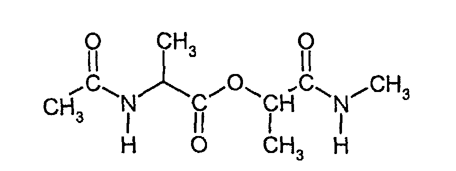 ausgeschlossen ist,
ferner mit der Maßgabe, daß die Verbindungen
Methyl-O-(N-carbobenzoxy-L-Leucyl)-hydroxyethanoat,
Methyl-O-(N-carbobenzoxy-L-Leucyl)-(S)-2-hydroxy-3-phenylpropanoat,
Methyl-O-(N-carbobenzoxy-L-Leucyl)-(S)-2-hydroxypropanoat,
Methyl-O-(N-carbobenzoxy-L-Leucyl)-(S)-2-hydroxy-4-methyl-pentanoat,
Ethyl-N-(benzyloxycarbonyl)-amino-2-(O-glycyl)-glycolat,
Ethyl-N-(benzyloxycarbonyl)-amino-2-(O-glycyl)-lactat,
Ethyl-N-(benzyloxycarbonyl)-amino-2-(O-alanyl)-glycolat,
Ethyl-N-(benzyloxycarbonyl)-amino-2-(O-alanyl)lactat
ausgeschlossen sind,
sowie deren optische Isomere und Racemate.

Die erfindungsgemäß zu verwendenden Verbindung der allgemeinen Formel (I) und deren Salze enthalten außerdem ein oder mehrere Chiralitätszentren und kön-nen somit in reinen Stereoisomeren oder in Form verschiedener Enantiomeren- und Diastereoisomerengemische vorliegen, die erforderlichenfalls in an sich bekannter Weise getrennt werden können. Die Erfindung betrifft daher sowohl die reinen Enantiomeren und Diastereomeren, als auch deren Gemische zur Bekämpfung von Endoparasiten, besonders auf dem Gebiet der Medizin und Veterinärmedizin.

Vorzugsweise werden jedoch die optisch aktiven, stereoisomeren Formen der Verbindungen der allgemeinen Formel (I) und deren Salze erfindungsgemäß verwendet.

Als geeignete Salze der Verbindungen der allgemeinen Formel (I) können übliche nicht toxische Salze, d. h. Salze mit verschiedenen Basen und Salze mit zugesetz-ten Säuren, genannt werden. Vorzugsweise sind Salze mit anorganischen, Basen wie Alkalimetallsalze, beispielsweise Natrium-, Kalium oder Cäsiumsalze, Erdalkalimetallsalze, beispielsweise Calzium- oder Magnesiumsalze, Ammoniumsalze, Salze mit organischen Basen sowie mit organischen Aminen, beispielsweise Triethylammonium-, Pyridinium-, Picolinium-, Ethanolammonium-, Triethanolammonium-, Dicyclohexylammonium- oder N,N'-Dibenzylethylendiammoniumsalze, Salze mit anorganischen Säuren, beispielsweise Hydrochloride, Hydrobromide, Dihydrosulfate oder Trihydrophosphate, Salze mit organischen Carbonsäuren oder organischen Sulfosäuren, beispielsweise Formiate, Acetate, Trifluoracetate, Maleate, Tartrate, Methansulfonate, Benzolsulfonate oder para-Toluolsulfonate, Salze mit basischen Aminosäuren oder sauren Aminosäuren, beispielsweise Arginate, Aspartate oder Glutamate, zu nennen.

Insbesondere seien die in den folgenden Tabellen 1 bis 84 genannten Gruppen von Verbindungen genannt.

Ganz besonders bevorzugt sind die Verbindungen der Formel (Ia-1) und deren Salze, bestehend aus der N-Methyl-aminosäure N-Methyl-alanin (R¹, R² = Methyl und R³ = Wasserstoff) und der 2-Hydroxycarbonsäure 2-Hydroxyessigsäure (R⁴, R⁵ = Wasserstoff) die in den nachfolgenden Tabellen 1 bis 13 angegeben sind.

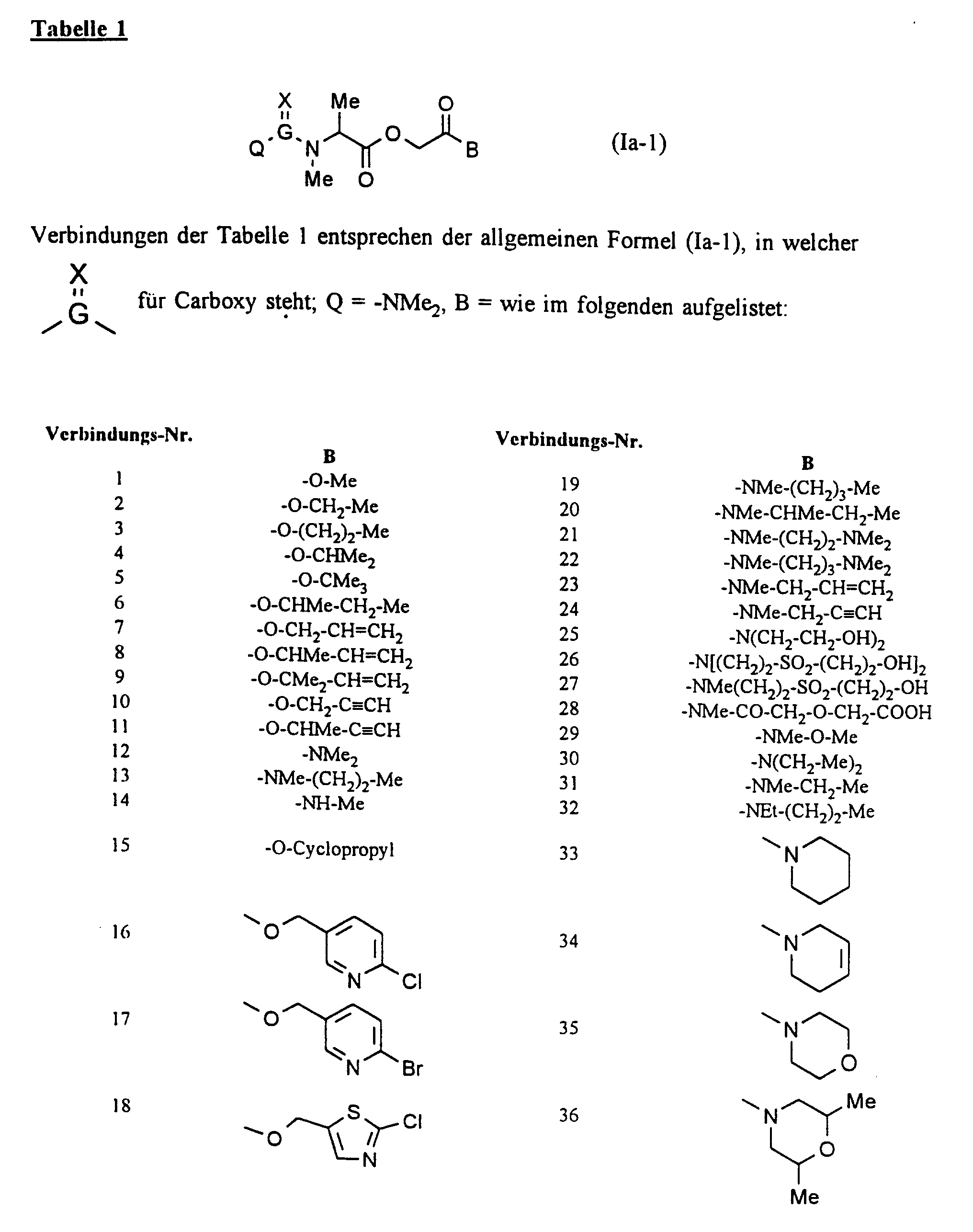

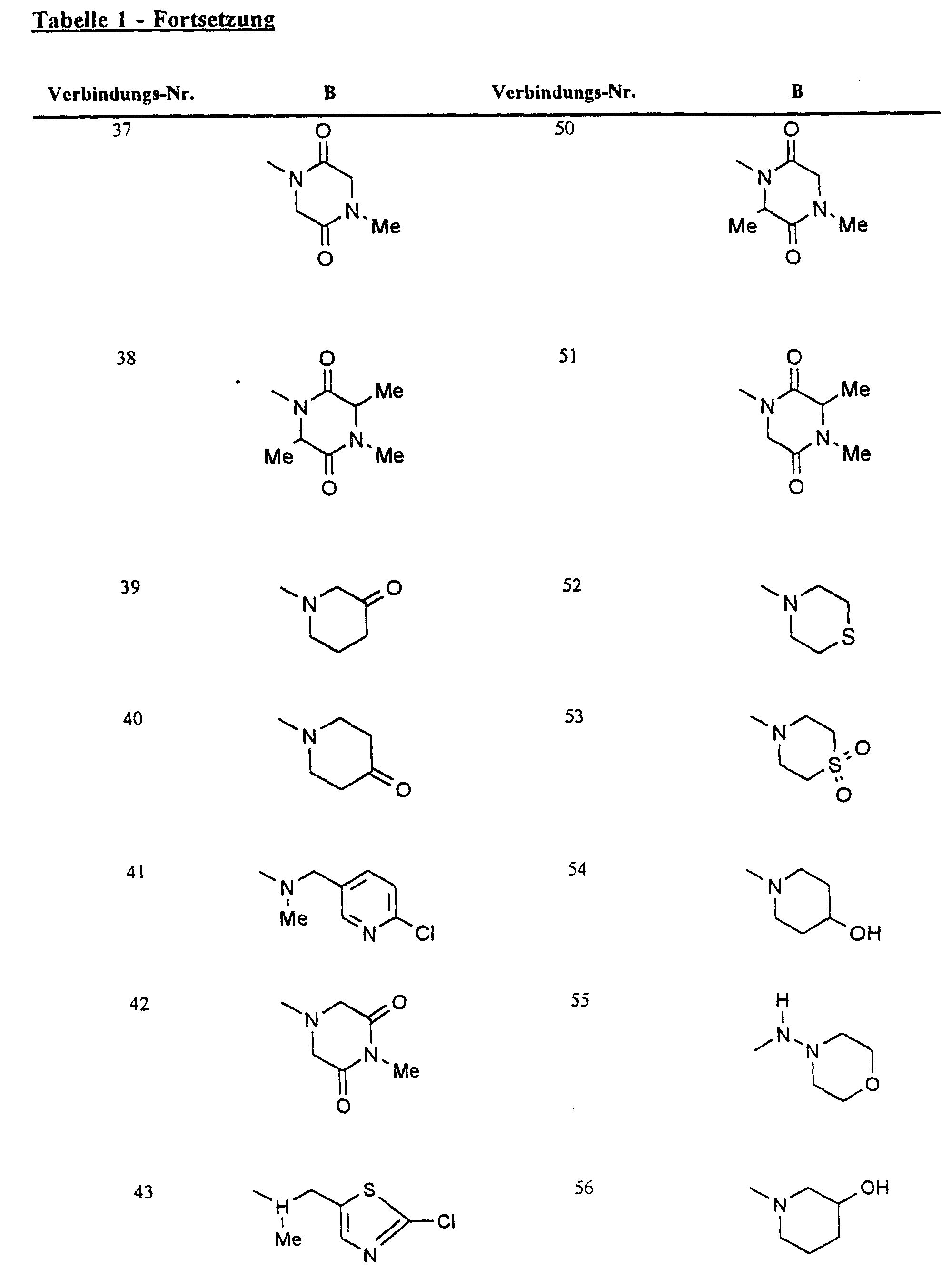

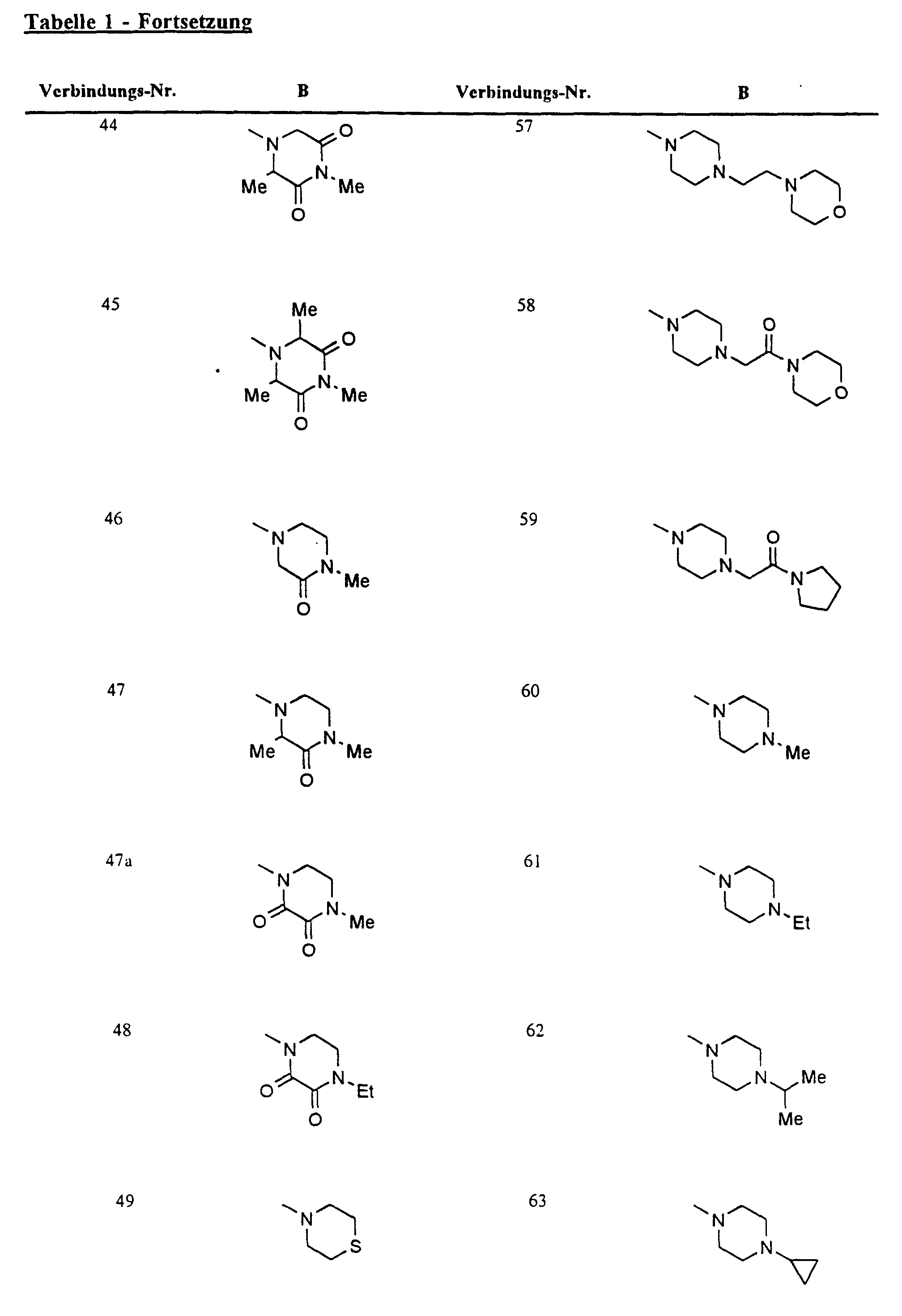

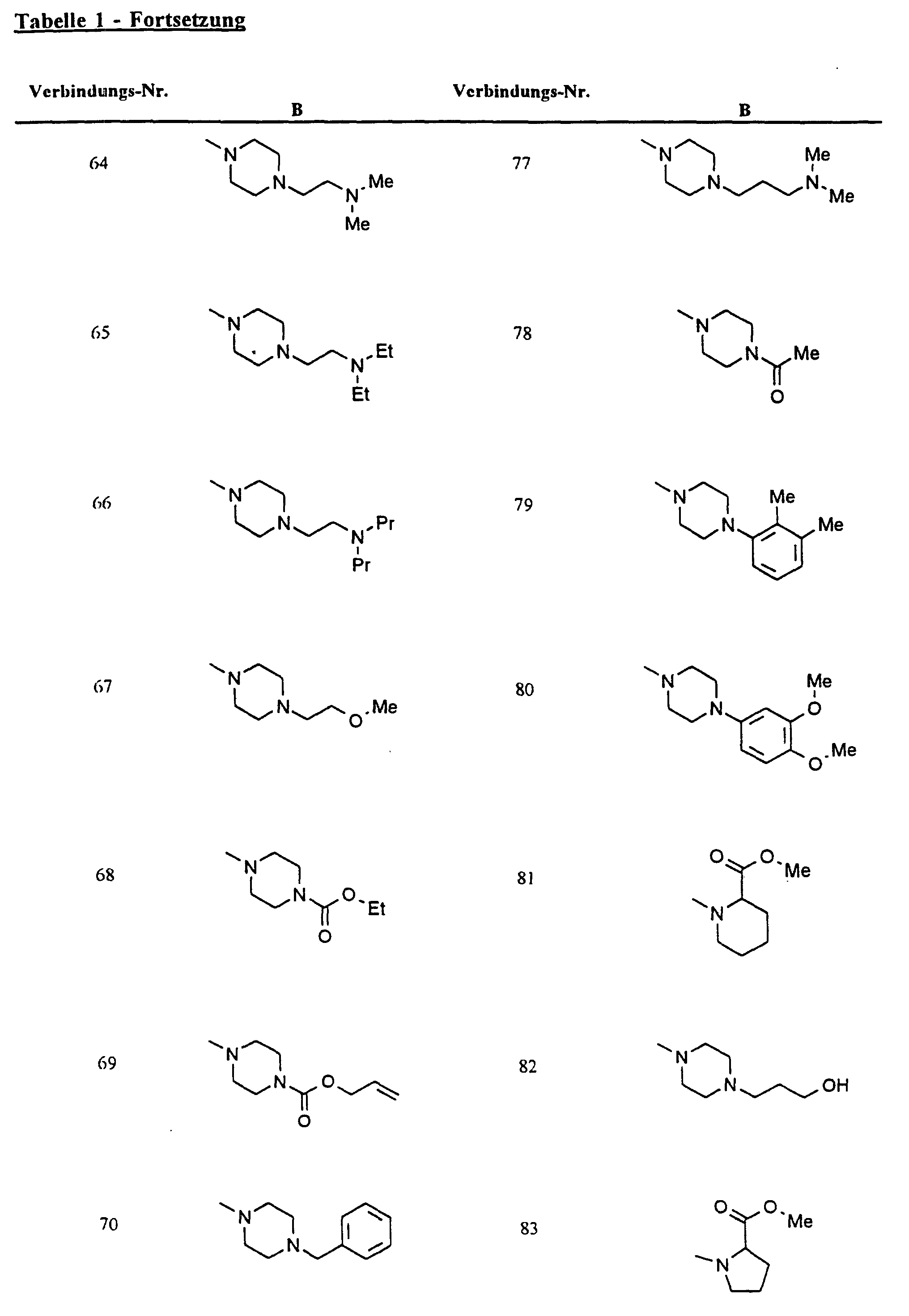

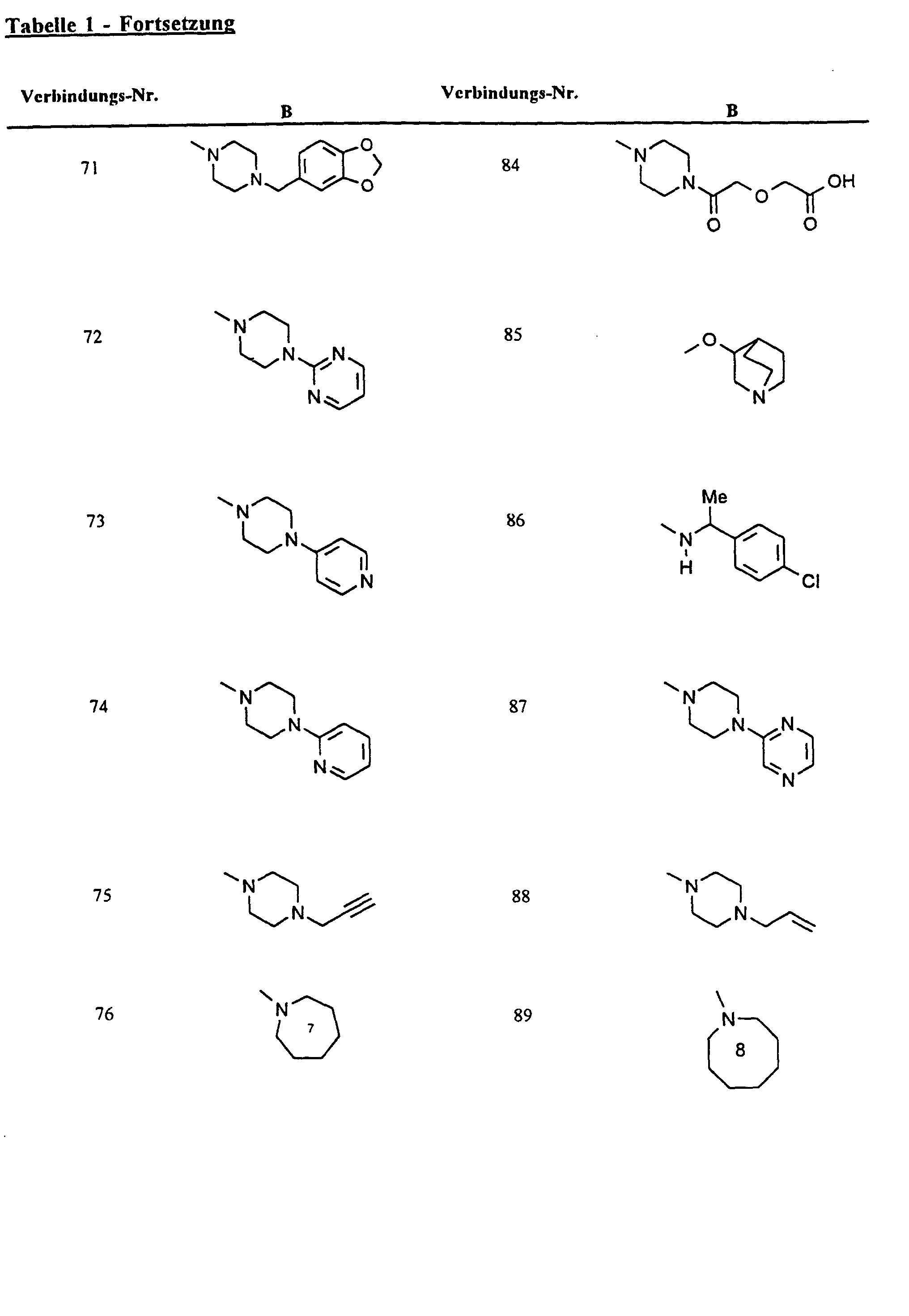

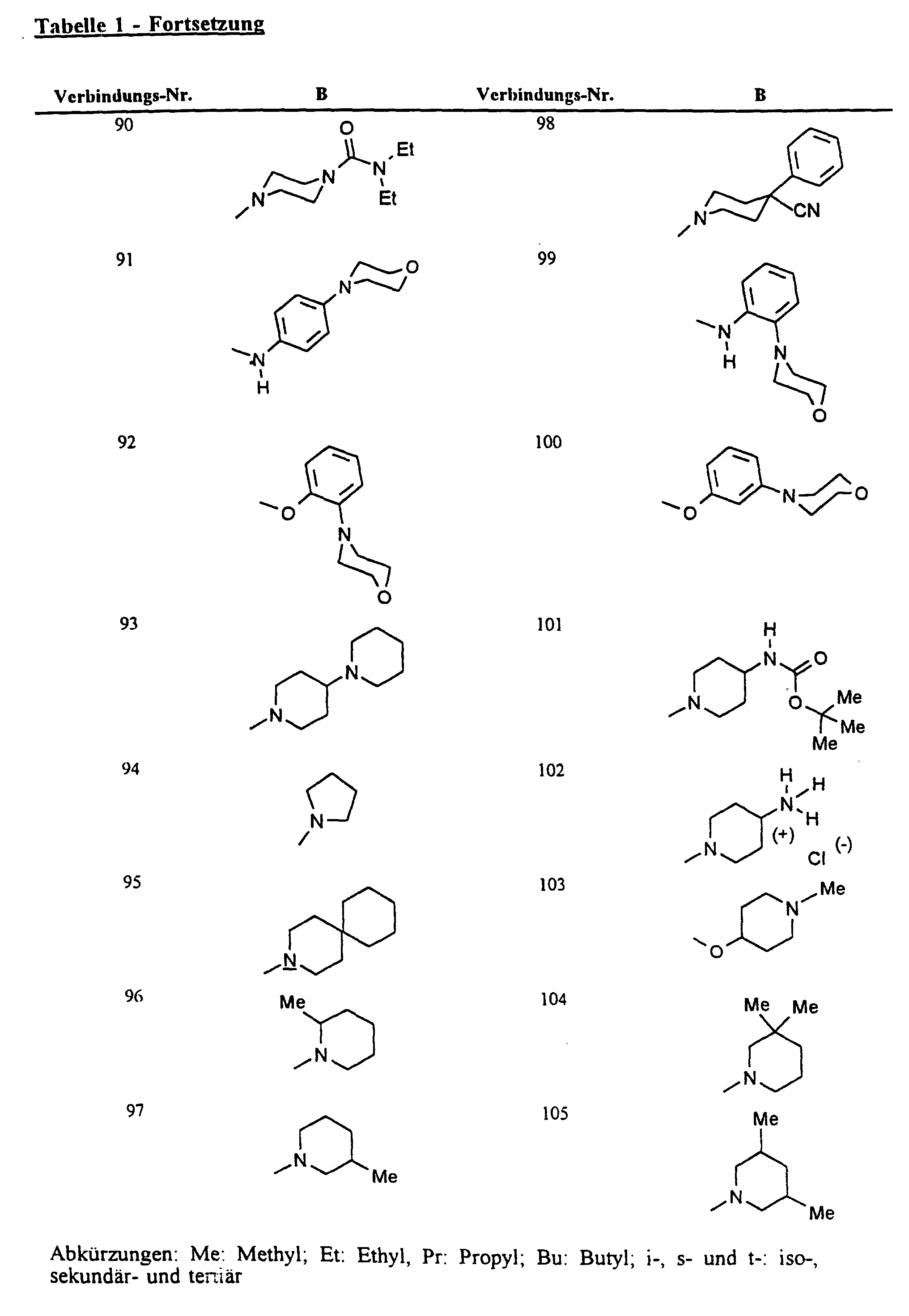

### Tabelle 2

Tabelle 2 enthält die Verbindungen der allgemeinen Formel (Ia-1), in welcher 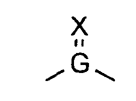 für Carboxy steht; Q für -NEt₂ steht und B die in Tabelle 1 aufgelisteten Bedeutungen besitzt.

### Tabelle 3

Tabelle 3 enthält die Verbindungen der allgemeinen Formel (Ia-1), in welcher 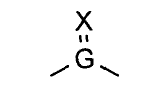 für Carboxy steht; Q für -O-Me steht und B die in Tabelle 1 aufgelisteten Bedeutungen besitzt.

### Tabelle 4

Tabelle 4 enthält die Verbindungen der allgemeinen Formel (Ia-1), in welcher 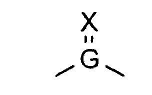 für Carboxy steht; Q für -O-CHMe-CH₂-Me steht und B die in Tabelle 1 aufgelisteten Bedeutungen besitzt.

### Tabelle 5

Tabelle 5 enthält die Verbindungen der allgemeinen Formel (Ia-1), in welcher 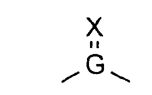 für Carboxy steht; Q für -O-CH₂-CH=CH₂ steht und B die in Tabelle 1 aufgelisteten Bedeutungen besitzt.

### Tabelle 6

Tabelle 6 enthält die Verbindungen der allgemeinen Formel (Ia-1), in welcher 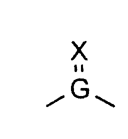 für Carboxy steht; Q für -O-CHMe-CH=CH₂ steht und B die in Tabelle 1 aufgelisteten Bedeutungen besitzt.

### Tabelle 7

Tabelle 7 enthält die Verbindungen der allgemeinen Formel (Ia-1), in welcher 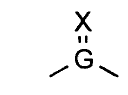 für Carboxy steht; Q = -O-CH₂-C≡CH steht und B die in Tabelle 1 aufgelisteten Bedeutungen besitzt.

### Tabelle 8

Tabelle 8 enthält die Verbindungen der allgemeinen Formel (Ia-1), in welcher 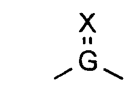 für Carboxy steht; Q für -O-CMe=CH₂ steht und B die in Tabelle 1 aufgelisteten Bedeutungen besitzt.

### Tabelle 9

Tabelle 9 enthält die Verbindungen der allgemeinen Formel (Ia-1), in welcher 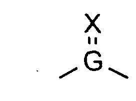 für Carboxy steht; Q für -NMe-CO-NMe₂ steht und B die in Tabelle 1 aufgelisteten Bedeutungen besitzt.

### Tabelle 10

Tabelle 10 enthält die Verbindungen der allgemeinen Formel (Ia-1), in welcher 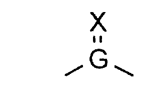 für Carboxy steht; Q für -NMe-CO-NEt₂ steht und B die in Tabelle 1 aufgelisteten Bedeutungen besitzt.

### Tabelle 11

Tabelle 11 enthält die Verbindungen der allgemeinen Formel (Ia-1), in welcher 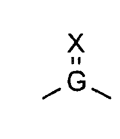 für Carboxy steht; Q für 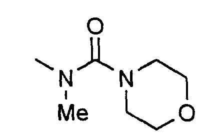 steht und B die in Tabelle 1 aufgelisteten Bedeutungen besitzt.

### Tabelle 12

Tabelle 12 enthält die Verbindungen der allgemeinen Formel (Ia-1), in welcher 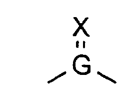 für Sulfonyl steht; Q für -NMe₂ steht und B die in Tabelle 1 aufgelisteten Bedeutungen besitzt.

### Tabelle 13

Tabelle 13 enthält die Verbindungen der allgemeinen Formel (Ia-1), in welcher 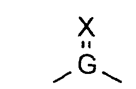 für Sulfonyl steht; Q für -NEt₂ steht und B die in Tabelle 1 aufgelisteten Bedeutungen besitzt.

### Tabelle 14

Tabelle 14 enthält die Verbindungen der allgemeinen Formel (Ia-1), in welcher 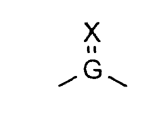 für Sulfonyl steht; Q für 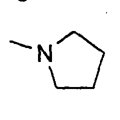 steht und B die in Tabelle 1 aufgelisteten Bedeutungen besitzt.

Weiterhin bevorzugt sind die Verbindungen der Formel (Ia-2) und deren Salze, bestehend aus der N-Methyl-aminosäure N-Methyl-alanin (R¹, R² = Methyl und R³ = Wasserstoff) und der 2-Hydroxycarbonsäure 2-Hydroxy-propionsäure (Milchsäure) (R⁴ = Wasserstoff; R⁵ = Methyl).

Ganz besonders bevorzugte Beispiele für diese neuen erfindungsgemäßen Verbindungen ( Ia-2 ) sind in den Tabellen 15-28 aufgeführt.

### Tabelle 15

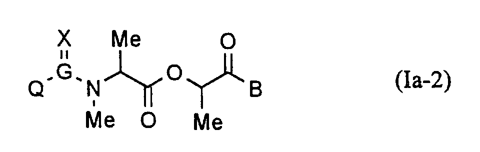

Verbindungen der Tabelle 15 entsprechen der allgemeinen Formel (Ia-2), in welcher 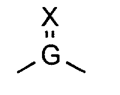 für Carboxy steht; Q für -NMe₂ steht und B die in Tabelle 1 aufgelisteten Bedeutungen besitzt.

### Tabelle 16

Tabelle 16 enthält die Verbindungen der allgemeinen Formel (Ia-2), in welcher 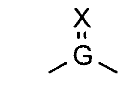 für Carboxy steht; Q für -NEt₂ steht und B die in Tabelle 1 aufgelisteten Bedeutungen besitzt.

### Tabelle 17

Tabelle 17 enthält die Verbindungen der allgemeinen Formel (Ia-2), in welcher 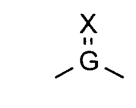 für Carboxy steht; Q für -O-Me steht und B die in Tabelle 1 aufgelisteten Bedeutungen besitzt.

### Tabelle 18

Tabelle 18 enthält die Verbindungen der allgemeinen Formel (Ia-2), in welcher 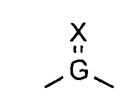 für Carboxy steht; Q für -O-CHMe-CH₂-Me steht und B die in Tabelle 1 aufgelisteten Bedeutungen besitzt.

### Tabelle 19

Tabelle 19 enthält die Verbindungen der allgemeinen Formel (Ia-2), in welcher 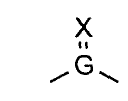 für Carboxy steht; Q für -O-CH₂-CH=CH₂ steht und B die in Tabelle 1 aufgelisteten Bedeutungen besitzt.

### Tabelle 20

Tabelle 20 enthält die Verbindungen der allgemeinen Formel (Ia-2), in welcher 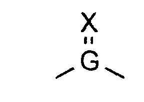 für Carboxy steht; Q für -O-CHMe-CH=CH₂ steht und B die in Tabelle 1 aufgelisteten Bedeutungen besitzt.

### Tabelle 21

Tabelle 21 enthält die Verbindungen der allgemeinen Formel (Ia-2), in welcher 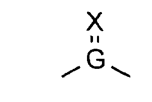 für Carboxy steht; Q für -O-CH₂-C≡CH steht und B die in Tabelle 1 aufgelisteten Bedeutungen besitzt.

### Tabelle 22

Tabelle 22 enthält die Verbindungen der allgemeinen Formel (Ia-2), in welcher 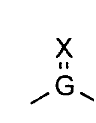 für Carboxy steht; Q für -O-CMe=CH₂ steht und B die in Tabelle 1 aufgelisteten Bedeutungen besitzt.

### Tabelle 23

Tabelle 23 enthält die Verbindungen der allgemeinen Formel (Ia-2), in welcher 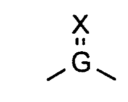 für Carboxy steht; Q für -NMe-CO-NMe₂ steht und B die in Tabelle 1 aufgelisteten Bedeutungen besitzt.

### Tabelle 24

Tabelle 24 enthält die Verbindungen der allgemeinen Formel (Ia-2), in welcher 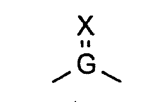 für Carboxy steht; Q für -NMe-CO-NEt₂ steht und B die in Tabelle 1 aufgelisteten Bedeutungen besitzt.

### Tabelle 25

Tabelle 25 enthält die Verbindungen der allgemeinen Formel (Ia-2), in welcher 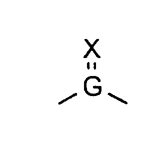 für Carboxy steht; Q für 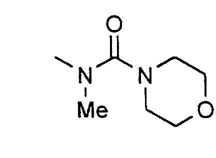 steht und B die in Tabelle 1 aufgelisteten Bedeutungen besitzt.

### Tabelle 26

Tabelle 26 enthält die Verbindungen der allgemeinen Formel (Ia-2), in welcher 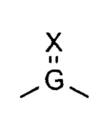 für Sulfonyl steht; Q für -NMe₂ steht und B die in Tabelle 1 aufgelisteten Bedeutungen besitzt.

### Tabelle 27

Tabelle 27 enthält die Verbindungen der allgemeinen Formel (Ia-2), in welcher 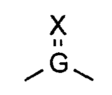 für Sulfonyl steht; Q für -NEt₂ steht und B die in Tabelle 1 aufgelisteten Bedeutungen besitzt.

### Tabelle 28

Tabelle 28 enthält die Verbindungen der allgemeinen Formel (Ia-2), in welcher 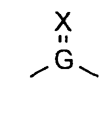 für Sulfonyl steht; Q für 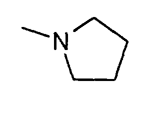 steht und B die in Tabelle 1 aufgelisteten Bedeutungen besitzt.

Weiterhin bevorzugt sind die Verbindungen der Formel (Ia-3) und deren Salze, bestehend aus der N-Methyl-aminosäure N-Methyl-alanin (R¹, R² = Methyl und R³ = Wasserstoff) und der 2-Hydroxycarbonsäure 2-Hydroxy-buttersäure (R⁴ = Wasserstoff; R⁵ = Ethyl).

Ganz besonders bevorzugte Beispiele für diese neuen erfindungsgemäßen Verbindungen ( Ia-3 ) sind in den Tabellen 29-42 aufgeführt.

### Tabelle 29

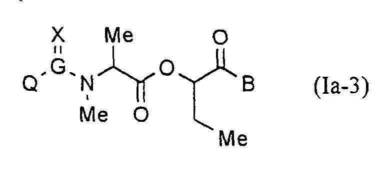

Verbindungen der Tabelle 29 entsprechen der allgemeinen Formel (Ia-3), in welcher 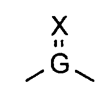 für Carboxy steht; Q für -NMe₂ steht und B die in Tabelle 1 aufgelisteten Bedeutungen besitzt.

### Tabelle 30

Tabelle 30 enthält die Verbindungen der allgemeinen Formel (Ia-3), in welcher 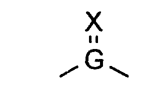 für Carboxy steht; Q für -NEt₂ steht und B die in Tabelle 1 aufgelisteten Bedeutungen besitzt.

### Tabelle 31

Tabelle 31 enthält die Verbindungen der allgemeinen Formel (Ia-3), in welcher 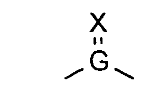 für Carboxy steht; Q für -O-Me steht und B die in Tabelle 1 aufgelisteten Bedeutungen besitzt.

### Tabelle 32

Tabelle 32 enthält die Verbindungen der allgemeinen Formel (Ia-3), in welcher 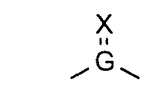 für Carboxy steht; Q für -O-CHMe-CH₂-Me steht und B die in Tabelle 1 aufgelisteten Bedeutungen besitzt.

### Tabelle 33

Tabelle 33 enthält die Verbindungen der allgemeinen Formel (Ia-3), in welcher 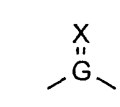 für Carboxy steht; Q für -O-CH₂-CH=CH₂ steht und B die in Tabelle 1 aufgelisteten Bedeutungen besitzt.

### Tabelle 34

Tabelle 34 enthält die Verbindungen der allgemeinen Formel (Ia-3), in welcher 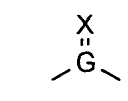 für Carboxy steht; Q für -O-CHMe-CH=CH₂ steht und B die in Tabelle 1 aufgelisteten Bedeutungen besitzt.

### Tabelle 35

Tabelle 35 enthält die Verbindungen der allgemeinen Formel (Ia-3), in welcher 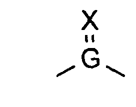 für Carboxy steht; Q für -O-CH₂-C≡CH steht und B die in Tabelle 1 aufgelisteten Bedeutungen besitzt.

### Tabelle 36

Tabelle 36 enthält die Verbindungen der allgemeinen Formel (Ia-3), in welcher 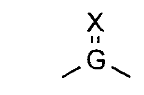 für Carboxy steht; Q für -O-CMe=CH₂ steht und B die in Tabelle 1 aufgelisteten Bedeutungen besitzt.

### Tabelle 37

Tabelle 37 enthält die Verbindungen der allgemeinen Formel (Ia-3), in welcher 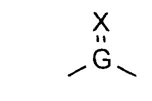 für Carboxy steht; Q für -NMe-CO-NMe₂ steht und B die in Tabelle 1 aufgelisteten Bedeutungen besitzt.

### Tabelle 38

Tabelle 38 enthält die Verbindungen der allgemeinen Formel (Ia-3), in welcher 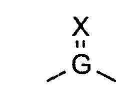 für Carboxy steht; Q für -NMe-CO-NEt₂ steht und B die in Tabelle 1 aufgelisteten Bedeutungen besitzt.

### Tabelle 39

Tabelle 39 enthält die Verbindungen der allgemeinen Formel (Ia-3), in welcher 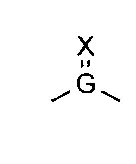 für Carboxy steht; Q für 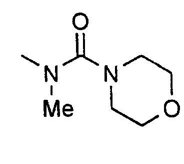 steht und B die in Tabelle 1 aufgelisteten Bedeutungen besitzt.

### Tabelle 40

Tabelle 40 enthält die Verbindungen der allgemeinen Formel (Ia-3), in welcher 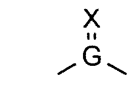 für Sulfonyl steht; Q für -NMe₂ steht und B die in Tabelle 1 aufgelisteten Bedeutungen besitzt.

### Tabelle 41

Tabelle 41 enthält die Verbindungen der allgemeinen Formel (Ia-3), in welcher 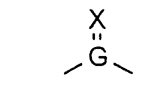 für Sulfonyl steht; Q für -NEt₂ steht und B die in Tabelle 1 aufgelisteten Bedeutungen besitzt.

### Tabelle 42

Tabelle 42 enthält die Verbindungen der allgemeinen Formel (Ia-3), in welcher 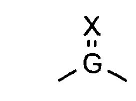 für Sulfonyl steht; Q für 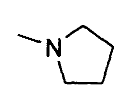 steht und B = diee in Tabelle 1 aufgelisteten Bedeutungen besitzt.

Weiterhin bevorzugt sind die Verbindungen der Formel (Ia-4) und deren Salze, bestehend aus der N-Methyl-aminosäure N-Methyl-aminobuttersäure (R¹ = Methyl, R² = Ethyl und R³ = Wasserstoff) und der 2-Hydroxycarbonsäure 2-Hydroxyessigsäure (R⁴, R⁵ = Wasserstoff).

Ganz besonders bevorzugte Beispiele für diese neuen erfindungsgemäßen Verbindungen ( Ia-4 ) sind in den Tabellen 43-56 aufgeführt.

### Tabelle 43

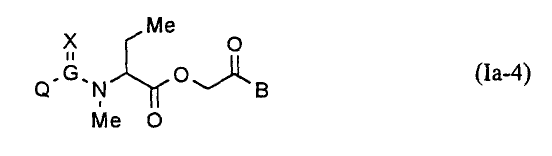

Verbindungen der Tabelle 43 entsprechen der allgemeinen Formel (Ia-4), in welcher 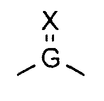 für Carboxy steht; Q für -NMe₂ steht und B die in Tabelle 1 aufgelisteten Bedeutungen besitzt.

### Tabelle 44

Tabelle 44 enthält die Verbindungen der allgemeinen Formel (Ia-4), in welcher 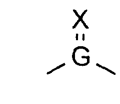 für Carboxy steht; Q für -NEt₂ steht und B die in Tabelle 1 aufgelisteten Bedeutungen besitzt.

### Tabelle 45

Tabelle 45 enthält die Verbindungen der allgemeinen Formel (Ia-4), in welcher 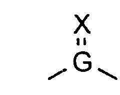 für Carboxy steht; Q für -O-Me steht und B die in Tabelle 1 aufgelisteten Bedeutungen besitzt.

### Tabelle 46

Tabelle 46 enthält die Verbindungen der allgemeinen Formel (Ia-4), in welcher 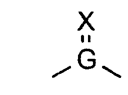 für Carboxy steht; Q für -O-CHMe-CH₂-Me steht und B die in Tabelle 1 aufgelisteten Bedeutungen besitzt.

### Tabelle 47

Tabelle 47 enthält die Verbindungen der allgemeinen Formel (Ia-4), in welcher 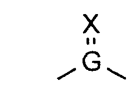 für Carboxy steht; Q für -O-CH₂-CH=CH₂ steht und B die in Tabelle 1 aufgelisteten Bedeutungen besitzt.

### Tabelle 48

Tabelle 48 enthält die Verbindungen der allgemeinen Formel (Ia-4), in welcher 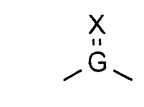 für Carboxy steht; Q für -O-CHMe-CH=CH₂ steht und B die in Tabelle 1 aufgelisteten Bedeutungen besitzt.

### Tabelle 49

Tabelle 49 enthält die Verbindungen der allgemeinen Formel (Ia-4), in welcher 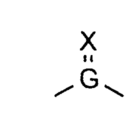 für Carboxy steht; Q für -O-CH₂-C≡CH steht und B die in Tabelle 1 aufgelisteten Bedeutungen besitzt.

### Tabelle 50

Tabelle 50 enthält die Verbindungen der allgemeinen Formel (Ia-4), in welcher 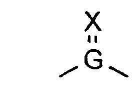 für Carboxy steht; Q für -O-CMe=CH₂ steht und B die in Tabelle 1 aufgelisteten Bedeutungen besitzt.

### Tabelle 51

Tabelle 51 enthält die Verbindungen der allgemeinen Formel (Ia-4), in welcher 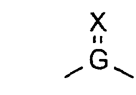 für Carboxy steht; Q für -NMe-CO-NMe₂ steht und B die in Tabelle 1 aufgelisteten Bedeutungen besitzt.

### Tabelle 52

Tabelle 52 enthält die Verbindungen der allgemeinen Formel (Ia-4), in welcher 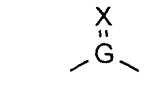 für Carboxy steht; Q für -NMe-CO-NEt₂ steht und B die in Tabelle 1 aufgelisteten Bedeutungen besitzt.

### Tabelle 53

Tabelle 53 enthält die Verbindungen der allgemeinen Formel (Ia-4), in welcher 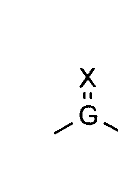 für Carboxy steht; Q für 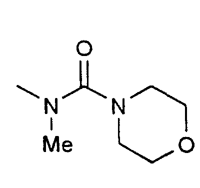 steht und B die in Tabelle 1 aufgelisteten Bedeutungen besitzt.

### Tabelle 54

Tabelle 54 enthält die Verbindungen der allgemeinen Formel (Ia-4), in welcher 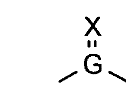 für Sulfonyl steht; Q für -NMe₂ steht und B die in Tabelle 1 aufgelisteten Bedeutungen besitzt.

### Tabelle 55

Tabelle 55 enthält die Verbindungen der allgemeinen Formel (Ia-4), in welcher 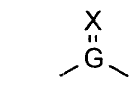 für Sulfonyl steht; Q für -NEt₂ steht und B die in Tabelle 1 aufgelisteten Bedeutungen besitzt.

### Tabelle 56

Tabelle 56 enthält die Verbindungen der allgemeinen Formel (Ia-4), in welcher 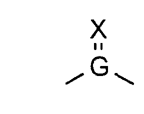 für Sulfonyl steht; Q für 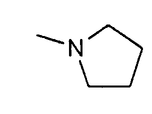 steht und B die in Tabelle 1 aufgelisteten Bedeutungen besitzt.

Weiterhin bevorzugt sind die Verbindungen der Formel (Ia-5) und deren Salze, bestehend aus der N-Methyl-aminosäure N-Methyl-aminobuttersäure (R¹ = Methyl, R² = Ethyl und R³ = Wasserstoff) und der 2-Hydroxycarbonsäure 2-Hydroxypropion-säure (Milchsäure) (R⁴ = Wasserstoff; R³ = Methyl ).

Ganz besonders bevorzugte Beispiele für diese neuen erfindungsgemäßen Verbindungen ( Ia-5 ) sind in den Tabellen 57-70 aufgeführt.

### Tabelle 57

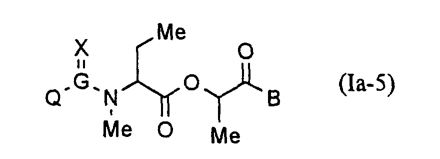

Verbindungen der. Tabelle 43 entsprechen der allgemeinen Formel (Ia-5), in welcher 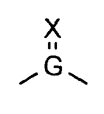 für Carboxy steht; Q für -NMe₂ steht und B die in Tabelle 1 aufgelisteten Bedeutungen besitzt.

### Tabelle 58

Tabelle 58 enthält die Verbindungen der allgemeinen Formel (Ia-5), in welcher 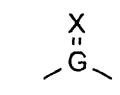 für Carboxy steht; Q für -NEt₂ steht und B die in Tabelle 1 aufgelisteten Bedeutungen besitzt.

### Tabelle 59

Tabelle 59 enthält die Verbindungen der allgemeinen Formel (Ia-5), in welcher 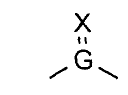 für Carboxy steht; Q für -O-Me steht und B die in Tabelle 1 aufgelisteten Bedeutungen besitzt.

### Tabelle 60

Tabelle 60 enthält die Verbindungen der allgemeinen Formel (Ia-5), in welcher 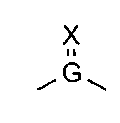 für Carboxy steht; Q für -O-CHMe-CH₂-Me steht und B die in Tabelle 1 aufgelisteten Bedeutungen besitzt.

### Tabelle 61

Tabelle 61 enthält die Verbindungen der allgemeinen Formel (Ia-5), in welcher 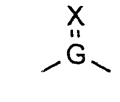 für Carboxy steht; Q für -O-CH₂-CH=CH₂ steht und B die in Tabelle 1 aufgelisteten Bedeutungen besitzt.

### Tabelle 62

Tabelle 62 enthält die Verbindungen der allgemeinen Formel (Ia-5), in welcher 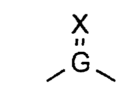 für Carboxy steht; Q für -O-CHMe-CH=CH₂ steht und B die in Tabelle 1 aufgelisteten Bedeutungen besitzt.

### Tabelle 63

Tabelle 63 enthält die Verbindungen der allgemeinen Formel (Ia-5), in welcher 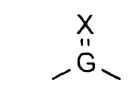 für Carboxy steht; Q für -O-CH₂-C≡CH steht und B die in Tabelle 1 aufgelisteten Bedeutungen besitzt.

### Tabelle 64

Tabelle 64 enthält die Verbindungen der allgemeinen Formel (Ia-5), in welcher 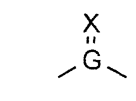 für Carboxy steht; Q für -O-CMe=CH₂ steht und B die in Tabelle 1 aufgelisteten Bedeutungen besitzt.

### Tabelle 65

Tabelle 65 enthält die Verbindungen der allgemeinen Formel (Ia-5), in welcher 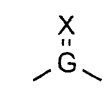 für Carboxy steht; Q für -NMe-CO-NMe₂ steht und B die in Tabelle 1 aufgelisteten Bedeutungen besitzt.

### Tabelle 66

Tabelle 66 enthält die Verbindungen der allgemeinen Formel (Ia-5), in welcher 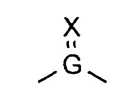 für Carboxy steht; Q für -NMe-CO-NEt₂ steht und B die in Tabelle 1 aufgelisteten Bedeutungen besitzt.

### Tabelle 67

Tabelle 67 enthält die Verbindungen der allgemeinen Formel (Ia-5), in welcher 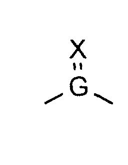 für Carboxy steht; Q für 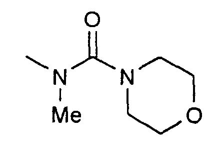 steht und B die in Tabelle 1 aufgelisteten Bedeutungen besitzt.

### Tabelle 68

Tabelle 68 enthält die Verbindungen der allgemeinen Formel (Ia-5), in welcher 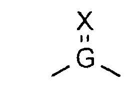 für Sulfonyl steht; Q für -NMe₂ steht und B die in Tabelle 1 aufgelisteten Bedeutungen besitzt.

### Tabelle 69

Tabelle 69 enthält die Verbindungen der allgemeinen Formel (Ia-5), in welcher 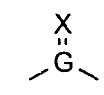 für Sulfonyl steht; Q für -NEt₂ steht und B die in Tabelle 1 aufgelisteten Bedeutungen besitzt.

### Tabelle 70

Tabelle 70 enthält die Verbindungen der allgemeinen Formel (Ia-5), in welcher 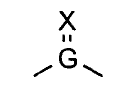 für Sulfonyl steht; Q für 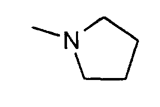 steht und B die in Tabelle 1 aufgelistet.

Weiterhin bevorzugt sind die Verbindungen der Formel (Ia-6) und deren Salze, bestehend aus der N-Methyl-aminosäure N-Methyl-aminobuttersäure (R¹ = Methyl, R² = Ethyl und R³ = Wasserstoff) und der 2-Hydroxycarbonsäure 2-Hydroxybuttersäu-re (R⁴ = Wasserstoff; R⁵ = Ethyl).

Ganz besonders bevorzugte Beispiele für diese neuen erfindungsgemäßen Verbindungen ( Ia-6 ) sind in den Tabellen 71-84 aufgeführt.

### Tabelle 71

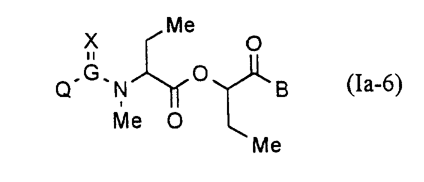

Verbindungen der Tabelle 71 entsprechen der allgemeinen Formel (Ia-6), in welcher 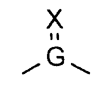 für Carboxy steht; Q für -NMe₂ steht und B die in Tabelle 1 aufgelisteten Bedeutungen besitzt.

### Tabelle 72

Tabelle 72 enthält die Verbindungen der allgemeinen Formel (Ia-6), in welcher 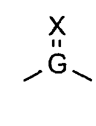 für Carboxy steht; Q für -NEt₂ steht und B die in Tabelle 1 aufgelisteten Bedeutungen besitzt.

### Tabelle 73

Tabelle 73 enthält die Verbindungen der allgemeinen Formel (Ia-6), in welcher 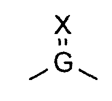 für Carboxy steht; Q für -O-Me steht und B die in Tabelle 1 aufgelisteten Bedeutungen besitzt.

### Tabelle 74

Tabelle 74 enthält die Verbindungen der allgemeinen Formel (Ia-6), in welcher 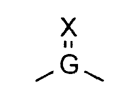 für Carboxy steht; Q für -O-CHMe-CH₂-Me steht und B die in Tabelle 1 aufgelisteten Bedeutungen besitzt.

### Tabelle 75

Tabelle 75 enthält die Verbindungen der allgemeinen Formel (Ia-6), in welcher 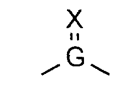 für Carboxy steht; Q für -O-CH₂-CH=CH₂ steht und B die in Tabelle 1 aufgelisteten Bedeutungen besitzt.

### Tabelle 76

Tabelle 76 enthält die Verbindungen der allgemeinen Formel (Ia-6), in welcher für Carboxy steht; Q für -O-CHMe-CH=CH₂ steht und B die in Tabelle 1 aufgelisteten Bedeutungen besitzt.

### Tabelle 77

Tabelle 77 enthält die Verbindungen der allgemeinen Formel (Ia-6), in welcher für Carboxy steht; Q für -O-CH₂-C≡CH steht und B die in Tabelle 1 aufgelisteten Bedeutungen besitzt.

### Tabelle 78

Tabelle 78 enthält die Verbindungen der allgemeinen Formel (Ia-6), in welcher 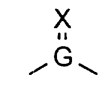 für Carboxy steht; Q für -O-CMe=CH₂ steht und B die in Tabelle 1 aufgelisteten Bedeutungen besitzt.

### Tabelle 79

Tabelle 79 enthält die Verbindungen der allgemeinen Formel (Ia-6), in welcher 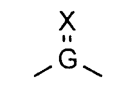 für Carboxy steht; Q für -NMe-CO-NMe₂ steht und B die in Tabelle 1 aufgelisteten Bedeutungen besitzt.

### Tabelle 80

Tabelle 80 enthält die Verbindungen der allgemeinen Formel (Ia-6), in welcher 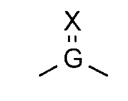 für Carboxy steht; Q für -NMe-CO-NEt₂ steht und B die in Tabelle 1 aufgelisteten Bedeutungen besitzt.

### Tabelle 81

Tabelle 81 enthält die Verbindungen der allgemeinen Formel (Ia-6), in welcher 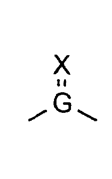 für Carboxy steht; Q für 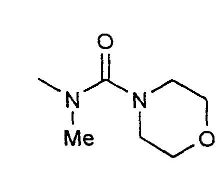 steht und B die in Tabelle 1 aufgelisteten Bedeutungen besitzt.

### Tabelle 82

Tabelle 82 enthält die Verbindungen der allgemeinen Formel (Ia-6), in welcher 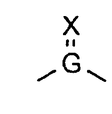 für Sulfonyl steht; Q für -NMe₂ steht und B die in Tabelle 1 aufgelisteten Bedeutungen besitzt.

### Tabelle 83

Tabelle 83 enthält die Verbindungen der allgemeinen Formel (Ia-6), in welcher 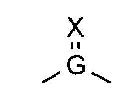 für Sulfonyl steht; Q für -NEt₂ steht und B die in Tabelle 1 aufgelisteten Bedeutungen besitzt.

### Tabelle 84

Tabelle 84 enthält die Verbindungen der allgemeinen Formel (Ia-6), in welcher 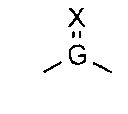 für Sulfonyl steht; Q für 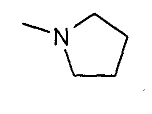 steht und B die in Tabelle 1 aufgelisteten Bedeutungen besitzt.

Im einzelnen seien folgende Verbindungen der allgemeinen Formel (Ia) genannt, in welcher die Reste R¹ bis R⁵, G, Q, X und B die folgende Bedeutung haben:

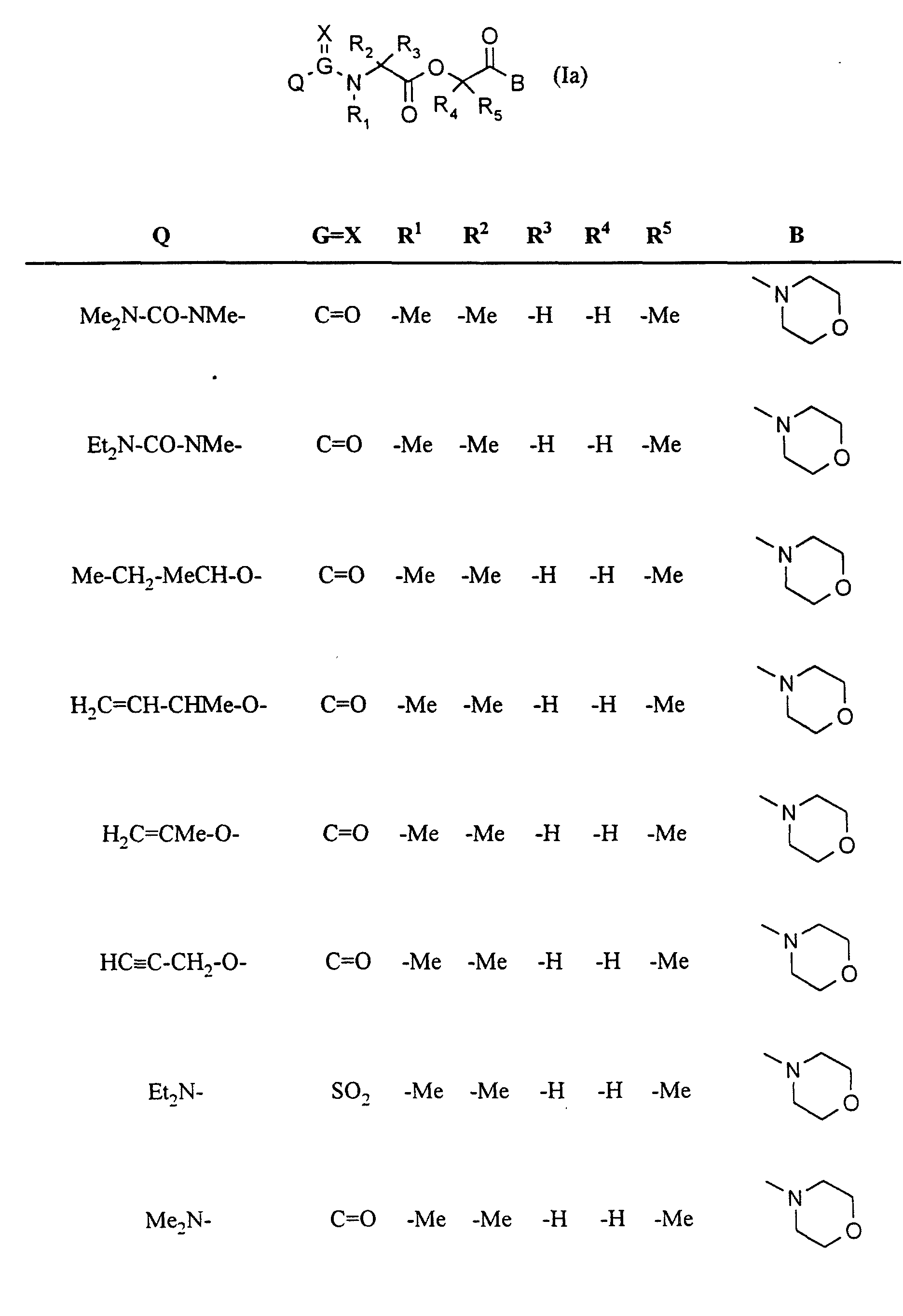

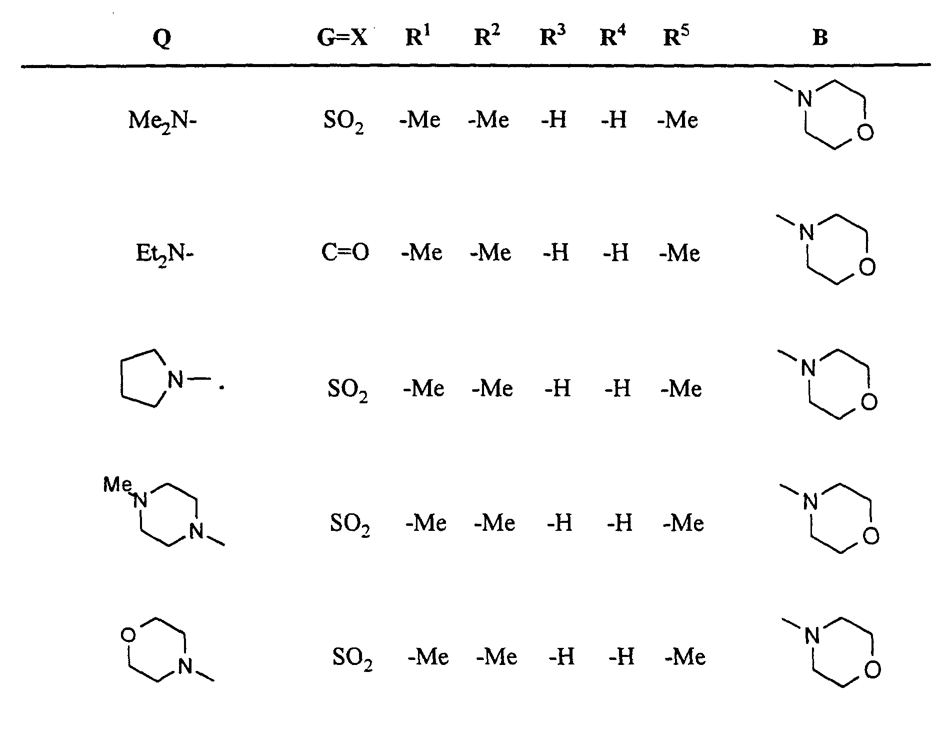

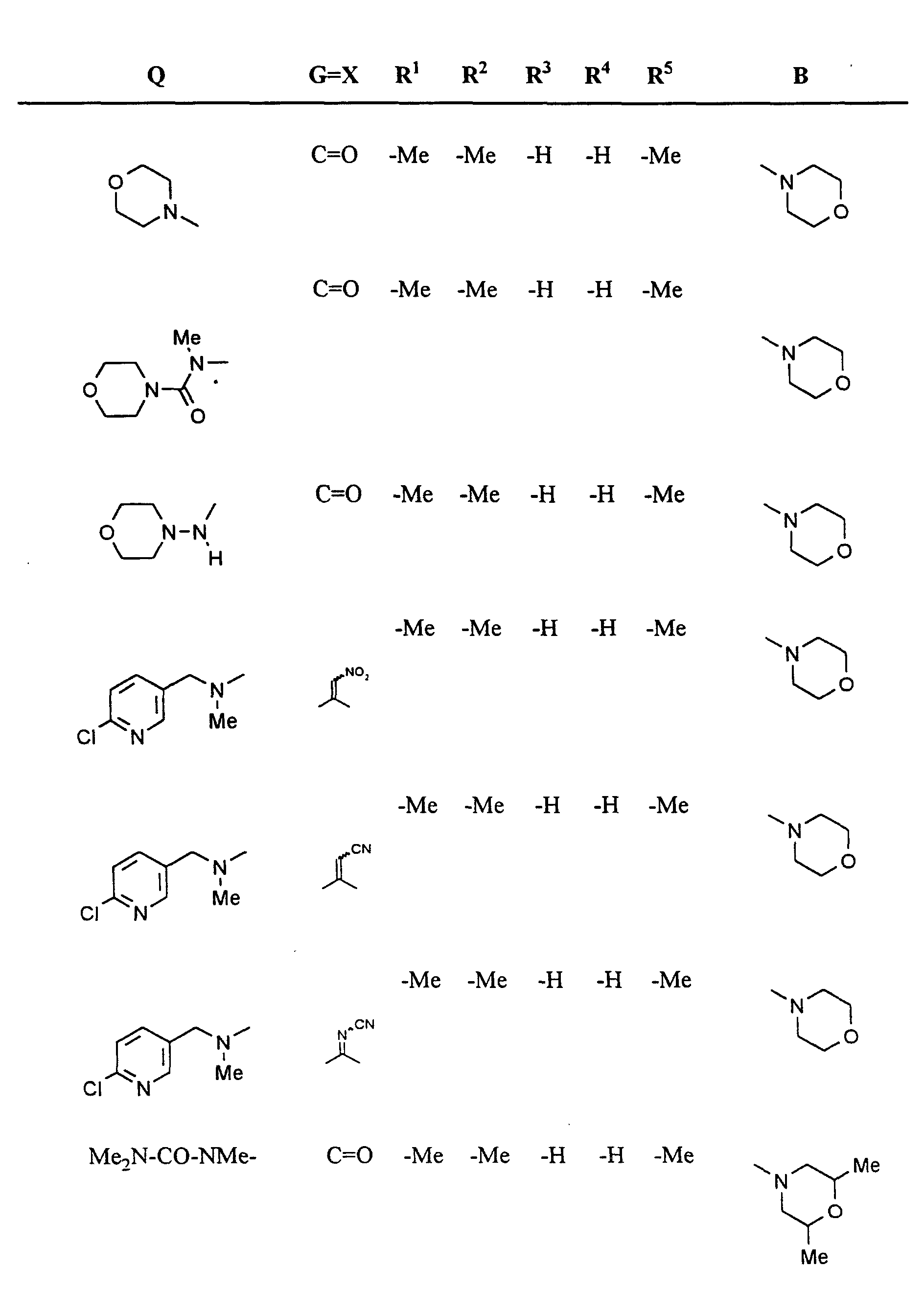

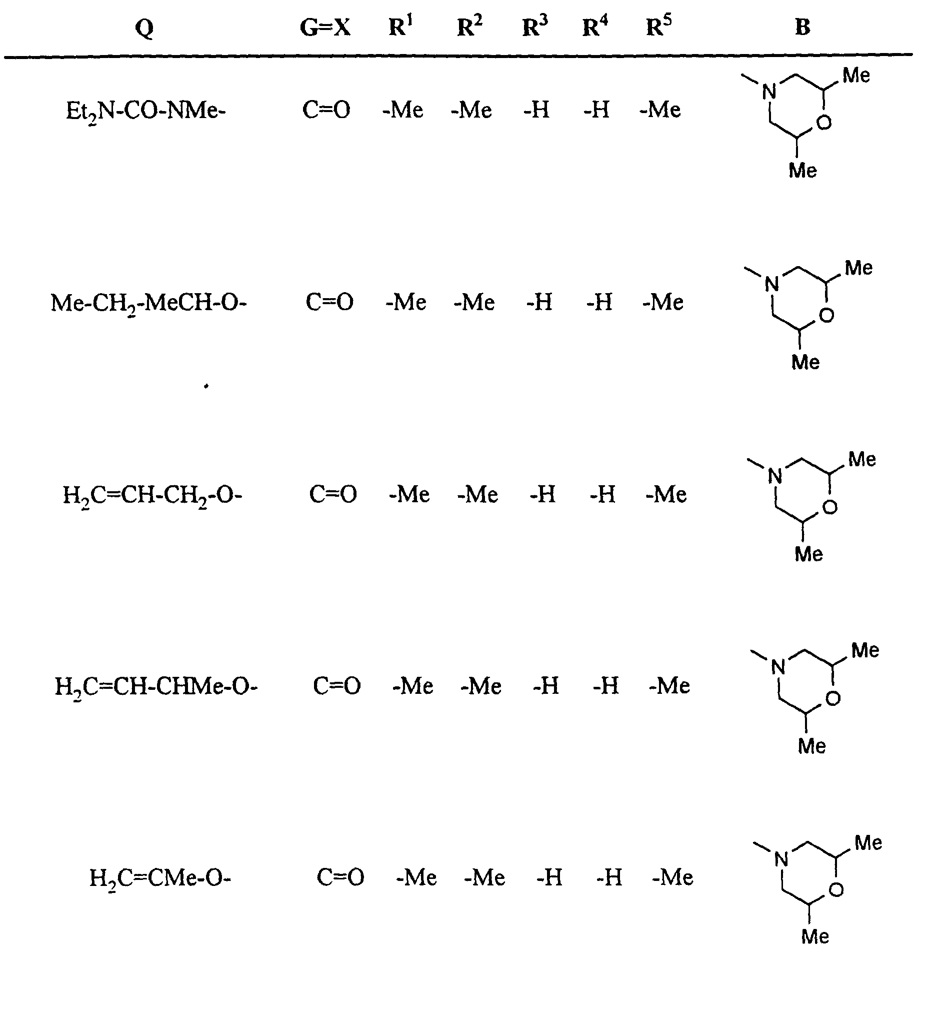

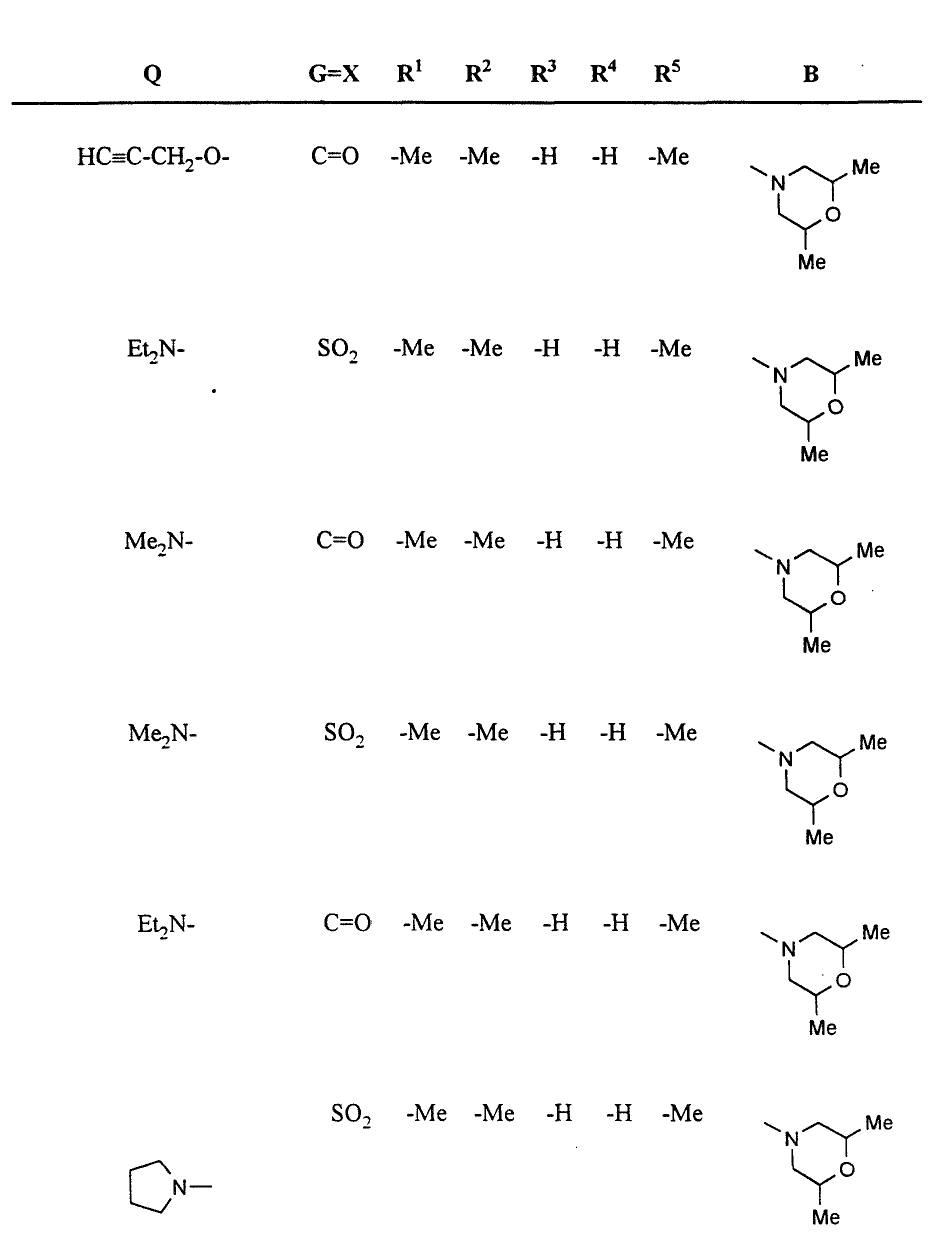

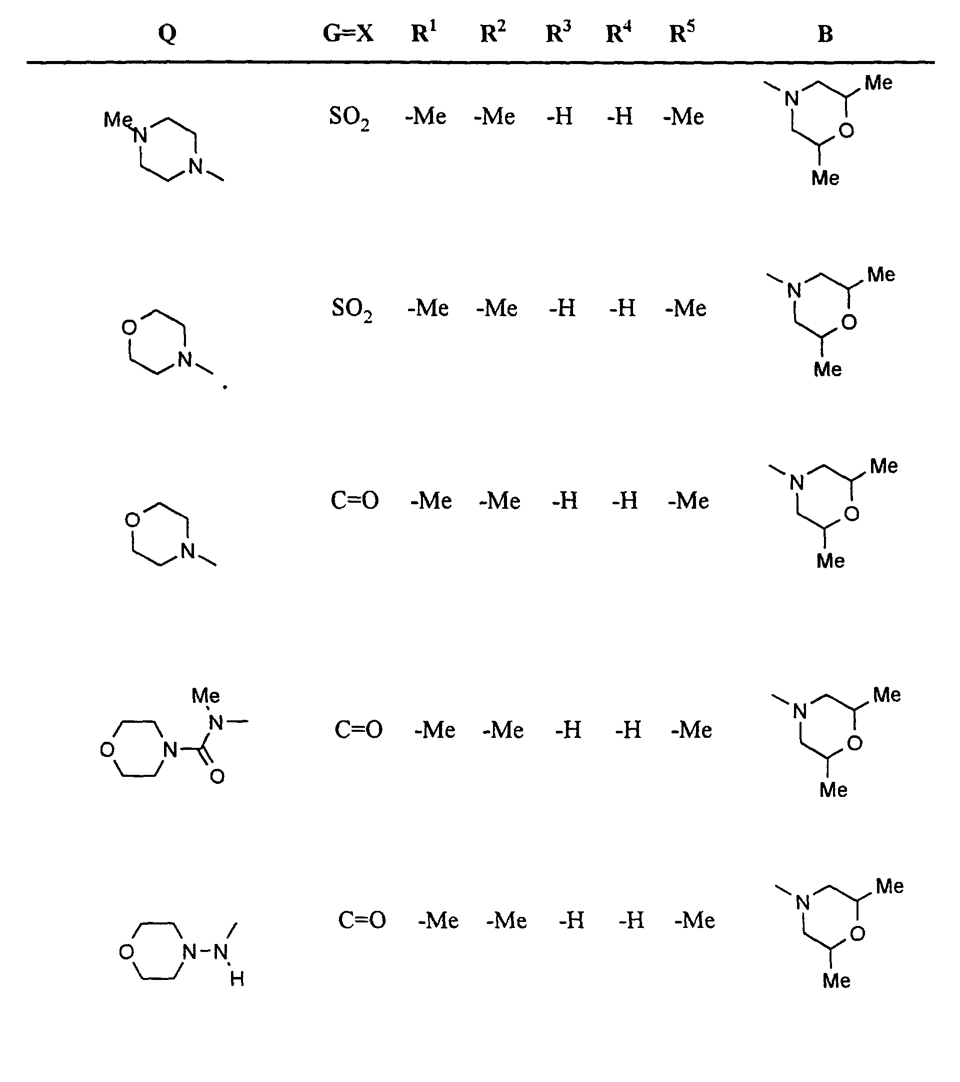

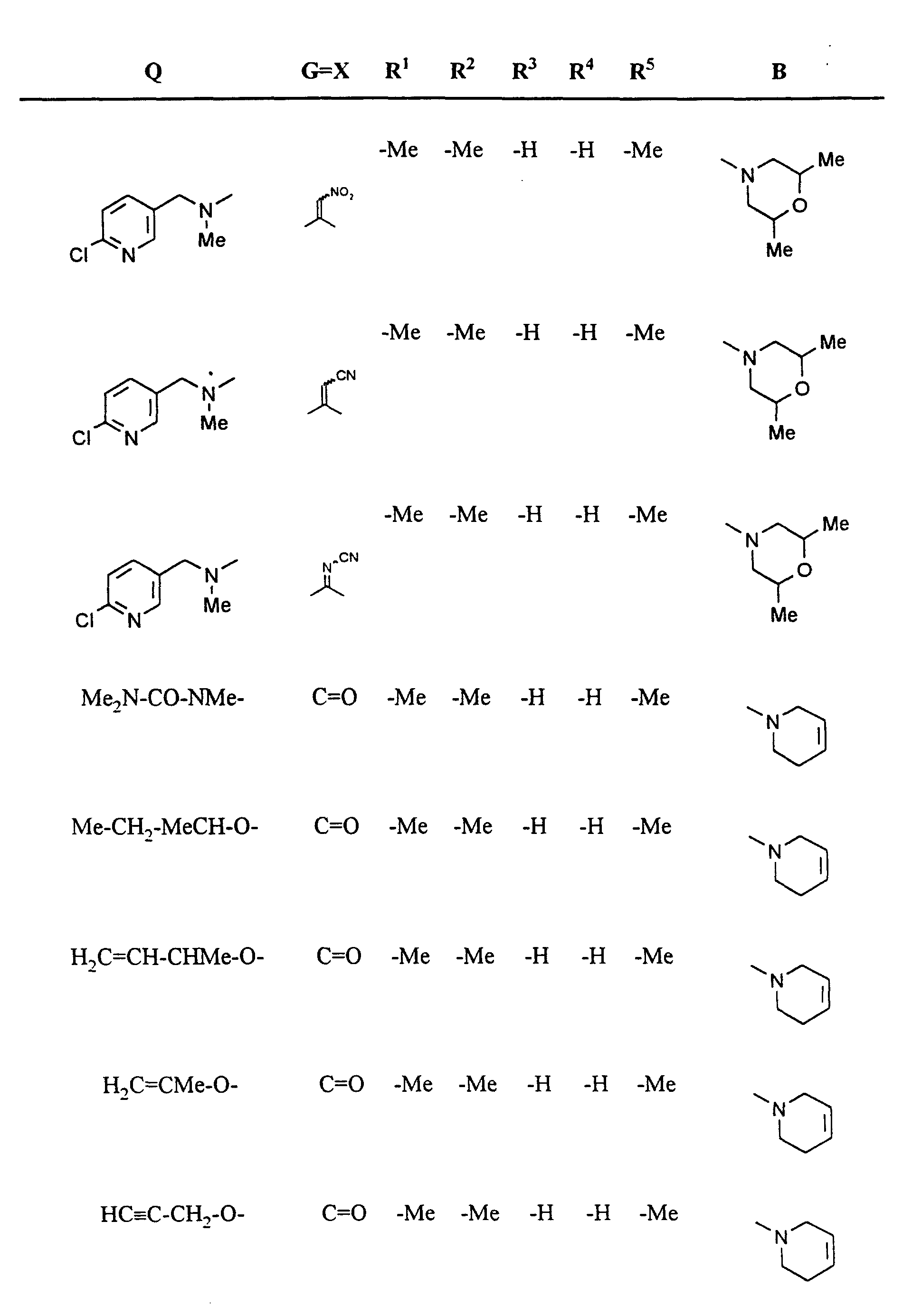

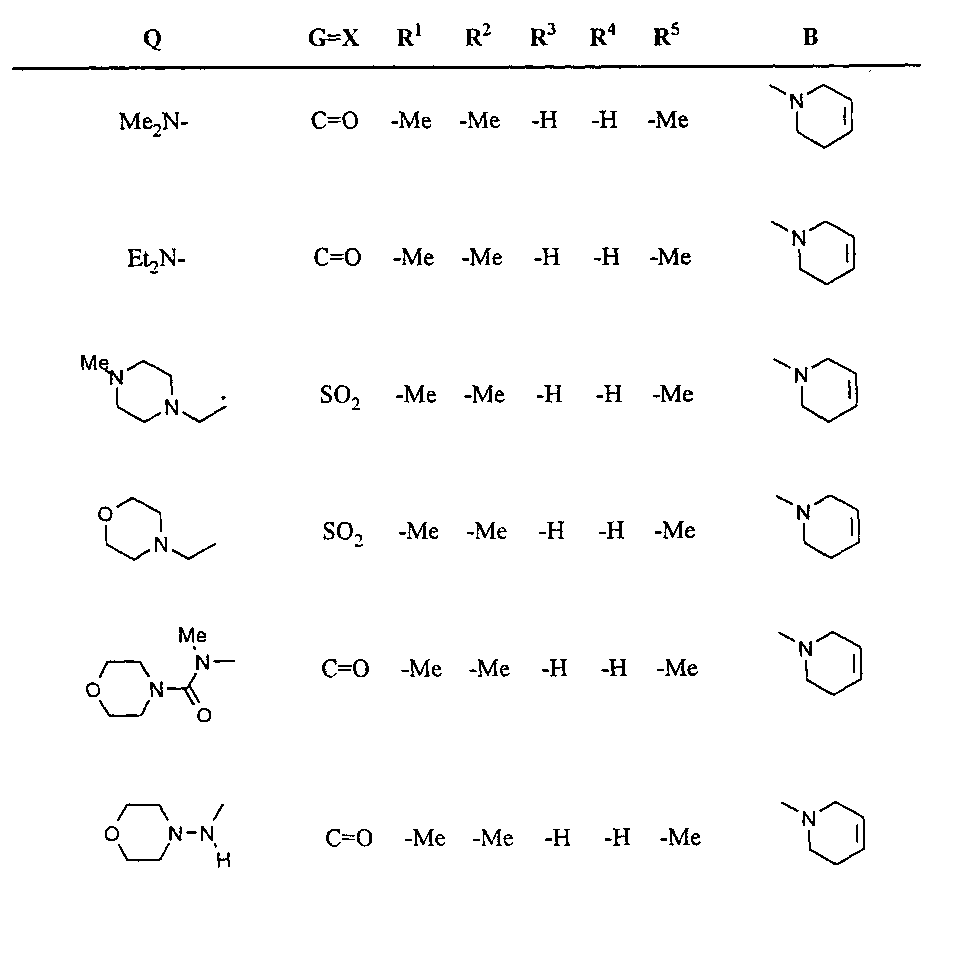

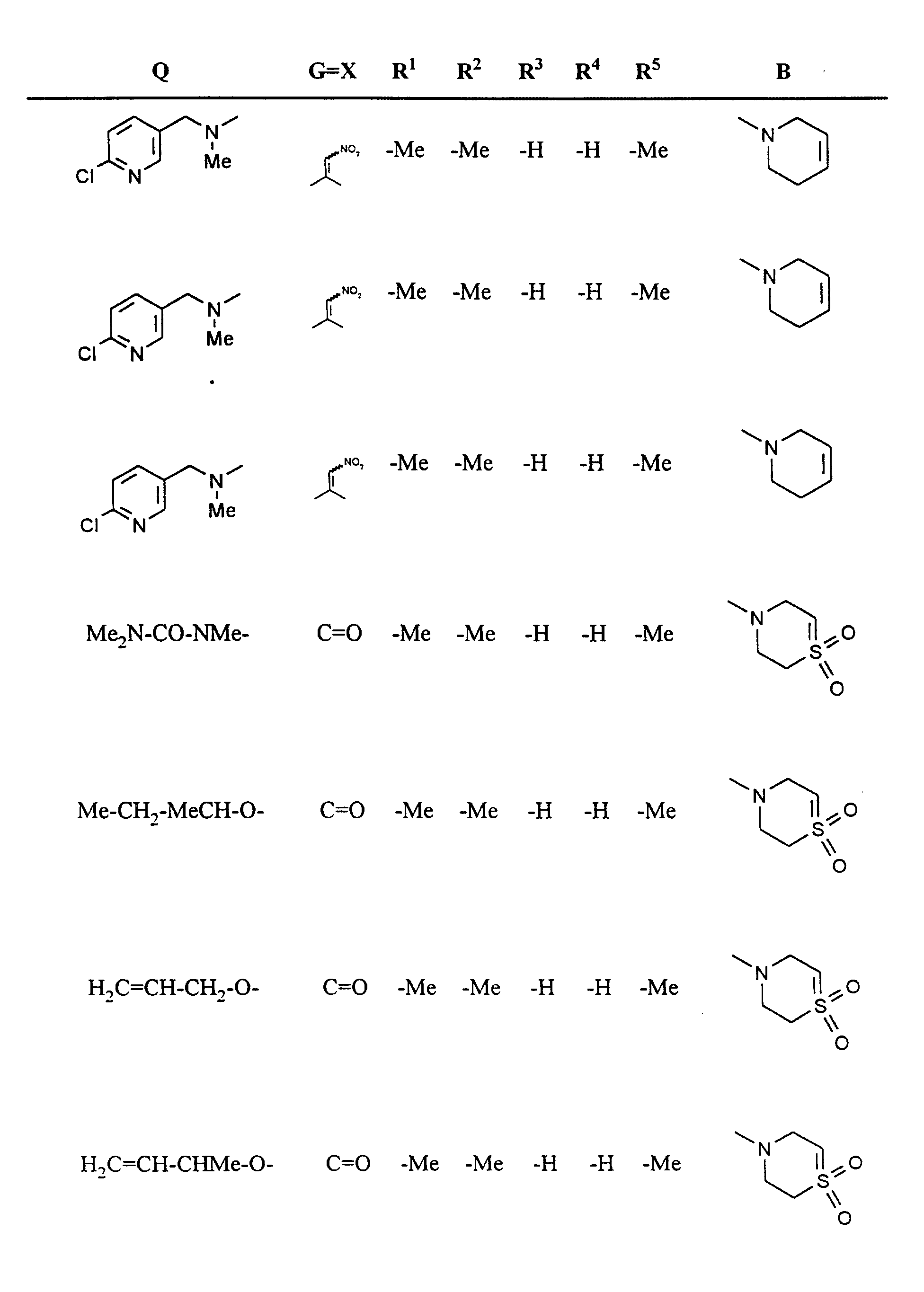

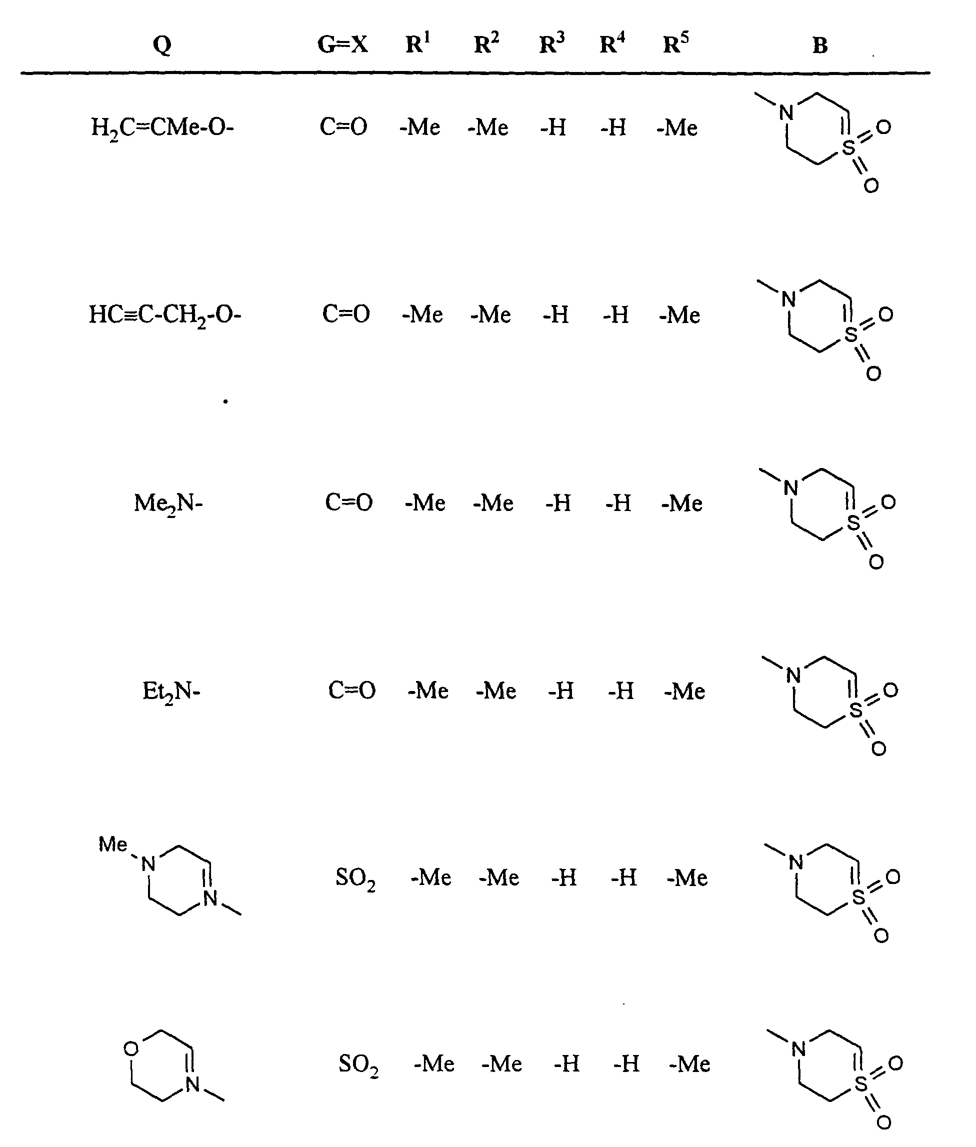

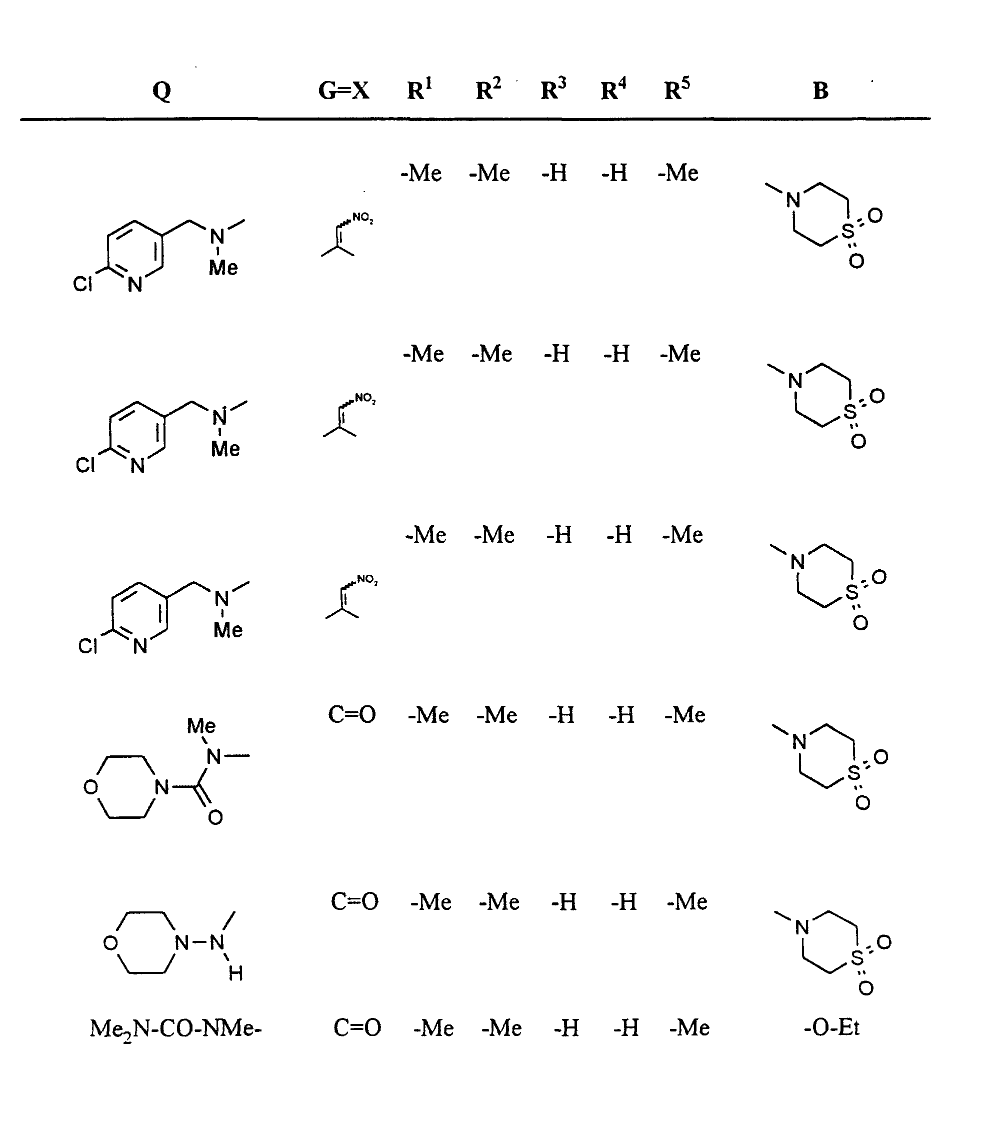

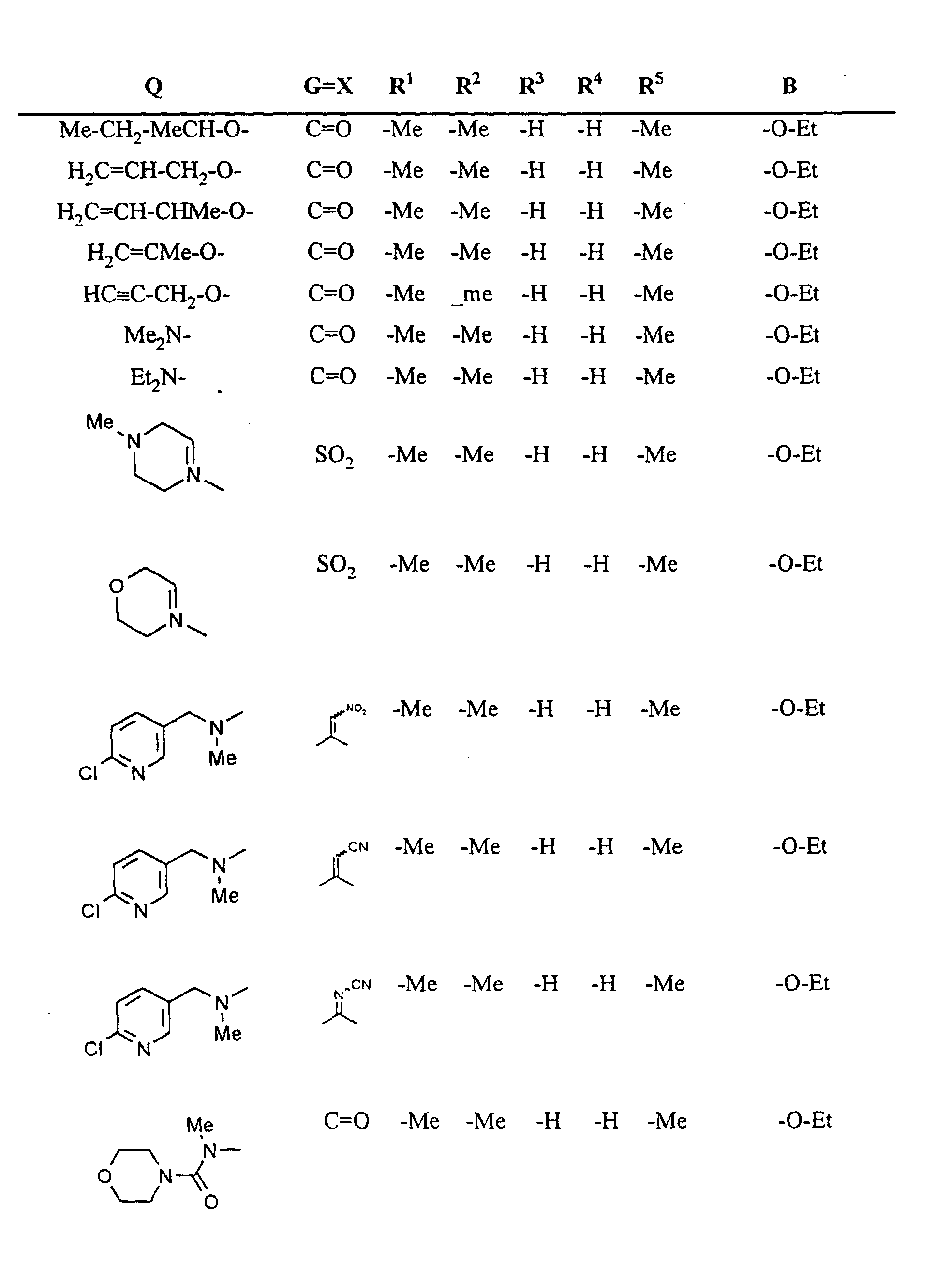

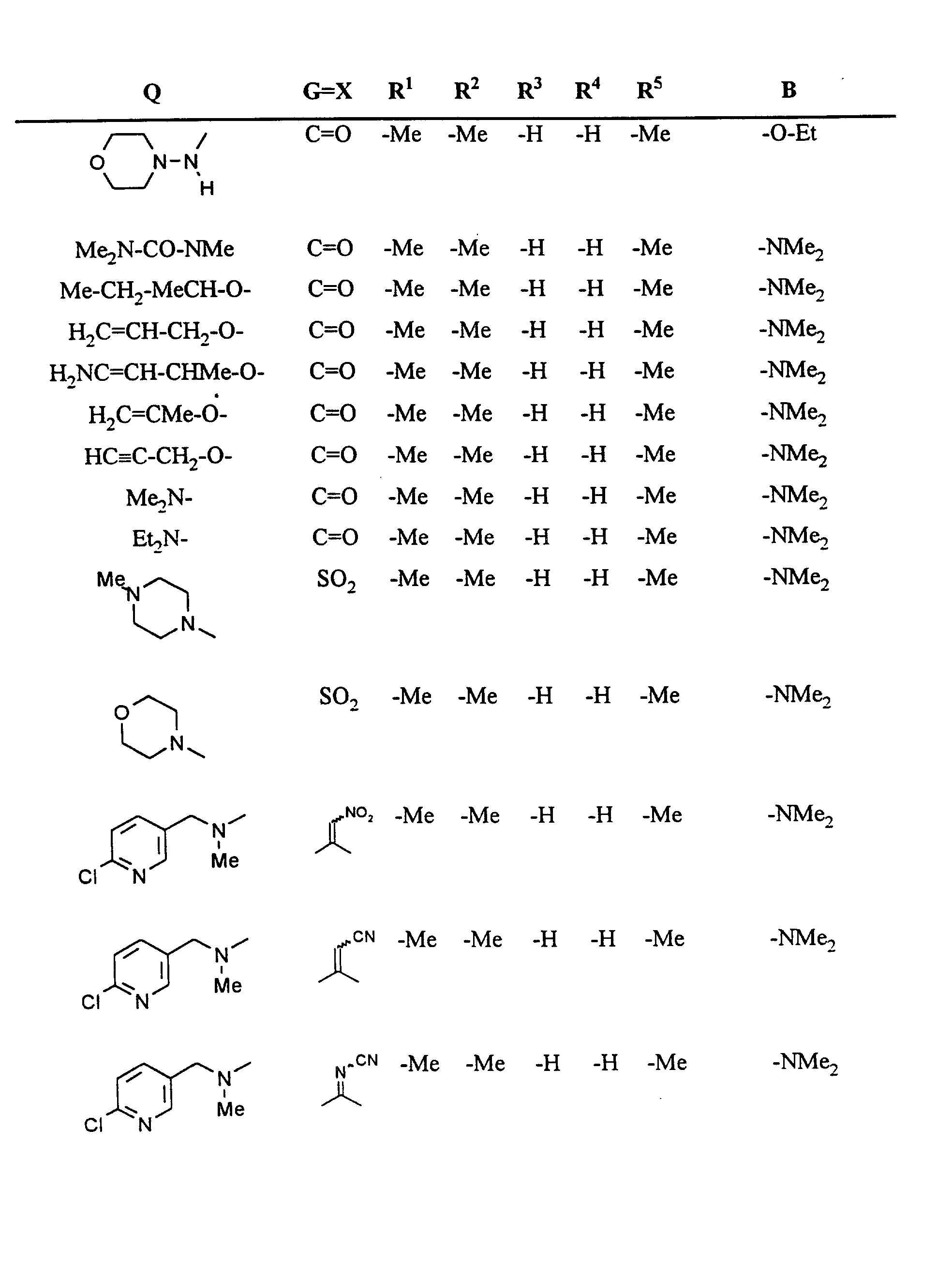

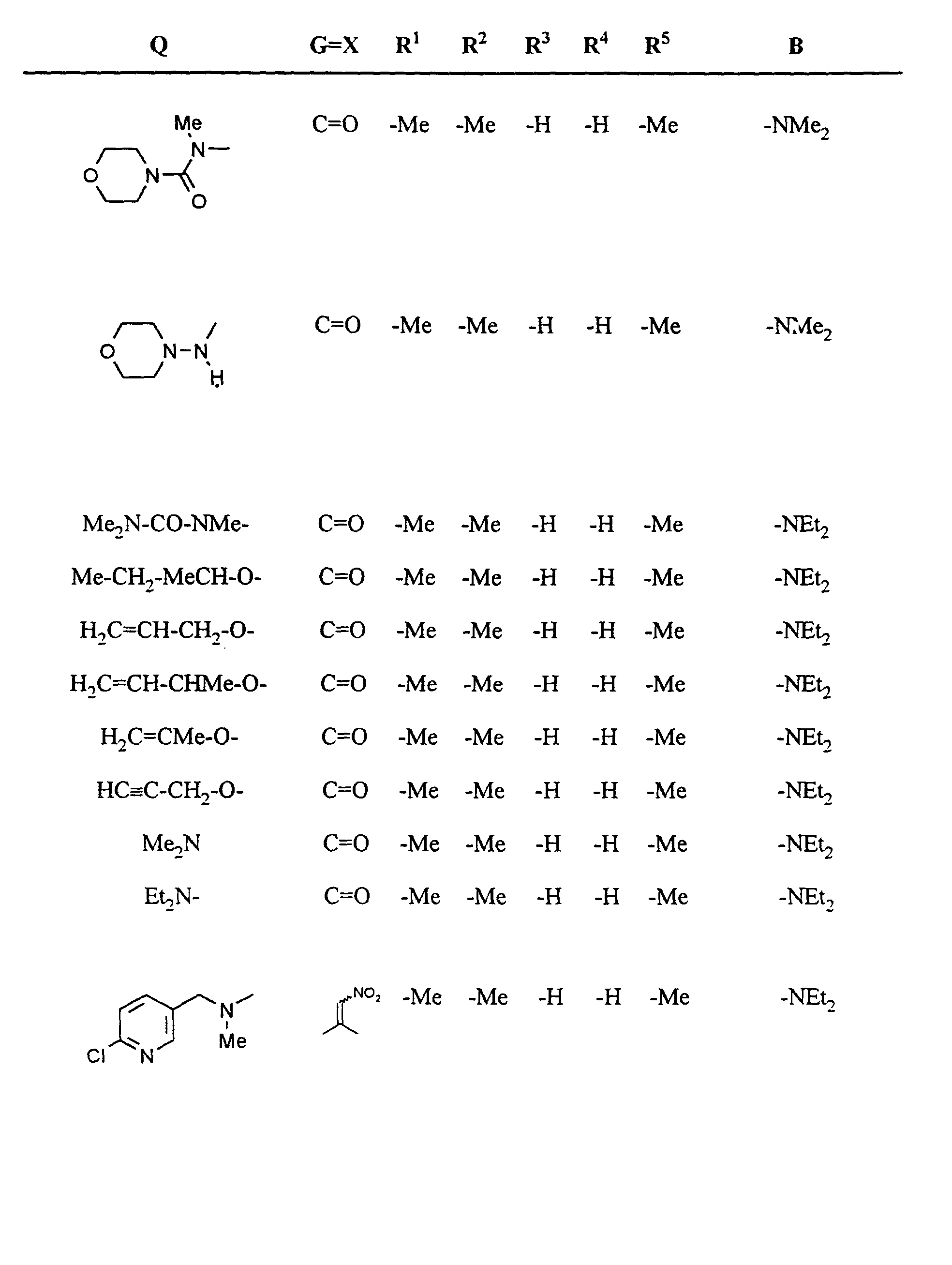

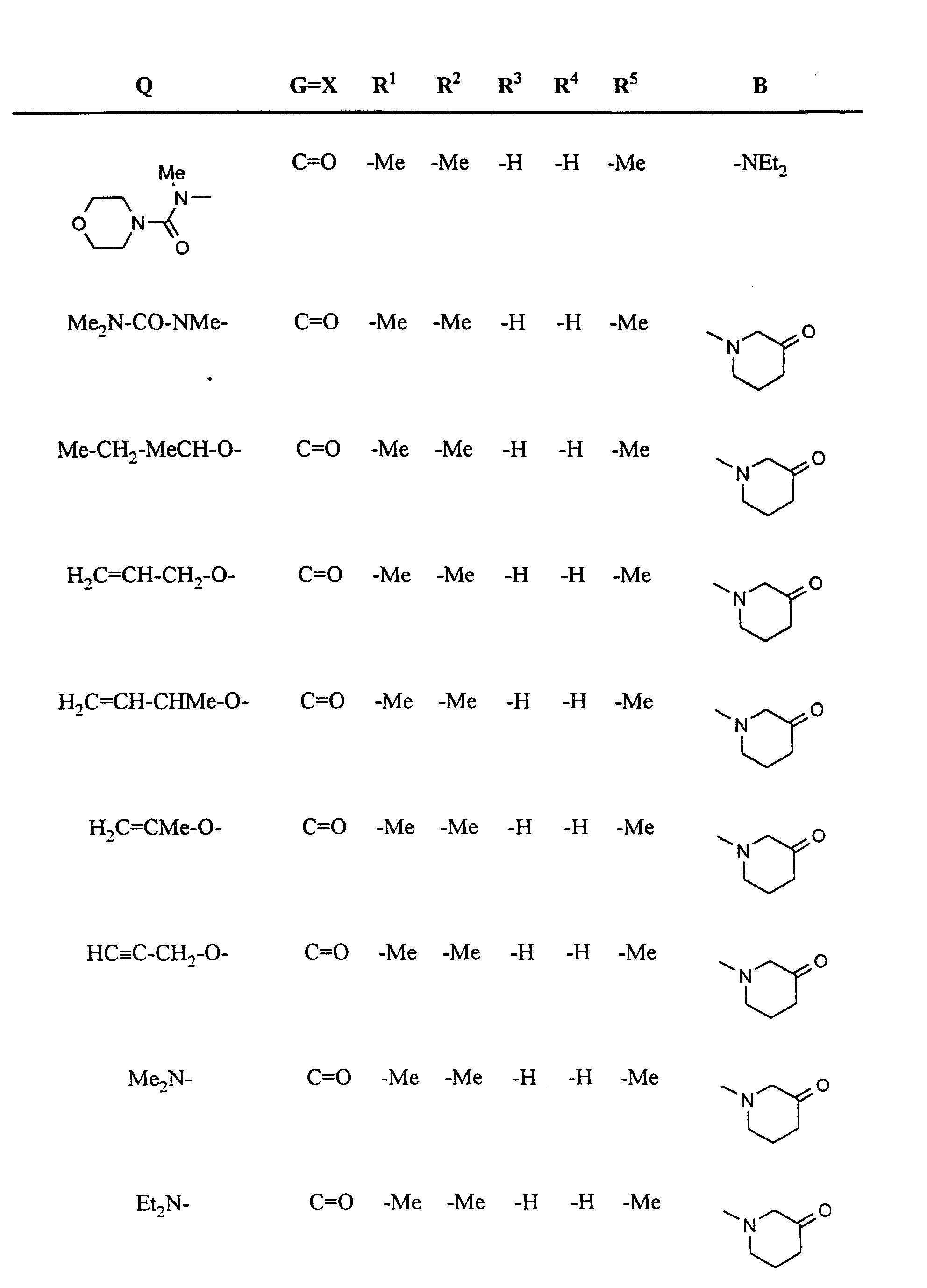

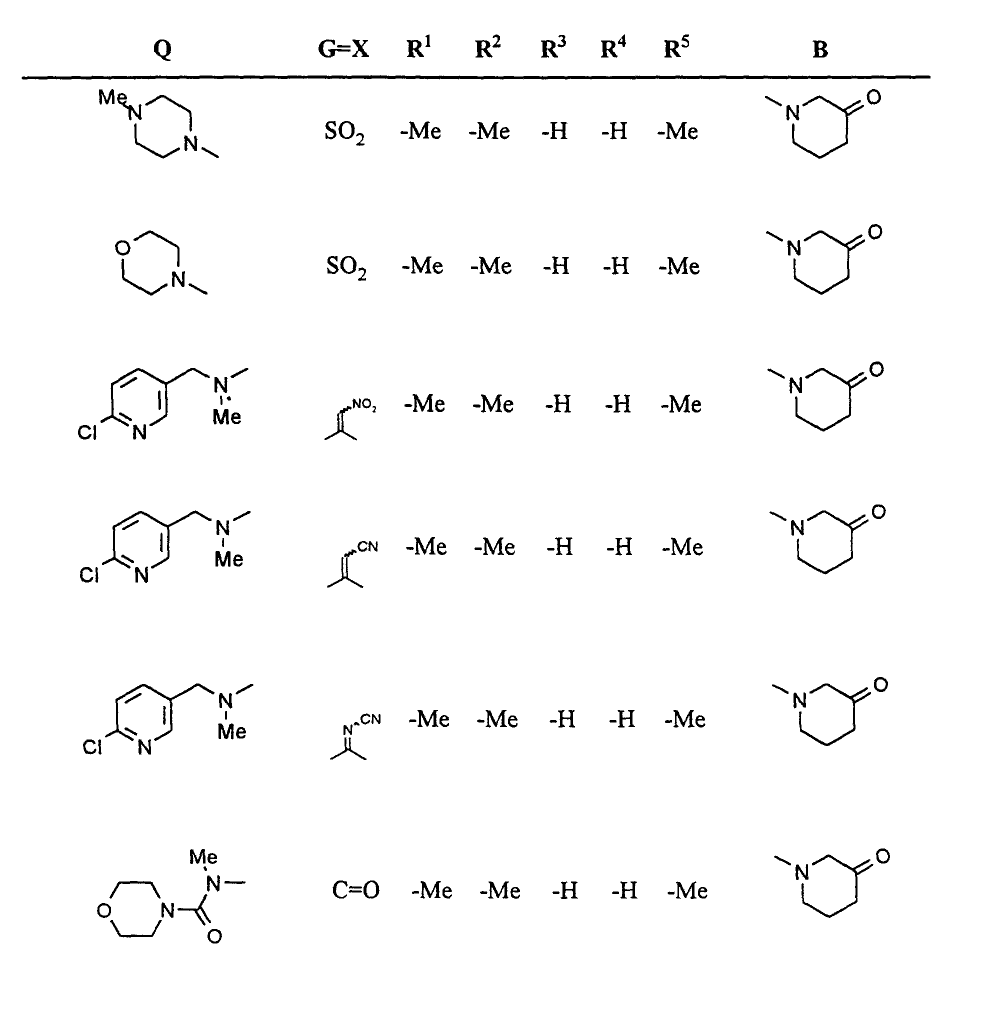

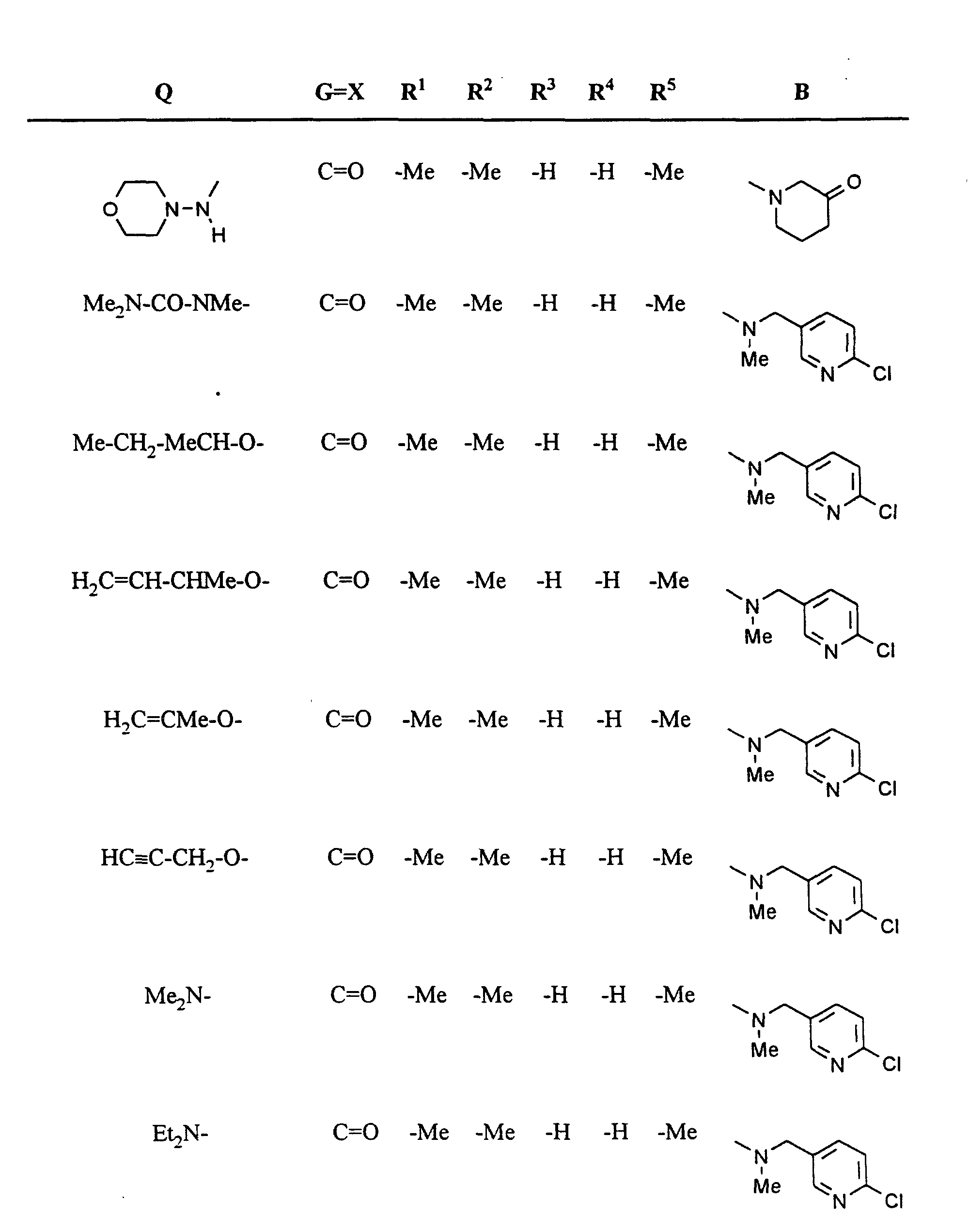

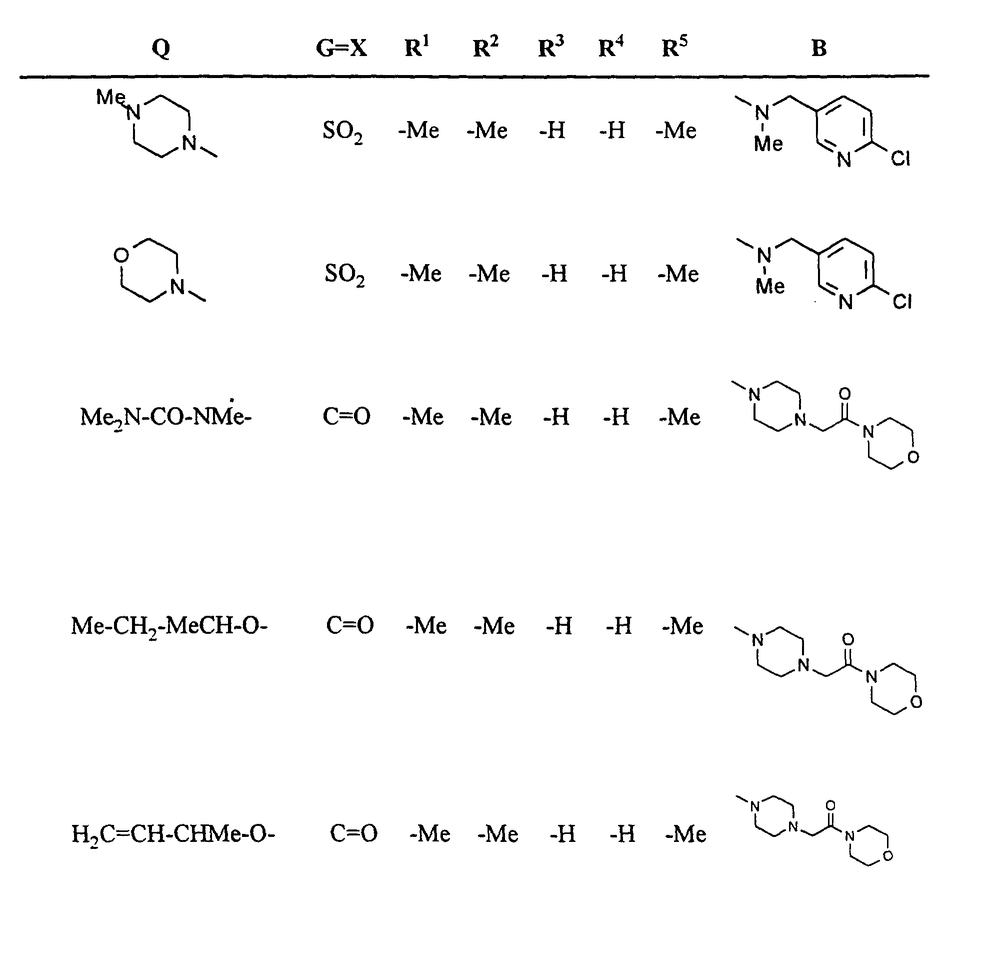

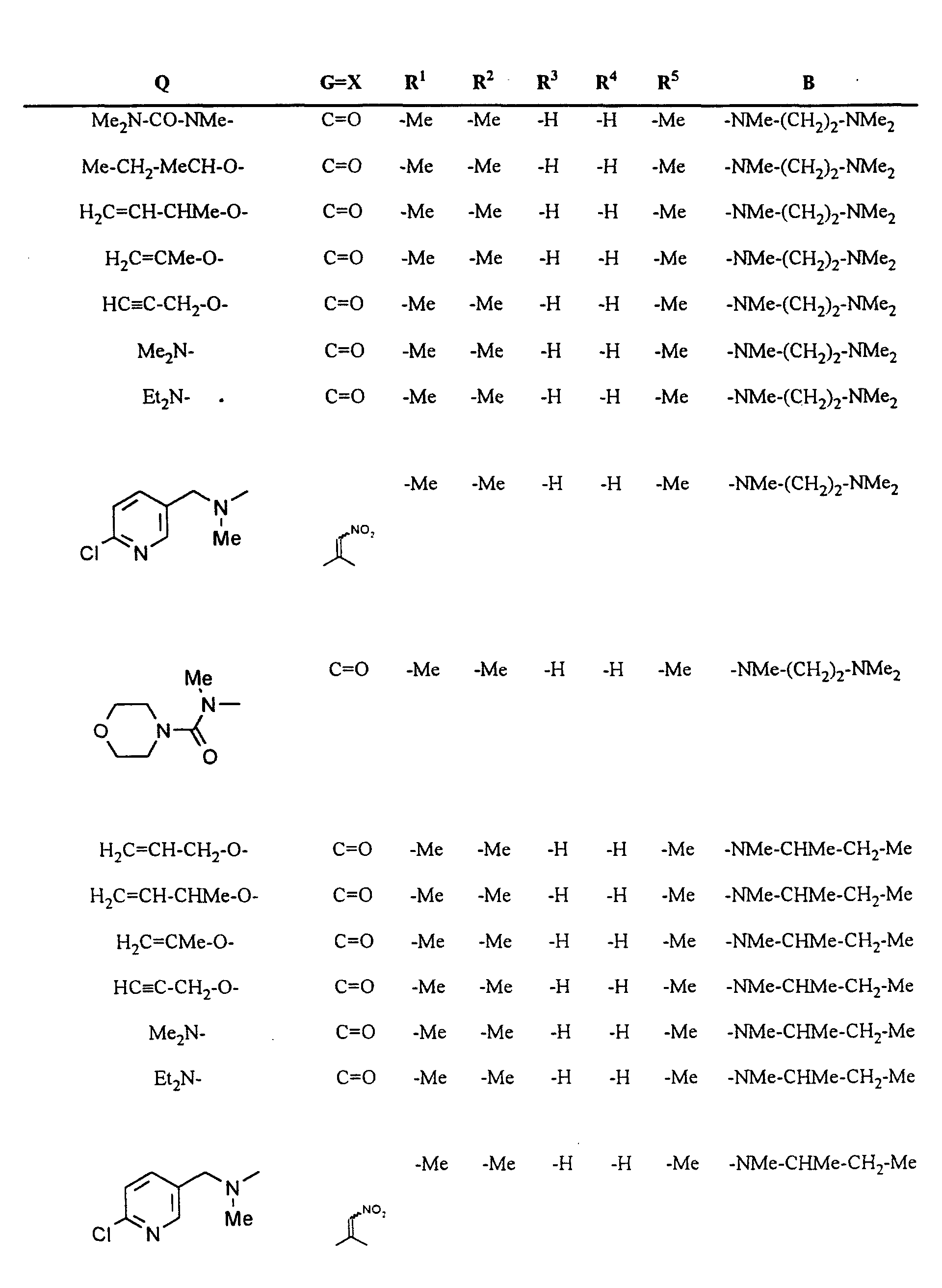

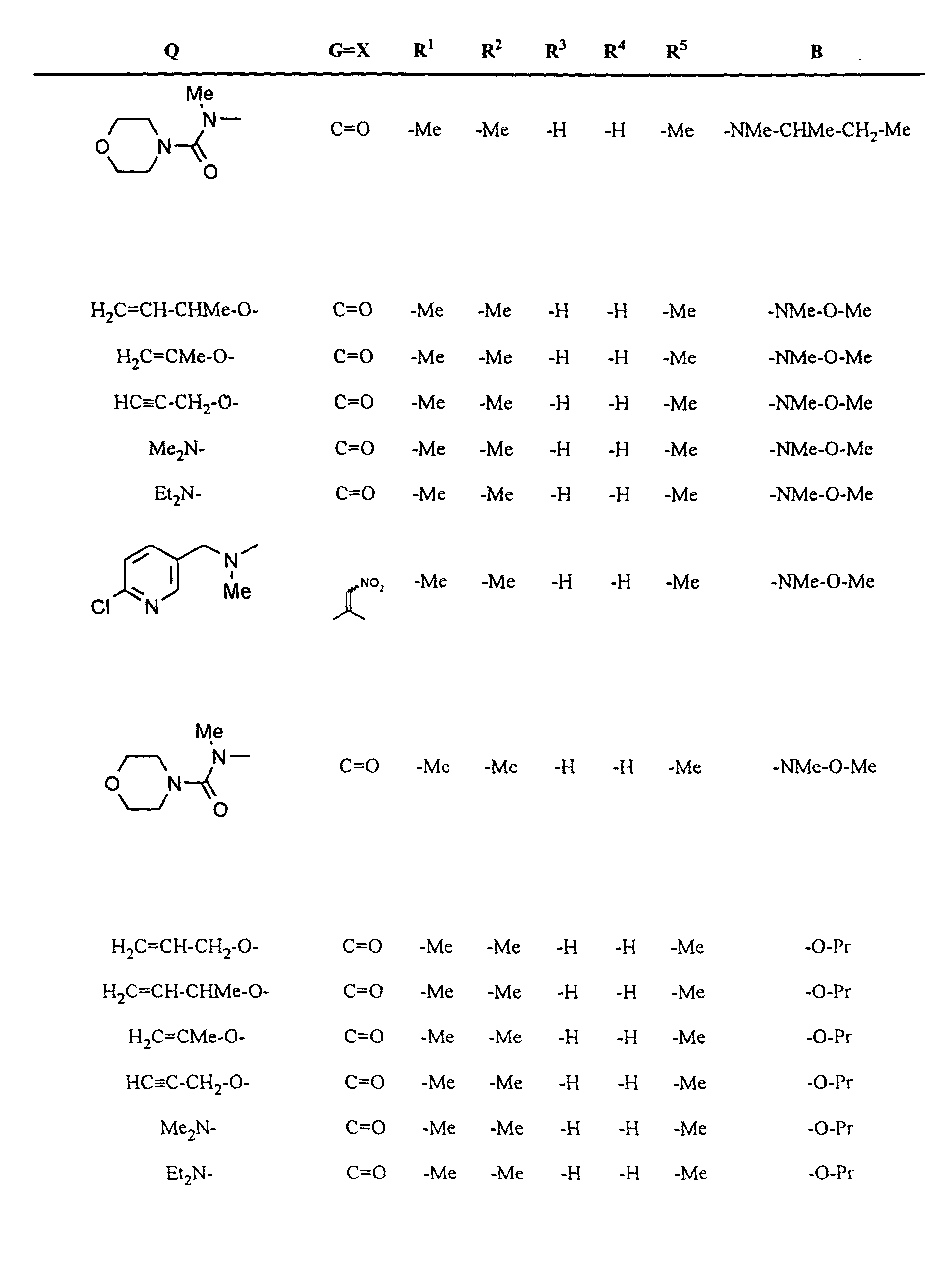

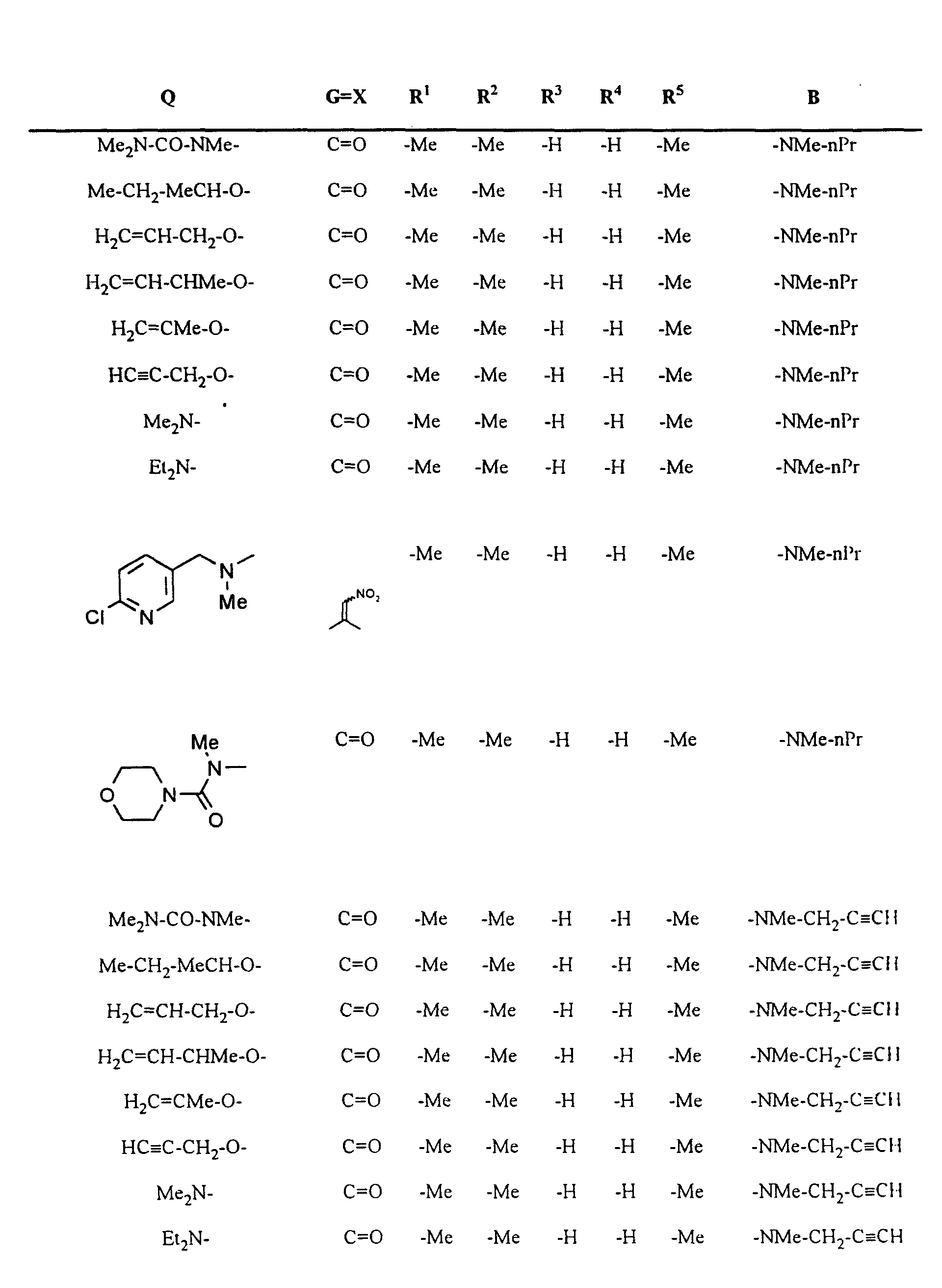

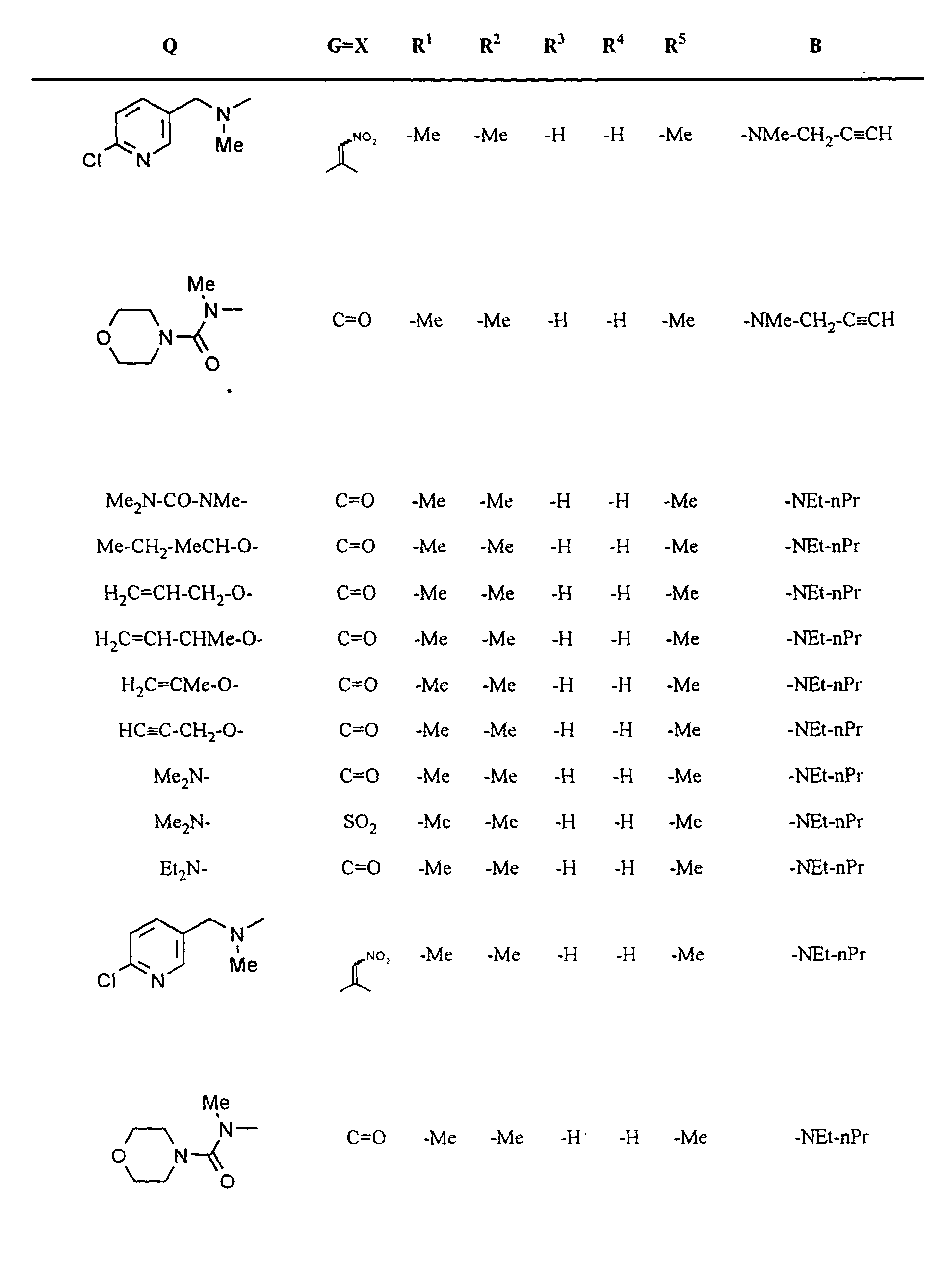

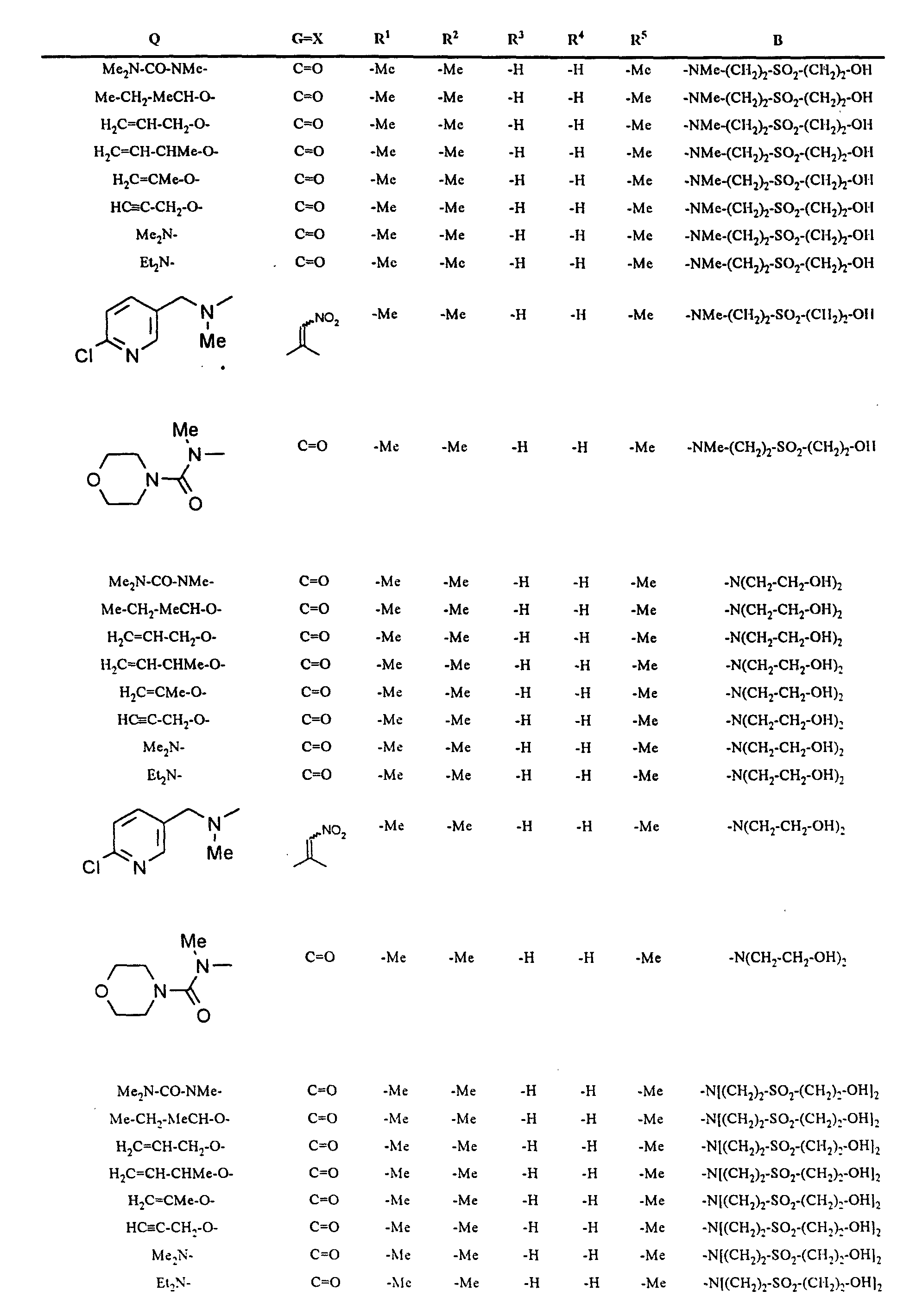

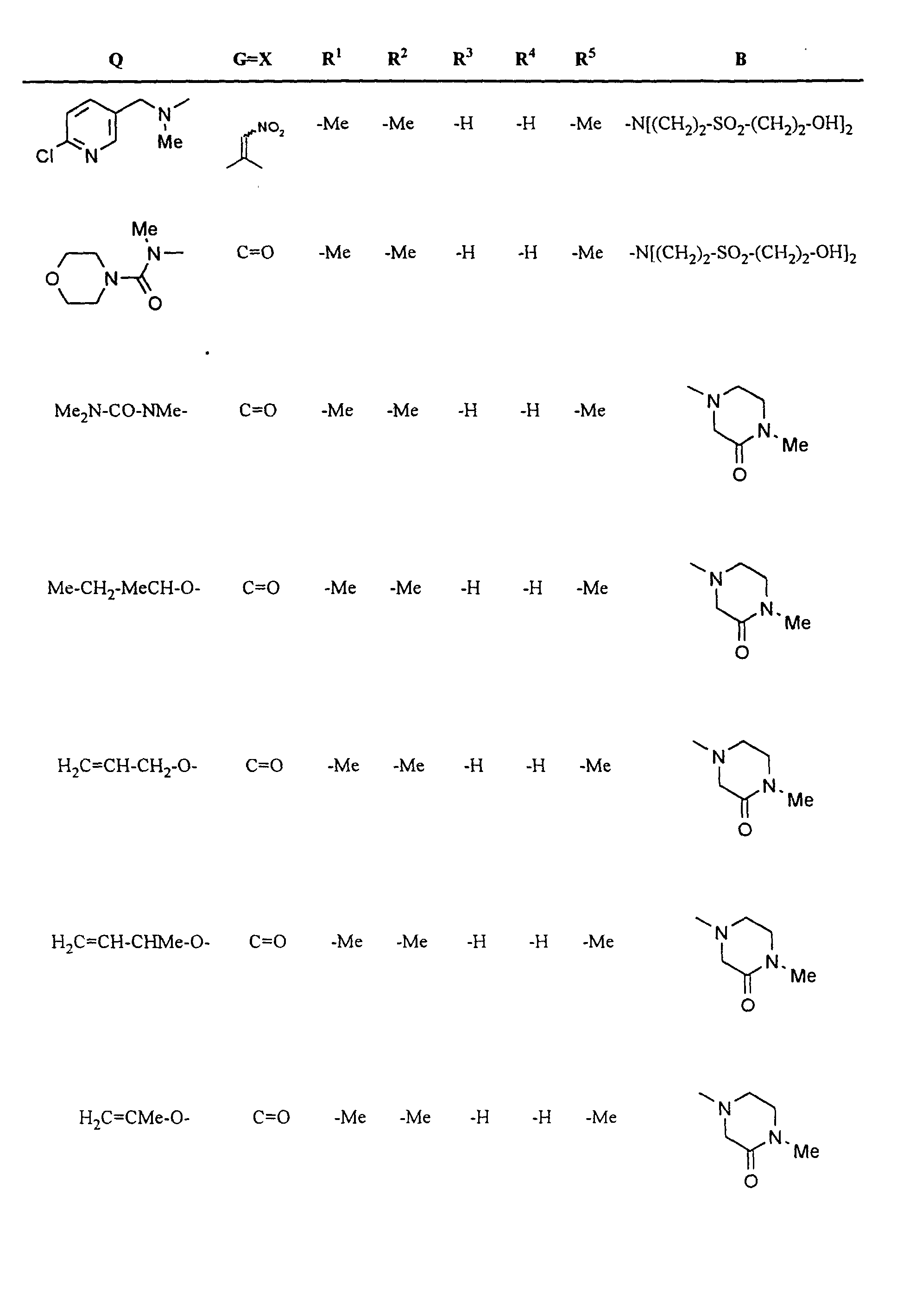

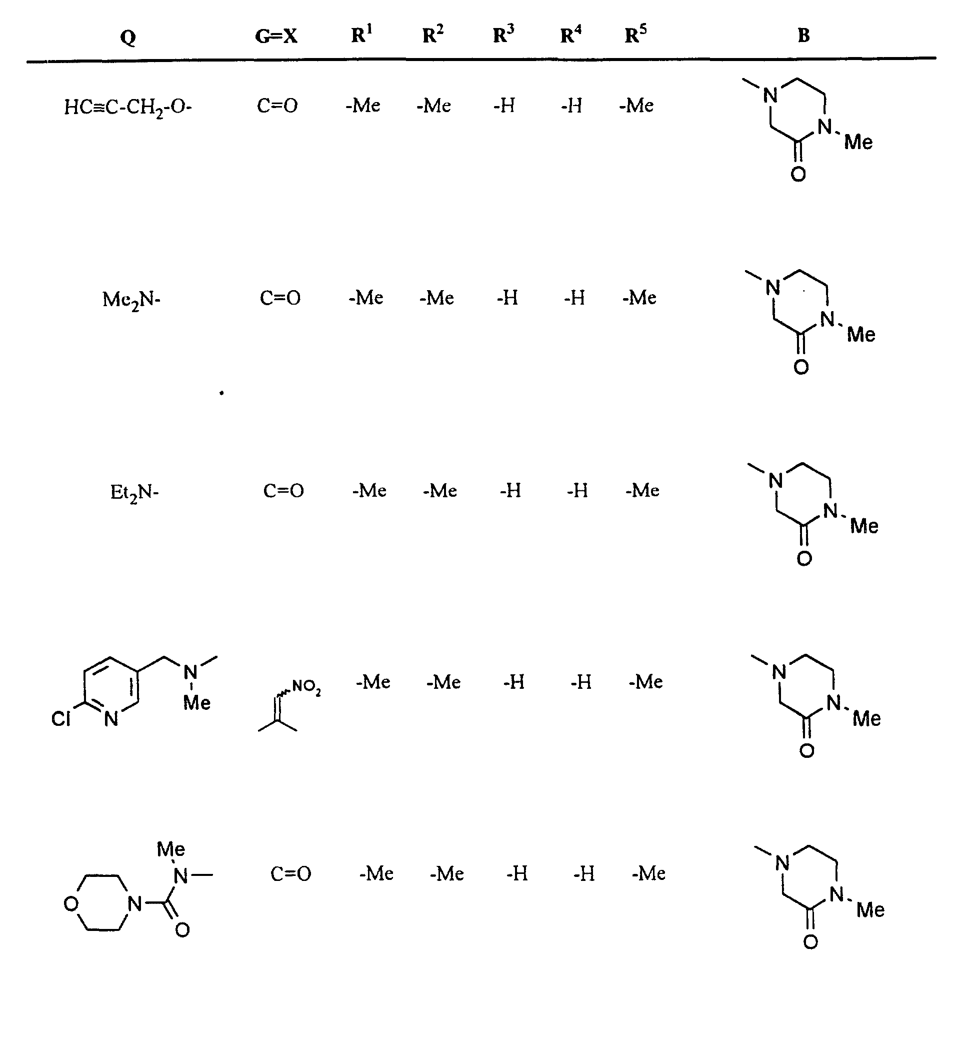

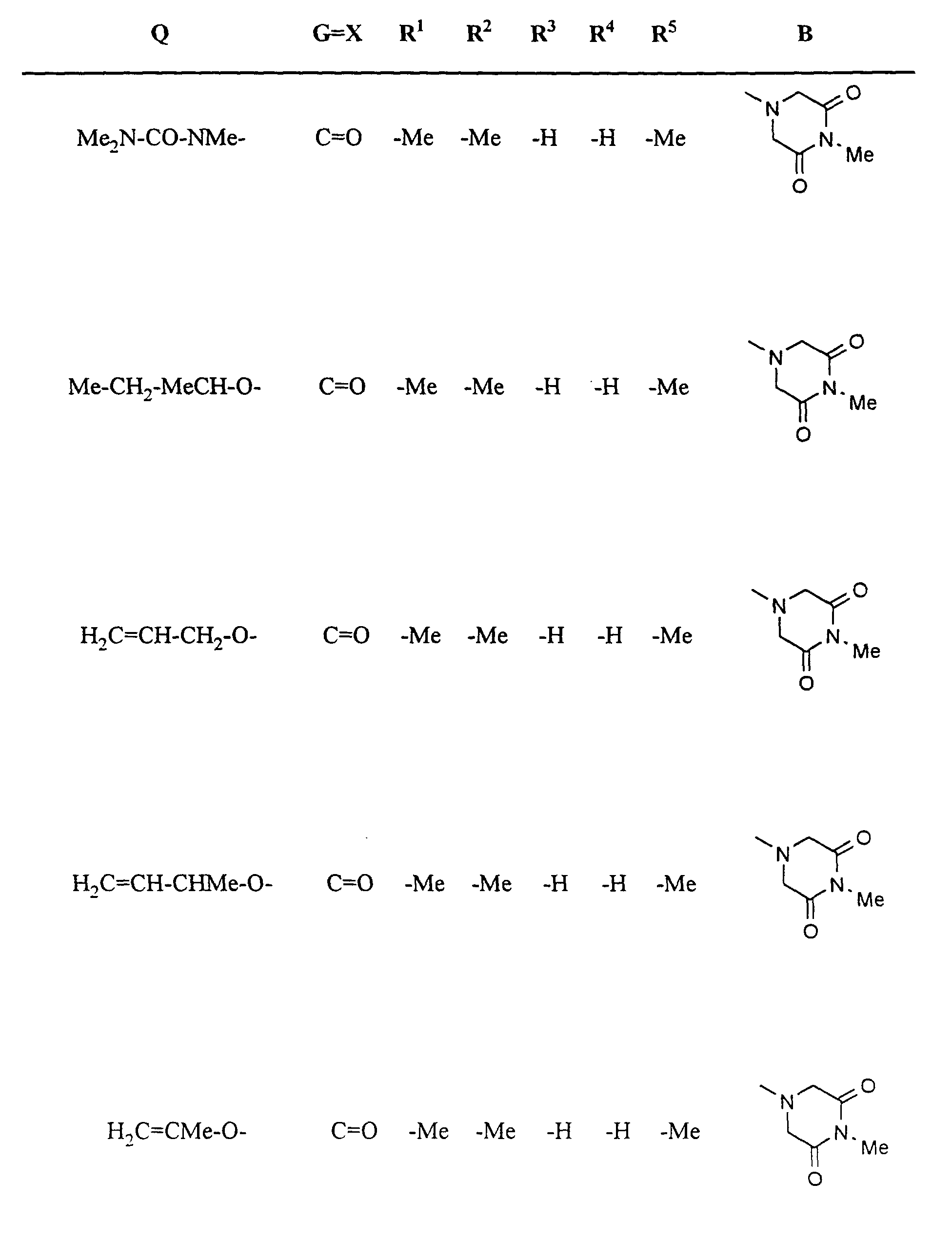

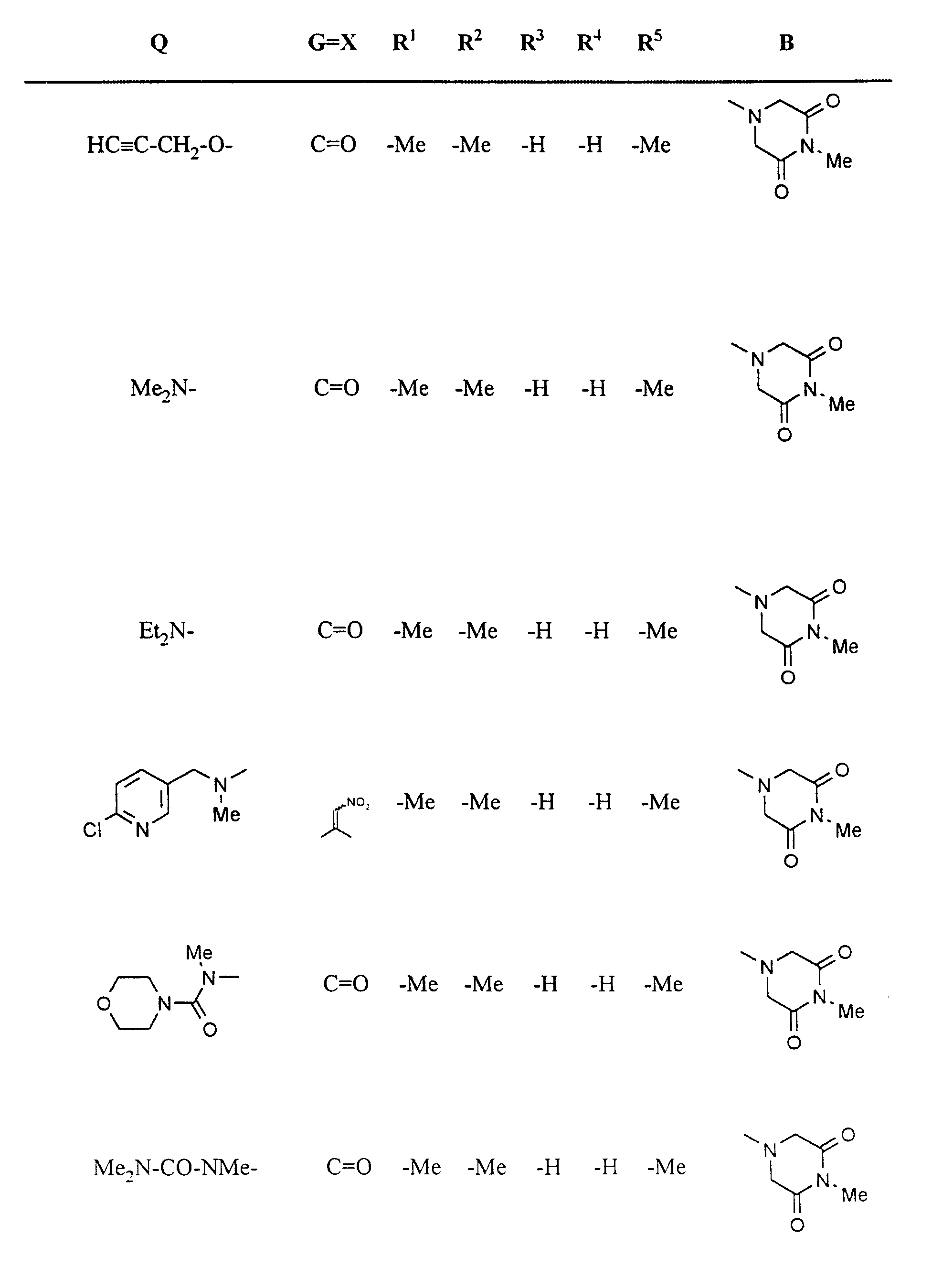

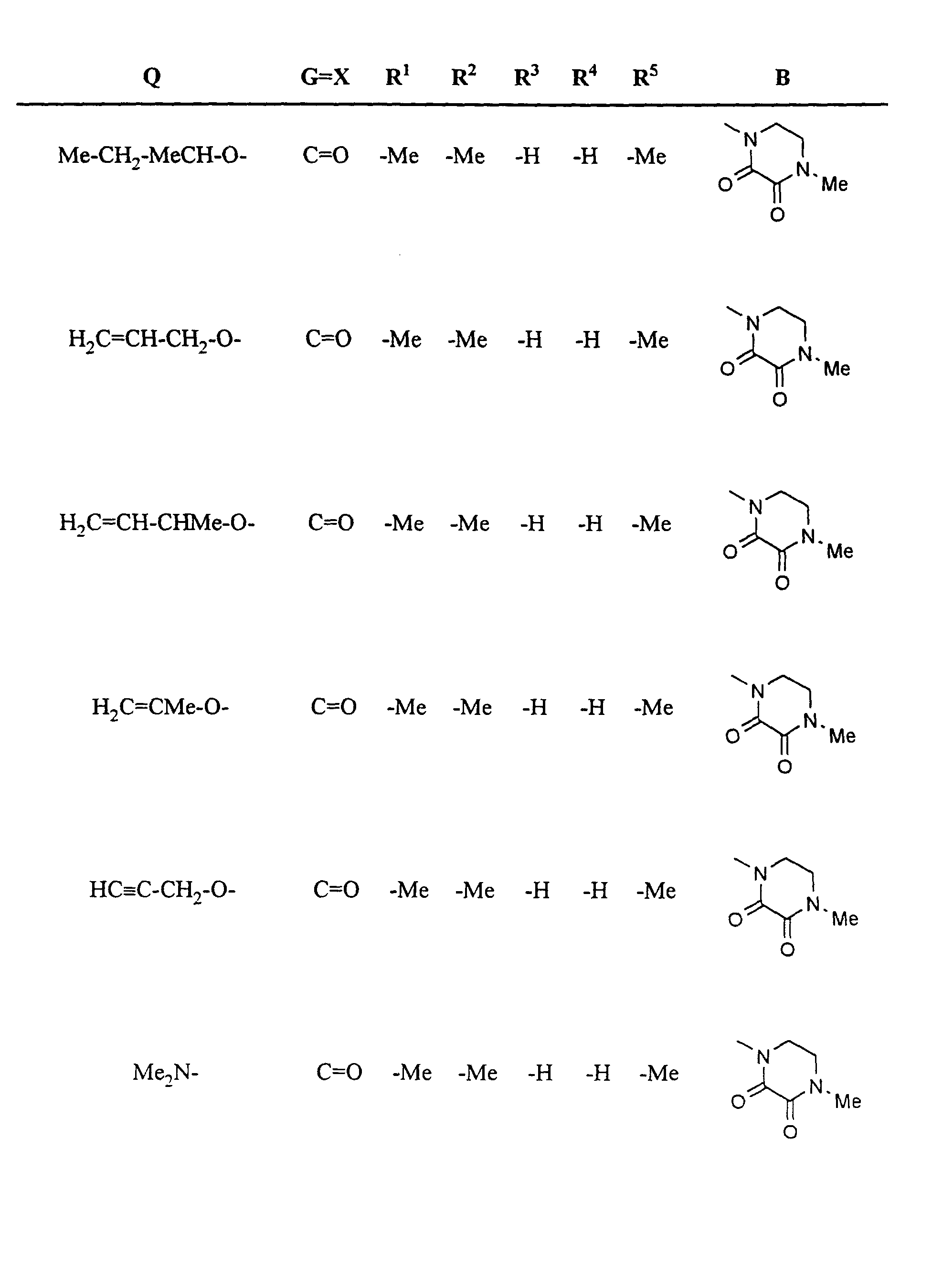

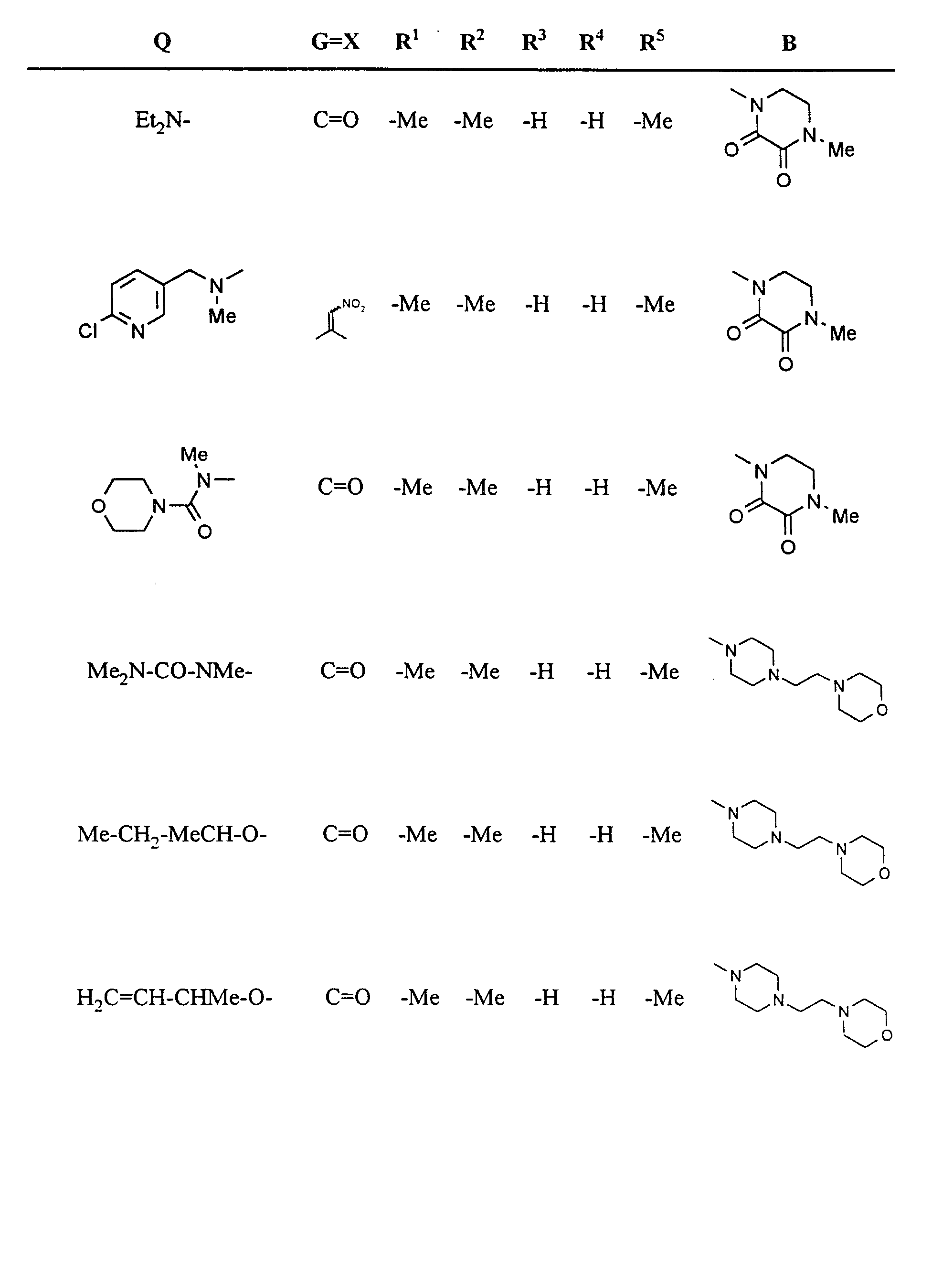

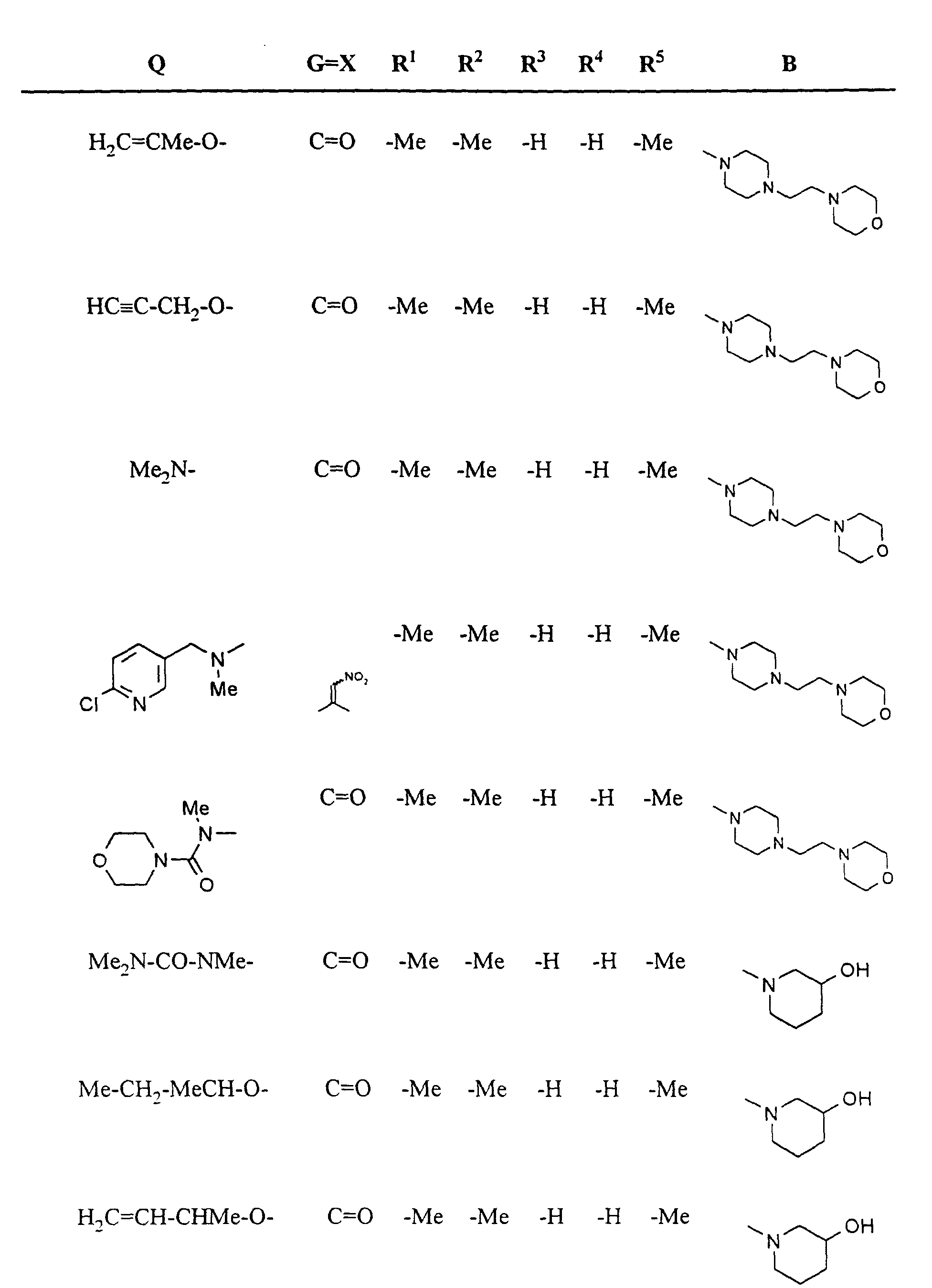

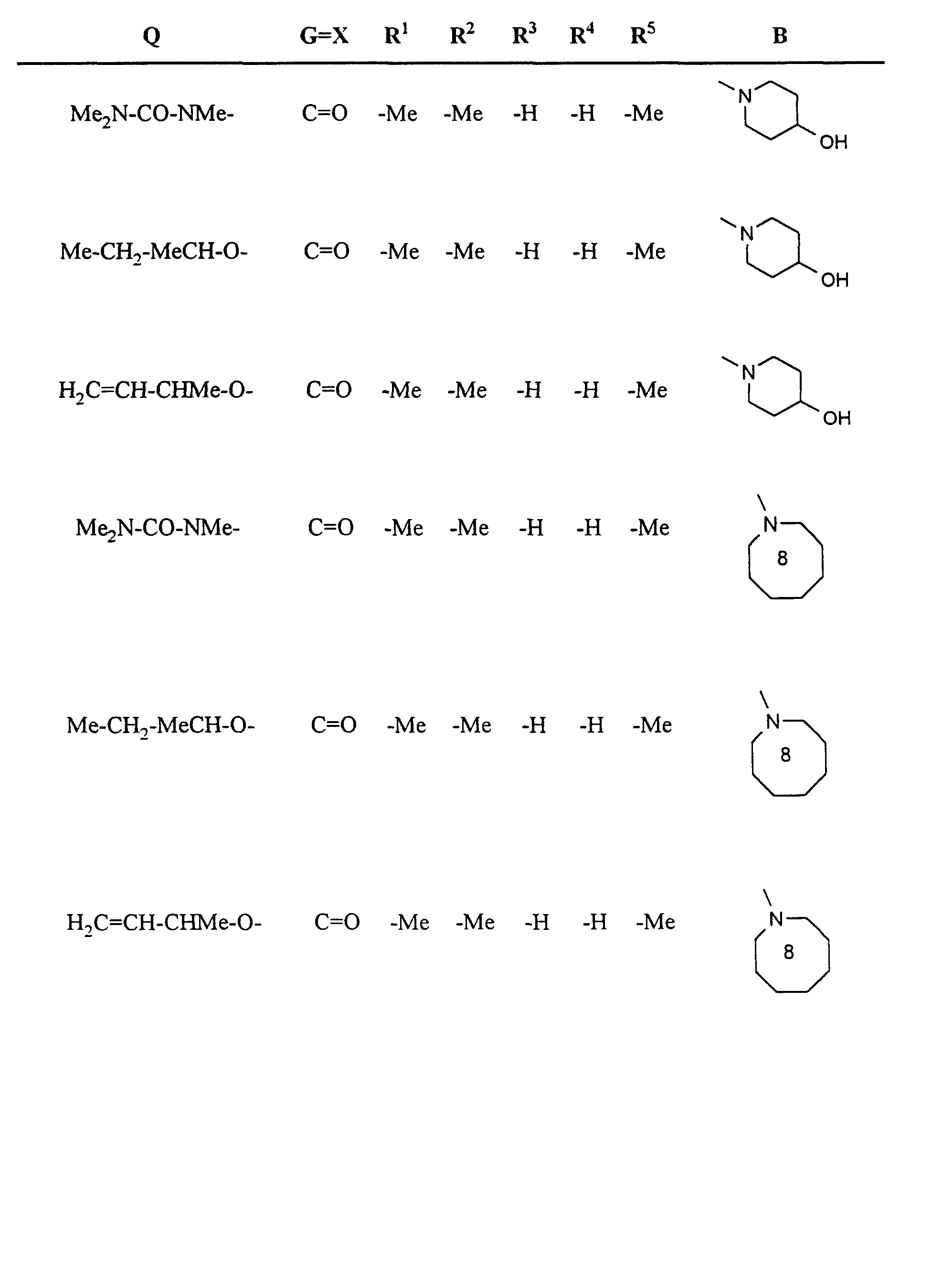

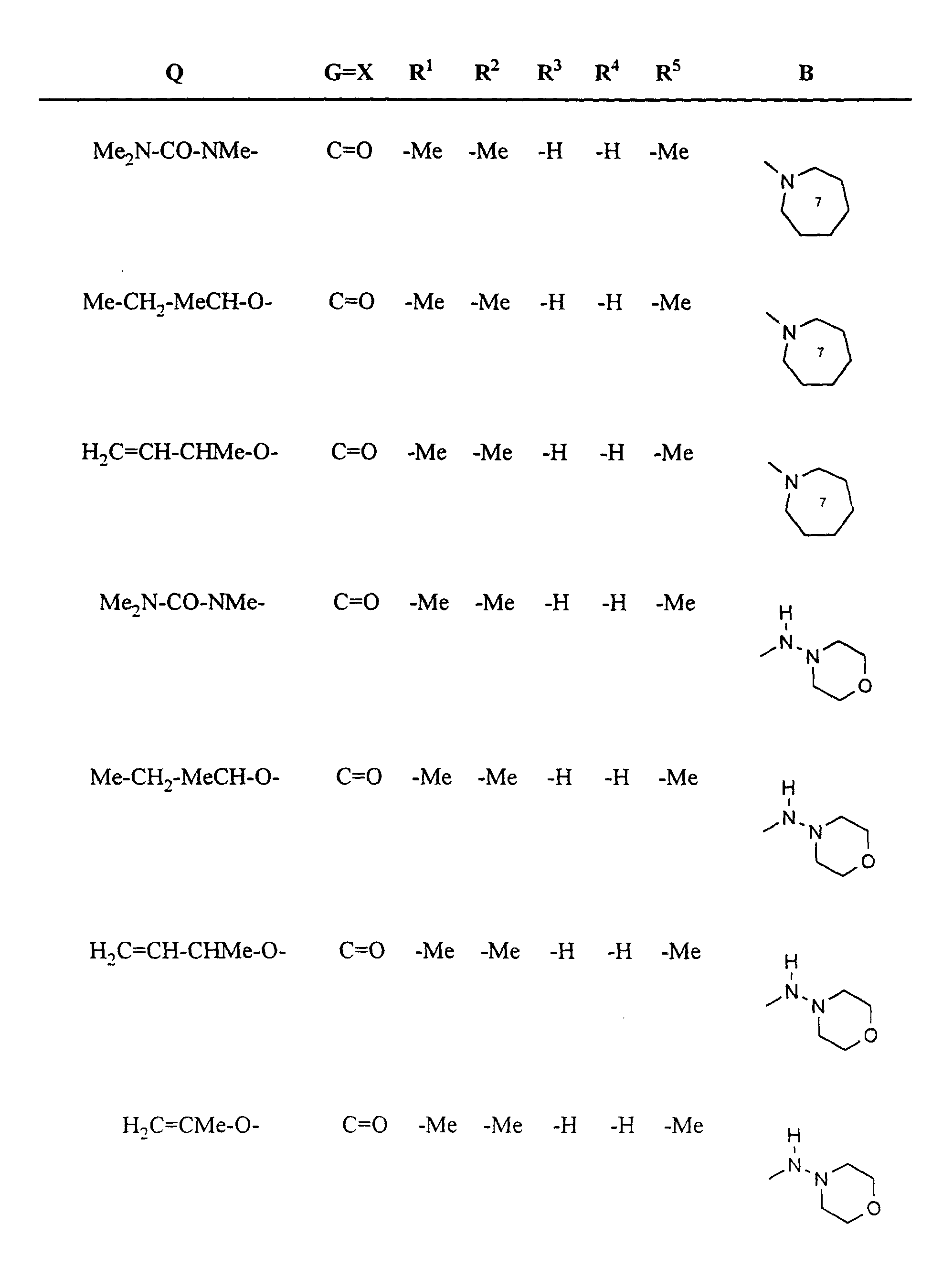

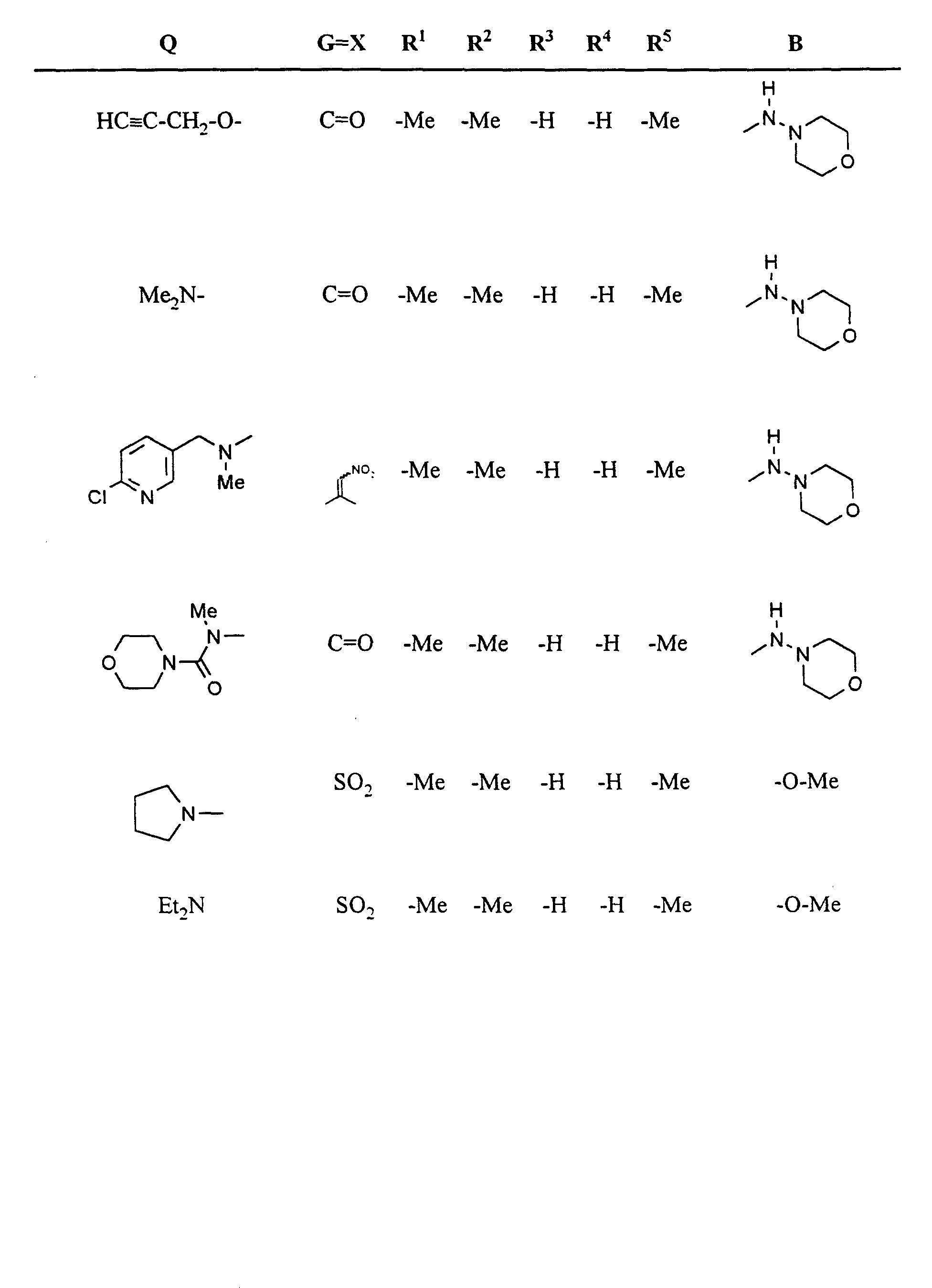

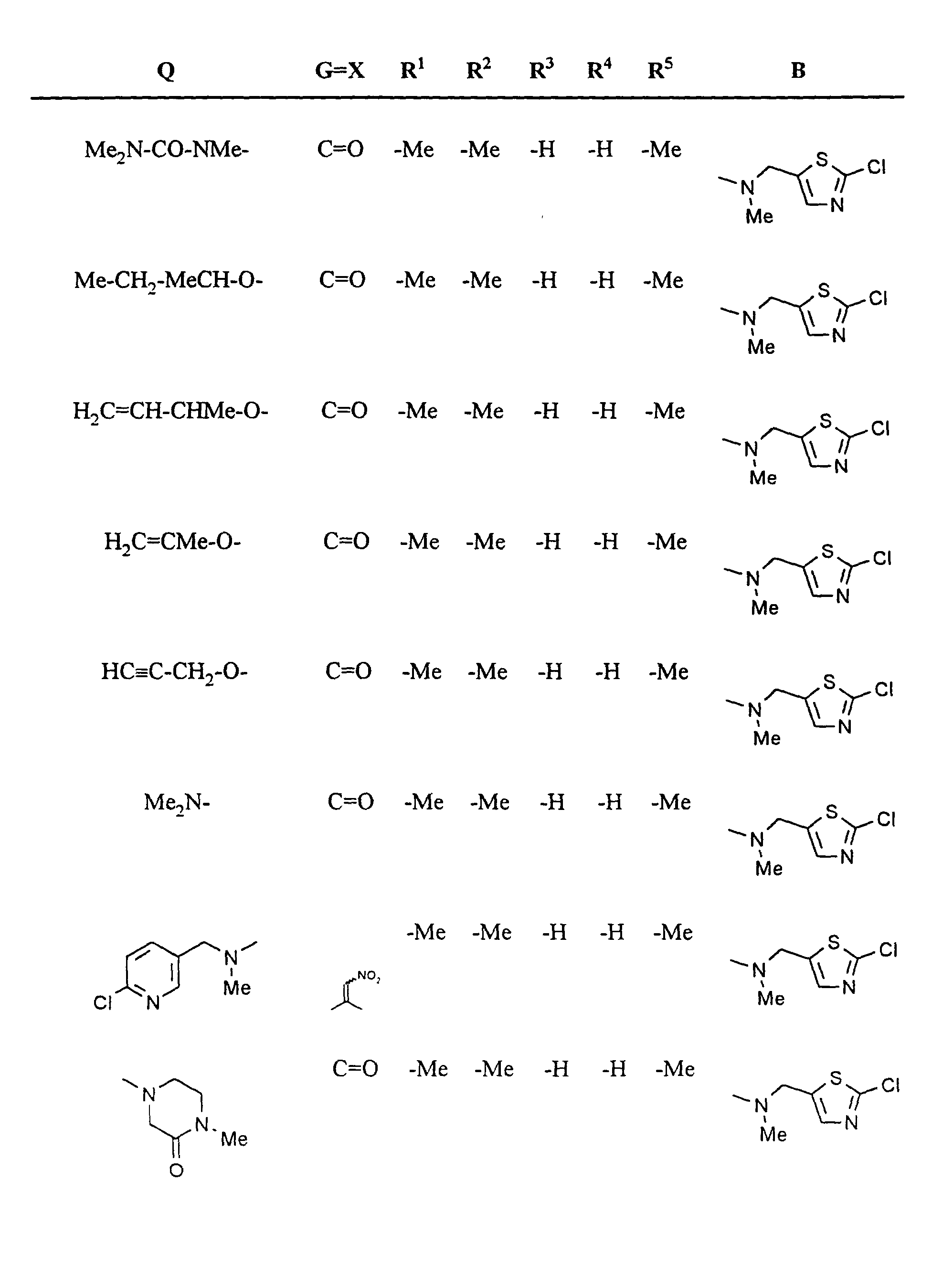

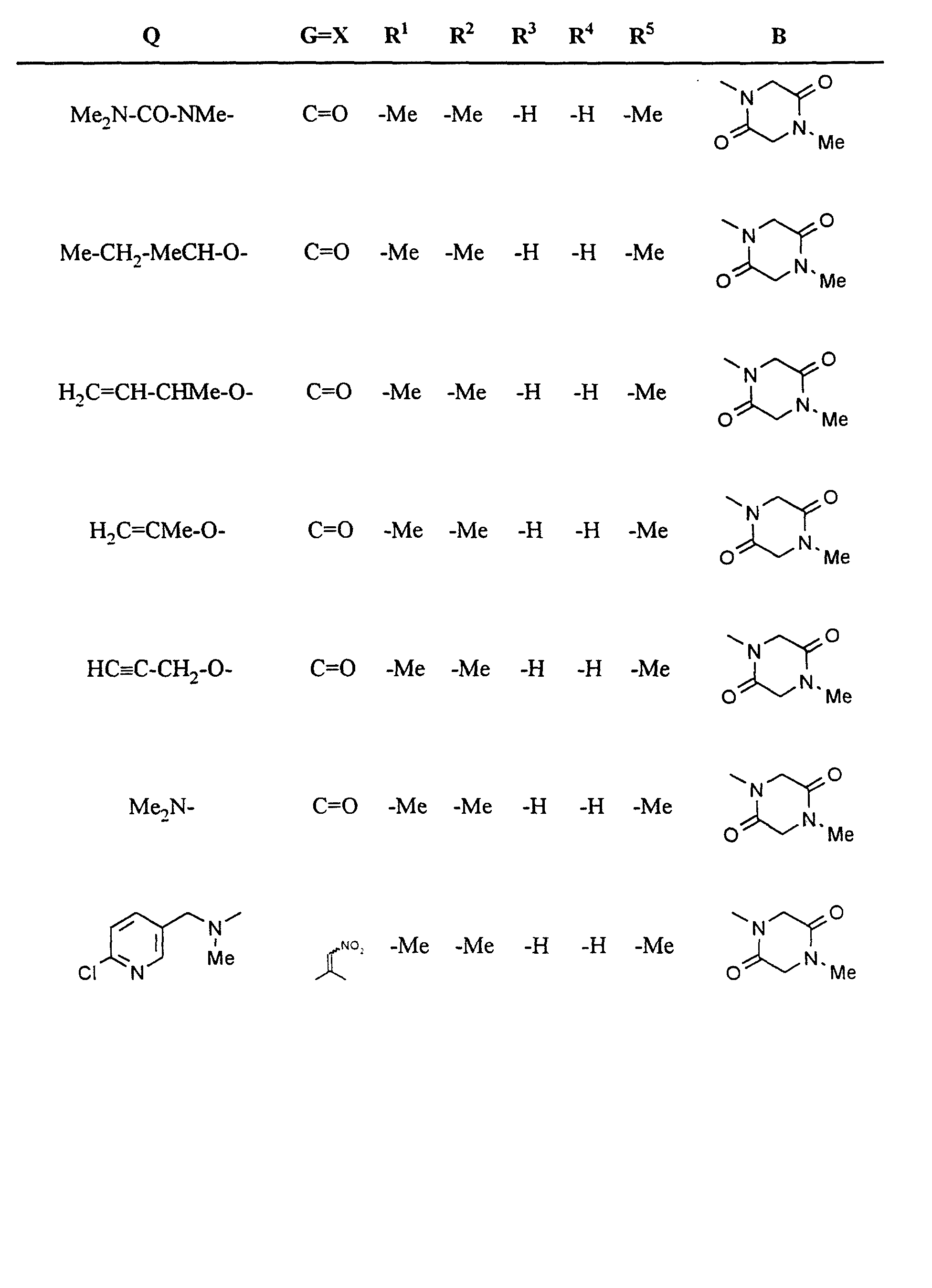

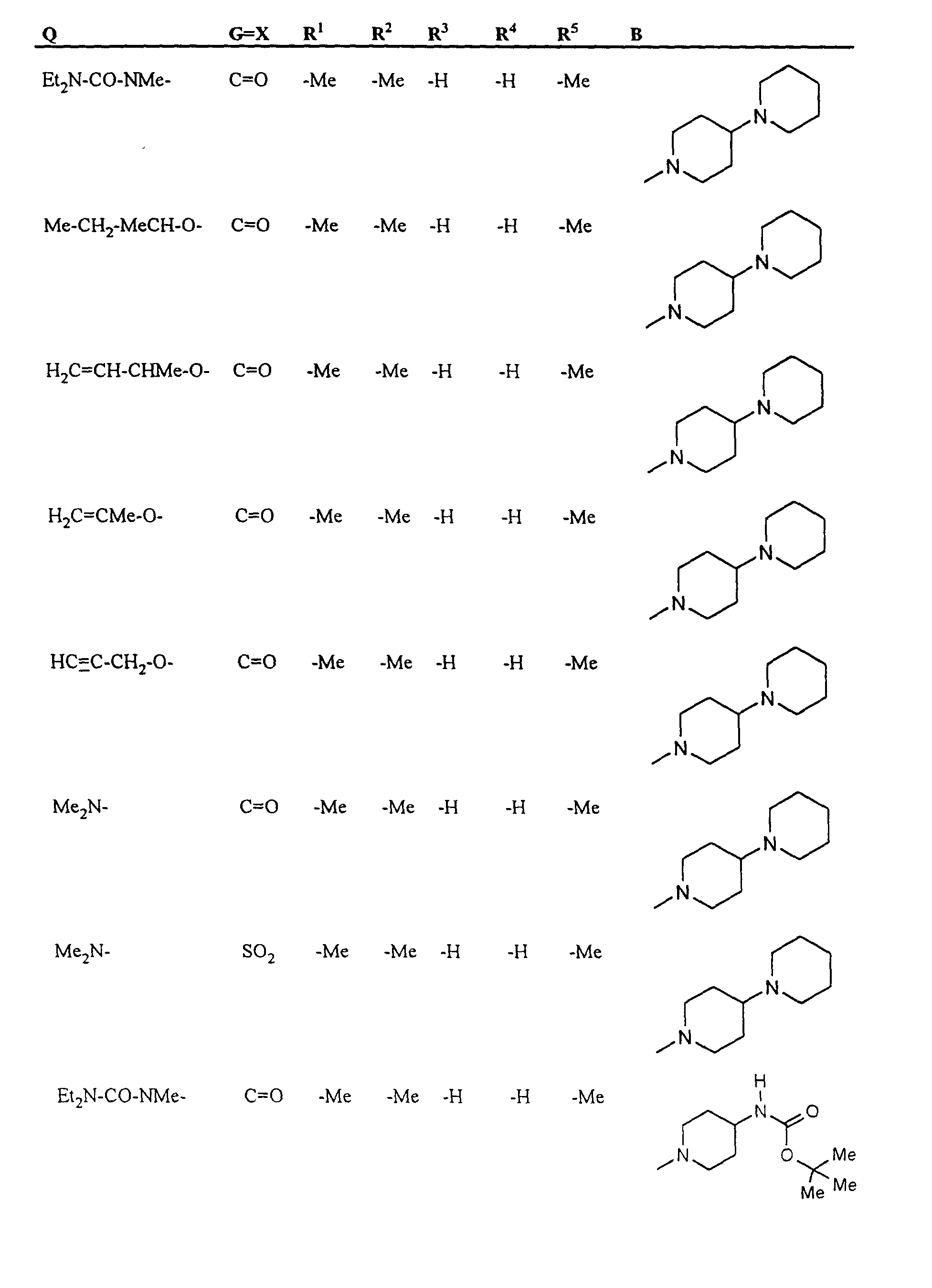

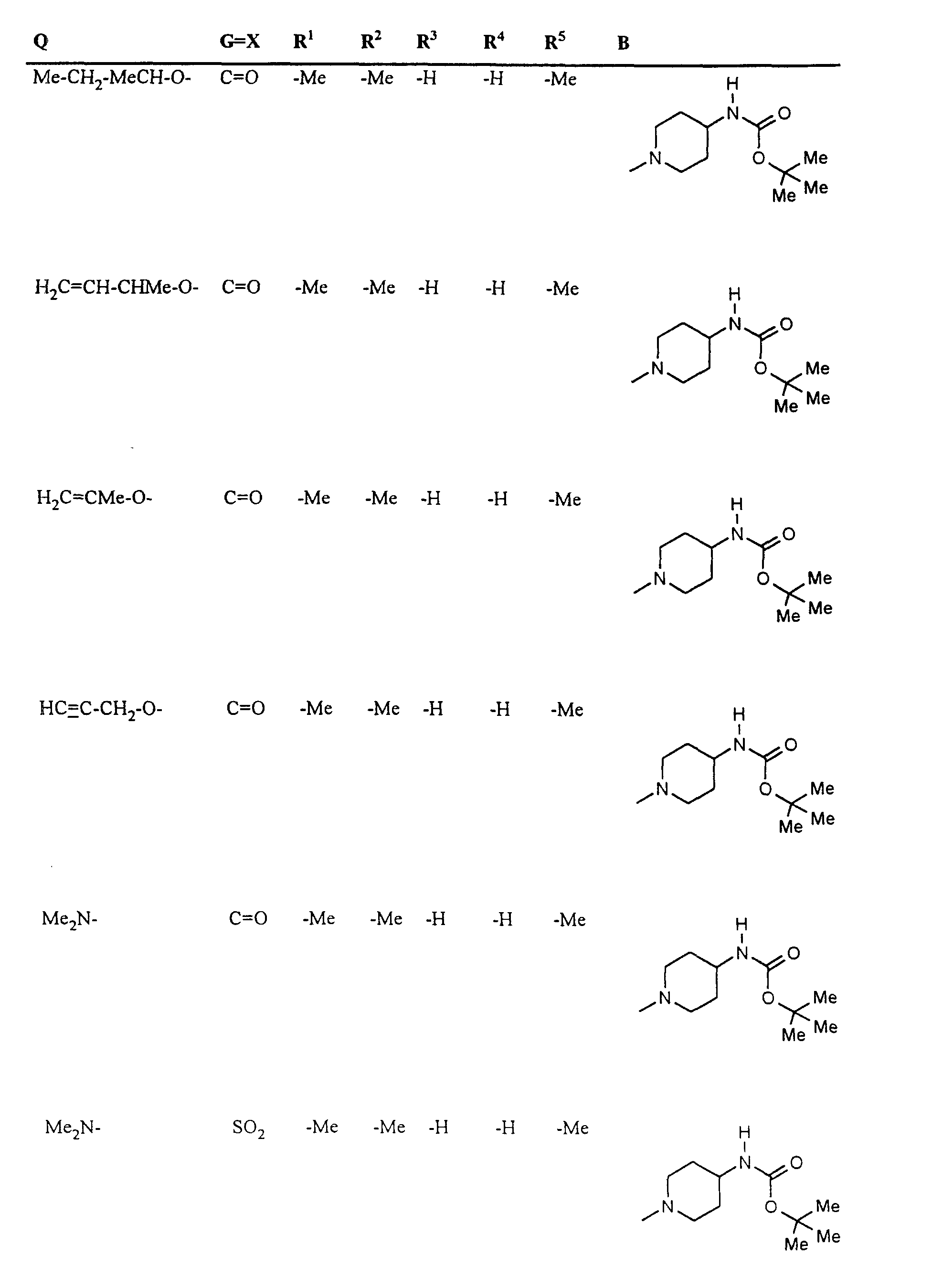

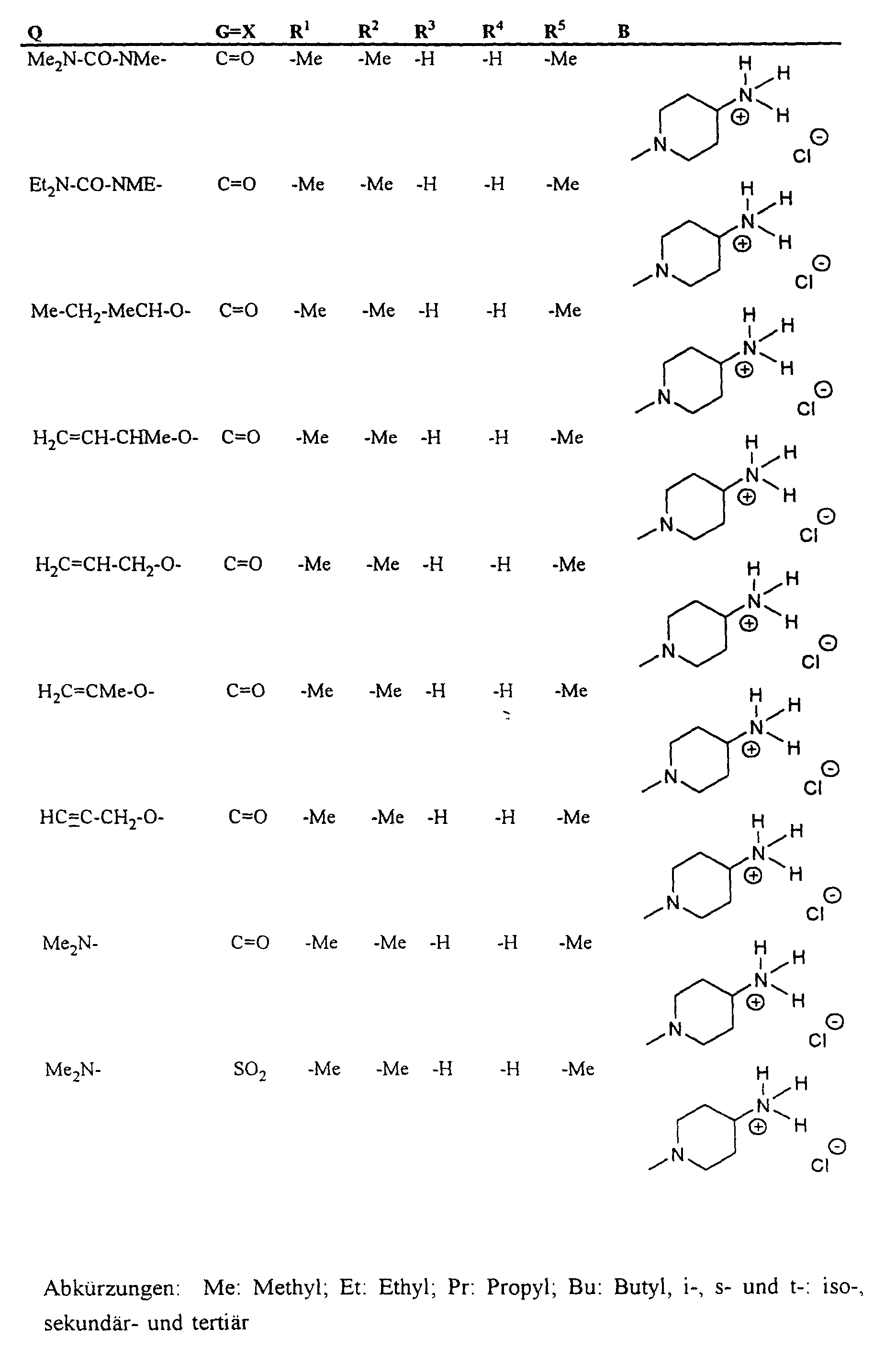

Setzt man bei Verfahren 3a zur Herstellung der neuen Didepsipeptide (Ia) als Verbindungen der allgemeinen Formel (II) N-Methyl-N-trimethylallophanoyl-Lalanin und als Verbindungen der allgemeinen Formel (III) D-Milchsäureisobutylester ein, so läßt sich das Verfahren durch folgendes Reaktionsschema wiedergeben:

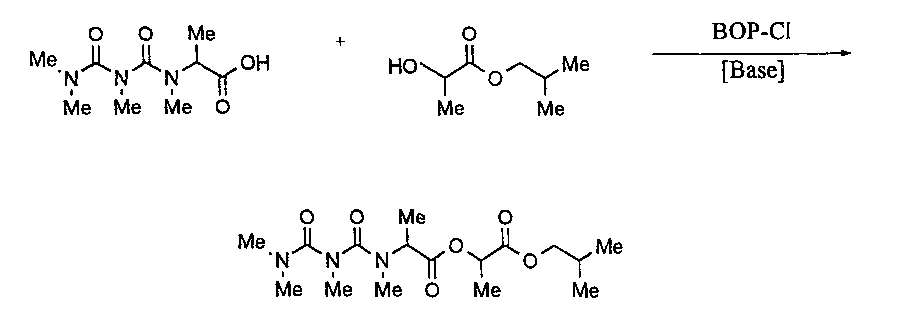

Die zur Durchführung des erfindungsgemäßen Verfahrens 3a als Ausgangsstoffe benötigten N-terminal acylierten N-Alkyl-aminosäuren sind durch die Formel (II) allgemein definiert. In dieser Formel stehen R¹, R², R³, G, Q und X vorzugsweise für diejenigen Reste, die bereits im Zusammenhang mit der Beschreibung der erfindungsgemäßen Stoffe der allgemeinen Formel (II) als bevorzugt für diese Substituenten genannt werden.

Die als Ausgangsmaterialien verwendeten N-acylierten N-Alkyl-aminosäuren der allgemeinen Formel (II) sind teilweise bekannt (vgl. z.B.: N-Methylaminosäuren: R. Bowmann et al. J. Chem. Soc. (1950) S. 1346; J. R. McDermott et al. Can. J. Chem. 51 (1973) S. 1915; H. Wurziger et al. Kontakte (Merck, Darmstadt) 3 (1987) S. 8) bzw. können nach den dort beschriebenen Verfahren erhalten werden.

Die außerdem für die Durchführung des erfindungsgemäßen Verfahrens 3a als Ausgangsstoffe zu verwendenden Carbonsäurederivate sind allgemein durch die Formel (III) definiert.

In der Formel (III) haben R⁴, R⁵, B und Z die Bedeutung die bereits im Zusammenhang mit der Beschreibung der erfindungsgemäßen Stoffe der allgemeinen Formel (Ia) als bevorzugt für diese Substituenten genannt wurden.

Die Verbindungen der Formel (III) sind allgemein bekannte Verbindungen der organischen Chemie bzw. können nach literaturbekannten Methoden erhalten werden (z.B.: 2-Hydroxycarbonsäurederivate: vgl. Houben-Weyl, Methoden der organischen Chemie, Band VIII; 2-Halogencarbonsäurederivate: S. M. Birnbaum et al. J. Amer. Chem. Soc. 76 (1954) S. 6054, C. S. Rondestvedt, Jr. et al. Org. Reactions 11 (1960) S. 189 [Review]) bzw. können nach den dort beschriebenen Verfahren erhalten werden.

Die Umsetzung der N-acylierten N-Alkylaminosäuren (II) mit 2-Hydroxycarbonsäurederivaten (III) führt man vorzugsweise in Gegenwart von Kupplungsreagenzien und in Gegenwart eines basischen Reaktionshilfsmittels unter Verwendung von Verdünnungsmitteln durch.

Als Kupplungsreagenzien zur Durchführung des Verfahrens 3a finden alle, die zur Herstellung einer Amidbindung geeignet sind (vgl. z.B.: Houben-Weyl, Methoden der organischen Chemie, Band 15/2; Bodanszky et al., Peptide Synthesis 2nd ed. (Wiley & Sons, New York 1976) oder Gross, Meienhofer, The Peptides: Analysis Synthesis, Biology (Academic Press, New York 1979), Verwendung. Vorzugsweise werden folgende Methoden herangezogen: Aktivestermethode mit Pentachlor- (Pcp) und Pentafluorphenol (Pfp), N-Hydroxysuccinimid, N-Hydroxy-5-norbornen-2,3-dicarbox-amid (HONB), 1-Hydroxy-benzotriazol (HOBt) oder 3-Hydroxy-4-oxo-3,4-dihydro1,2,3-benzotriazin als Alkoholkomponente, Kupplung mit Carbodiimiden wie Dicyclohexyl-carbodiimid (DCC) nach dem DCC-Additiv-Verfahren, oder mit n-Propanphosphonsäureanhydrid (PPA) und Gemischt-Anhydrid-Methode mit Pivaloylchlorid, Ethyl- (EEDQ) und Isobutyl-chlorformiat (IIDQ) oder Kupplung mit Phosphoniumreagenzien, wie Benzotriazol-1-yl-oxytris(dimethylamino-phosphonium)-hexafluorophosphat (BOP), Bis(2-oxo-3-oxazolidinyl)phosphoniumsäurechlorid (BOP-Cl), oder mit Phosphonsäureesterreagenzien, wie Cyanphosphonsäurediethylester (DEPC) und Diphenylphosphorylazid (DPPA) oder Uroniumreagenzien, wie 2-(1H-Benzotriazol-1-yl)-1,1,3,3-tetramethyluroniumtetrafluoroborat (TBTU).

Bevorzugt ist die Kupplung mit Phosphoniumreagenzien wie Bis(2-oxo-3-oxazolidinyl)-phosphoniumsäurechlorid (BOP-Cl), Benzotriazol-1-yl-oxy-tris(dimethylamino-phosphonium)-hexafluorophosphat (BOP) und Phosphonsäureesterreagenzien, wie Cyanphosphonsäurediethylester (DEPC) oder Diphenylphosphorylazid (DPPA).

Als basische Reaktionshilfsmittel zur Durchführung der erfindungsgemäßen Verfahrens 3a können alle geeigneten Säurebindemittel eingesetzt werden wie Amine, insbesondere tertiäre Amine sowie Alkali- und Erdalkaliverbindungen.

Beispielhaft seien dafür erwähnt die Hydroxide, Oxide und Carbonate des Lithiums, Natriums, Kaliums, Magnesiums, Calciums und Bariums, ferner weitere basische Verbindungen wie Amidinbasen oder Guanidinbasen wie 7-Methyl-1,5,7-triazabicyclo(4.4.0)dec-5-en (MTBD); Diazabicyclo(4.3.0)nonen (DBN), Diazabicyclo(2.2.2)-octan (DABCO), 1,8-Diaza-bicyclo(5.4.0)undecen (DBU) Cyclohexyl-tetrabutylguanidin (CyTBG), Cyclohexyltetramethylguanidin (CyTMG), N,N,N,N-Tetramethyl-1,8-naphthalindiamin, Pentamethylpiperidin, tertiäre Amine wie Triethylamin, Trimethylamin, Tribenzylamin, Triisopropylamin, Tributylamin, Tribenzylamin, Tricyclohexylamin, Triamylamin, Trihexylamin, N,N-Dimethyl-anilin, N,N-Dimethyl-toluidin, N,N-Dimethyl-p-aminopyridin, N-Methyl-pyrrolidin, N-Methyl-piperidin, N-Methyl-imidazol, N-Methyl-pyrrol, N-Methyl-morpholin, N-Methyl-hexamethylenimin, Pyridin, 4-Pyrrolidinopyridin, 4-Dimethylamino-pyridin, Chinolin, α-Picolin, α-Picolin, Isochinolin, Pyrimidin, Acridin, N,N,N',N'-Tetramethylendiamin, N,N',N'-Tetra-ethylendiamin, Chinoxalin, N-Propyl-diisopropylamin, N-Ethyl-diisopropylamin, N,N'-Dimethylcyclohexylamin, 2,6-Lutidin, 2,4-Lutidin oder Triethylendiamin.

Vorzugsweise finden tertiären Amine, insbesondere Trialkylamine wie Triethylamin, N,N-Diisopropylethylamin, N-Propyl-diisopropylamin, N,N'-Dimethyl-cyclohexylamin oder N-Methylmorpholin, Verwendung.

Im allgemeinen ist es vorteilhaft das erfindungsgemäße Verfahren 3a in Gegenwart von Verdünnungssmitteln durchzuführen. Verdünnungsmittel werden vorteilhafterweise in einer solchen Menge eingesetzt, daß das Reaktionsgemisch während des ganzen Verfahrens gut rührbar bleibt. Als Verdünnungsmittel zur Durchführung des erfindungsgemäßen Verfahrens 3a kommen alle inerten organischen Lösungsmittel in Frage.

Als Beispiele sind zu nennen: Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Dichlorbenzol, Chlortoluol, Trichlorbenzol; Alkohole wie Methanol, Ethanol, Isopropanol, Butanol; Ether wie Ethylpropylether, Methyl-tert.-butylether, n-Butylether, Anisol, Phenetol, Cyclohexylmethylether, Dimethylether, Diethylether, Dipropylesther, Diisopropylether, Di-n-butylether, Diisobutylether, Diisoamylether, Ethylenglycoldimethylether, Tetrahydrofuran, Dioxan, Dichlordiethylether und Polyether des Ethylenoxids und/oder Propylenoxids; Amine wie Trimethyl-, Triethyl-, Tripropyl-, Tributylamin, N-Methyl-morpholin, Pyridin und Tetramethylendiamin, Nitrokohlenwasserstoffe wie Nitromethan, Nitroethan, Nitropropan, Nitrobenzol, Chlornitrobenzol, o-Nitrotoluol; Nitrile wie Acetonitril, Propionitril, Butyronitril, Isobutyronitril, Benzonitril, m-Chlor-benzonitril sowie Verbindungen wie Tetrahydrothiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid; Sulfone wie Dimethyl-, Diethyl-, Dipropyl-, Dibutyl-, Diphenyl-, Dihexyl-, Methylethyl-, Ethylpropyl-, Ethylisobutyl- und Pentamethylensulfon; aliphatische, cycloaliphatische oder aromatische Kohlenwasserstoffe wie Pentan, Hexan, Heptan, Oktan, Nonan und technische Kohlenwasserstoffe, beispielsweise sogenannte White Spirits mit Komponenten mit Siedepunkten im Bereich beilspielsweise 40 bis 250°C, Cymol, Benzinfraktionen innerhalb eines Siedepunktintervalles von 70°C bis 190°C, Cyclohexan, Methylcyclohexan, Petrolether, Ligroin, Octan, Benzol, Toluol, Xylol; Ester wie Methyl-, Ethyl-, Butyl-, Isobutylacetat, sowie Dimethyl-, Dibutyl-, Ethylencarbonat; Amide wie Hexamethylenphosphortriamid, Formamid, N-Methylformamid, N,N-Dimethylformamid, N,N-Dipropylformamid, N,N-Dibutylformamid, N-Methyl-pyrrolidon, N-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, N-Formylpiperidin, N,N'-1,4-Diformylpiperazin; Ketone wie Aceton, Methylethylketon, Methylbutylketon.

Selbstverständlich kann man in das erfindungsgemäße Verfahren auch Gemische der genannten Lösungs- und Verdünnungsmittel einsetzen.

Bevorzugte Verdünnungsmittel sind Halogenkohlenwasserstoffe, insbesondere Chlorkohlenwasserstoffe, wie Methylenchlorid oder 1,2-Dichlorethan und Gemische von diesen mit anderen genannten Verdünnungsmitteln.

Das Verfahren 3a wird im allgemeinen so durchgeführt, indem man Verbindungen der Formel (II) in Gegenwart eines der angegebenen Kupplungsreagenzien und in Gegenwart eines der angegebenen basischen Reaktionshilfsmittel mit Verbindungen der allgemeinen Formel (III) in einem der angegebenen Verdünnungsmittel umsetzt. Die Reaktionsdauer beträgt 4 bis 72 Stunden. Die Umsetzung erfolgt bei Temperaturen zwischen -10°C und +120°C, bevorzugt zwischen -5°C und +50°C, besonders bevorzugt bei 0 °C bis Raumtemperatur. Es wird unter Normaldruck gearbeitet.

Zur Durchführung des erfindungsgemäßen Verfahrens 3a setzt man pro Mol an N-acylierter N-Alkylaminosäure der Formel (II) im allgemeinen 1,0 bis 3,0 Mol, vorzugsweise 1,0 bis 1,5 Mol Kupplungsreagenz, ein.

Nach vollendeter Umsetzung wird die Reaktionslösung gewaschen, die organische Phase abgetrennt, getrocknet und im Vakuum eingeengt. Die anfallenden Produkte lassen sich in üblicher Weise durch Umkristallisieren, Vakuumdestillation oder Säulenchromatographie reinigen (vgl. auch die Herstellungsbeispiele).

Alternativ können die erfindungsgemäßen Didepsipeptide auch nach klassischen Verfahren hergestellt werden, beispielsweise demjenigen, wie es von H.-G. Lerchen und H. Kunz (Tetrahedron Lett. 26 (43) (1985) S. 5257-5260; 28 (17) (1987) S. 1873-1876) unter Ausnutzung der Veresterungsmethode nach B. F. Gisin (Helv. Chim. Acta 56 (1973) s. 1476) beschrieben ist.

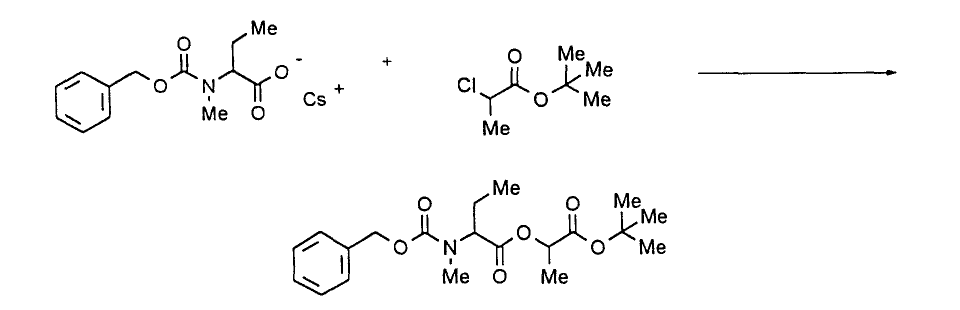

Setzt man bei Verfahren 3b zur Herstellung der neuen Didepsipeptide (Ia) als Verbindungen der allgemeinen Formel (Ib) N-Methyl-L-alanyl-D-milchsäuremethylester und als Verbindungen der allgemeinen Formel (IV) Trimethylallophansäurechlorid ein, so läßt sich das Verfahren durch folgendes Reaktionsschema wiedergeben:

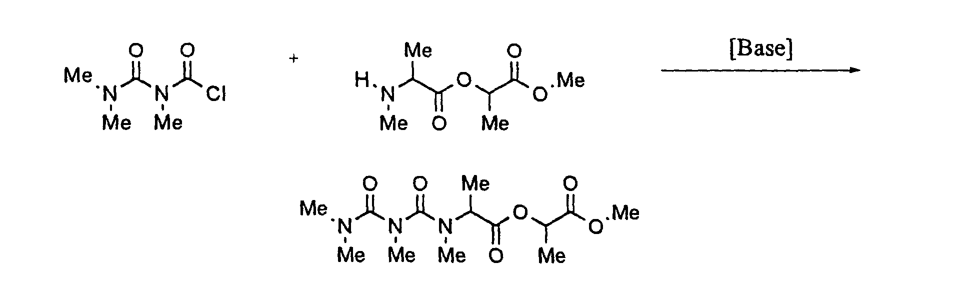

Die zur Durchführung des erfindungsgemäßen Verfahrens 3b als Ausgangsstoffe benötigten N-terminal deblockierten Didepsipeptide sind durch die Formel (Ib) allgemein definiert. In dieser Formel stehen R¹, R², R³, R⁴, R⁵ und B vorzugsweise für diejenigen Reste, die bereits im Zusammenhang mit der Beschreibung der erfindungsgemäßen Stoffe der allgemeinen Formel (Ia) als bevorzugt für diese Substituenten genannt werden.

Die als Ausgangsmaterialien verwendeten N-terminal deblockierten Didepsipeptide der allgemeinen Formel (Ib) sind teilweise bekannt (vgl. DE-OS 4 341 991, DE-OS 4 341 992, DE-OS 4 341 992) bzw. können nach den dort beschriebenen Verfahren aus N-terminal geschützten Didepsipeptiden erhalten werden.

Die außerdem für die Durchführung des erfindungsgemäßen Verfharens 3b als Ausgangsstoffe zu verwendenden Verbindungen sind allgemein durch die Formel (IV) definiert.

In der Formel (IV) haben G, X, Y, und W die Bedeutung die bereits im Zusammenhang mit der Beschreibung der erfindungsgemäßen Stoffe der allgemeinen Formel (Ia) als bevorzugt für diese Substituenten genannt wurden.

Die Verbindungen der Formel (IV) sind allgemein bekannte Verbindungen der organischen Chemie bzw. können nach literaturbekannten Methoden erhalten werden (z.B.: persubstituierte Allophansäurehalogenide: DE-OS 2 008 116; Carbamoylchloride: Liebigs Ann. 299, S. 85; Carbamate: Houben-Weyl, Methoden der organischen Chemie, Band E 4).

Die Umsetzung der Verbindungen (Ib) mit (IV) führt man vorzugsweise in Gegenwart eines basischen Reaktionshilfsmittels unter Verwendung von Verdünnungsmitteln durch.

Als Verdünnungsmittel zur Durchführung des erfindungsgemäßen Verfahrens 3b finden die bei Verfahren 3a genannten inerten, aprotischen Lösungsmittel wie z.B. Dioxan, Acetonitril oder Tetrahydrofuran aber auch Halogenkohlenwasserstoffe, insbesondere Chlorkohlenwasserstoffe, wie Methylenchlorid, Verwendung.

Als basische Reaktionshilfsmittel zur Durchführung des erfindungsgemäßen Verfahrens 3b können alle bei Verfahren 3a genannten Säurebindemittel, vorzugsweise jedoch tertiären Amine, insbesondere Trialkylamine wie Triethylamin, N,N-Diisopropylethylamin, N-Propyl-diisopropylamin, N,N'-Dimethyl-cyclohexylamin oder N-Methylmorpholin, Verwendung finden.

Das Verfahren 3b wird durchgeführt, indem man Verbindungen der allgemeinen Formel (Ib) in Gegenwart eines basischen Reaktionshilfsmittels mit Verbindungen der allgemeinen Formel (IV) in einem der angegebenen Verdünnungsmittel umsetzt.

Die Reaktionsdauer beträgt 4 bis 72 Stunden. Die Umsetzung erfolgt bei Temperaturen zwischen -10°C und +150°C, bevorzugt zwischen -5°C und +80°C, besonders bevorzugt bei 0°C bis Raumtemperatur. Es wird unter Normaldruck gearbeitet. Zur Durchführung des erfindungsgemäßen Verfahrens 3b setzt man pro Mol an N-Alkylaminosäure der Formel (Ib) im allgemeinen 1,0 bis 3,0 Mol, vorzugsweise 1,0 bis 1,5 Mol Acylierungsagenz, ein.

Nach vollendeter Umsetzung wird die Reaktionslösung gewaschen, die organische Phase abgetrennt, getrocknet und im Vakuum eingeengt. Die anfallenden Produkte lassen sich in üblicher Weise durch Umkristallisieren, Vakuumdestillation oder Säulenchromatographie reinigen (vgl. auch die Herstellungsbeispiele).

Setzt man bei Verfahren 3c zur Herstellung der neuen Didepsipeptide (Ia) als Verbindungen der allgemeinen Formel (Ib) N-Methyl-L-alanyl-D-milchsäure-tert-butylester und als Verbindungen der allgemeinen Formel (V) Trichloracetylisocyanat ein, so läßt sich das Verfahren durch folgendes Reaktionsschema wiedergeben:

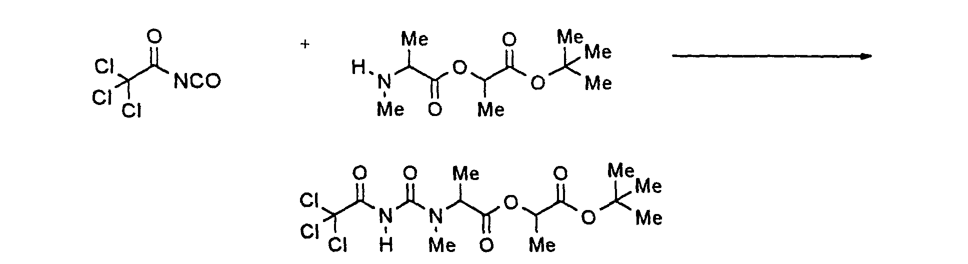

Die zur Durchführung des erfindungsgemäßen Verfahrens 3c als Ausgangsstoffe benötigten N-terminal deblockierten Didepsipeptide sind durch die Formel (Ib) allgemein definiert. In dieser Formel stehen R¹, R², R³, R⁴, R⁵ und B vorzugsweise für diejenigen Reste, die bereits im Zusammenhang mit der Beschreibung der erfindungsgemäßen Stoffe der allgemeinen Formel (Ia) als bevorzugt für diese Substituenten genannt werden.

Die als Ausgangsmaterialien verwendeten N-terminal deblockierten Didepsipeptide der allgemeinen Formel (Ib) sind teilweise bekannt (vgl. DE-OS 4 341 991, DE-OS 4 341 992, DE-OS 4 341 992) bzw. können nach den dort beschriebenen Verfahren aus N-terminal geschützten Didepsipeptiden erhalten werden.

Die außerdem für die Durchführung des erfindungsgemäßen Verfahrens 3c als Ausgangsstoffe zu verwendenden Verbindungen sind allgemein durch die Formeln (V) und (VI) definiert.

In der Formel (VI) haben R⁸, Y, G¹, X¹ und X die Bedeutung die bereits im Zusammenhang mit der Beschreibung der erfindungsgemäßen Stoffe der allgemeinen Formel (Ia) als bevorzugt für diesen Substituenten genannt wurden.

Die Verbindungen der Formel (VI) sind allgemein bekannte Verbindungen der organischen Chemie, können z.T. kommerziell oder nach literaturbekannten Methoden erhalten werden (Houben-Weyl, Methoden der organischen Chemie, Band E4).

Die Umsetzung der Verbindungen (Ib) mit (VI) nach dem erfindungsgemäßen Verfahren 3c führt man vorzugsweise in Gegenwart von Verdünnungsmitteln, gegebenenfalls in Gegenwart eines basischen Reaktionshilfsmittels, durch.

Als Verdünnungsmittel zur Durchführung des erfindungsgemäßen Verfahrens 3c finden die bei Verfahren 3a genannten Lösungsmittel wie z.B. Nitrile wie Acetonitril, Propionitril, Butyronitril, insbesondere Acetonitril, und Ether wie Ethylpropylether, n-Butylether, Diethylether, Dipropylesther, Diisopropylether, Di-n-butylether, Diisobutylether, Diisoamylether, Tetrahydrofuran, Dioxan, insbesondere Terahydrofuran und Dioxan, Verwendung.

Das Verfahren 3c kann auch in Gegenwart von basischen Reaktionshilfsmitteln durchgeführt werden. Als solche basischen Reaktionshilfsmittel zur Durchführung des erfindungsgemäßen Verfahrens 3c können alle bei Verfahren 3a genannten Säurebindemittel, vorzugsweise jedoch tertiären Amine, insbesondere Trialkylamine wie Triethylamin, N,N-Diisopropylethylamin oder N-Methylmorpholin, und Amidinbasen oder Guanidinbasen wie Diazabicyclo-(4.3.0)nonen (DBN), Diazabicyclo (2.2.2)-octan (DABCO), 1,8-Diaza-bicyclo(5.4.0)-undecen (DBU) insbesondere 1,8-Diazabicyclo(5.4.0)-undecen (DBU), Verwendung.

Das Verfahren 3c wird durchgeführt, indem Verbindungen der allgemeinen Formel (Ib) mit äquimolaren Mengen einer Verbindung der Formel (VI) in einem der weiter oben angegebenen Verdünnungsmittel, gegebenenfalls in Gegenwart eines basischen Reaktionshilfsmittels, zusammengegeben werden. Die Reaktionsdauer beträgt 1 bis 72 Stunden. Die Reaktion wird bei Temperaturen zwischen -50°C bis +200°C, bevorzugt in einem Temperaturbereich zwischen -20°C und +150°C, insbesondere in einem Temperaturbereich zwischen -10°C und +120°C durchgeführt.

Es kann grundsätzlich unter Normaldruck gearbeitet, aber auch bei erhöhtem oder erniedrigtem Druck gearbeitet werden. Vorzugsweise arbeitet man bei Normaldruck oder bei Drucken bis zu 15 bar. Bei höheren Temperaturen ist es vorteilhaft, unter erhöhtem Druck zu arbeiten, gegebenenfalls auch über 15 bar.

Nach vollendeter Umsetzung wird das Reaktionsgemisch nach allgemein üblichen Methoden aufgearbeitet (vgl. auch die Herstellungsbeispiele).

Verfahren zur Herstellung von organischen Carbamaten aus einem basisch reagierenden Amin, Kohlendioxid und einem Alkylierungsmittel in Gegenwart von basischen Alkalimetall-, Erdalkalimetall- oder Ammoniumsalzen sind bekannt (vgl. EP-OS 511 948, EP-OS 628 542 und dort zitierte Lit.).

Es wurde nun gefunden, daß auch die schwach basischen, erfindungsgemäßen, N-terminal deblockierten Didepsipeptide der Formel (Ib) als Aminoverbindungen mit Kohlendioxid und einem Alkylierungsmittel in Gegenwart von Metallcarbonaten zu Carbamaten der allgemeinen Formel (Ia) reagieren.

Setzt man bei Verfahren 3d zur Herstellung der neuen Didepsipeptide (Ia) als Verbindungen der allgemeinen Formel (Ib) N-Methyl-L-alanyl-D-milchsäure-tert-butylester, Kohlendioxid, Kaliumkarbonat und als Verbindungen der allgemeinen Formel (IX) Butylbromid ein, so läßt sich das Verfahren durch folgendes Reaktionsschema wiedergeben:

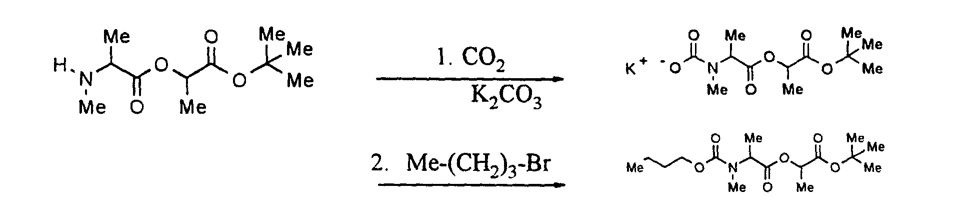

Bevorzugt werden bei bei Verfahren 3d die Didepsipeptide der Formel (Ib) eingesetzt, bei denen die Reste R¹, R², R³, R⁴, R⁵ und B die bei den Verbindungen der allgemeinen Formel (Ia) bevorzugten und besonders bevorzugten Bedeutungen besitzen.

Die als Ausgangsmaterialien verwendeten N-terminal deblockierten Didepsipeptide der allgemeinen Formel (Ib) sind teilweise bekannt (vgl. DE-OS 4 341 991, DE-OS 4 341 992, DE-OS 4 341 992) bzw. können nach den dort beschriebenen Verfahren aus N-terminal geschützten Didepsipeptiden erhalten werden.

Als Kohlendioxid kann in das erfindungsgemäße Verfahren das übliche Handelsprodukt, gegebenenfalls auch sogenanntes "Trockeneis", eingesetzt werden.

Die außerdem für die Durchführung des erfindungsgemäßen Verfahrens 3d als Ausgangsstoffe zu verwendenden Alkylierungsmittel der Formel (IX) sind allgemein bekannte Verbindungen der organischen Chemie. In der Formel (IX) hat R⁸ die Bedeutung die bereits im Zusammenhang mit der Beschreibung der erfindungsgemäßen Stoffe der allgemeinen Formel (Ia) als bevorzugt für diesen Substituenten genannt wurde und Hal hat die Bedeutung einer elektronenziehenden Abgangsgruppe.

Geeignete Abgangsgruppen sind z. B. Halogen wie Fluor, Chlor, Brom und Iod, Sulfonat wie Aryl- und Perfluoralkylsulfonat, monosubstituiertes Diazo und monosubstituiertes Nitrato, sowie die zusätzlich in J. March, Advanced Organic Chemistry, 3rd ed., John Wiley & Sons, New York 1985, S. 310-316 aufgeführten.

Als basisch reagierende Verbindungen können in der vorliegenden Erfindung eine oder mehrere basische Verbindungen der Elemente Lithium, Natrium, Magnesium, Kalium, Kalzium, Rubidium, Strontium, Cäsium, Barium, und/oder des Ammoniums Verwendung finden. Als basische Verbindungen kommen z.B. basisch reagierende Salze, Oxide, Hydride und Hydroxide in Frage. Beispielsweise seien genannt: Lithiumhydrid, Natriumhydrid, Kaliumhydrid, Kalziumhydrid, Lithiumhydroxid, Natriumhydroxid, Kaliumhydroxid, Rubidiumhydroxid, Magnesiumhydroxid, Kalziumhydroxid, Strontiumhydroxid, Bariumhydroxid, Lithiumoxid, Natriumperoxid, Kaliumoxid, Kaliumperoxid, Kalziumoxid, Bariumoxid, Magnesiumoxid, Strontiumoxid, Lithiumcarbonat, Lithiumhydrogencarbonat, Rubidiumcarbonat, Rubidiumhydrogencarbonat, Cäsiumhydrogencarbonat, Cäsiumcarbonat, Lithiumcyanid, Natriumcyanid, Kaliumcyanid, Rubidiumcyanid, Ammoniumhydrogencarbonat, Cäsiumcarbonat, Ammoniumcarbamat, Kaliumsulfid, Kaliumhydrogensulfid, Natriumsulfid, Natriumhydrogensulfid und/oder deren natürlich vorkommende oder synthetisch erhältliche Gemische, wie beispielsweise Dolomit oder Magnesiumoxidcarbonat und/oder Verbindungen, die Natrium- oder Kaliummetall auf den entsprechenden Carbonaten in dispergierter Form enthalten.

Bevorzugt sind jedoch Alkalicarbonate und/oder -hydrogencarbonate, ganz besonders bevorzugt Cäsiumcarbonat oder Kaliumcarbonat.

Die basisch reagierenden Verbindungen können in wasserfreier Form oder, soweit es sich um Salze handelt, die mit Hydratwasser kristallisieren, auch in hydratisierter Form eingesetzt werden. Bevorzugt werden jedoch wasserfreie Verbindungen verwendet.

Als Verdünnungsmittel zur Durchführung des erfindungsgemäßen Verfahrens 3d finden die bei Verfahren 3a genannten Lösungsmittel wie z. B. Amide wie Hexamethylenphosphortriamid, N,N-Dimethylformamid, N,N-Dipropylformamid, N,N-Dibutylformamid, N-Methyl-pyrrolidon oder N-Methyl-caprolactam, insbesondere N,N-Dialkylformamide, wie N,N-Dimethylformamid, und Sulfoxide wie Dimethylsulfoxid, Tetramethylensulfoxid, Dipropylsulfoxid, Benzylmethylsulfoxid, Diisobutylsulfoxid, Dibutylsulfoxid, Diisoamylsulfoxid, insbesondere Dimethylsulfoxid, Verwendung.

Alternativ kann man das Verfahren 3d auch in Gegenwart von basischen Reaktionshilfsmitteln durchführen, d.h. in Gegenwart von weiteren Basen, beispielsweise in einer Menge von weniger als 0,5 Mol, bezogen auf die eingesetzte Base.

Als solche basischen Reaktionshilfsmittel zur Durchführung des erfindungsgemäßen Verfahrens 3b können alle bei Verfahren 3a genannten Säurebindemittel, vorzugsweise jedoch tertiären Amine, insbesondere Trialkylamine wie Triethylamin, N,N-Diisopropylethylamin oder N-Methylmorpholin, und Amidinbasen oder Guanidinbasen wie 7-Methyl-1,5,7-triazabicyclo(4.4.0)dec-5-en (MTBD); Diazabicyclo-(4.3.0)nonen (DBN), Diazabicyclo(2.2.2)-octan (DABCO), 1,8-Diaza-bicyclo(5.4.0)-undecen (DBU) Cyclohexyltetrabutylguanidin (CyTBG), Cyclohexyltetramethylguanidin (CyTMG), Cyclohexyltetrabutylguanidin, N,N,N,N-Tetramethyl-1,8-naphthalindiamin, insbesondere Cyclohexyltetramethylguanidin (CyTMG) und Cyclohexyltetrabutylguanidin (CyTBG), Verwendung.

Das Verfahren 3d wird durchgeführt, indem Verbindungen der allgemeinen Formel (Ib) in Gegenwart von Kohlendioxid, einem 2- bis 3-fachen Überschuß an Alkalimetallcarbonat der Formel ( VII ) und einem Alkylierungsmittel der Formel (IX) in einem der weiter oben angegebenen Verdünnungsmittel, gegebenenfalls in Gegenwart eines basischen Reaktionshilfsmittels, bei Raumtemperatur zusammengegeben werden. In einem zweiten Reaktionsschritt erfolgt die Alkylierung der in situ gebildeten Alkalimetallsalze der Formel (VIII) mit Verbindungen der Formel (IX) während einer Reaktionszeit von 1 bis 72 Stunden und einer Reaktionstemperatur zwischen -50 und +180°C, bevorzugt sind Temperaturen im Bereich zwischen -30 und +150°C, insbesondere solche im Bereich -10 bis +100°C.

Es kann grundsätzlich unter Normaldruck gearbeitet, aber auch bei erhöhtem oder erniedrigtem Druck gearbeitet werden. Vorzugsweise arbeitet man bei Normaldruck oder bei Drucken bis zu 15 bar. Bei höheren Temperaturen ist es vorteilhaft, unter erhöhtem Druck zu arbeiten, gegebenenfalls auch über 15 bar.

Die Aufarbeitung und Isolierung der Reaktionsprodukte wird nach allgemein üblichen Methoden durchgeführt (vgl. auch die Herstellungsbeispiele).

Setzt man bei Verfahren 3e zur Herstellung der neuen Didepsipeptide (Ia) als Verbindungen der allgemeinen Formel (Ic) N-Methyl-N-(4-nitro-phenoxycarbonyl)-Lalanyl-D-milchsäure-tert-butylester und als Nucleophil der allgemeinen Formel (X) Morpholin ein, so läßt sich das Verfahren durch folgendes Reaktionsschema wiedergeben:

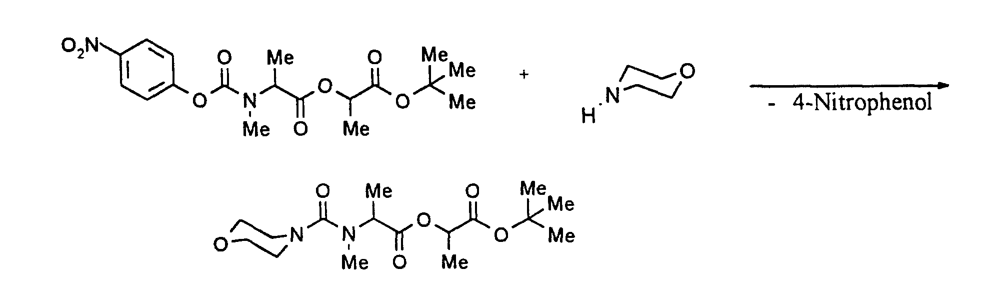

Die zur Durchführung des erfindungsgemäßen Verfahrens 3e als Ausgangsstoffe benötigten N-terminal acylierten Didepsipeptide sind durch die Formel (Ie) allgemein definiert. In dieser Formel stehen R¹, R², R³, R⁴, R⁵, G, W, X und B vorzugsweise für diejenigen Reste, die bereits im Zusammenhang mit der Beschreibung der erfindungsgemäßen Stoffe der allgemeinen Formel (Ia) als bevorzugt für diese Substituenten genannt werden.

Die als Ausgangsmaterialien verwendeten N-terminal acylierten Didepsipeptide der allgemeinen Formel (Ib) sind teilweise bekannt (vgl. DE-OS 4 341 991, DE-OS 4 341 992, DE-OS 4 341 992), können nach den dort beschriebenen Verfahren erhalten werden oder lassen sich nach dem weiter oben aufgezeigten erfindungsgemäßen Verfahren 3b darstellen.

Die außerdem für die Durchführung des erfindungsgemäßen Verfahrens 3e als Ausgangsstoffe zu verwendenden nucleophilen Agenzien der Formel (X) sind allgemein bekannte Verbindungen der organischen Chemie. In der Formel (X) haben R⁸ und Y die Bedeutung die bereits im Zusammenhang mit der Beschreibung der erfindungsgemäßen Stoffe der allgemeinen Formel (Ia) als bevorzugt genannt wurden.

Das Verfahren 3e wird durchgeführt, indem Verbindungen der allgemeinen Formel (Ie) in Gegenwart eines nucleophilen Agenz der Formel (X) in einem der weiter oben angegebenen Verdünnungsmittel umsetzt. Die Reaktionsdauer beträgt 4 bis 72 Stunden. Die Umsetzung erfolgt bei Temperaturen zwischen +10°C und +200°C, bevorzugt zwischen +20°C und +150°C, besonders bevorzugt bei Siedetemperatur des Verdünnungsmittels.

Es kann grundsätzlich unter Normaldruck gearbeitet, aber auch bei erhöhtem oder erniedrigtem Druck gearbeitet werden. Vorzugsweise arbeitet man bei Normaldruck oder bei Drucken bis zu 15 bar. Bei höheren Temperaturen ist es vorteilhaft, unter erhöhtem Druck zu arbeiten, gegebenenfalls auch über 15 bar.

Nach vollendeter Umsetzung wird die Reaktionslösung gewaschen, die organische Phase abgetrennt, getrocknet und im Vakuum eingeengt. Die anfallenden Produkte lassen sich in üblicher Weise durch Umkristallisieren, Vakuumdestillation oder Säulenchromatographie reinigen (vgl. auch die Herstellungsbeispiele).

Setzt man bei Verfahren 3f zur Herstellung der neuen Didepsipeptide (Ia) als Verbindungen der allgemeinen Formel (Id) N-Methyl-N-trimethylallophanoyl-L-alanyl-D-milchsäure und als Verbindungen der allgemeinen Formel (XI) L-Homoprolinmethylester Hydrochlorid (H-Pec-OMe ^{·} HCl) ein, so läßt sich das Verfahren durch folgendes Reaktionsschema wiedergeben:

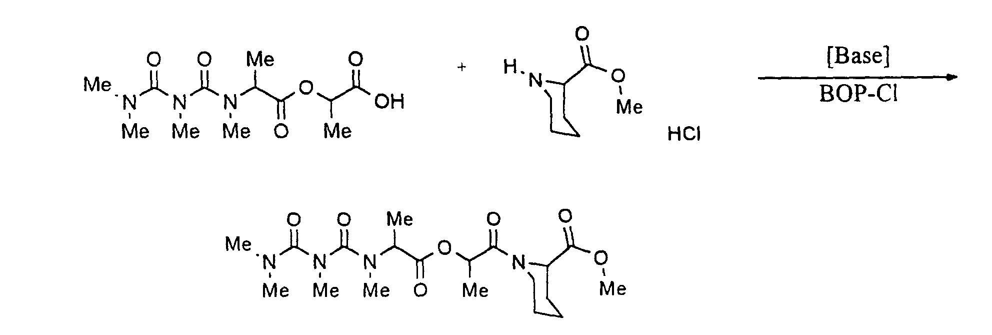

Die zur Durchführung des erfindungsgemäßen Verfahrens 3e als Ausgangsstoffe benötigten C-terminal deblockierten Didepsipeptide sind durch die Formel (Id) allgemein definiert. In dieser Formel stehen R¹, R², R³, R⁴, R⁵, G, X und Q vorzugsweise für diejenigen Reste, die bereits im Zusammenhang mit der Beschreibung der erfindungsgemäßen Stoffe der allgemeinen Formel (Ia) als bevorzugt für diese Substituenten genannt werden.

Die als Ausgangsmaterialien verwendeten C-terminal deblockierten Didepsipeptide der allgemeinen Formel ( Id) sind teilweise bekannt (vgl. DE-OS 4 341 991, DE-OS 4 341 992, DE-OS 4 341 992) bzw. können nach den dort beschriebenen Verfahren erhalten werden.

Beispielsweise lassen sich die als Ausgangsmaterialien verwendeten C-terminal deblockierten Didepsipeptide ( Id ) mittels üblicher Methoden einer C-terminaler Deblockierung wie Acidolyse, beispielsweise im Falle eines tert-Butylesters, oder katalytischer Hydrierung, beispielsweise im Falle eines Benzylesters, darstellen.

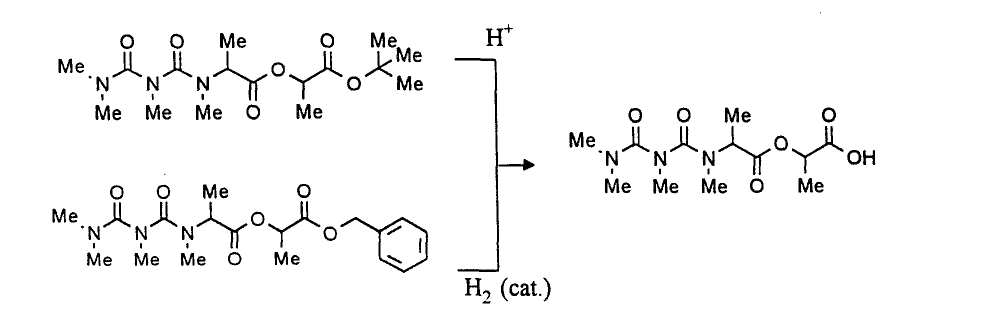

Die außerdem für die Durchführung des erfindungsgemäßen Verfahrens 3e als Ausgangsstoffe zu verwendenden Nucleophile sind allgemein durch die Formel (XI) definiert.

In der Formel (XI) hat B die Bedeutung die bereits im Zusammenhang mit der Beschreibung der erfindungsgemäßen Stoffe der allgemeinen Formel (Ia) als bevorzugt für diesen Substituenten genannt wurden.

Die Verbindungen der Formel (XI) sind allgemein bekannte Verbindungen der organischen Chemie, können z.T. kommerziell oder nach literaturbekannten Methoden erhalten werden.

Die Umsetzung der C-terminal deblockierten Didepsipeptide der Formel (Id) mit Verbindungen der Formel (XI) führt man vorzugsweise in Gegenwart von Kupplungsreagenzien und in Gegenwart eines basischen Reaktionshilfsmittels unter Verwendung von Verdünnungsmitteln durch.

Als Kupplungsreagenzien zur Durchführung des Verfahrens 3f finden alle zur Herstellung einer Amidbindung geeigneten und bereits bei dem weiter oben genannten Verfahren 3a eingesetzten Kupplungsreagenzien Verwendung.

Bevorzugt ist die Kupplung mit Phosphoniumreagenzien wie Bis(2-oxo-3-oxazolidinyl)-phosphoniumsäurechlorid (BOP-Cl), Benzotriazol-1-yl-oxytris(dimethylaminophosphonium)-hexafluorophosphat (BOP) und Phosphonsäureesterreagenzien, wie Cyanphosphonsäurediethylester (DEPC) oder Diphenylphosphorylazid (DPPA).

Als basische Reaktionshilfsmittel zur Durchführung der erfindungsgemäßen Verfahrens 3f können ebenso alle für das Verfahren 3a geeigneten Säurebindemittel eingesetzt werden.

Vorzugsweise kommen tertiären Amine, insbesondere Trialkylamine wie Triethylamin, N,N-Diisopropylethylamin, N-Propyl-diisopropylamin, N,N'-Dimethyl-cyclohexylamin oder N-Methylmorpholin in Frage.

Als Verdünnungsmittel zur Durchführung des erfindungsgemäßen Verfahrens 3f finden die bei Verfahren 3a genannten Lösungsmittel wie z. B. Halogenkohlenwasserstoffe, insbesondere Chlorkohlenwasserstoffe, wie Methylenchlorid oder 1,2-Dichlorethan und Gemische von diesen mit anderen genannten Verdünnungsmitteln, Verwendung.

Das Verfahren 3j wird im allgemeinen so durchgeführt, indem man Verbindungen der Formel (Id) in Gegenwart eines der angegebenen Kupplungsreagenzien und in Gegenwart eines der angegebenen basischen Reaktionshilfsmittel mit Verbindungen der allgemeinen Formel (XI) in einem der angegebenen Verdünnungsmittel umsetzt. Die Reaktionsdauer beträgt 4 bis 72 Stunden. Die Umsetzung erfolgt bei Temperaturen zwischen -10°C und +120°C, bevorzugt zwischen -5°C und +50°C, besonders bevorzugt bei 0°C bis Raumtemperatur. Es wird unter Normaldruck gearbeitet.

Zur Durchführung des erfindungsgemäßen Verfahrens 3f setzt man pro Mol an C-terminal deblockiertem Didepsipeptid der Formel (Id) im allgemeinen 1,0 bis 3,0 Mol, vorzugsweise 1,0 bis 1,5 Mol Kupplungsreagenz, ein.

Nach vollendeter Umsetzung wird die Reaktionslösung gewaschen, die organische Phase abgetrennt, getrocknet und im Vakuum eingeengt. Die anfallenden Produkte lassen sich in üblicher Weise durch Umkristallisieren, Vakuumdestillation oder Säulenchromatographie reinigen (vgl. auch die Herstellungsbeispiele).

Mit den erfindungsgemäßen Verfahren 3a bis 3f sind, aus den einzelnen Bausteinen mit sowohl L- als auch D-Konfiguration, Didepsipeptide unter Beibehaltung, aber auch unter Inversion der ursprünglichen Konfiguration der Ausgangsstoffe erhältlich.

Mit den in den vorstehenden Verfahrensvarianten 3a bis 3f bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Die Wirkstoffe eignen sich bei günstiger Warmblütertoxizität zur Bekämpfung von pathogenen Endoparasiten, die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist. Zu den pathogenen Endoparasiten zählen Cestoden, Trematoden, Nematoden, Acantocephalen, insbesondere:

Aus der Ordnung der Pseudophyllidea z.B.: Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., 'Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp., Metorchis spp., Heterophyes spp., Metagonismus spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp., Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Ascaridia z.B.: Ascaris spp., Toxascaris spp., Toxocara spp., Parascaris spp., Anisakis spp., Ascaridia spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Ordnung der Gigantorhynchida z.B.: Filicollis spp., Moniliformis spp., Macracanthorhynchus spp., Prosthenorchis spp..

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Tabletten, Kapseln, Pasten, Tränken, Granulaten, oral applizierbaren Lösungen, Suspensionen und Emulsionen, Boli, medikiertem Futter oder Trinkwasser. Die dermale Anwendung geschieht z.B. in Form des Tauchens (Dippen), Sprühens (Sprayen) oder Aufgießens (pour-on and spot-on). Die parenterale Anwendung geschieht z.B. in Form der Injektion (intramusculär, subcutan, intravenös, intraperitoneal) oder durch Implantate.

Geeignete Zubereitungen sind:
Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;
Emulsionen und Suspensionen zur oralen oder dermalen Anwendung sowie zur Injektion; halbfeste Zubereitungen;
Formulierungen, bei denen der Wirkstoff in einer Salbengrundlage oder in einer Öl in Wasser oder Wasser in Öl Emulsionsgrundlage verarbeitet ist;
Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylalkohol, Glycerin, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt oder aufgesprüht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Methacrylate.

Gele werden auf die Haut aufgetragen oder aufgestrichen oder in Körperhöhlen eingebracht. Gele werden hergestellt, indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff die Haut durchdringt und systemisch wirkt.

Aufgieß-Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl /Caprinsäure-biglycerid, Triglyceridgemisch mit Pflanzenfettsäuren der Kettenlänge C₈₋₁₂ oder anderen speziell ausgewählten natürlichen Fettsäuren. Partialglyceridgemische gesättigter oder ungesättigter ebentuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Capryl/Caprinsäureester von gesättigten Fettalkoholen der Kettenlänge C₁₂-C₁₈, Isopropylstearat, Ölsäureoleylester, Ölsäuredecylester, Ethyloleat, Milchsäureethylester, wachsartige Fettsäureester wie künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.
Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethynolaminsalz.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebenen Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenylimidazothiazol, Benzimidazolcarbamate, Praziquantel, Pyrantel, Febantel.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen, die vor Anwendung verdünnt werden, enthalten den Wirkstoff in Konzentrationen von 0,5 - 90 Gew.-%, bevorzugt von 5 - 50 Gew.-%.

Im allgemeinen hat es sich als vorteilhaft erwiesen, Mengen von etwa 1 bis etwa 100 mg Wirkstoff je kg Körpergewicht pro Tag zur Erzielung wirksamer Ergebnisse zu verabreichen.

### Beispiel A

### In vivo Nematodentest

### Trichostrongylus colubriformis / Schaf

Experimentell mit Trichostrongylus colubriformis infizierte Schafe wurden nach Ablauf der Präpatenzzeit des Parasiten behandelt. Die Wirkstoffe wurden als reiner Wirkstoff oral und/oder intravenös appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich.

| **Wirkstoff Beispiel-Nr.** | **Dosis effectiva in [mg/kg]** |
|---|---|
| 2 | 5 |
| 9 | 5 |
| 11 | 5 |
| 12 | 5 |
| 13 | 5 |
| 18 | 5 |
| I - 7 | 5 |
| 46 | 5 |
| 68 | 5 |

### Beispiel B

### In vivo Nematodentest

### Haemonchus contortus / Schaf

Experimentell mit Haemonchus contortus infizierte Schafe wurden nach Ablauf der Präpatenzzeit des Parasiten behandelt. Die Wirkstoffe wurden als reiner Wirkstoff oral und/oder intravenös appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus den nachfolgenden Tabellen ersichtlich.

| **Wirkstoff Beispiel-Nr.** | **Dosis effectiva in [mg/kg]** | | **Wirkstoff Beispiel-Nr.** | **Dosis effectiva in [mg/kg]** |
|---|---|---|---|---|
| 2 | 5 | | 36 | 5 |
| 8 | 5 | | 38 | 5 |
| 9 | 5 | | 45 | 5 |
| 10 | 5 | | 46 | 5 |
| 11 | 5 | | 47 | 5 |
| 12 | 5 | | 49 | 5 |
| 19 | 5 | | 50 | 5 |
| 20 | 5 | | 61 | 5 |
| 30 | 5 | | 68 | 5 |
| 31 | 5 | | I - 4 | 5 |
| 34 | 5 | | I - 5 | 5 |
| 35 | 5 | | I - 7 | 5 |

### Herstellungsbeispiele

### Herstellung nach Verfahren 3a

### Beispiel 1:

### N-Methyl-N-trimethylallophanoyl-L-alanyl-D-milchsäureisobutylester

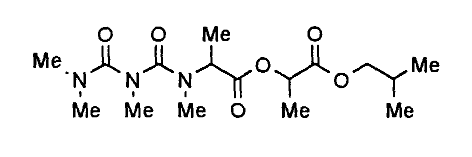

Zu einer Lösung von 2,4 g (10,4 mMol) N-Methyl-N-trimethylallophanoyl-Lalanin und 1,5 g (10,4 mMol) D-Milchsäureisobutylester in 20 ml Methylenchlorid werden bei 0°C 2,9 g (22,9 mMol) N,N-Diisopropylethylamin ("Hünig's Base") sowie 2,9 g (11,4 mMol) Bis(2-oxo-3-oxazolidinyl)-phosphoniumsäurechlorid (BOP-Cl) zugegeben und 18 bei Raumtemperatur gerührt. Die Reaktionslösung wird zweimal mit Wasser geschüttelt, die organische Phase abgetrennt und nach dem Trocknen über Natriumsulfat im Vakuum eingeengt. Das zurückbleibende Rohprodukt wird über eine Kieselgelsäule (Kieselgel 60 - Merck, Korngröße : 0,04 bis 0,063 mm) mit dem Fließmittel Cyclohexan : Aceton (4:1) chromatographiert. Man erhält 1,6 g (57,2 % der Theorie) N-Methyl-N-trimethylallophanoyl-L-alanyl-D-milchsäureisobutylester.
¹H-NMR (400 MHz, CDCl₃, δ): 0,92; 0,94 (d, 6H, 2 x -CH₃; J = 6,7 Hz); 2,91; 2,92; 2,94 (3s, 9H, 3 x -N-CH₃); 3,06 (s, 3H, -N-CH₃); 3,92 (m, 3H, -O-CH₂-); 5,14 (m, 1H, CH) ppm
GC-MS m/z (%): 360 (MH⁺,100); 271 (38); 214 (62).

### Herstellung nach Verfahren 3b

### Beispiel 2:

### N-Methyl-N-trimethylallophanoyl-L-alanyl-D-milchsäuremethylester

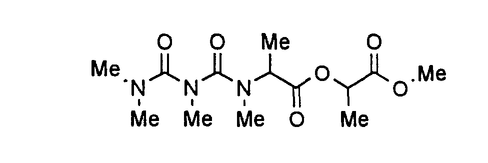

Zu einer Lösung von 2,0 g (10,6 mMol) N-Methyl-L-alanyl-Dmilchsäuremethylester in 100 ml Methylenchlorid werden bei 0°C 3,8 g (29,1 mMol) N,N-Diisopropylethylamin ("Hünig's Base") sowie 2,1 g (12,7 mMol) Trimethylallophansäurechlorid zugegeben, zwei Stunden bei 0°C und anschließend ca. 18 Stunden bei Raumtemperatur gerührt. Die Reaktionslösung wird zweimal mit Wasser geschüttelt, die organische Phase abgetrennt und nach dem Trocknen über Natriumsulfat im Vakuum eingeengt. Das zurückbleibende Rohprodukt wird über eine Kieselgelsäule (Kieselgel 60 - Merck, Korngröße : 0,04 bis 0,063 mm) mit dem Fließmittel Cyclohexan : Essigsäureethylester (3:1) chromatographiert. Man erhält 1,35 g (40,2 % der Theorie) N-Methyl-N-trimethylallophanoyl-L-alanyl-D-milchsäuremethylester.
¹H-NMR (400 MHz, CDCl₃, δ): 1,46; 1,49; (2d, 6H, 2 x -CH₃; J = 7,2 Hz); 2,91; 2,92; 2,93 (3s, 9H, 3 x -N-CH₃); 3,06 (s, 3H, -N-CH₃); 3,75 (s, 3H, -O-CH₃); 4,49; 5,13 (2q, 2H, 2 x CH; J = 7,2 Hz) ppm
EI-MS m/z (%): 317 (M⁺,1); 286 (M⁺-OMe,1); 273 (M⁺-CO₂,7); 214 (3); 186 (40); 72 (100).

### Herstellung nach Verfahren 3c

### Beispiel 3:

### N-Methyl-N-trichloracetylaminocarbonyl-L-alanyl-D-milchsäure-tert-butylester

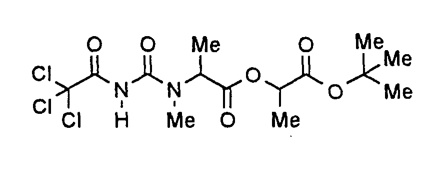

Zu einer Lösung von 3,0 g (12,9 mMol) N-Methyl-L-alanyl-D-milchsäure-tert-butylester in 30 ml Acetonitril werden bei 0°C 2,4 g (12,9 mMol) Trichloracetylisocyanat gegeben, zwei Stunden bei 0°C und anschließend 18 Stunden Raumtemperatur gerührt. Die Reaktionslösung wird zweimal mit Wasser geschüttelt, die organische Phase abgetrennt und nach dem Trocknen über Natriumsulfat im Vakuum eingeengt. Das zurückbleibende Rohprodukt wird über eine Kieselgelsäule (Kieselgel 60 - Merck, Korngröße : 0,04 bis 0,063 mm) mit dem Fließmittel Cyclohexan : Aceton (4: 1) chromatographiert. Man erhält 4,2 g (77,2 % der Theorie) N-Methyl-N-trichlor-acetyl-L-alanyl-D-milchsäure-tertbutylester.
¹H-NMR (400 MHz, CDCl₃, δ): 1,49 (s, 9H, -O-tBu); 1,40; 1,47; (2d, 6H, 2 x -CH₃; J = 6,9 Hz); 2,95 (s, 3H, -N-CH₃); 3,98; 4,22 (2q, 2H, 2 x CH; J = 6,9 Hz); 9,05 (br., 1H, -CO-NH-CO-) ppm

### Herstellung nach Verfahren 3d

### Beispiel 4:

### N-Butyloxycarbonyl-N-methyl-L-alanyl-D-milchsäure-tert-butylester

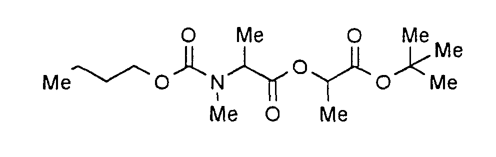

2,0 g (8,6 mMol) N-Methyl-L-alanyl-D-milchsäure-tert-butylester, 2,1 g (15,2 mmol) Kaliumcarbonat und 30 ml Dimethylsulfoxid werden eine Stunde lang mit Kohlendioxid begast und anschließend mit 1,2 g (8,6 mMol) Butylbromid versetzt. Die Reaktionsmischung wurde 48 Stunden bei Raumtemperatur gerührt, danach vom Feststoff abgetrennt, unter vermindertem Druck die flüchtigen Komponenten abgezogen, mit Chloroform - Wasser extrahiert und die organische Phase abgetrennt. Anschließend wird die organische Phase über Natriumsulfat getrocknet, im Vakuum eingeengt und das zurückbleibende Öl über eine Kieselgelsäule (Kieselgel 60 - Merck, Korngröße : 0,04 bis 0,063 mm) chromatographiert.
¹H-NMR (400 MHz, CDCl₃, δ): 1,46 (s, 9H, -O-tBu) ppm
EI-MS m/z (%): 331 (M⁺,0,5); 275 (M⁺-H₂C=CMe₂, 5); 158 (100).

### Herstellung nach Verfahren 3e

### Beispiel 5:

### N-Methyl-N-(4-nitro-phenoxy)carbonyl-L-alanyl-D-milchsäure-tert-butylester

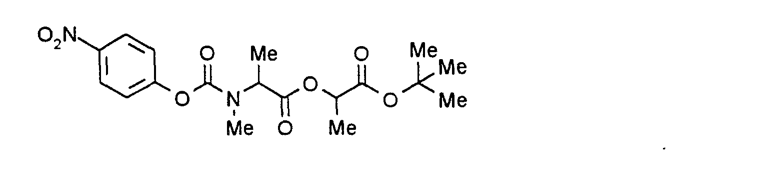

Zu einer Lösung von 5,0 g (21,6 mMol) N-Methyl-L-alanyl-D-milchsäure-tert-butylester in 150 ml Methylenchlorid werden bei 0°C 6,15 g (47,5 mMol) N,N-Diisopropylethyl-amin ("Hünig's Base") sowie 4,4 g (21,6 mMol) Chlorameisensäure-(4-nitrophenyl)-ester zugegeben, zwei Stunden bei 0°C und anschließend ca. 18 Stunden bei Raumtemperatur gerührt. Die Reaktionslösung wird zweimal mit Wasser geschüttelt, die organische Phase abgetrennt und nach dem Trocknen über Natriumsulfat im Vakuum eingeengt. Das zurückbleibende Rohprodukt wird über eine Kieselgelsäule (Kieselgel 60 - Merck, Korngröße : 0,04 bis 0,063 mm) mit dem Fließmittel Cyclohexan : Aceton (10:1) chromatographiert.
¹H-NMR (400 MHz, CDCl₃, δ): 7,31; 8,24 (2d, 4H, 4-NO₂-Phenoxy) ppm
EI-MS m/z (%): 340 (M⁺-H₂C=CMe₂, 2); 202 (100).

### Beispiel 6:

### N-Methyl-N-Morpholinocarbonyl-L-alanyl-D-milchsäure-tert-butylester

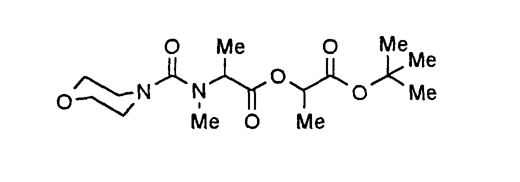

Zu einer Lösung von 2,0 g (5,0 mMol) N-Methyl-N-(4-nitro-phenoxy)carbonyl-Lalanyl-D-milchsäure-tert-butylester in 40 ml Methylenchlorid werden bei Raumtemperatur 0,65 g (5,0 mMol) N,N-Diisopropylethylamin ("Hünig's Base") sowie 0,45 g (5,0 m Mol) Morpholin zugegeben und ca. 18 Stunden bei Rückflußtemperatur gerührt. Dabei tritt eine kräftige Gelbfärbung des Reaktionsgemisches auf. Die Reaktionslösung wird zweimal mit Wasser geschüttelt, die organische Phase abgetrennt und nach dem Trocknen über Natriumsulfat im Vakuum eingeengt. Das zurückbleibende Rohprodukt wird über eine Kieselgelsäule (Kieselgel 60 - Merck, Korngröße : 0,04 bis 0,063 mm) mit dem Fließmittel Cyclohexan : Essigsäureethylester (1:1) chromatographiert.
¹H-NMR (400 MHz, CDCl₃, δ); 1,42; 1,46 (s/2d, 15H, -O-tBu/-CH₃); 2,90 (s, 3H, -N-Me) 3,25 (m, 4H, -CH₂-O-CH₂-); 3,68 (m, 4H, -CH₂-N-CH₂-); 4,69; 4,96 (2q, 2H, -2 x -CH-) ppm
EI-MS m/z (%): 344 (M⁺,4); 171 (100).

### Herstellung nach Verfahren 3f

### Beispiel 7:

### N-Methyl-N-trimethylallophanoyl-L-alanyl-D-milchsäure

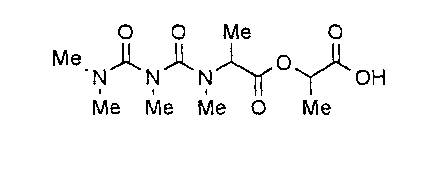

In eine auf 0°C gekühlte Lösung von 11,8 g (32,8 mmol) N-Methyl-N-trimethylallophanoyl-L-alanyl-D-milchsäure-tert-butylester in 250 ml absolutem Methylenchlorid wird 20 Minuten trockenes Chlorwasserstoffgas eingeleitet. Anschließend rührt man ca. 16 Stunden bei Raumtemperatur und engt den gesamten Reaktionsansatz im Vakuum ein.

Man erhält 10,0 g (100 % der Theorie) N-Methyl-N-trimethylallophanoyl-L-alanyl-D-milchsäure, die ohne weitere Reinigung weiter umgesetzt werden kann.
¹H-NMR (400 MHz, CDCl₃, δ): 1,46; 1,51 (2d, 2H, 2 x -CH₃; J = 7,2 Hz); 2,91; 2,94; 3,05 (3s, 12H, 4 x N-Me); 4,84; 5,17 (2q, 2H, -CH-; J = 7,2 Hz) ppm
EI-MS m/z (%): 304 (MH⁺, 0,1); 259 (2); 214 (1); 186 (15); 129 (15); 72 (52); 58 (100).

### Beispiel 8:

### N-Methyl-N-trimethylallophanoyl-L-alanyl-D-lactyl-L-homoprolin-methylester

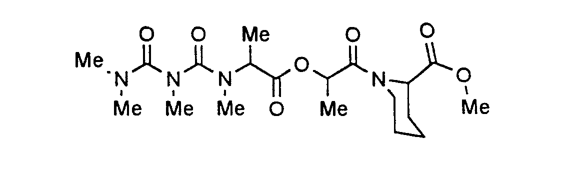

Zu einer Lösung von 2,0 g (6,6 mMol) N-Methyl-N-trimethylallophanoyl-L-alanyl-D-milchsäure und 1,3 g (7,25 mMol) L-Homoprolin-methylester Hydrochlorid (H-Pec-OMe ^{·} HCl) in 100 ml Methylenchlorid werden bei 0°C 2,8 g (21,8 mMol) N,N-Diisopropylethylamin ("Hünig's Base") sowie 1,85 g (7,25 mMol) Bis(2-oxo-3-oxa-zolidinyl)-phosphoniumsäurechlorid (BOP-Cl) zugegeben und 30 Minuten bei 0 °C, anschließend 18 Stunden bei Raumtemperatur gerührt. Die Reaktionslösung wird zweimal mit Wasser geschüttelt, die organische Phase abgetrennt und nach dem Trocknen über Natriumsulfat im Vakuum eingeengt. Das zurückbleibende Rohprodukt wird über eine LiChroprep RP-18 Säule (LiChroprep RP-18 - Merck, Korngröße : 0,04 bis 0,063 mm) mit dem Fließmittel Acetonitril - Wasser (3 : 7) chromatographiert. Man erhält 0,32 g (11,3 % der Theorie).
EI-MS m/z (%): 428 (M⁺, 2); 397 (2); 352 (17); 186 (38); 72 (100).

Analog zu den Verfahren 3a-f können die in der nachstehenden Tabelle 1 aufgeführten Verbindungen der allgemeinen Formel (Ia) hergestellt werden.

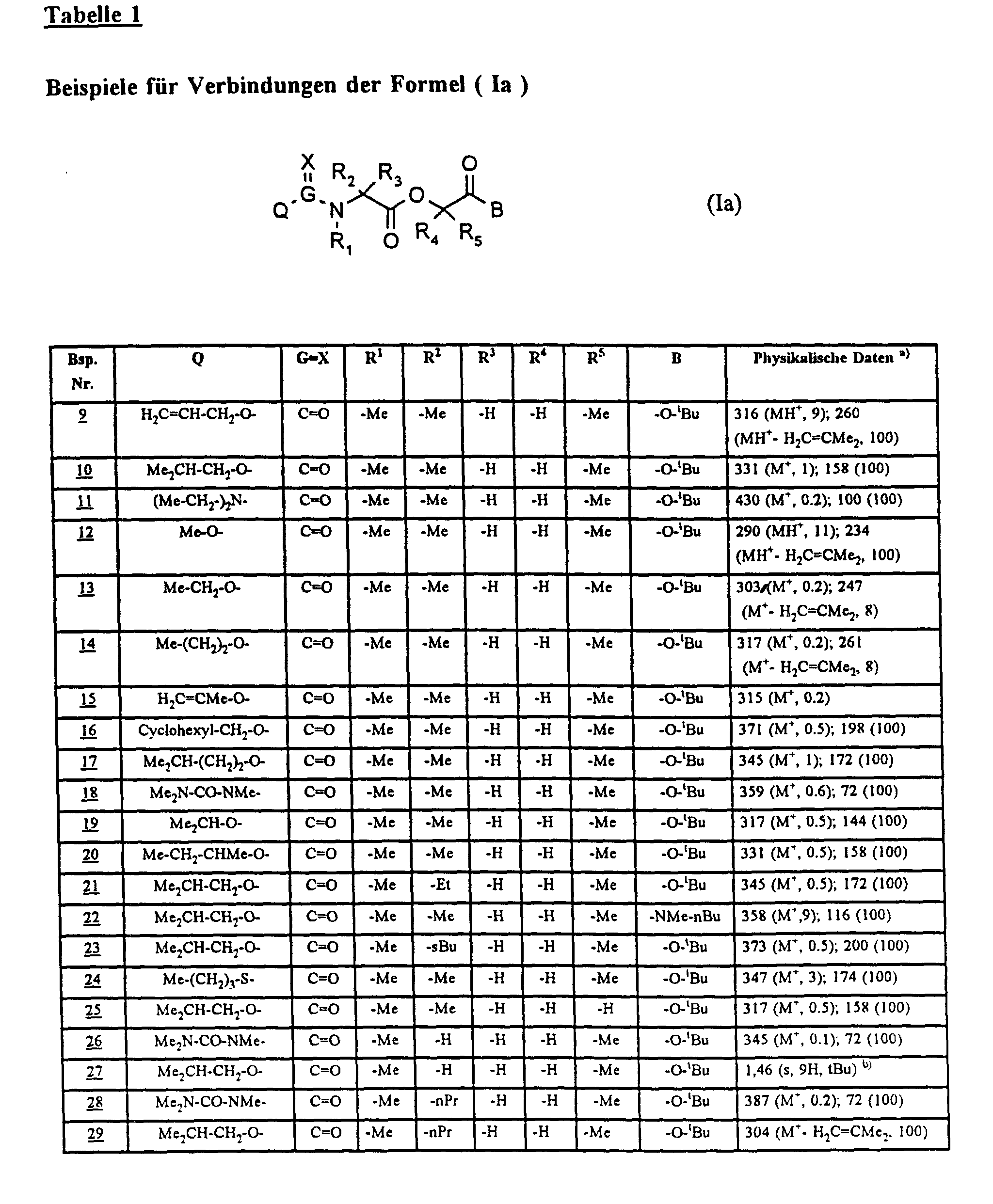

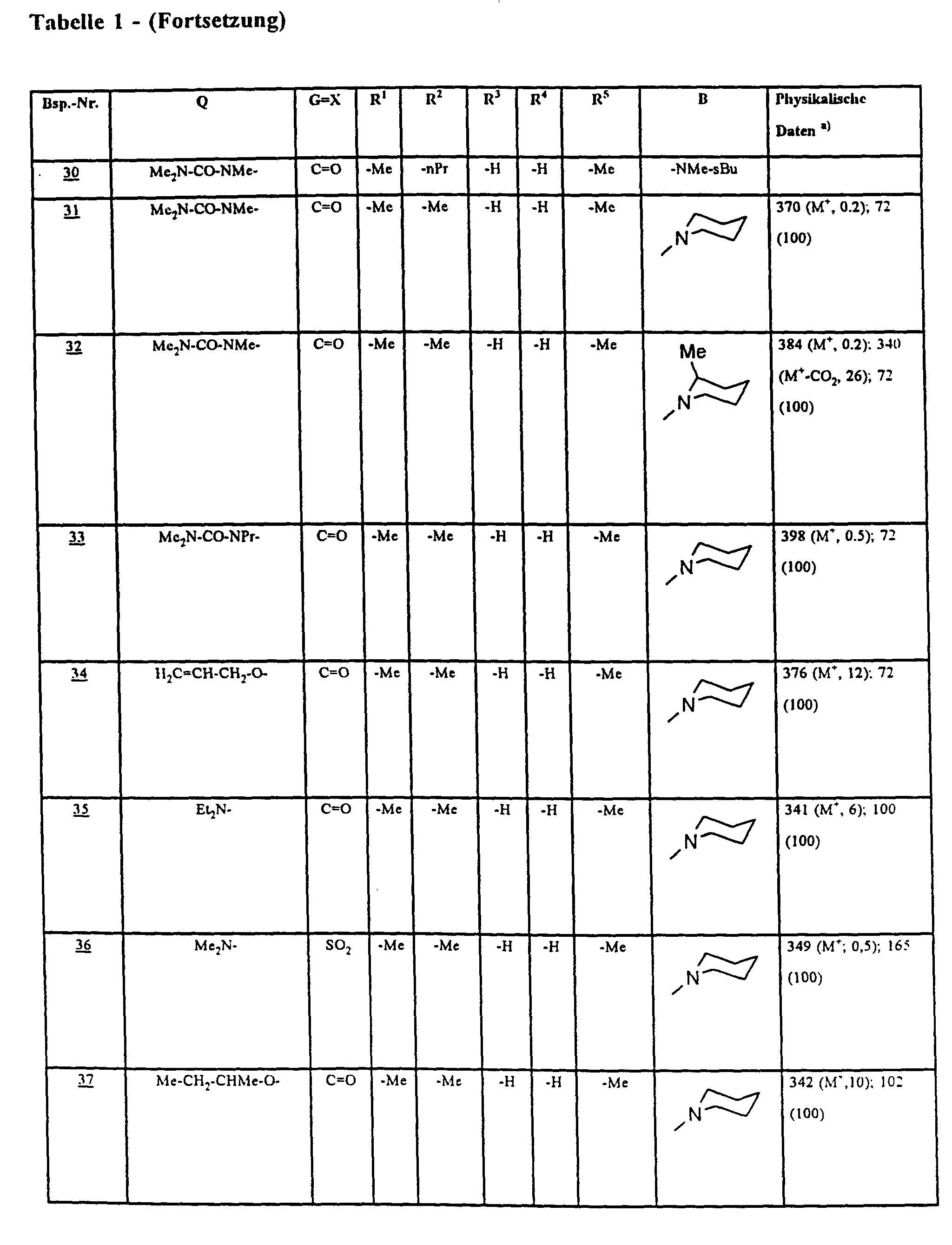

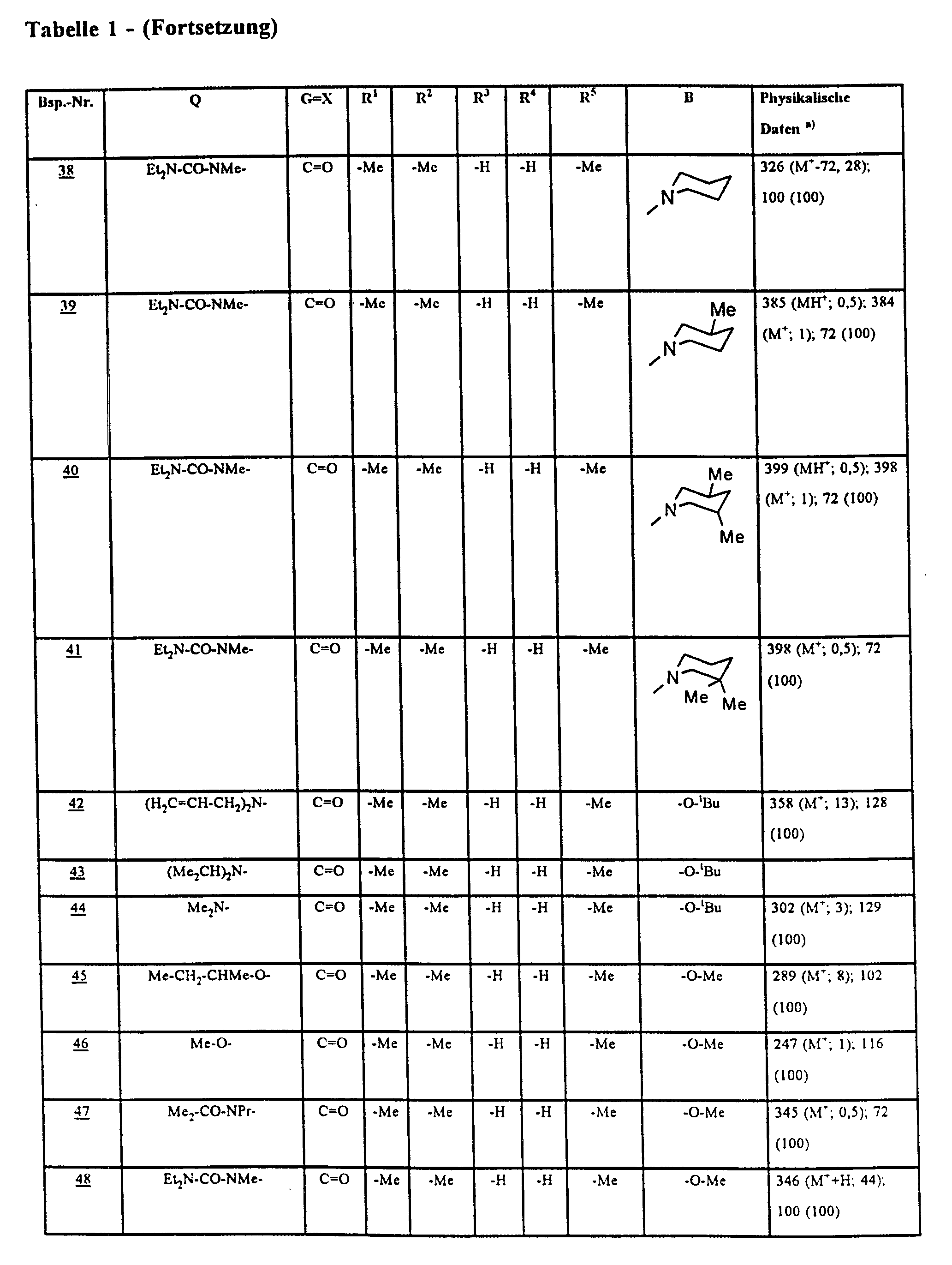

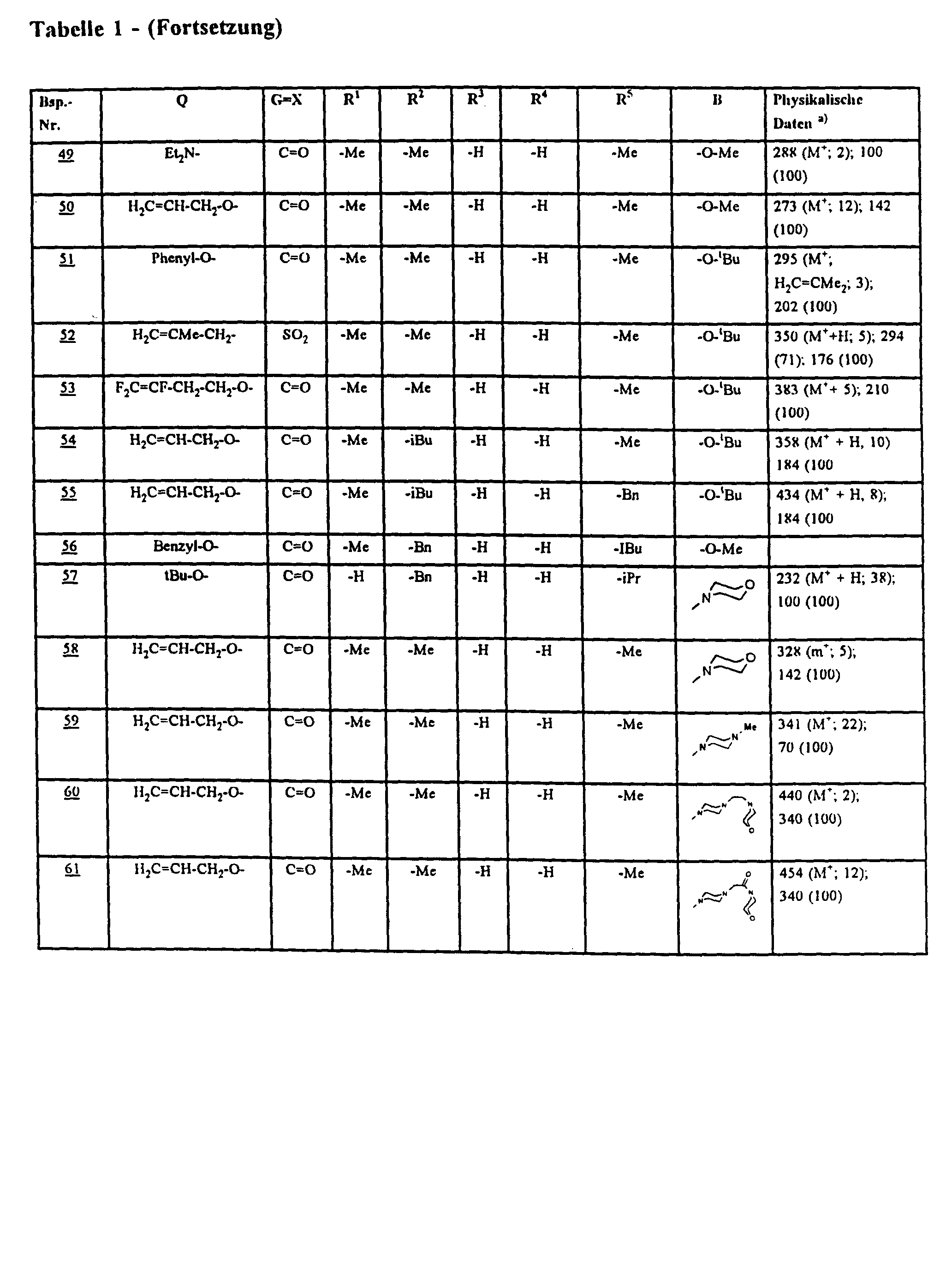

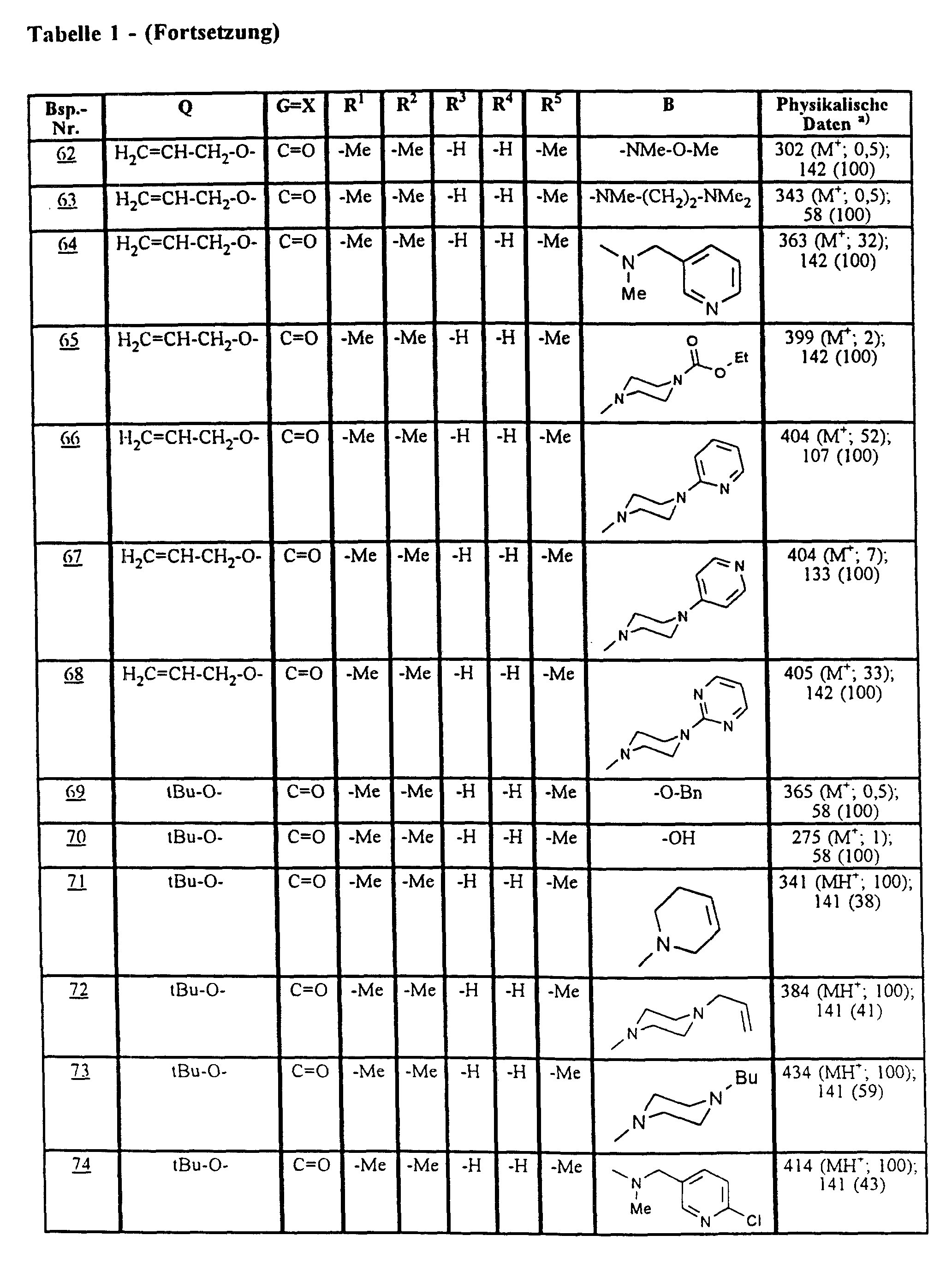

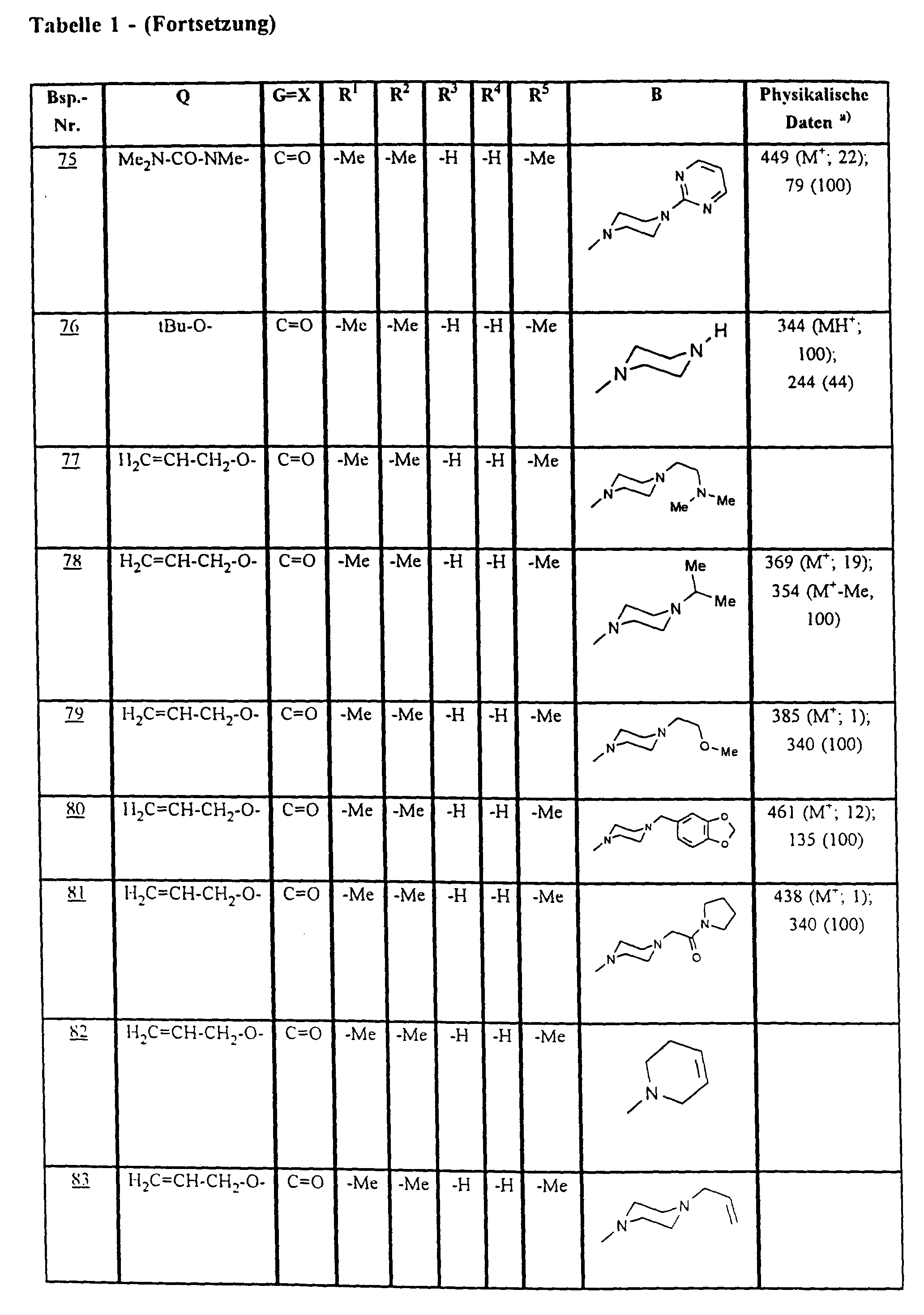

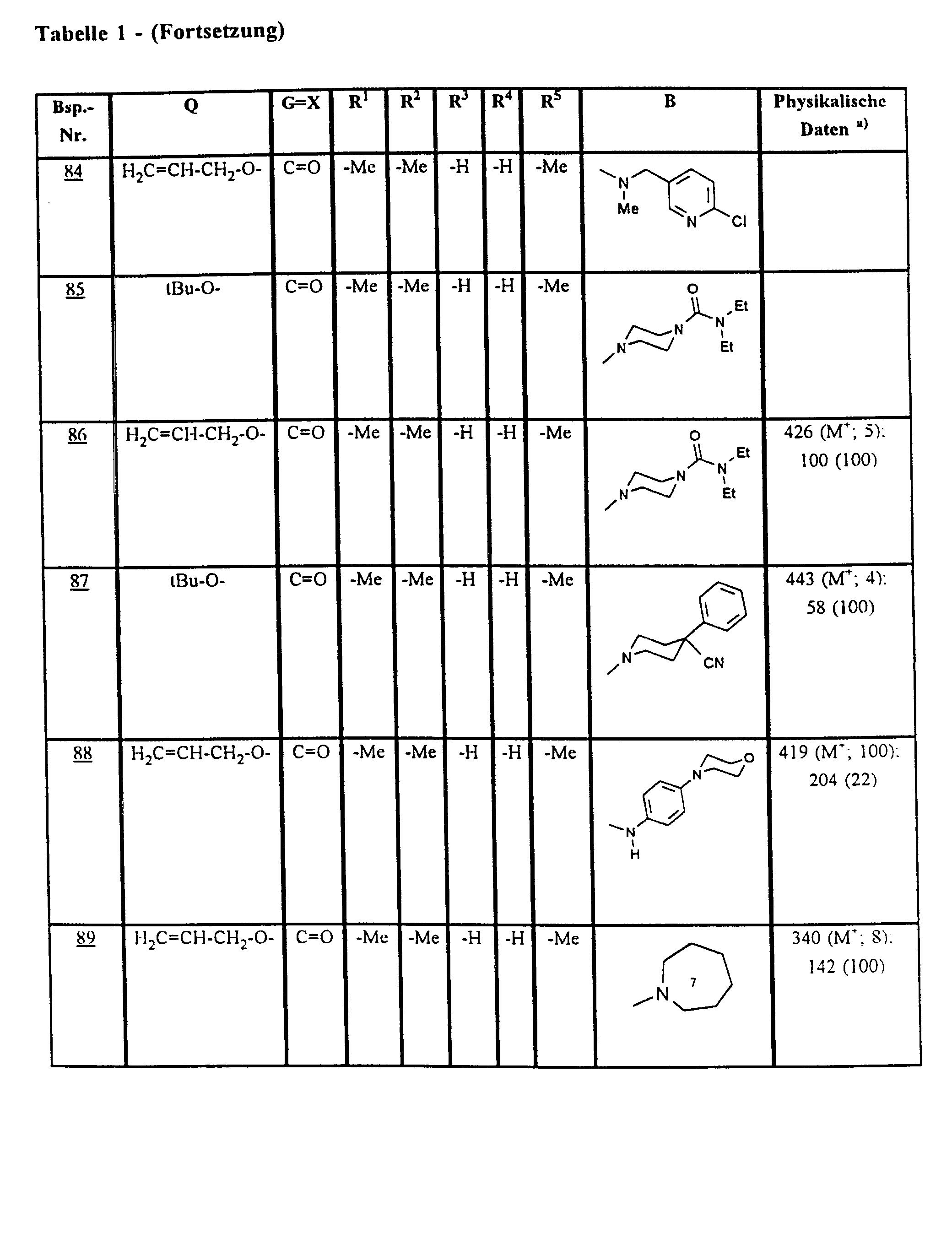

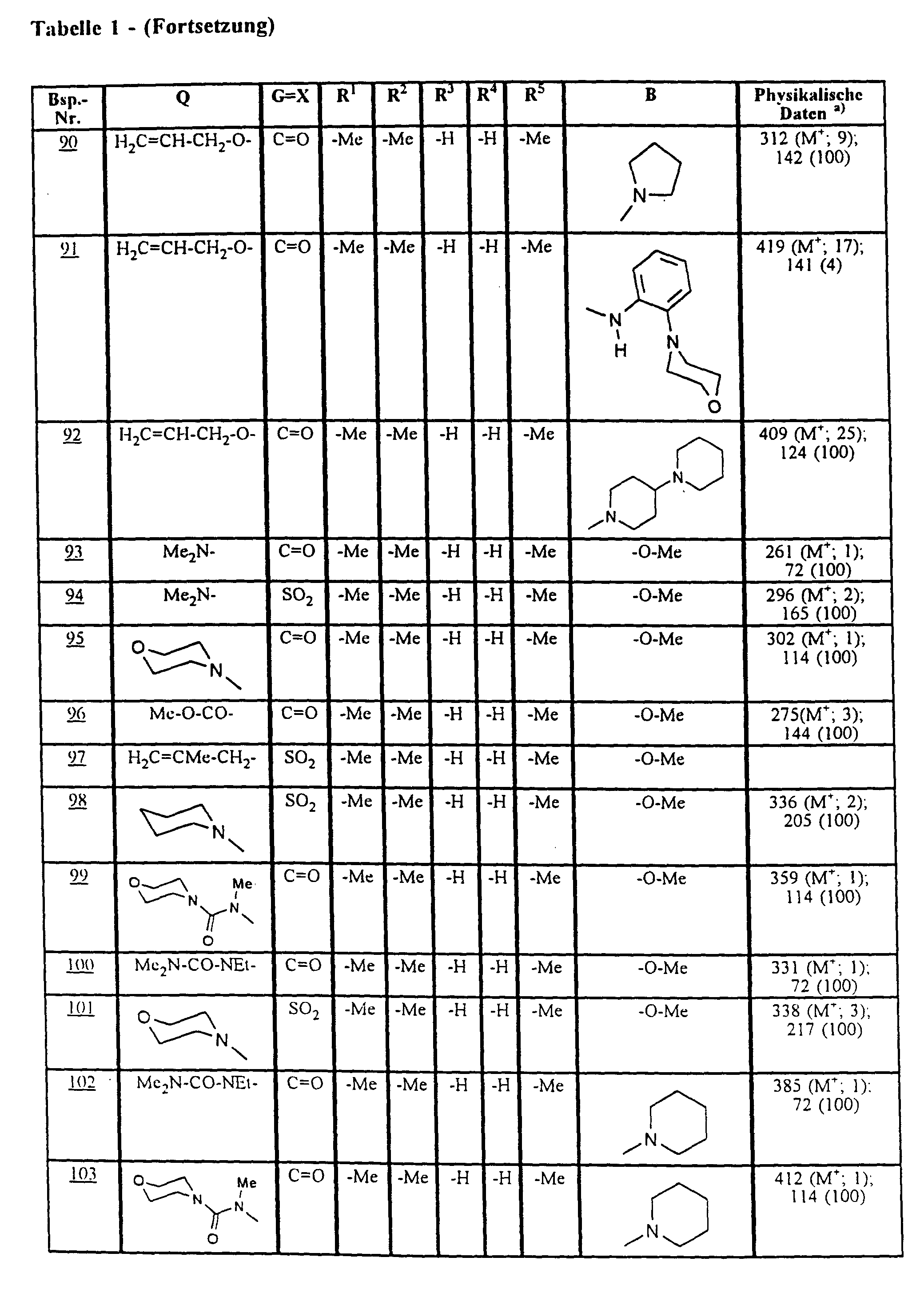

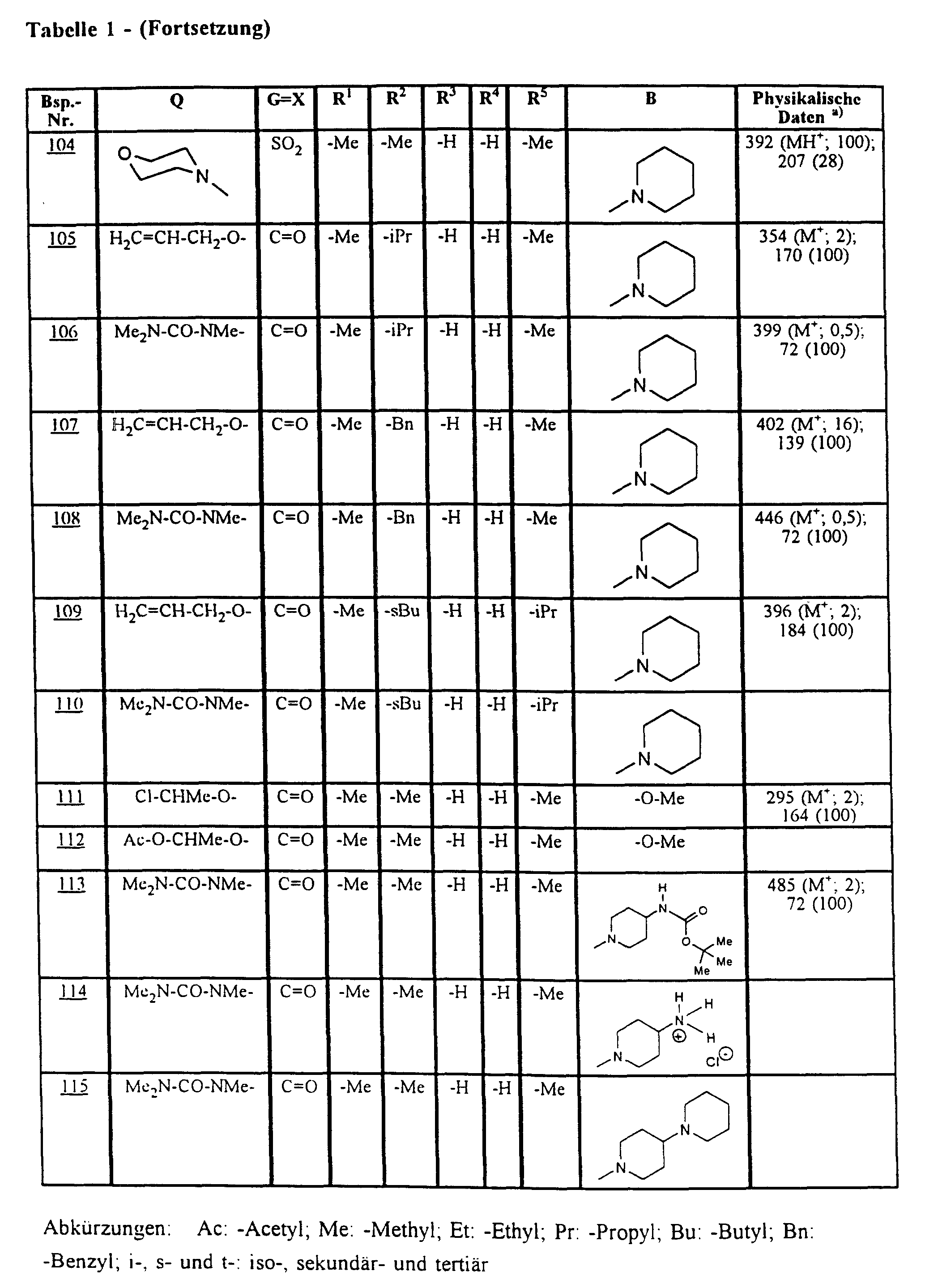

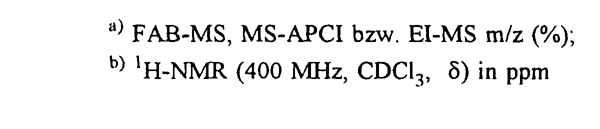

### Ausgangsstoffe der Formel ( I )

### Beispiel (I-1)

### N-Benzyl-N-methyl-L-alanyl-D-milchsäurepiperidid

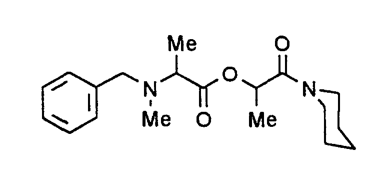

Zu einer Lösung von 5,0 g (18,8 mMol) N-Benzyl-N-methyl-L-alanyl-Dmilchsäure und 1,6 g (20,7 mMol) Piperidin in 150 ml Methylenchlorid werden bie 0°C 5,4 g (41,4 mMol) N,N-Diisopropylethylamin ("Hünigs Base") sowie 5,3 g (20,7 mMol) Bis(2-oxo-3-oxazolidinyl)-phosphoniumsäurechlorid (BOP-Cl) zugegeben, zwei Stunden bei 0°C und anschließend 18 Stunden bei Raumtemperatur gerührt. Das zurückbleibende Rohprodukt wird über eine Kieselgelsäule (Kieselgel 60 - Merck, Korngröße : 0,04 bis 0,063 mm) mit dem Fließmittel Cyclohexan : Essigsäureethyl-ester (3 : 1) chromatographiert. Man erhält 3,5 g (55,8 % der Theorie) N-Benzyl-N-methyl-L-alanyl-D-milchsäurepiperidid.
EI-MS m/z (%): 332 (M⁺, 1); 213 (2); 192 (2); 148 (100); 120 (35); 91 (55)

### Beispiel (I-2)

### N-Methyl-L-alanyl-D-milchsäurepiperidid

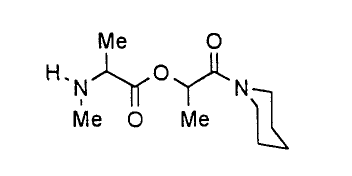

3,3 g (9,9 mMol) N-Benzyl-N-methyl-L-alanyl-D-milchsäurepiperidid werden in 100 ml Ethanol in Gegenwart von 0,35 g Pd(OH)₂-Kohle [20% Pd Gehalt] bis zur Beendigung der Wasserstoffaufnahme hydriert (ca. 4 Stunden). Nach Abfiltrieren des Katalysators wird die gesamte Reaktionslösung im Vakuum eingeengt. Man erhält 2,4 g (100 % der Theorie) N-Methyl-L-alanyl-D-milchsäurepiperidid.
GC-MS m/z (%): 243 (MH⁺, 100); 158 (32)

### Beispiel ( I-3 )

### N-Benzyl-N-methyl-L-alanyl-D-milchsäuremethylester

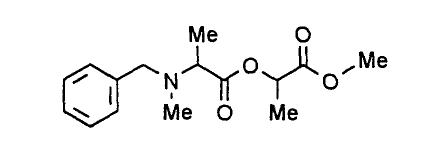

Die Kupplungsreaktion erfolgr analog der Reaktionsvorschrift des Beispiels 1 unter Verwendung von:
18,5 g (95,7 mMol) N-Benzyl-N-methyl-L-alanin,
10,0 g (95,7 mMol) (R)-(+)-Milchsäuremethylester,
300 ml absol. Methylenchlorid,
36,7 g (284,5 mMol) N,N-Diisopropylethylamin ("Hünigs Base"),
29,0 g Bis(2-oxo-3-oxazolidinyl)-phosphoniumsäurechlorid (BOP-Cl).

Das zurückbleibende Rohprodukt wird über eine Kieselgelsäule (Kieselgel 60 - Merck, Korngröße : 0,04 bis 0,063 mm) mit dem Fließmittel Cyclohexan : Essigsäureethylester (3 : 1 ) chromatographiert. Man erhält 5,6 g (20,9 % der Theorie) N-Benzyl-N-methyl-L-alanyl-D-milchsäure-methylester.
EI-MS m/z (%): 279 (M⁺, 11); 148 (Ph-CH₂-NMe-CHMe, 100); 91 (Ph-CH₂-; 99).

Analog zu den Beispielen (I-1) bis (I-3) können die in der nachstehenden Tabelle 2 aufgeführten Ausgangsstoffe der allgemeinen Formel (I) hergestellt werden.

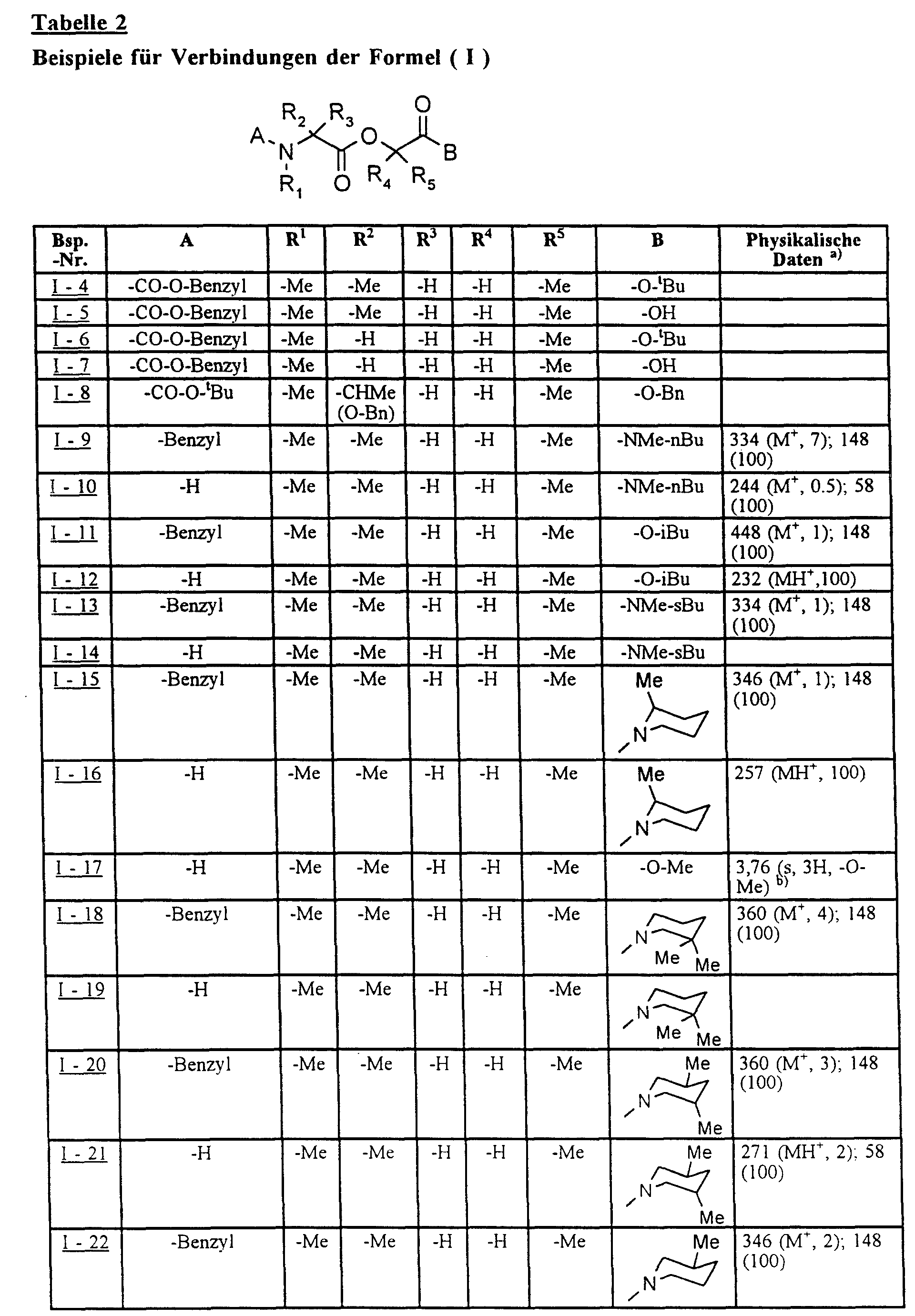

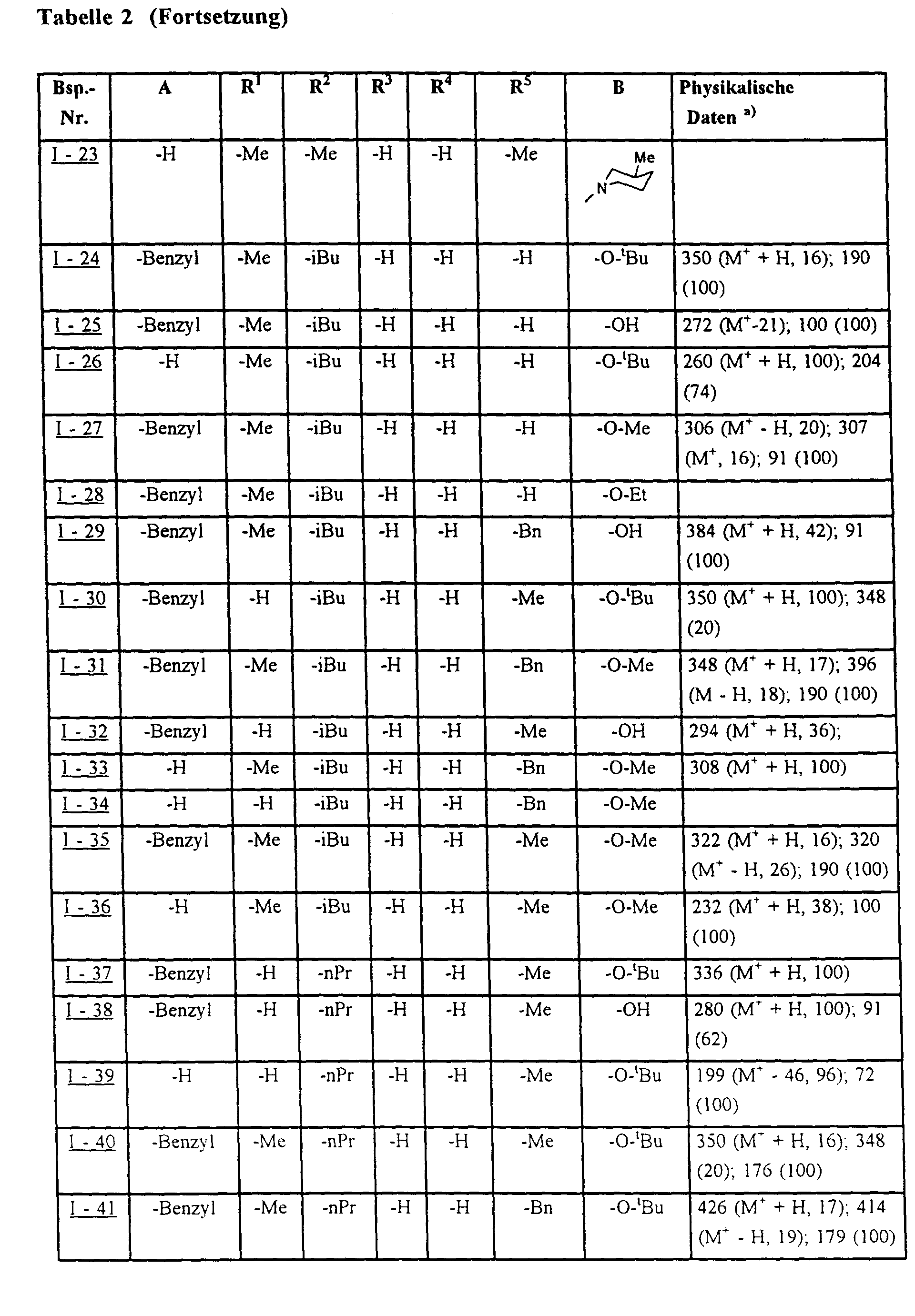

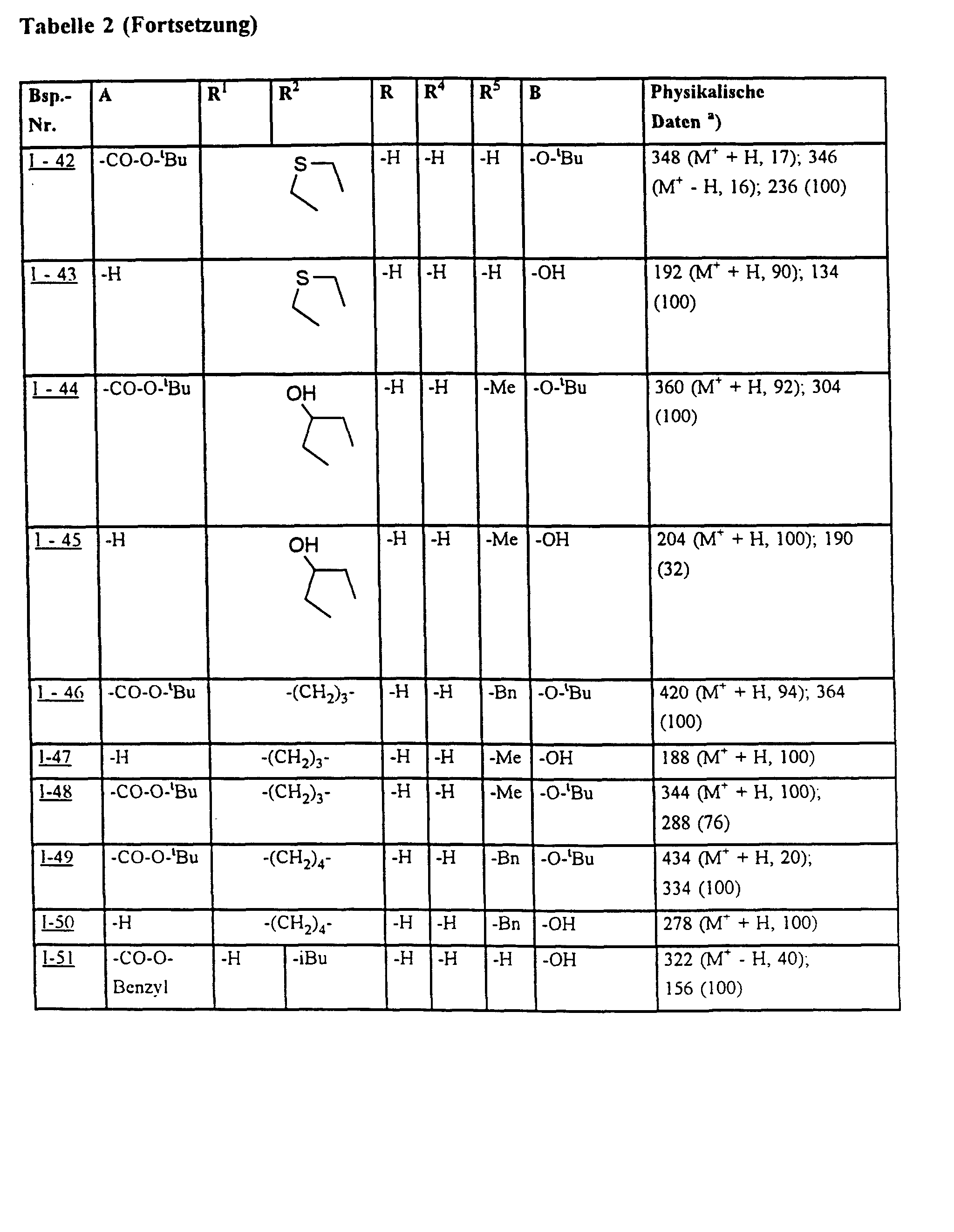

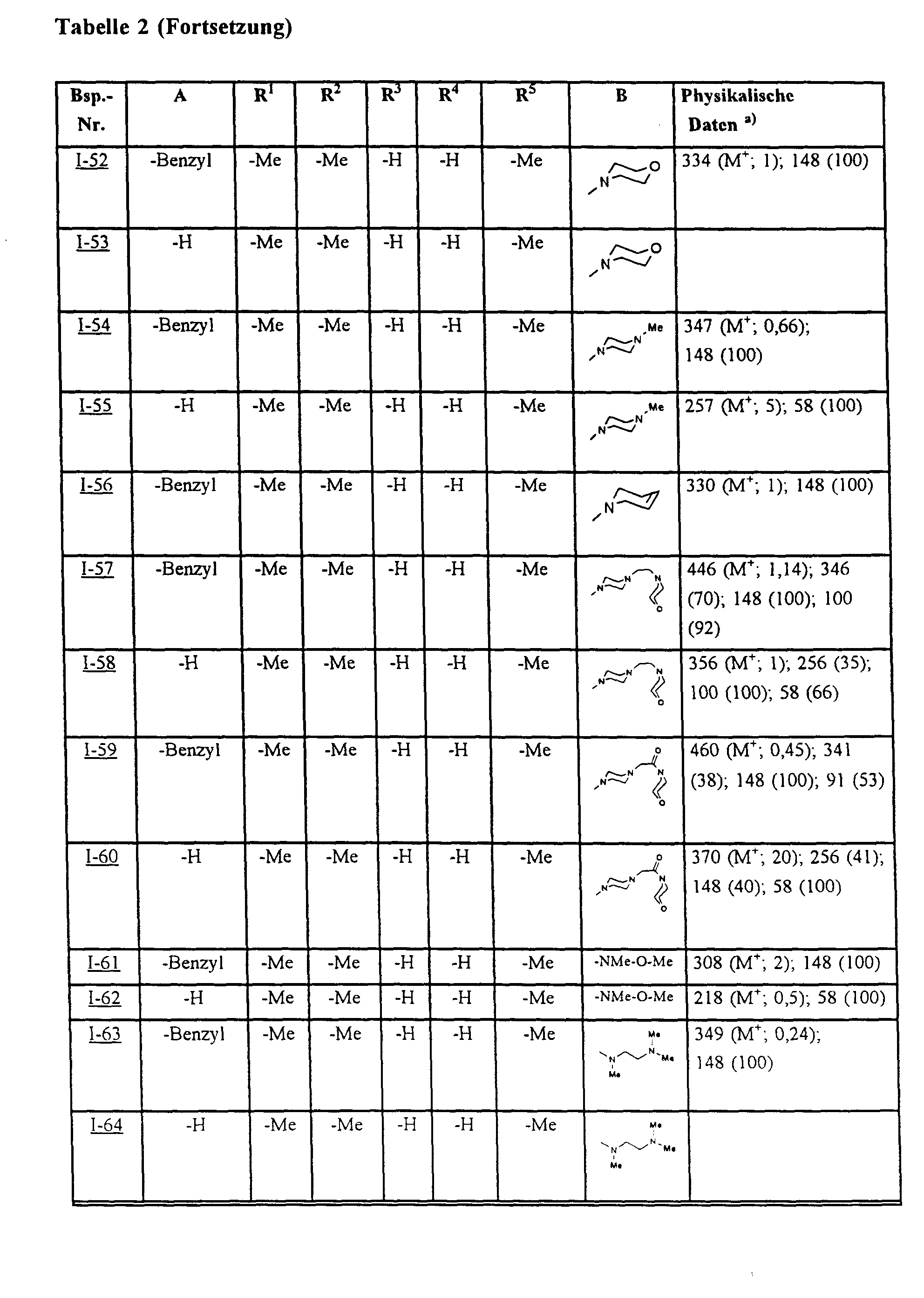

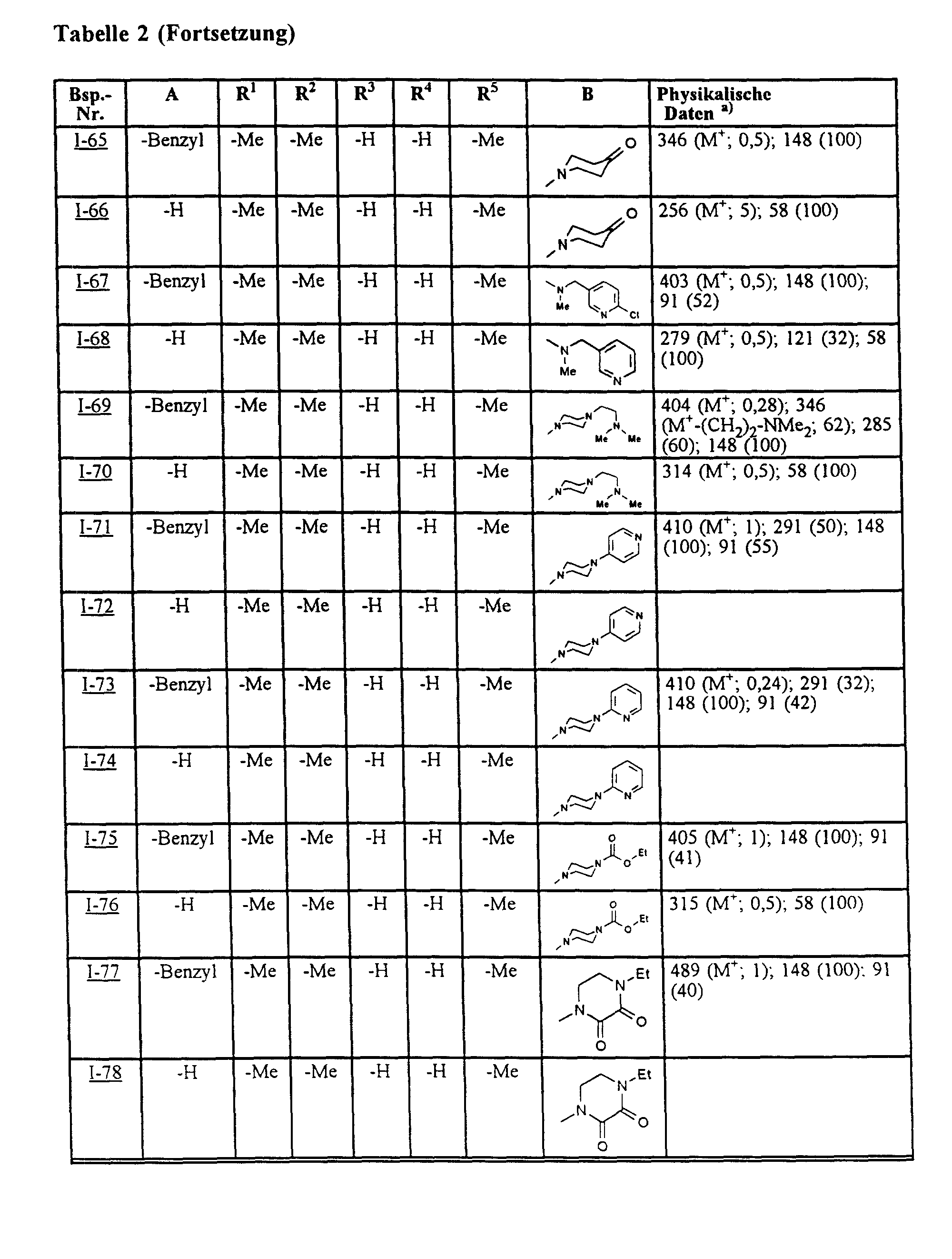

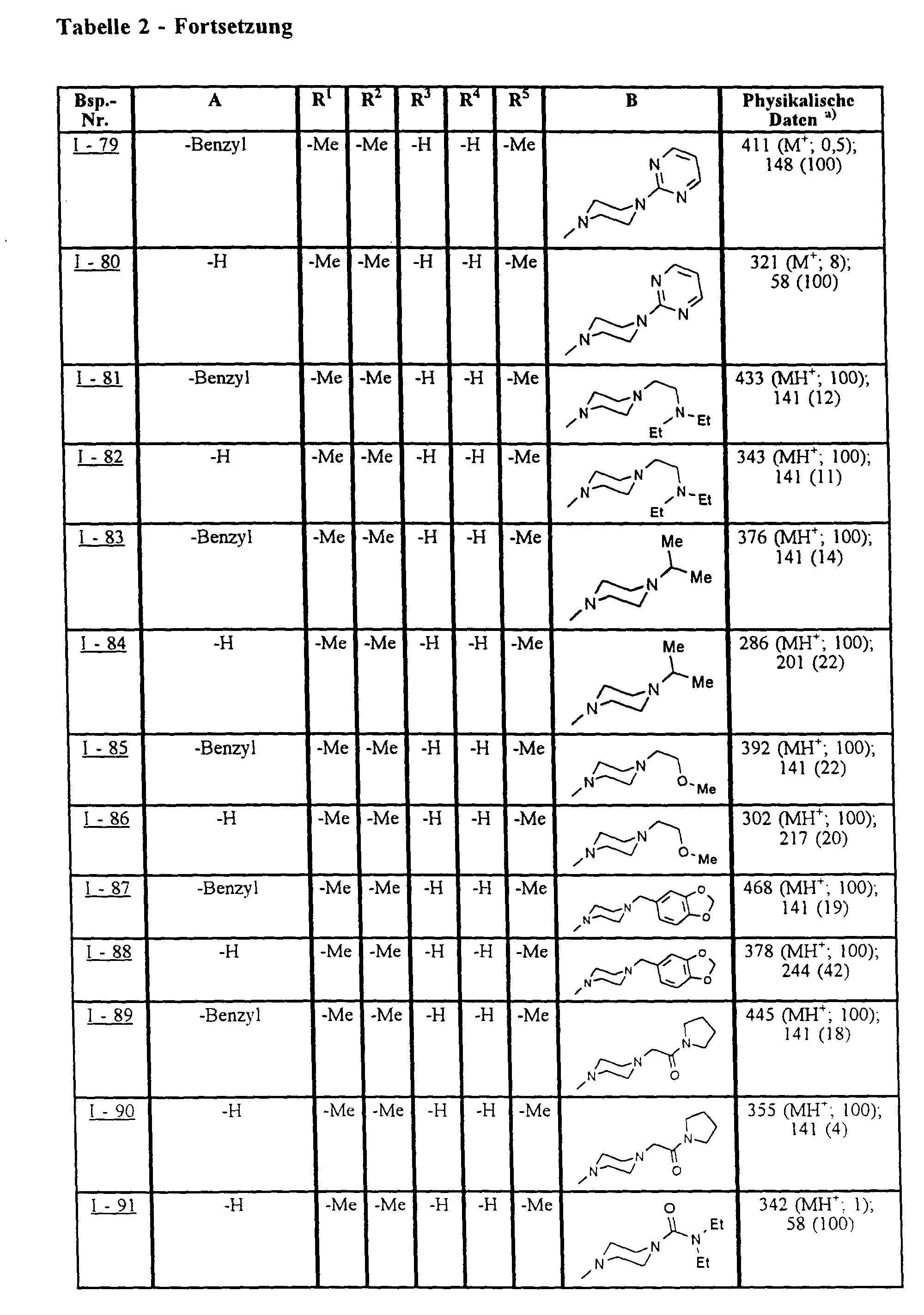

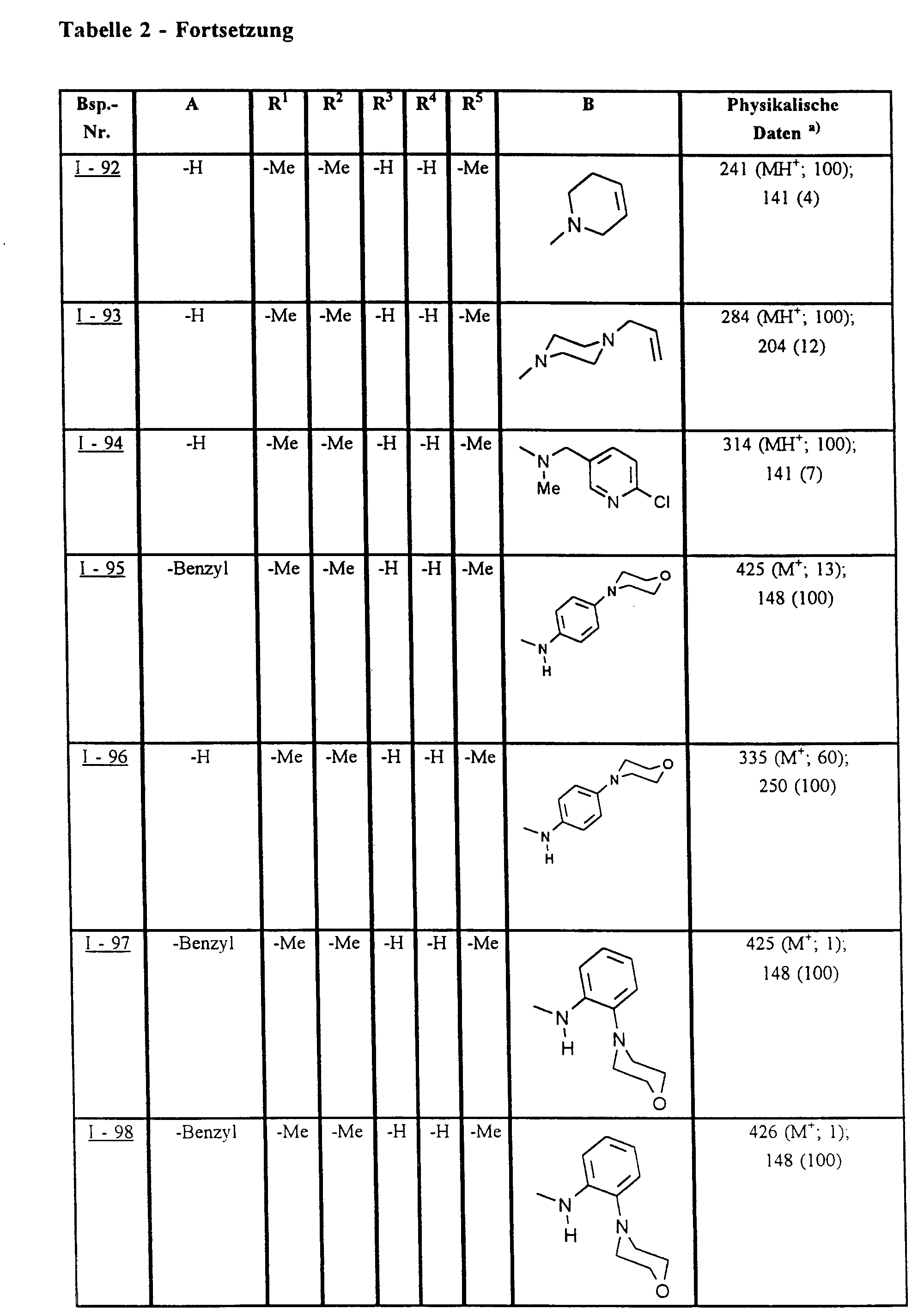

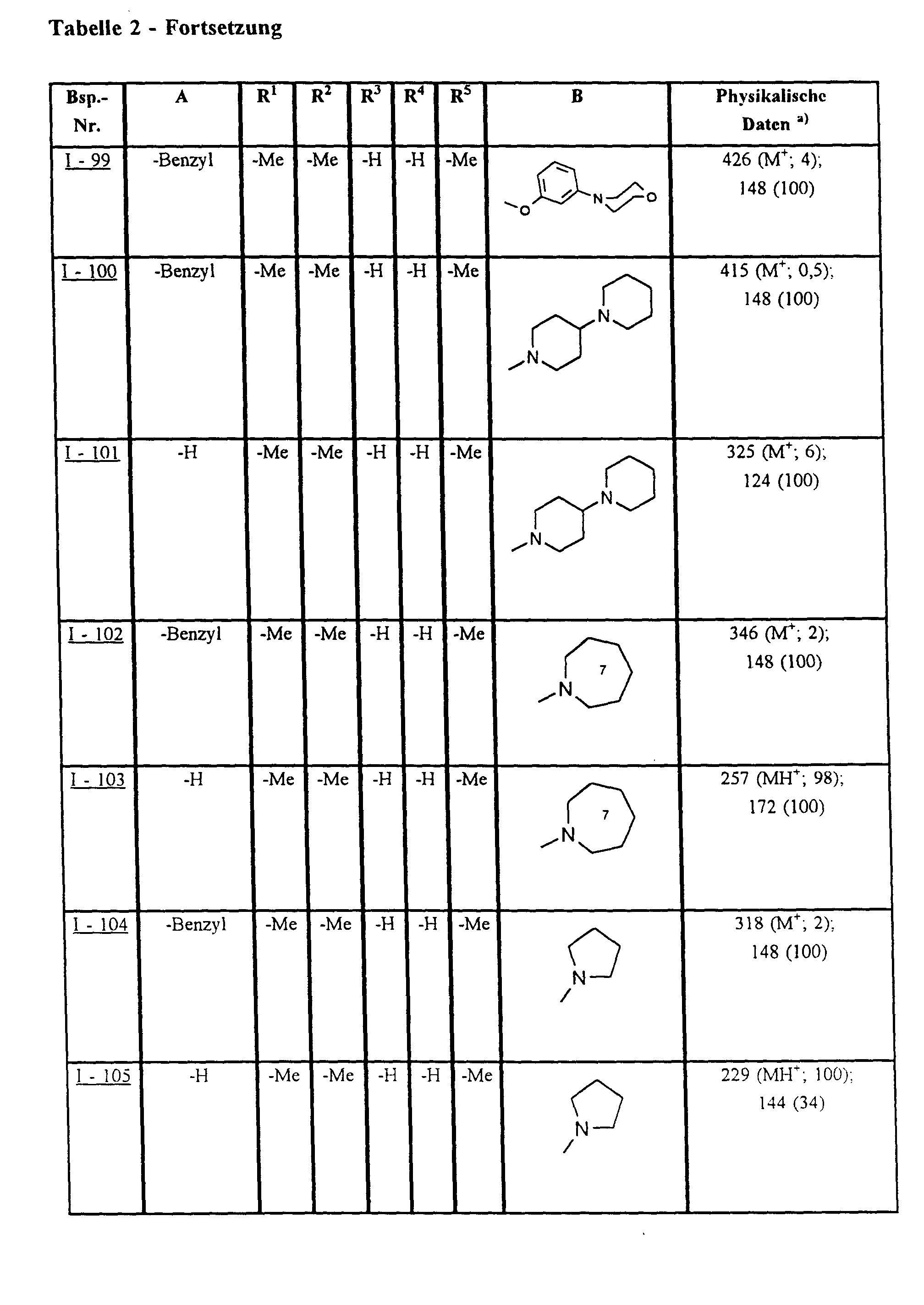

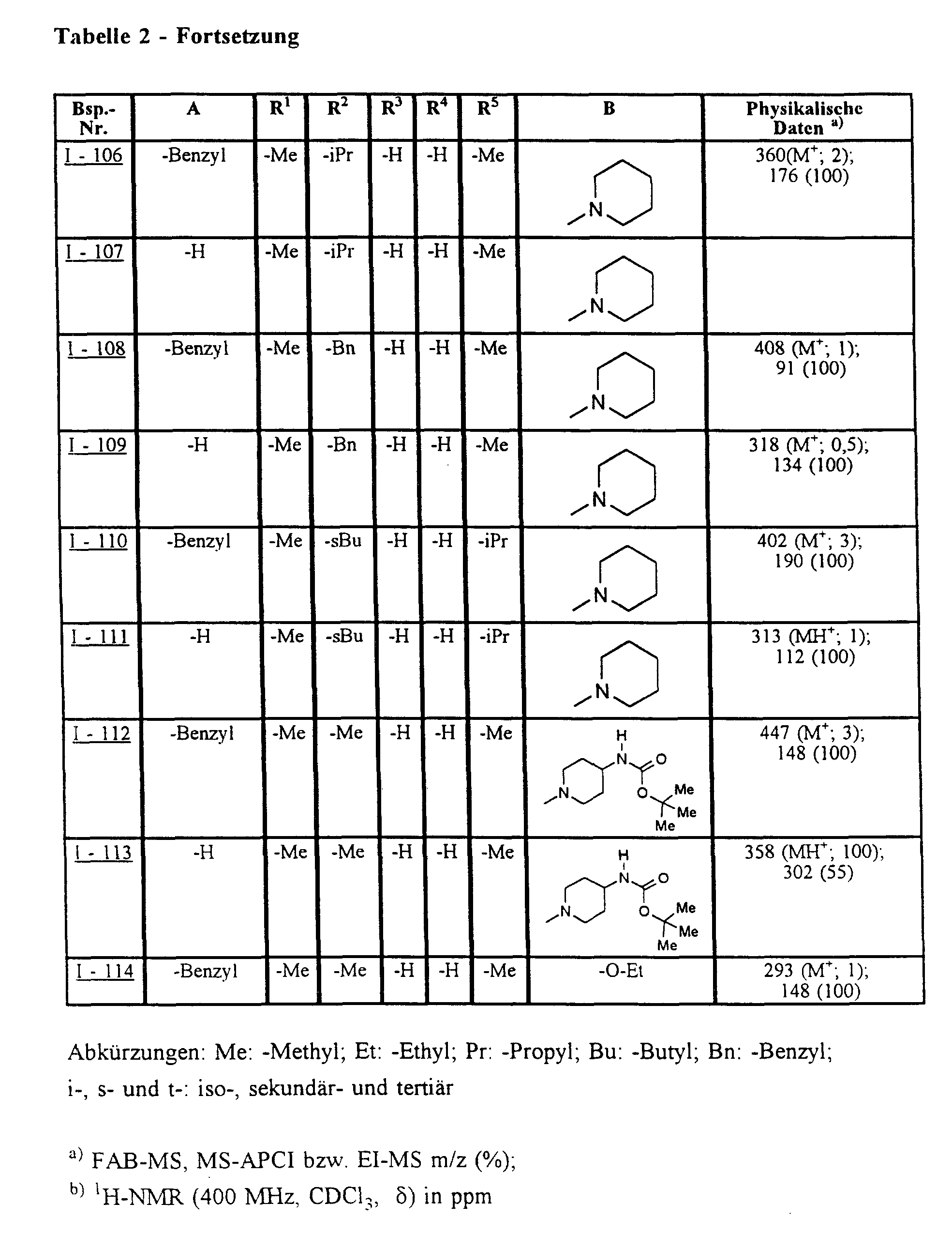

## Patentansprüche

1. Verwendung von Didepsipeptiden der allgemeinen Formel (I) und deren Salze, 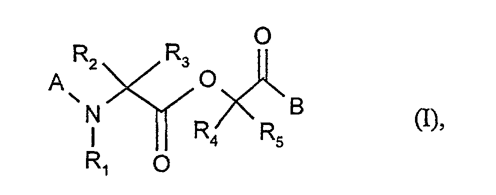 in welcher
R¹ für Wasserstoff oder geradkettiges oder verzweightes Alkl, steht,
R¹ und R² gemeinsam mit den Atomen, an die sie gebunden sind, für einen 5- oder 6-gliedrigen Ring stehen, der gegebenfalls durch Sauerstoff, Schwefel, Sulfoxyl oder Sulfonyl unterbrochen sein kann und gegebenenfalls substituiert ist,
R² und R³ unabhängig voneinander für Wasserstoff, geradkettiges oder verzweigtes Alkyl, Alkenyl, Cycloalkyl, Cycloalkylalkyl, Aryl, Arylalkyl, Heteroaryl, Heteroarylalkyl stehen, die gegebenenfalls substituiert sind, oder
R² und R³ gemeinsam für einen spirocyclischen Ring stehen, der gegebenenfalls substituiert ist,
R⁴ und R⁵ unabhängig voneinander für Wasserstoff, geradkettiges oder verzweigtes Alkyl, Alkenyl, Cycloalkyl, Cycloalkylalkyl, Aryl, Arylalkyl, Heteroaryl, Heteroarylalkyl stehen, die gegebenenfalls substituiert sind, oder
R⁴ und R⁵ gemeinsam für einen spirocyclischen Ring stehen, der gegebenenfalls substituiert ist,
A für Wasserstoff, Alkyl, Aralkyl, Formyl, Alkoxydicarbonyl oder für einen Rest der Gruppe G¹ 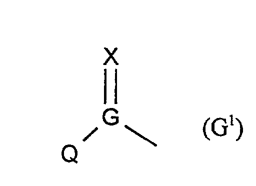 steht,
worin 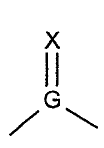 Carboxy, Thiocarboxy, -C=CH-NO₂, -C=CH-CN,
-C=N-R⁶, Sulfoxyl, Sulfonyl, -P(O)-OR⁷ oder P(S)-OR⁷ bedeuten kann,
R⁶ für Wasserstoff, Hydroxy, Alkoxy, Alkylcarbonyl, Halogenalkylcarbonyl, Alkylsulfonyl, Nitro oder Cyan steht, und
R⁷ für Wasserstoff oder Alkyl steht, und
Q für geradkettiges oder verzweigtes Alkyl, Alkenyl, Alkinyl, Cycloalkyl, Aryl, Arylalkyl, Hetaryl oder Hetarylalkyl, die gegebenenfalls substituiert sind, oder gegebenenfalls für einen Rest aus der Gruppe G² und G³
R⁸-Y- (G²) 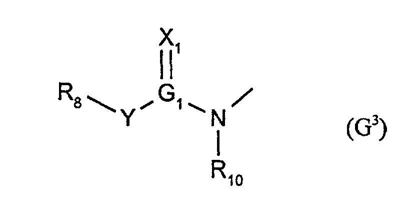
steht,
worin 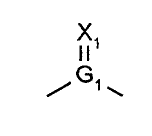 Carboxy, Thiocarboxy oder Sulfonyl bedeuten kann,
Y für Sauerstoff, Schwefel oder -NR⁹ steht,
R⁸ für den Fall, daß Y für Stickstoff steht, eine über ein Stickstoffatom verknüpfte cyclische Aminogruppe bedeuten kann,
R⁸ und R⁹ unabhängig voneinander für Wasserstoff, geradkettiges oder verzweigtes Alkyl, Alkenyl, Alkinyl, Cycloalkyl, Cycloalkylalkyl, Aryl, Arylalkyl, Hetaryl, Hetarylalkyl stehen, die gegebenenfalls substituiert sind, oder
R⁸ und R⁹ gemeinsam mit dem angrenzenden N-Atom für ein carbocyclisches 5-, 6- oder 7-gliedriges Ringsystem oder für ein 7 bis 10-gliedriges bicyclisches Ringsystem, das gegebenenfalls auch durch Sauerstoff, Schwefel, Sulfoxyl, Sulfonyl, Carbonyl, -N-O-, -N=, -NR¹¹- oder durch quaternisierten Stickstoff unterbrochen sein kann und gebenenfalls substituiert ist,
R¹⁰ für Wasserstoff oder Alkyl steht,
R¹¹ für Wasserstoff, geradkettiges oder verzweigtes Alkyl, Alkenyl, Alkinyl, Cycloalkyl, Cycloalkylalkyl, Alkoxycarbonyl, Alkylcarbonyl, Cycloalkylcarbonyl, Cyan, Aryl, Arylalkyl, Hetaryl, Hetarylalkyl stehen, die gegebenenfalls substituiert sind, steht, und
B für Hydroxy, Alkoxy, Alkenyloxy, Alkinyloxy, Cycloalkyloxy, Cycloalkylalkyloxy, Aryloxy-, Arylalkyloxy, Hetaryloxy, Hetarylalkyloxy stehen, die gegebenenfalls substituiert sind, steht, oder
für die Reste -NR¹²R¹³, -NR¹⁴-NR¹²R¹³ und -NR¹⁵-OR¹⁶ steht,
in denen
R¹² und R¹³ unabhängig voneinander für Wasserstoff, geradkettiges oder verzweigtes Alkyl, Alkylcarbonyl, Alkylsulfonyl, Alkenyl, Alkinyl, Cycloalkyl, Cycloalkylalkyl, Aryl, Arylcarbonyl, Arylsulfonyl, Arylalkyl, Hetaryl, Hetarylcarbonyl, Hetarylsulfonyl oder Hetarylalkyl, die gegebenenfalls substituiert sind, stehen oder
R¹² und R¹³ gemeinsam mit dem angrenzenden N-Atom für ein carbocyclisches 5-, 6-, 7- oder 8-gliedriges Ringsystem oder für ein 7 bis 10-gliedriges bicyclisches Ringsystem, das gegebenenfalls auch durch Sauerstoff, Schwefel, Sulfoxyl, Sulfonyl, Carbonyl, -N-O-, -N=, -NR¹¹- oder durch quaternisierten Stickstoff unterbrochen sein kann und gebenenfalls substituiert ist,
R¹⁴ für Wasserstoff, geradkettiges oder verzweigtes Alkyl, Cycloalkyl, Arylalkyl oder Hetarylalkyl, die gegebenenfalls substituiert sind, steht,
R¹⁵ und R¹⁶ unabhängig voneinander Wasserstoff, geradkettiges oder verzweigtes Alkyl, Alkylcarbonyl, Alkenyl, Alkinyl, Cycloalkyl, Cycloalkylalkyl, Arylalkyl oder Hetarylalkyl, die gegebenenfalls substituiert sind, bedeuten,
R¹⁵ und R¹⁶ gemeinsam mit der angrenzenden N-O-Gruppe für einen carbocyclischen 5-, 6- oder 7-gliedrigen Ring stehen,
sowie deren optische Isomere und Racemate,
zur Herstellung eines Mittels
zur Bekämpfung von Endoparasiten in der Medizin und Tiermedizin.

2. Neue Didepsipeptide der allgemeinen Formel (Ia) und deren Salze, 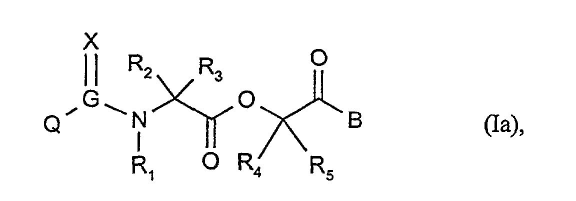 in welcher
R¹ für Wasserstoff oder geradkettiges oder verzweigtes C₁₋₄-Alkyl steht, und
R¹ und R² gemeinsam mit den Atomen, an die sie gebunden sind, für einen 5- oder 6-gliedrigen Ring stehen, der gegebenfalls durch Sauerstoff, Schwefel, Sulfoxyl oder Sulfonyl unterbrochen sein kann und gegebenenfalls substituiert ist,
R² für Wasserstoff, geradkettiges oder verzweigtes Alkyl mit bis zu 6 Kohlenstoffatomen, Alkenyl mit bis zu 4 Kohlenstoffatomen, C₃₋₆-Cycloalkyl, C₃₋₆-Cycloalkyl-C₁₋₂-alkyl, Aryl, Aryl-C₁₋₂-alkyl, Hetaryl, Hetaryl-C₁₋₂-alkyl die gegebenenfalls substituiert sind, steht,
R³ und R⁴ für Wasserstoff stehen,
R⁵ für Wasserstoff, geradkettiges oder verzweigtes Alkyl mit bis zu 6 Kohlenstoffatomen, Alkenyl mit bis zu 4 Kohlenstoffatomen, C₃₋₆-Cycloalkyl, C₃₋₆-Cycloalkyl-C₁₋₂-alkyl, Aryl, Aryl-C₁₋₂-alkyl, Hetaryl, Hetaryl-C₁₋₂-alkyl die gegebenenfalls substituiert sind, steht, 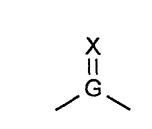 für Carboxy, Thiocarboxy, -C=CH-NO₂, -C=CH-CN,
-C=N-R⁶, Sulfoxyl, Sulfonyl, -P(O)-OR⁷ oder P(S)-OR⁷ steht,
R⁶ für Wasserstoff, Hydroxy, C₁₋₄-Alkoxy, C₁₋₄-Alkylcarbonyl, C₁₋₄-Halogenalkylcarbonyl, C₁₋₄-Alkylsulfonyl, Nitro oder Cyan steht, und
R⁷ für Wasserstoff oder C₁₋₄-Alkyl steht, und
Q für geradkettiges oder verzweigtes C₁₋₆-Alkyl, C₂₋₆-Alkenyl, C₂₋₆-Alkinyl, C₃₋₆-Cycloalkyl oder Hetaryl-C₁₋₂-alkyl, die gegebenenfalls substituiert sind, oder gegebenenfalls für einen Rest aus der Gruppe G² und G³
R⁸-Y- (G²) 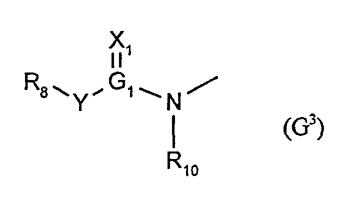
steht,
worin 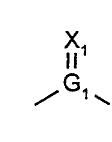 Carboxy, Thiocarboxy oder Sulfonyl bedeuten kann,
Y für Sauerstoff, Schwefel oder -NR⁹ steht,
R⁸ für den Fall, daß Y für Stickstoff steht, eine über ein Stickstoffatom verknüpfte cyclische Aminogruppe bedeuten kann,
R⁸ und R⁹ unabhängig voneinander für Wasserstoff, geradkettiges oder verzweigtes C₁₋₆-Alkyl, C₂₋₆-Alkenyl, C₂₋₆-Alkinyl, C₃₋₆-Cycloalkyl, C₃₋₆-Cycloalkyl-C₁₋₂-alkyl, Hetaryl oder Hetaryl-C₁₋₂-alkyl, die gegebenenfalls substituiert sind, steht, oder
R⁸ und R⁹ gemeinsam mit dem angrenzenden N-Atom für ein carbocyclisches 5-, 6- oder 7-gliedriges Ringsystem oder für ein 7 bis 10-gliedriges bicyclisches Ringsystem stehen, das gegebenenfalls auch durch Sauerstoff, Schwefel, Sulfoxyl, Sulfonyl, Carbonyl, -N-O-, -N=, -NR¹¹- oder durch quaternisierten Stickstoff unterbrochen sein kann und gebenenfalls substituiert ist,
R¹⁰ für Wasserstoff oder C₁₋₄-Alkyl steht, und
R¹¹ für Wasserstoff, geradkettiges oder verzweigtes C₁₋₆-Alkyl, C₃₋₆-Cycloalkyl, C₂₋₆-Alkenyl, C₂₋₆-Alkinyl, C₃₋₆-Cycloalkyl, C₃₋₆-Cycloalkyl-C₁₋₂-alkyl, C₁₋₄-Alkoxycarbonyl, C₁₋₄-Alkylcarbonyl, C₃₋₆-Cycloalkylcarbonyl, Cyan, Aryl, Aryl-C₁₋₂-alkyl, Hetaryl oder Hetaryl-C₁₋₂-alkyl, die gegebenenfalls substituiert sind, steht, und
B für C₁₋₆-Alkoxy, C₂₋₆-Alkenyloxy, C₂₋₆-Alkinyloxy, C₃₋₇-Cycloalkyloxy, C₃₋₇-Cycloalkyl-C₁₋₂-alkyloxy, Aryloxy-, Aryl-C₁₋₂-alkyloxy, Hetaryloxy, Hetaryl-C₁₋₂-alkyloxy, die gegebenenfalls substituiert sind, steht, oder
für die Aminoreste -NR¹²R¹³, -NR¹⁴-NR¹²R¹³ und -NR¹⁵-OR¹⁶ steht, in denen
R¹² und R¹³ unabhängig voneinander für Wasserstoff, geradkettiges oder verzweigtes C₁₋₆-Alkyl, C₁₋₆-Alkylcarbonyl, C₁₋₆-Alkylsulfonyl, C₂₋₆-Alkenyl, C₂₋₆-Alkinyl, C₃₋₈-Cycloalkyl, C₃₋₈-Cycloalkyl-C₁₋₂-alkyl, Aryl, Arylcarbonyl, Arylsulfonyl, Aryl-C₁₋₂-alkyl, Hetaryl, Hetarylcarbonyl, Hetarylsulfonyl oder Hetaryl-C₁₋₂-alkyl, die gegebenenfalls substituiert sind, stehen, oder
R¹² und R¹³ gemeinsam mit dem angrenzenden N-Atom für ein carbocyclisches 5-, 6-, 7- oder 8-gliedriges Ringsystem oder für ein 7 bis 10-gliedriges bicyclisches Ringsystem stehen, das gegebenenfalls auch durch Sauerstoff, Schwefel, Sulfoxyl, Sulfonyl, Carbonyl, -N-O-, -N=, -NR¹¹- oder durch quaternisierten Stickstoff unterbrochen sein kann und gebenenfalls substituiert ist,
R¹⁴ für Wasserstoff, geradkettiges oder verzweigtes C₁₋₆-Alkyl, C₃₋₆-Cycloalkyl, die gegenenenfalls substituiert sind, steht,
R¹⁵ und R¹⁶ unabhängig voneinander Wasserstoff, geradkettiges oder verzweigtes C₁₋₆-Alkyl, C₁₋₆-Alkylcarbonyl, C₂₋₆-Alkenyl, C₂₋₆-Alkinyl, oder C₃₋₆-Cycloalkyl, die gegebenenfalls substituiert sind, bedeuten, und
R¹⁵ und R¹⁶ gemeinsam mit der angrenzenden N-O-Gruppe für einen carbocyclischen 5-, 6- oder 7-gliedrigen Ring stehen,
mit der Maßgabe für den Fall, daß in Formel (Ia) R¹, R⁵ und =G=X gemeinsam für folgende Reste stehen:
R¹ steht für Wasserstoff und Methyl,
R⁵ steht für Wasserstoff, 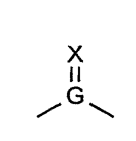
steht für Carboxy,
die Reste Q und B folgende Bedingung erfüllen müssen:
Q steht für andere Reste als Methyl,
B steht für andere Reste als -NH₂
sowie mit der Maßgabe für den Fall, daß in Formel (Ia) G² und =G=X gemeinsam für folgende Reste stehen: 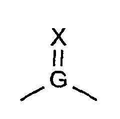 steht für Carboxy,
G² steht für tert-Butyloxy, Benzyloxy und 4-Nitro-benzyloxy,
der Rest B für andere Reste als tert-Butyloxy, Benzyloxy und 4-Nitrobenzyloxy steht, sowie mit der Maßgabe, daß wenn in Formel (Ia) R⁴ und R⁵ für Wasserstoff stehen, B nicht für NH₂ stehen darf,
sowie mit der Maßgabe, daß wenn in Formel (Ia) R¹ bis R⁵ für B Wasserstoff stehen und 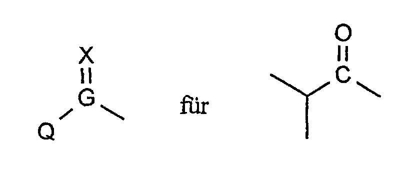 steht,
B nicht für Methylamino steht,
sowie mit der Maßgabe, daß wenn in Formel (Ia) 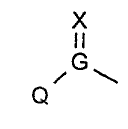 für Benzyloxycarbonyl steht, oder 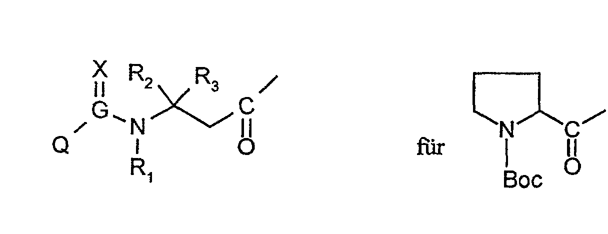 steht,
B nicht für Benzylamino steht,
sowie mit der Maßgabe, daß für den Fall, daß in Formel (Ia) 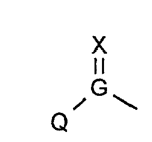 für tert.-Butyloxycarbonyl steht, und R¹ bis R³ für H, und entweder R⁴ oder R⁵ für Methyl und das andere für H steht,
B nicht für Ethoxy steht,
sowie mit der Maßgabe, daß die Verbindung der Formel 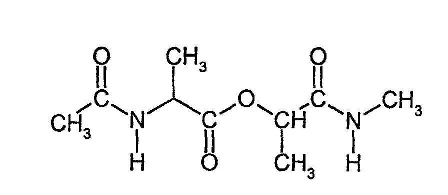 ausgeschlossen ist,
ferner mit der Maßgabe, daß die Verbindungen
Methyl-O-(N-carbobenzoxy-L-Leucyl)-hydroxyethanoat,
Methyl-O-(N-carbobenzoxy-L-Leucyl)-(S)-2-hydroxy-3-phenylpropanoat,
Methyl-O-(N-carbobenzoxy-L-Leucyl)-(S)-2-hydroxypropanoat,
Methyl-O-(N-carbobenzoxy-L-Leucyl)-(S)-2-hydroxy-4-methyl-pentanoat,
Ethyl-N-(benzyloxycarbonyl)-amino-2-(O-glycyl)-glycolat,
Ethyl-N-(benzyloxycarbonyl)-amino-2-(O-glycyl)-lactat,
Ethyl-N-(benzyloxycarbonyl)-amino-2-(O-alanyl)-glycolat,
Ethyl-N-(benzyloxycarbonyl)-amino-2-(O-alanyl)lactat
ausgeschlossen sind,
sowie deren optische Isomere und Racemate.

3. Verfahren zur Herstellung der neuen Didepsipeptide der allgemeinen Formel (Ia) und deren Salze gemäß Anspruch 2 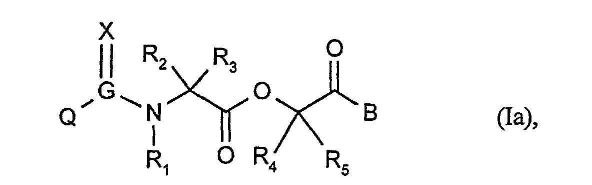 in welcher
die Reste R¹, R², R³, R⁴, R⁵, G, Q, X und B die in Anspruch 2 angegebene Bedeutung haben,
**dadurch gekennzeichnet, daß** man
a) N-terminal substituierte Aminosäuren der allgemeinen Formel (II) 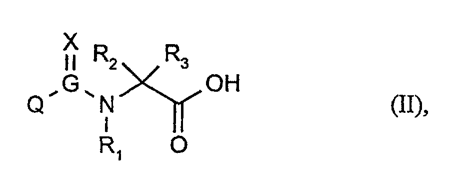 in welcher
die Reste R¹, R², R³, G, Q, und X die in Anspruch 2 angegebene Bedeutung haben, bzw. deren carboxyaktivierten Derivate oder deren Alkalimetallsalze, gegebenenfalls in Gegenwart eines Katalysators, gegebenenfalls in Gegenwart eines Säurebindemittels und gegebenenfalls in Gegenwart eines Vedünnungsmittels, mit Carbonsäurederivaten der allgemeinen Formel (III) 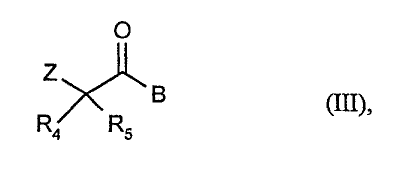 in welcher
die Reste R⁴, R⁵ und B die in Anspruch 2 angegebene Bedeutung haben und Z für eine geeignete Abgangsgruppe beispielsweise Halogen wie Brom, Chlor Fluor, oder für Hydroxyl steht, umsetzt oder
b) N-terminal deblockierte Didepsipeptide der allgemeinen Formel (Ib) 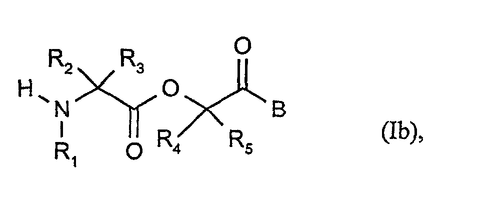 in welcher
die Reste R¹, R², R³, R⁴, R⁵ und B die in Anspruch 2 angegebene Bedeutung haben, gegebenenfalls in Gegenwart eines Katalysators, gegebenenfalls in Gegenwart eines Säurebindemittels und gegebenenfalls in Gegenwart eines Vedünnungsmittels, mit Verbindungen der allgemeinen Formel (IV) 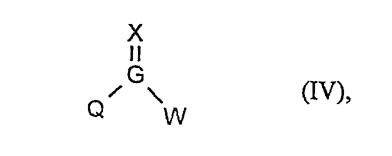 in welcher
die Reste G, Q, W und X die in Anspruch 2 angegebene Bedeutung haben und W für eine geeignete Abgangsgruppe, wie beispielsweise Halogen, Alkoxy, Alkylthio oder Aryloxy steht, umsetzt oder
c) N-terminal deblockierte Didepsipeptide der allgemeinen Formel (Ib) 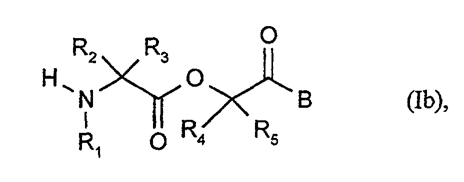 in welcher
die Reste R¹, R², R³, R⁴, R⁵ und B die in Anspruch 2 angegebene Bedeutung haben, gegebenenfalls in Gegenwart eines Katalysators, gegebenenfalls in Gegenwart eines Säurebindemittels und gegebenenfalls in Gegenwart eines Vedünnungsmittels, mit Verbindungen der allgemeinen Formeln (V) oder (VI)
R⁸-N=C=X (V) 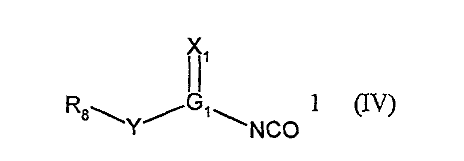
in welchen
die Reste R⁸, G¹, X, X¹ und Y die in Anspruch 2 angegebene Bedeutung haben, umsetzt oder
zur Darstellung der Depsipeptide der allgemeinen Formel (Ia) und deren Salze
in der die Gruppe 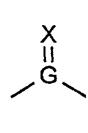 für Carboxy und Y für Sauerstoff steht,
d) N-terminal deblockierte Didepsipeptide der allgemeinen Formel (Ib) 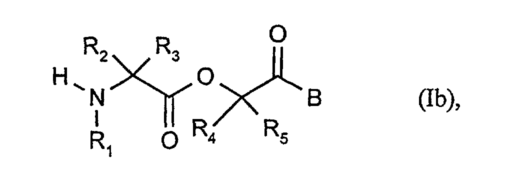 in welcher
die Reste R¹, R², R³, R⁴, R⁵ und B die in Anspruch 2 angegebene Bedeutung haben,
in einem ersten Reaktionsschritt mit Kohlendioxid und einem Alkalimetallcarbonat der Formel (VII)
M₂CO₃ (VII)
in welcher
M für ein einwertiges Alkalimetallkation, vorzugsweise Lithium, Natrium, Kalium oder Cäsium, insbesondere Kalium oder Cäsium steht,
dann in einem zweiten Reaktionsschritt das resultierende Alkalimetallsalz der Formel (VIII) 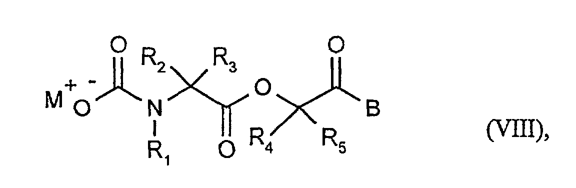 in welcher
die Reste R¹, R², R³, R⁴, R⁵ und B die in Anspruch 2 angegebene Bedeutung haben,
M für ein salzartig gebundenes Metallkationenäquivalent steht,
mit Alkylierungsmitteln der Formel ( IX )
R⁸-Hal (IX),
in welcher
R⁸ die in Anspruch 2 angegebene Bedeutung besitzt und
Hal für ein Halogen wie Fluor, Chlor, Brom oder Iod steht,
gegebenenfalls in Gegenwart eines Verdünnungsmittels und gegebenenfalls in Gegenwart eines basischen Reaktionshilfsmittels umsetzt, oder
e) Didepsipeptide der allgemeinen Formel (Ic) 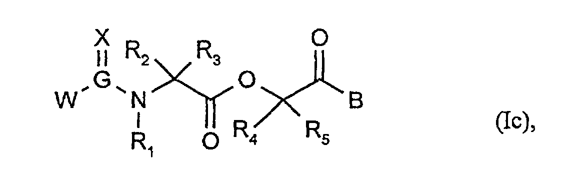 in welcher
die Reste R¹, R², R³, R⁴, R⁵, G, W, X und B und die in Anspruch 2 und 3b angegebene Bedeutung haben, gegebenenfalls in Gegenwart eines Katalysators, gegebenenfalls in Gegenwart eines Säurebindemittels und gegebenenfalls in Gegenwart eines Verdünnungsmittels, mit Verbindungen der allgemeinen Formel (X)
R⁸-Y-H (X)
in welcher
die Reste R⁸ und Y die weiter oben in Anspruch 2 angegebene Bedeutung haben, umsetzt oder
f) Didepsipeptide der allgemeinen Formel (Id) 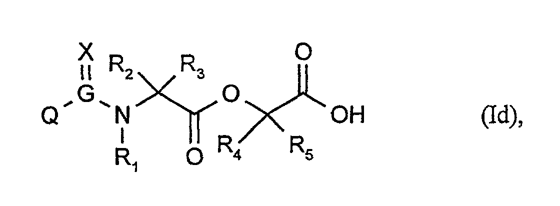
in welcher
die Reste R¹, R², R³, R⁴, R⁵, G, Q, X und B die in Anspruch 2 angegebene Bedeutung haben bzw. deren carboxyaktivierten Derivate oder deren Alkalimetallsalze, gegebenenfalls in Gegenwart eines Katalysators, gegebenenfalls in Gegenwart eines Säurebindemittels und gegebenenfalls in Gegenwart eines Verdünnungsmittels, mit Verbindungen der allgemeinen Formel (XI)
H-B (XI)
in welcher
der Rest B die weiter oben in Anspruch 2 angegebene Bedeutung hat, umsetzt.

4. Endoparasitizide Mittel, **gekennzeichnet durch** einen Gehalt an mindestens einem Didepsipeptid der Formel (I) gemäß Anspruch 1.

5. Verfahren zur Herstellung von endoparasitiziden Mitteln, **dadurch gekennzeichnet, daß** man Didepsipeptide der Formel (I) gemäß Anspruch 1 mit Streckmitteln und/oder oberflächenaktiven Mitteln vermischt.

## Claims

1. Use of didepsipeptides of the general formula (I) and their salts 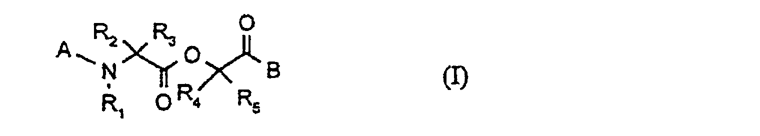 in which
R¹ represents hydrogen or straight-chain or branched alkyl,
R¹ and R² together with the atoms to which they are bonded represent a 5- or 6-membered ring which can optionally be interrupted by oxygen, sulphur, sulphoxyl or sulphonyl and is optionally substituted,
R² and R³ independently of one another represent hydrogen, straight-chain or branched alkyl, alkenyl, cycloalkyl, cycloalkylalkyl, aryl, arylalkyl, heteroaryl, heteroarylalkyl, each of which is optionally substituted, or
R² and R³ together represent a spirocyclic ring, which is optionally substituted,
R⁴ and R⁵ independently of one another represent hydrogen, straight-chain or branched alkyl, alkenyl, cycloalkyl, cycloalkylalkyl, aryl, arylalkyl, heteroaryl, heteroarylalkyl, each of which is optionally substituted, or
R⁴ and R⁵ together represent a spirocyclic ring, which is optionally substituted,
A represents hydrogen, alkyl, aralkyl, formyl, alkoxydicarbonyl or a radical of the group G¹ 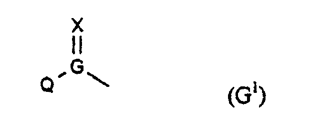 in which 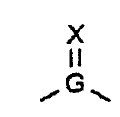 can denote carboxyl, thiocarboxyl, -C=CH-NO₂,
-C=CH-CN, -C=N-R⁶, sulphoxyl, sulphonyl, -P(O)-OR⁷ or P(S)-OR⁷,
R⁶ represents hydrogen, hydroxyl, alkoxy, alkylcarbonyl, halogenoalkylcarbonyl, alkylsulphonyl, nitro or cyano, and
R⁷ represents hydrogen or alkyl, and
Q represents straight-chain or branched alkyl, alkenyl, alkinyl, cycloalkyl, aryl, arylalkyl, hetaryl or hetarylalkyl, each of which is optionally substituted, or optionally represents a radical from the group G² and G³
R⁸-Y- (G²) 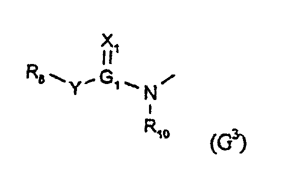
in which 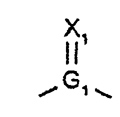 can denote carboxyl, thiocarboxyl or sulphonyl,
Y represents oxygen, sulphur or -NR⁹,
R⁸ in the case where Y represents nitrogen can denote a cyclic amino group linked via a nitrogen atom,
R⁸ and R⁹ independently of one another represent hydrogen, straight-chain or branched alkyl, alkenyl, alkinyl, cycloalkyl, cycloalkylalkyl, aryl, arylalkyl, hetaryl, hetarylalkyl, each of which is optionally substituted, or
R⁸ and R⁹ together with the adjacent N atom represent a carbocyclic 5-, 6- or 7-membered ring system or a 7 to 10-membered bicyclic ring system which can optionally also be interrupted by oxygen, sulphur, sulphoxyl, sulphonyl, carbonyl, -N-O-, -N=, -NR¹¹- or by quaternized nitrogen and is optionally substituted,
R¹⁰ represents hydrogen or alkyl,
R¹¹ represents hydrogen, straight-chain or branched alkyl, alkenyl, alkinyl, cycloalkyl, cycloalkylalkyl, alkoxycarbonyl, alkylcarbonyl, cycloalkylcarbonyl, cyano, aryl, arylalkyl, hetaryl, hetarylalkyl, each of which is optionally substituted, and
B represents hydroxyl, alkoxy, alkenyloxy, alkinyloxy, cycloalkyloxy, cycloalkylalkyloxy, aryloxy, arylalkyloxy, hetaryloxy, hetarylalkyloxy, each of which is optionally substituted, or
represents the radicals -NR¹²R¹³, -NR¹⁴-NR¹²R¹³ and -NR¹⁵-OR¹⁶,
in which
R¹² and R¹³ independently of one another represent hydrogen, straight-chain or branched alkyl, alkylcarbonyl, alkylsulphonyl, alkenyl, alkinyl, cycloalkyl, cycloalkylalkyl, aryl, arylcarbonyl, arylsulphonyl, arylalkyl, hetaryl, hetarylcarbonyl, hetarylsulphonyl or hetarylalkyl, each of which is optionally substituted, or
R¹² and R¹³ together with the adjacent N atom represent a carbocyclic 5-, 6-, 7- or 8-membered ring system or a 7 to 10-membered bicyclic ring system which can optionally also be interrupted by oxygen, sulphur, sulphoxyl, sulphonyl, carbonyl, -N-O-, -N=, -NR¹¹- or by quaternized nitrogen and is optionally substituted,
R¹⁴ represents hydrogen, straight-chain or branched alkyl, cycloalkyl, arylalkyl or hetarylalkyl, each of which is optionally substituted,
R¹⁵ and R¹⁶ independently of one another denote hydrogen, straight-chain or branched alkyl, alkylcarbonyl, alkenyl, alkinyl, cycloalkyl, cycloalkylalkyl, arylalkyl or hetarylalkyl, each of which is optionally substituted,
R¹⁵ and R¹⁶ together with the adjacent N-O- group represent a carbocyclic 5-, 6- or 7-membered ring,
and their optical isomers and racemates,
for the production of a composition for the control of endoparasites in medicine and veterinary medicine.

2. New didepsipeptides of the general formula (Ia) and their salts 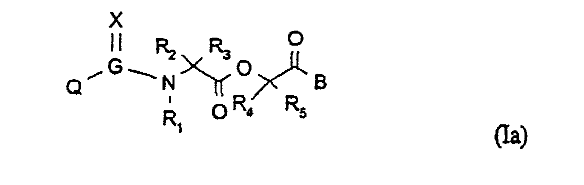 in which
R¹ represents hydrogen or straight-chain or branched C₁₋₄-alkyl, and
R¹ and R² together with the atoms to which they are bonded represent a 5- or 6-membered ring which can optionally be interrupted by oxygen, sulphur, sulphoxyl or sulphonyl and is optionally substituted,
R² represents hydrogen, straight-chain or branched alkyl having up to 6 carbon atoms, alkenyl having up to 4 carbon atoms, C₃₋₆-cycloalkyl, C₃₋₆-cycloalkyl-C₁₋₂-alkyl, aryl, aryl-C₁₋₂-alkyl, hetaryl, hetaryl-C₁₋₂-alkyl, each of which is optionally substituted,
R³ and R⁴ represent hydrogen,
R⁵ represents hydrogen, straight-chain or branched alkyl having up to 6 carbon atoms, alkenyl having up to 4 carbon atoms, C₃₋₆-cycloalkyl, C₃₋₆-cycloalkyl-C₁₋₂-alkyl, aryl, aryl-C₁₋₂-alkyl, hetaryl, hetaryl-C₁₋₂-alkyl, each of which is optionally substituted, 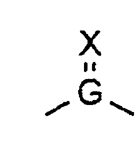 represents carboxyl, thiocarboxyl, -C=CH-NO₂,
-C=CH-CN, -C=N-R⁶, sulphoxyl, sulphonyl, -P(O)-OR⁷ or P(S)-OR⁷,
R⁶ represents hydrogen, hydroxyl, C₁₋₄-alkoxy, C₁₋₄-alkylcarbonyl, C₁₋₄-halogenoalkylcarbonyl, C₁₋₄-alkylsulphonyl, nitro or cyano, and
R⁷ represents hydrogen or C₁₋₄-alkyl, and
Q represents straight-chain or branched C₁₋₆-alkyl, C₂₋₆-alkenyl, C₂₋₆-alkinyl, C₃₋₆-cycloalkyl or hetaryl-C₁₋₂-alkyl, each of which is optionally substituted, or optionally represents a radical of the group G² and G³
R⁸-Y- (G²) 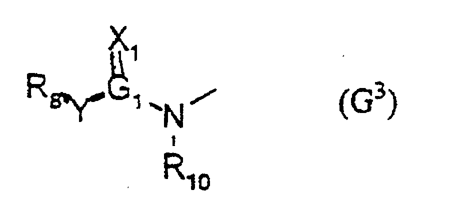
in which 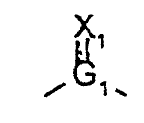 can denote carboxyl, thiocarboxyl or sulphonyl,
Y represents oxygen, sulphur or -NR⁹,
R⁸ in the case where Y represents nitrogen can denote a cyclic amino group linked via a nitrogen atom,
R⁸ and R⁹ independently of one another represent hydrogen, straight-chain or branched C₁₋₆-alkyl, C₂₋₆-alkenyl, C₂₋₆-alkinyl, C₃₋₆-cycloalkyl, C₃₋₆-cycloalkyl-C₁₋₂-alkyl, hetaryl or hetaryl-C₁₋₂-alkyl, each of which is optionally substituted, or
R⁸ and R⁹ together with the adjacent N atom represent a carbocyclic 5-, 6- or 7-membered ring system or a 7 to 10-membered bicyclic ring system which can optionally also be interrupted by oxygen, sulphur, sulphoxyl, sulphonyl, carbonyl, -N-O-, -N=, -NR¹¹- or by quatemized nitrogen and is optionally substituted,
R¹⁰ represents hydrogen or C₁₋₄-alkyl, and
R¹¹ represents hydrogen, straight-chain or branched C₁₋₆-alkyl, C₃₋₆-cycloalkyl, C₂₋₆-alkenyl, C₂₋₆-alkinyl, C₃₋₆-cycloalkyl, C₃₋₆-cycloalkyl-C₁₋₂-alkyl, C₁₋₄-alkoxycarbonyl, C₁₋₄-alkylcarbonyl, C₃₋₆-cycloalkylcarbonyl, cyano, aryl, aryl-C₁₋₂-alkyl, hetaryl or hetaryl-C₁₋₂-alkyl, each of which is optionally substituted, and
B represents C₁₋₆-alkoxy, C₂₋₆-alkenyloxy, C₂₋₆-alkinyloxy, C₃₋₇-cycloalkyloxy, C₃₋₇-cycloalkyl-C₁₋₂-alkyloxy, aryloxy, aryl-C₁₋₂-alkyloxy, hetaryloxy, hetaryl-C₁₋₂-alkyloxy, each of which is optionally substituted, or
represents the amino radicals -NR¹²R¹³, -NR¹⁴-NR¹²R¹³ and -NR¹⁵-OR¹⁶, in which
R¹² and R¹³ independently of one another represent hydrogen, straight-chain or branched C₁₋₆-alkyl, C₁₋₆-alkylcarbonyl, C₁₋₆-alkylsulphonyl, C₂₋₆-alkenyl, C₂₋₆-alkinyl, C₃₋₈-cycloalkyl, C₃₋₈-cycloalkyl-C₁₋₂-alkyl, aryl, arylcarbonyl, arylsulphonyl, aryl-C₁₋₂-alkyl, hetaryl, hetarylcarbonyl, hetarylsulphonyl or hetaryl-C₁₋₂-alkyl, each of which is optionally substituted, or
R¹² and R¹³ together with the adjacent N atom represent a carbocyclic 5-, 6-, 7- or 8-membered ring system or a 7 to 10-membered bicyclic ring system, which can optionally also be interrupted by oxygen, sulphur, sulphoxyl, sulphonyl, carbonyl, -N-O-, -N=, -NR¹¹- or by quaternized nitrogen and is optionally substituted,
R¹⁴ represents hydrogen, straight-chain or branched C₁₋₆-alkyl, C₃₋₆-cycloalkyl, each of which is optionally substituted,
R¹⁵ and R¹⁶ independently of one another denote hydrogen, straight-chain or branched C₁₋₆-alkyl, C₁₋₆-alkylcarbonyl, C₂₋₆-alkenyl, C₂₋₆-alkinyl or C₃₋₆-cycloalkyl, each of which is optionally substituted, and
R¹⁵ and R¹⁶ together with the adjacent N-O-group represent a carbocyclic 5-, 6- or 7-membered ring,
with the proviso in the case where in formula (Ia) R¹, R⁵ and =G=X together represent the following radicals:
R¹ represents hydrogen or methyl,
R⁵ represents hydrogen, 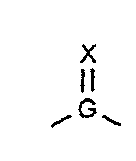
represents carboxyl,
the radicals Q and B must fulfil the following condition:
Q represents radicals other than methyl,
B represents radicals other than -NH₂,
and with the proviso in the case where in formula (Ia) G² and =G=X together represent the following radicals: 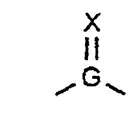 represents carboxyl,
G² represents tert-butyloxy, benzyloxy or 4-nitrobenzyloxy,
the radical B represents radicals other than tert-butyloxy, benzyloxy and 4-nitrobenzyloxy, and with the proviso that, if in formula (Ia) R⁴ and R⁵ are hydrogen, B must not represent NH₂,
and with the proviso that, if in formula (Ia) R¹ to R⁵ represent hydrogen and 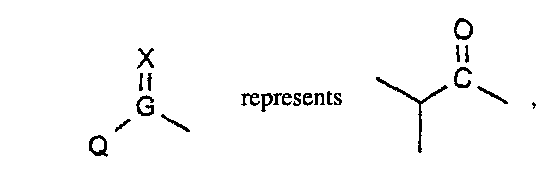
B does not represent methylamino,
and with the proviso that, if in formula (Ia) 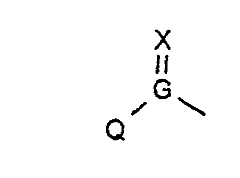 represents benzyloxycarbonyl or 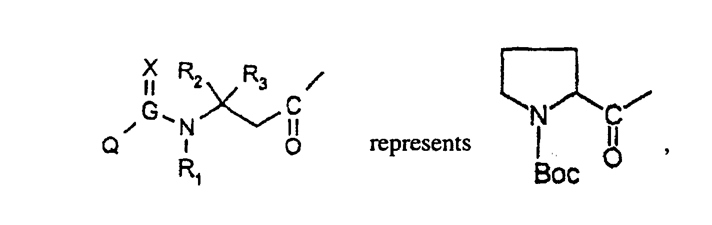
B does not represent benzylamino,
and with the proviso that, if in formula (Ia) 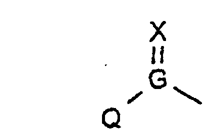 represents tert-butyloxycarbonyl and R¹ to R³ represent H and either R⁴ or R⁵ represents methyl and the other radical represents H,
B does not represent ethoxy,
and with the proviso that the compound of formula 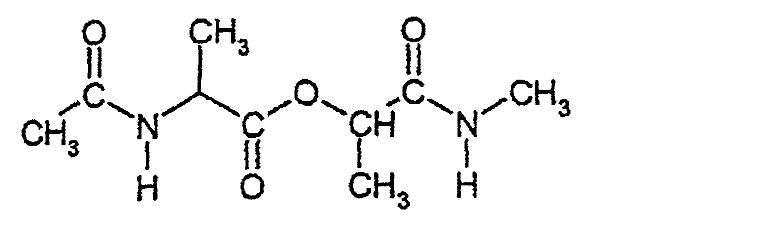 is excluded,
furthermore with the proviso that the compounds
methyl O-(N-carbobenzoxy-1-leucyl)-hydroxyethanoate,
methyl O-(N-carbobenzoxy-1-leucyl)-(S)-2-hydroxy-3-phenylpropanoate,
methyl O-(N-carbobenzoxy-1-leucyl)-(S)-2-hydroxypropanoate,
methyl O-(N-carbobenzoxy-1-leucyl)-(S)-2-hydroxy-4-methylpentanoate,
ethyl N-(benzyloxycarbonyl)-amino-2-(O-glycyl)-glycolate,
ethyl N-(benzyloxycarbonyl)-amino-2-(O-glycyl)-lactate,
ethyl N-(benzyloxycarbonyl)-amino-2-(O-alanyl)-glycolate,
ethyl N-(benzyloxycarbonyl)-amino-2-(O-alanyl)lactate
are excluded,
and their optical isomers and racemates.

3. Process for the preparation of the new didepsipeptides of the general formula (Ia) and their salts according to Claim 2 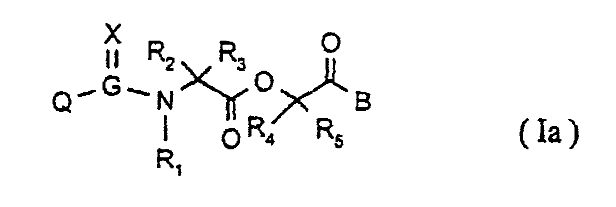 in which
the radicals R¹, R², R³, R⁴, R⁵, G, Q, X and B have the meaning indicated in Claim 2,
**characterized in that**
a) N-terminal-substituted amino acids of the general formula (II) 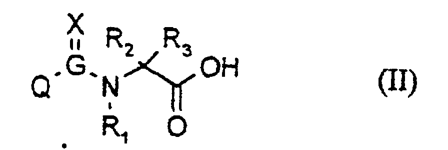 in which the radicals R¹, R², R³, G, Q, and X have the meaning indicated in Claim 2, or their carboxyl-activated derivatives or their alkali metal salts, are reacted, if appropriate in the presence of a catalyst, if appropriate in the presence of an acid-binding agent and if appropriate in the presence of a diluent, with carboxylic acid derivatives of the general formula (III) 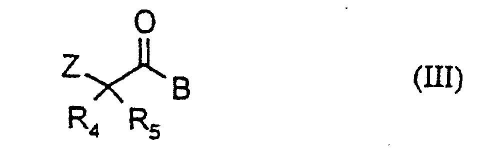 in which
the radicals R⁴, R⁵ and B have the meaning indicated in Claim 2 and Z represents a suitable leaving group, for example halogen such as bromine, chlorine, fluorine, or hydroxyl, or
b) N-terminal-deblocked didepsipeptides of the general formula (Ib) 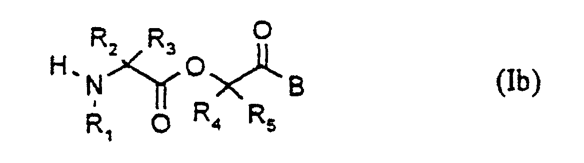 in which
the radicals R¹, R², R³, R⁴, R⁵ and B have the meaning indicated in Claim 2, are reacted, if appropriate in the presence of a catalyst, if appropriate in the presence of an acid-binding agent and if appropriate in the presence of a diluent, with compounds of the general formula (IV) 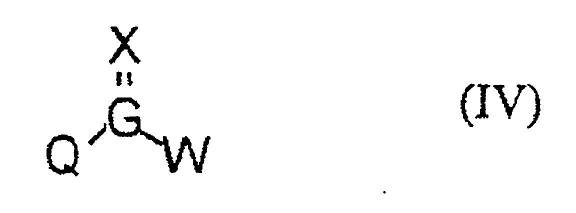 the radicals G, Q, W and X have the meaning indicated in Claim 2 and W represents a suitable leaving group, for example halogen, alkoxy, alkylthio or aryloxy, or
c) N-terminal-deblocked didepsipeptides of the general formula (Ib) 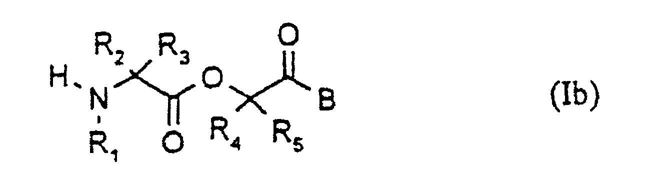 in which
the radicals R¹, R², R³, R⁴, R⁵ and B have the meaning indicated in Claim 2, are reacted, if appropriate in the presence of a catalyst, if appropriate in the presence of an acid-binding agent and if appropriate in the presence of a diluent, with compounds of the general formula (V) or (VI)
R⁸-N=C=X (V) 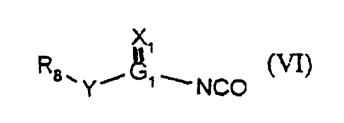
in which
the radicals R⁸, G¹, X, X¹ and Y have the meaning indicated in Claim 2, or
for the preparation of the depsipeptides of the general formula (Ia) and their salts
in which the group 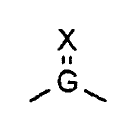 represents carboxyl and Y represents oxygen,
d) N-terminal-deblocked didepsipeptides of the general formula (Ib) 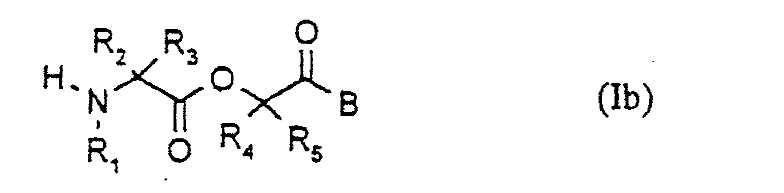 in which
the radicals R¹, R², R³, R⁴, R⁵ and B have the meaning indicated in Claim 2,
are reacted in a first reaction step with carbon dioxide and an alkali metal carbonate of the formula (VII)
M₂CO₃ (VII)
in which
M represents a monovalent alkali metal cation, preferably lithium, sodium, potassium or caesium, in particular potassium or caesium,
then in a second reaction step the resulting alkali metal salt of the formula (VIII) 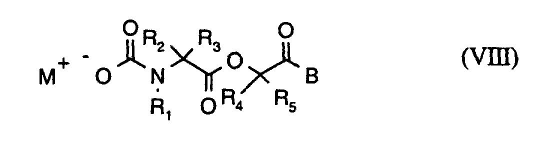 in which
the radicals R¹, R², R³, R⁴, R⁵ and B have the meaning indicated in Claim 2,
M represents a metal cation equivalent bound like a salt,
is reacted with alkylating agents of the formula (IX)
R⁸-Hal (IX)
in which
R⁸ has the meaning indicated in Claim 2 and
Hal represents a halogen such as fluorine, chlorine, bromine or iodine,
if appropriate in the presence of a diluent and if appropriate in the presence of a basic reaction auxiliary, or
e) didepsipeptides of the general formula (Ic) 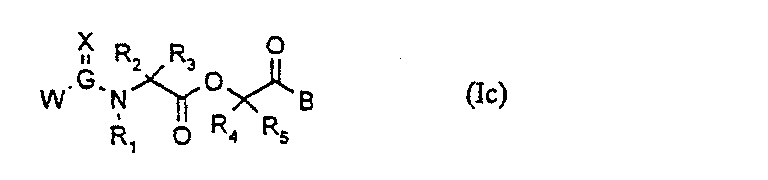 in which
the radicals R¹, R², R³, R⁴, R⁵, G, W, X and B have the meaning indicated in Claim 2 and 3b, are reacted, if appropriate in the presence of a catalyst, if appropriate in the presence of an acid-binding agent and if appropriate in the presence of a diluent, with compounds of the general formula (X)
R⁸-Y-H (X)
in which
the radicals R⁸ and Y have the meaning indicated above in Claim 2, or
f) didepsipeptides of the general formula (Id) 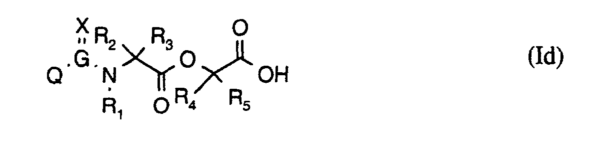 in which
the radicals R¹, R², R³, R⁴, R⁵, G, Q, X and B have the meaning indicated in Claim 2 or their carboxyl-activated derivatives or their alkali metal salts are reacted, if appropriate in the presence of a catalyst, if appropriate in the presence of an acid-binding agent and if appropriate in the presence of a diluent, with compounds of the general formula (XI)
H-B (XI)
in which
the radical B has the meaning indicated above in Claim 2.

4. Endoparasiticidal compositions, **characterized in that** they contain at least one didepsipeptide of the formula (I) according to Claim 1.

5. Process for the production of endoparasiticidal compositions, **characterized in that** didepsipeptides of the formula (I) according to Claim 1 are mixed with extenders and/or surface-active agents.

## Revendications

1. Utilisation de didepsipeptides de formule générale (I) 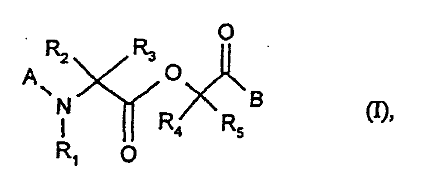 et de leurs sels,
formule dans laquelle
R¹ représente l'hydrogène ou un groupe alkyle linéaire ou ramifié,
R¹ et R² forment conjointement avec les atomes auxquels ils sont liés un noyau pentagonal ou hexagonal qui peut être interrompu éventuellement par de l'oxygène, du soufre, un groupe sulfoxyle ou sulfonyle et qui est éventuellement substitué,
R² et R³ représentent indépendamment l'un de l'autre l'hydrogène, un reste alkyle linéaire ou ramifié, alcényle, cycloalkyle, cycloalkylalkyle, aryle, arylalkyle, hétéroaryle, hétéroarylalkyle, ces restes étant éventuellement substitués, ou bien,
R² et R³ forment ensemble un noyau spirocyclique qui est éventuellement substitué,
R⁴ et R⁵ représentent indépendamment l'un de l'autre l'hydrogène, un reste alkyle linéaire ou ramifié, alcényle, cycloalkyle, cycloalkylalkyle, aryle, arylalkyle, hétéroaryle, hétéroarylalkyle, ces restes étant éventuellement substitués,
R⁴ et R⁵ forment ensemble un noyau spirocyclique qui est éventuellement substitué,
A représente l'hydrogène, un reste alkyle, aralkyle, formyle, alkoxydicarbonyle ou un reste du groupe G¹ 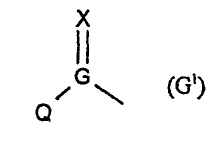 où 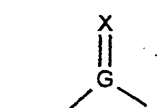 est un groupe carboxy, thiocarboxy, -C=CH-NO₂, -C=CH-CN, -C=N-R⁶ , sulfoxyle, sulfonyle, -P(O)-OR⁷ ou P(S)-OR⁷,
R⁶ est l'hydrogène, un groupe hydroxy, alkoxy, alkylcarbonyle, halogénalkylcarbonyle, alkylsulfonyle, nitro ou cyano, et
R⁷ est l'hydrogène ou un groupe alkyle, et
Q représente un reste alkyle linéaire ou ramifié, alcényle, alcynyle, cycloalkyle, aryle, arylalkyle, hétaryle ou hétarylalkyle, ces restes étant éventuellement substitués, ou le cas échéant un reste des groupes G² et G³
R⁸-Y- (G²) 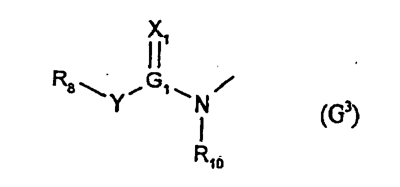
où 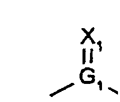 peut représenter un groupe carboxy, thiocarboxy ou sulfonyle,
Y est l'oxygène, le soufre ou un groupe -NR⁹,
R⁸, au cas où Y est l'azote, peut désigner un groupe amino cyclique lié par l'intermédiaire d'un atome d'azote,
R⁸ et R⁹ représentent indépendamment l'un de l'autre l'hydrogène, un reste alkyle linéaire ou ramifié, alcényle, alcynyle, cycloalkyle, cycloalkylalkyle, aryle, arylalkyle, hétaryle, hétarylalkyle, ces restes étant éventuellement substitués, ou bien
R⁸ et R⁹ forment conjointement avec l'atome contigu d'azote un noyau carbocyclique pentagonal, hexagonal ou heptagonal ou un noyau bicyclique de 7 à 10 atomes qui peut être interrompu éventuellement aussi par de l'oxygène, du soufre, un groupe sulfoxyle, sulfonyle, carbonyle, -N-O-, -N=, -NR¹¹- ou par de l'azote quaternisé et qui est éventuellement substitué,
R¹⁰ représente l'hydrogène ou un groupe alkyle,
R¹¹ représente l'hydrogène, un reste alkyle linéaire ou ramifié, alcényle, alcynyle, cycloalkyle, cycloalkylalkyle, alkoxycarbonyle, alkylcarbonyle, cycloalkylcarbonyle, cyano, aryle, arylalkyle, hétaryle, hétarylalkyle, ces restes étant éventuellement substitués,
et
B représente un reste hydroxy, alkoxy, alcényloxy, alcynyloxy, cycloalkyloxy, cycloalkylalkyloxy, aryloxy-, arylalkyloxy; hétaryloxy, hétarylalkyloxy, ces restes étant éventuellement substitués, ou représente les restes -NR¹²R¹³, -NR¹⁴-NR¹²R¹³ et -NR¹⁵-OR¹⁶,
dans lesquels,
R¹² et R¹³ représentent indépendamment l'un de l'autre l'hydrogène, un groupe alkyle linéaire ou ramifié, alkylcarbonyle, alkylsulfonyle, alcényle, alcynyle, cycloalkyle, cycloalkylalkyle, aryle, arylcarbonyle, arylsulfonyle, arylalkyle, hétaryle, hétarylcarbonyle, hétarylsulfonyle ou hétarylalkyle, ces groupes étant éventuellement substitués, ou
R¹² et R¹³ forment conjointement avec l'atome contigu d'azote un noyau carbocyclique pentagonal, hexagonal, heptagonal ou octogonal ou un noyau bicyclique de 7 à 10 atomes qui peut être interrompu le cas échéant aussi par de l'oxygène, du soufre, un groupe sulfoxyle, sulfonyle, carbonyle, -N-O-, -N=, -NR¹¹- ou par de l'azote quaternisé et qui est éventuellement substitué,
R¹⁴ représente l'hydrogène, un reste alkyle linéaire ou ramifié, cycloalkyle, arylalkyle ou hétarylalkyle, ces restes étant éventuellement substitués,
R¹⁵ et R¹⁶ représentent indépendamment l'un de l'autre l'hydrogène, un reste alkyle linéaire ou ramifié, alkylcarbonyle, alcényle, alcynyle, cycloalkyle, cycloalkylalkyle, arylalkyle ou hétarylalkyle, ces restes étant éventuellement substitués,
R¹⁵ et R¹⁶ forment avec le groupe N-O- contigu un noyau carbocyclique pentagonal, hexagonal ou heptagonal,
ainsi que de leurs isomères optiques et de leurs racémates, pour la préparation d'une composition destinée à combattre des endoparasites en médecine humaine et en médecine vétérinaire.

2. Nouveaux didepsipeptides de formule générale (Ia) 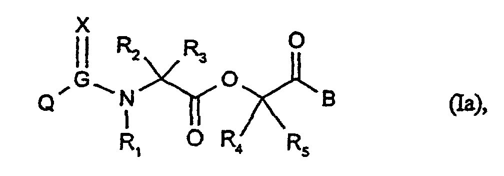 et leurs sels,
formule dans laquelle
R¹ représente l'hydrogène ou un reste alkyle linéaire ou ramifié en C₁ à C₄, et
R¹ et R² forment conjointement avec les atomes auxquels ils sont liés un noyau pentagonal ou hexagonal qui peut être interrompu par de l'oxygène, du soufre, un groupe sulfoxyle ou sulfonyle et qui est éventuellement substitué,
R² représente l'hydrogène, un reste alkyle linéaire ou ramifié ayant jusqu'à 6 atomes de carbone, alcényle ayant jusqu'à 4 atomes de carbone, cycloalkyle en C₃ à C₆, (cycloalkyle en C₃ à C₆)-(alkyle en C₁ ou C₂), aryle, aryl-(alkyle en C₁ ou C₂), hétaryle, hétaryl-(alkyle en C₁ ou C₂), ces restes étant éventuellement substitués, ou bien,
R³ et R⁴ représentent l'hydrogène,
R⁵ représente l'hydrogène, un reste alkyle linéaire ou ramifié ayant jusqu'à 6 atomes de carbone, alcényle ayant jusqu'à 4 atomes de carbone, cycloalkyle en C₃ à C₆, (cycloalkyle en C₃ à C₆)-(alkyle en C₁ ou C₂), aryle, aryl-(alkyle en C₁ ou C₂), hétaryle, hétaryl-(alkyle en C₁ ou C₂), ces restes étant éventuellement substitués, 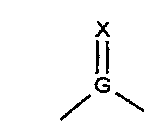 représente un reste carboxy, thiocarboxy, -C=CH-NO₂, -C=CH-CN, -C=N-R⁶, sulfoxyle, sulfonyle, -P(O)-OR⁷ ou P(S)-OR⁷,
R⁶ est l'hydrogène, un groupe hydroxy, alkoxy en C₁ à C₄, (alkyle en C₁ à C₄)-carbonyle, (halogénalkyle en C₁ à C₄)-carbonyle, alkylsulfonyle en C₁ à C₄, nitro ou cyano, et
R⁷ est l'hydrogène ou un reste alkyle en C₁ à C₄, et
Q représente un reste alkyle en C₁ à C₆ linéaire ou ramifié, alcényle en C₂ à C₆, alcynyle en C₂ à C₆, cycloalkyle en C₃ à C₆ ou hétaryl-(alkyle en C₁ ou C₂), ces restes étant éventuellement substitués, ou le cas échéant un reste des groupes G² et G³
R⁸-Y- (G²) 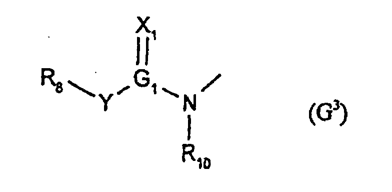
où 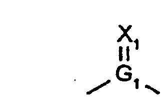 peut représenter un groupe carboxy, thiocarboxy ou sulfonyle,
Y est l'oxygène, le soufre où un groupe -NR⁹,
R⁸, au cas où Y représente l'azote, peut désigner un groupe amino cyclique lié par l'intermédiaire d'un atome d'azote,
R⁸ et R⁹ représentent indépendamment l'un de l'autre l'hydrogène, un reste alkyle en C₁ à C₆ linéaire ou ramifié, alcényle en C₂ à C₆, alcynyle en C₂ à C₆, cycloalkyle en C₃ à C₆, (cycloalkyle en C₃ à C₆)-(alkyle en C₁ ou C₂), hétaryle ou hétaryl-(alkyle en C₁ ou C₂), ces restes étant éventuellement substitués, ou bien
R⁸ et R⁹ forment conjointement avec l'atome contigu d'azote un noyau carbocyclique pentagonal, hexagonal ou heptagonal ou un noyau bicyclique de 7 à 10 atomes, qui peut être interrompu le cas échéant par de l'oxygène, du soufre, un groupe sulfoxyle, sulfonyle, carbonyle, -N-O-, -N=, -NR¹¹- ou par un atome d'azote quaternisé ou qui est éventuellement substitué,
R¹⁰ représente l'hydrogène ou un groupe alkyle en C₁ à C₄, et
R¹¹ représente l'hydrogène, un reste alkyle en C₁ à C₆ linéaire ou ramifié, cycloalkyle en C₃ à C₆, alcényle en C₂ à C₆, alcynyle en C₂ à C₆, cycloalkyle en C₃ à C₆, (cycloalkyle en C₃ à C₆) - (alkyle en C₁ ou C₂), (alkoxy en C₁ à C₄)-carbonyle, (alkyle en C₁ à C₄)-carbonyle, (cycloalkyle en C₃ à C₆)-carbonyle, cyano, aryle, aryl-(alkyle en C₁ ou C₂), hétaryle ou hétaryl-(alkyle en C₁ ou C₂), ces restes étant éventuellement substitués,
et
B représente un reste alkoxy en C₁ à C₆, alcényloxy en C₂ à C₆, alcynyloxy en C₂ à C₆, cycloalkyloxy en C₃ à C₇, (cycloalkyle en C₃ à C₇)-(alkyloxy en C₁ ou C₂), aryloxy-, aryl-(alkyloxy en C₁ ou C₂), hétaryloxy, hétaryl-(alkyloxy en C₁ ou C₂), ces restes étant éventuellement substitués, ou bien représente les restes amino -NR¹²R¹³, -NR⁴-NR¹²R¹³ et -NR¹⁵-OR¹⁶, dans lesquels,
R¹² et R¹³ représentent indépendamment l'un de l'autre l'hydrogène, un groupe alkyle en C₁ à C₆ linéaire ou ramifié, (alkyle en C₁ à C₆)-carbonyle, alkylsulfonyle en C₁ à C₆, alcényle en C₂ à C₆, alcynyle en C₂ à C₆, cycloalkyle en C₃ à C₈, (cycloalkyle en C₃ à C₈)-(alkyle en C₁ ou C₂), aryle, arylcarbonyle, arylsulfonyle, aryl-(alkyle en C₁ ou C₂), hétaryle, hétarylcarbonyle, hétarylsulfonyle ou hétaryl-(alkyle en C₁ ou C₂), ces restes étant éventuellement substitués, ou bien
R¹² et R¹³ forment conjointement avec l'atome contigu d'azote un noyau carbocyclique pentagonal, hexagonal, heptagonal ou octogonal ou un noyau bicyclique de 7 à 10 atomes qui peut être interrompu le cas échéant aussi par de l'oxygène, du soufre, un groupe sulfoxyle, sulfonyle, carbonyle, -N-O-, -N=, -NR¹¹- ou par de l'azote quaternisé et qui est éventuellement substitué,
R¹⁴ représente l'hydrogène, un reste alkyle en C₁ à C₆ linéaire ou ramifié, cycloalkyle en C₃ à C₆, ces restes étant éventuellement substitués,
R¹⁵ et R¹⁶ représentent indépendamment l'un de l'autre l'hydrogène, un reste alkyle en C₁ à C₆ linéaire ou ramifié, (alkyle en C₁ à C₆)-carbonyle, alcényle en C₂ à C₆, alcynyle en C₂ à C₆ ou cycloalkyle en C₃ à C₆, ces restes étant éventuellement substitués,
R¹⁵ et R¹⁶ forment conjointement avec le groupe N-O-contigu un noyau carbocyclique pentagonal, hexagonal ou heptagonal,
sous réserve que lorsque dans la formule (Ia), R¹, R⁵ et =G=X représentent ensemble les restes suivants :
R¹ représente l'hydrogène et un reste méthyle,
R⁵ représente l'hydrogène, 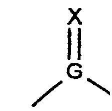 représente un groupe carboxy, les restes Q et B doivent satisfaire la condition suivante :
Q représente des restes autres que le reste méthyle,
B représente des restes autres que le groupe -NH₂, de même que, sous réserve que lorsque dans la formule (Ia) G² et =G=X représentent ensemble les restes suivants : 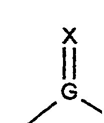 représente un groupe carboxy,
G² représente un groupe tertio-butyloxy, benzyloxy ou 4- nitro-benzyloxy,
le reste B représente des restes autres que les restes tertio-butyloxy, benzyloxy et 4-nitro-benzyloxy, ainsi que, sous réserve que lorsque dans la formule (Ia) R⁴ et R⁵ représentent l'hydrogène, B ne puisse pas être un groupe NH₂,
ainsi que, sous réserve que lorsque dans la formule (Ia), R¹ à R⁵ représentent de l'hydrogène et 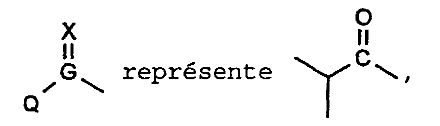
B ne représente pas un groupe méthylamino,
ainsi que, sous réserve que lorsque dans la formule (Ia) 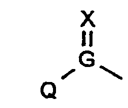 est un groupe benzyloxycarbonyle ou bien 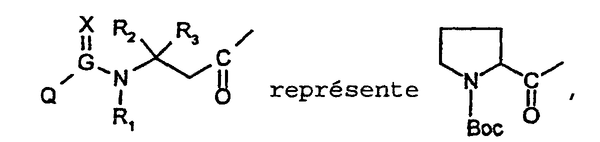
B ne représente pas un groupe benzylamino,
ainsi que, sous réserve que lorsque dans la formule (Ia) 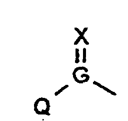 représente un groupe tertio-butyloxycarbonyle et R¹ à R³ représentent H, et ou bien l'un de R⁴ et R⁵ est un groupe méthyle et l'autre représente H,
B ne représente pas un groupe éthoxy,
de même que, sous réserve que le composé de formule 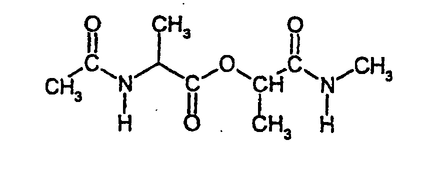 soit exclu,
sous réserve en outre de l'exclusion des composés : O- (N-carbobenzoxy-L-leucyl)-hydroxyéthanoate de méthyle,
O-(N-carbobenzoxy-L-leucyl)-(S)-2-hydroxy-3-phénylpropanoate de méthyle,
O-(N-carbobenzoxy-L-leucyl)-(S)-2-hydroxypropanoate de méthyle,
O-(N-carbobenzoxy-L-leucyl)-(S)-2-hydroxy-4-méthylpentanoate de méthyle,
N-(benzyloxycarbonyl)-amino-2-(O-glycyl)-glycolate d'éthyle,
N-(benzyloxycarbonyl)-amino-2-(O-glycyl)-lactate d'éthyle,
N-(benzyloxycarbonyl)-amino-2-(O-alanyl)-glycolate d'éthyle,
N-(benzyloxycarbonyl)-amino-2-(O-alanyl)-lactate d'éthyle,
de même que leurs isomères optiques et leurs racémates.

3. Procédé de production des nouveaux didepsipeptides de formule générale (Ia) et de leurs sels suivant la revendication 2, 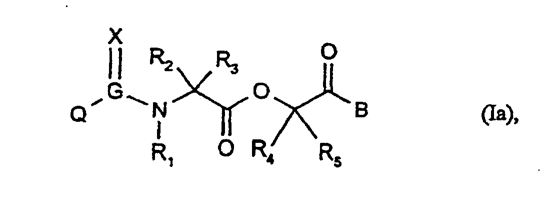 formule dans laquelle
les restes R¹, R², R³, R⁴, R⁵, G, Q, X et B ont la définition indiquée dans la revendication 2,
**caractérisé en ce que** :
a) on fait réagir des aminoacides substitués sur l'extrémité terminale N de formule générale (II) 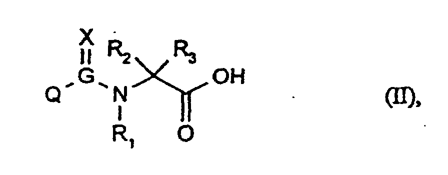 dans laquelle
les restes R¹, R², R³, G, Q et X ont la définition indiquée dans la revendication 2, ou leurs dérivés carboxy-activés ou leurs sels de métaux alcalins, le cas échéant en présence d'un catalyseur, en présence éventuelle d'un accepteur d'acide et le cas échéant en présence d'un diluant, avec des dérivés d'acides carboxyliques de formule générale (III) 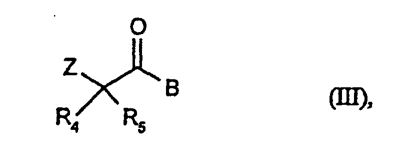 formule dans laquelle
les restes R⁴, R⁵ et B ont la définition indiquée dans la revendication 2, et Z représente un groupe partant approprié, par exemple un halogène tel que le brome, le chlore, le fluor, ou un groupe hydroxyle, ou bien
b) on fait réagir des didepsipeptides libérés à l'extrémité terminale N, de formule générale (Ib) 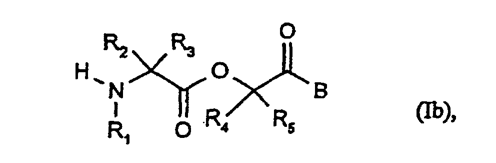 dans laquelle
les restes R¹, R², R³, R⁴, R⁵ et B ont la définition indiquée dans la revendication 2, éventuellement en présence d'un catalyseur, le cas échéant en présence d'un accepteur d'acide et en présence éventuelle d'un diluant, avec des composés de formule générale (IV) 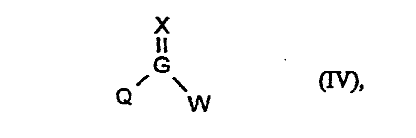 dans laquelle
les restes G, Q, W et X ont la définition indiquée dans la revendication 2 et W désigne un groupe partant approprié tel que par exemple un halogène, un groupe alkoxy, alkylthio ou aryloxy, ou bien
c) on fait réagir des didepsipeptides libérés à l'extrémité terminale N, de formule générale (Ib) 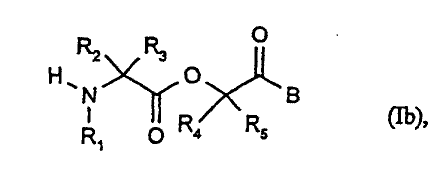 dans laquelle
les restes R¹, R², R³, R⁴, R⁵ et B ont la définition indiquée dans la revendication 2, éventuellement en présence d'un catalyseur, le cas échéant en présence d'un accepteur d'acide et en présence éventuelle d'un diluant, avec des composés de formules générales (V) ou (VI)
R⁸-N=C=X (V) 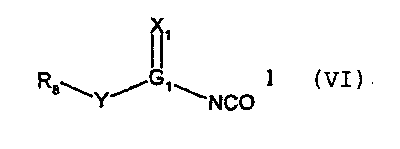
formules dans lesquelles
les restes R⁸, G¹, X, X¹ et Y ont la définition indiquée dans la revendication 2, ou bien
pour la préparation des depsipeptides de formule générale (Ia) et de leurs sels
où le groupe 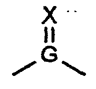 représente un groupe carboxy et Y est un atome d'oxygène,
d) on fait réagir des didepsipeptides libérés à l'extrémité terminale N, de formule générale (Ib) 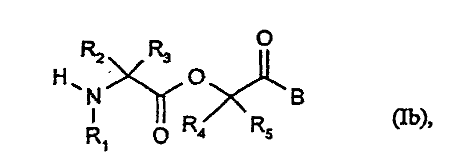 dans laquelle
les restes R¹, R², R³, R⁴, R⁵ et B ont la définition indiquée dans la revendication 2,
dans une première étape réactionnelle, avec le dioxyde de carbone et un carbonate de métal alcalin de formule (VII)
M₂CO₃ (VII)
dans laquelle
M est un cation de métal alcalin monovalent, de préférence lithium, sodium, potassium ou césium, en particulier potassium ou césium,
puis dans une seconde étape réactionnelle, on fait réagir le sel de métal alcalin résultant de formule (VIII) 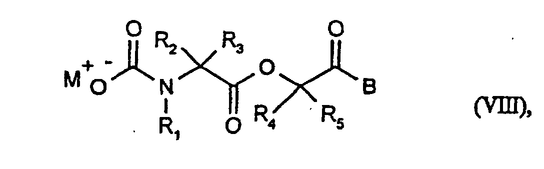 dans laquelle
les restes R¹, R², R³, R⁴, R⁵ et B ont la définition indiquée dans la revendication 2,
M est un équivalent de cation métallique lié à la manière d'un sel,
avec des agents d'alkylation de formule (IX)
R⁸-Hal (IX),
dans laquelle
R⁸ a la définition indiquée dans la revendication 2 et
Hal représente un halogène tel que le fluor, le chlore, le brome ou l'iode,
éventuellement en présence d'un diluant et en présence éventuelle d'un auxiliaire basique de réaction, ou bien
e) on fait réagir des didepsipeptides de formule générale (Ic) 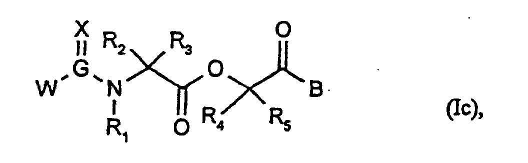 dans laquelle
les restes R¹, R², R³, R⁴, R⁵, G, W, X et B ont la définition indiquée dans la revendication 2 et en b dans la revendication 3, le cas échéant en présence d'un catalyseur, éventuellement en présence d'un accepteur d'acide et en présence éventuelle d'un diluant, avec des composés de formule générale (X)
R⁸-Y-H (X)
dans laquelle
les restes R⁸ et Y ont la définition indiquée ci-dessus dans la revendication 2, ou bien
f) on fait réagir des didepsipeptides de formule générale (Id) 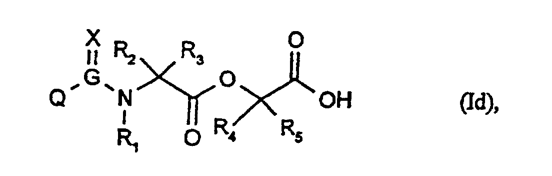 dans laquelle
les restes R¹, R², R³, R⁴, R⁵, G, Q, X et B ont la définition indiquée dans la revendication 2 ou leurs dérivés carboxy-activés ou leurs sels de métaux alcalins, éventuellement en présence d'un catalyseur, le cas échéant en présence d'un accepteur d'acide et en présence éventuelle d'un diluant, avec des composés de formule générale (XI)
H-B (XI)
dans laquelle
le reste B a la définition indiquée ci-dessus dans la revendication 2.

4. Composition endoparasiticide, **caractérisée par** une teneur en au moins un didepsipeptide de formule (I) suivant la revendication 1.

5. Procédé de préparation de compositions endoparasiticides, **caractérisé en ce qu'**on mélange des didepsipeptides de formule (I) suivant la revendication 1 avec des diluants et/ou des agents tensio-actifs.
